(19) **Europäisches Patentamt / European Patent Office / Office européen des brevets**

(11) **EP 4 664 463 A1**

(12) **EUROPEAN PATENT APPLICATION**

(43) Date of publication:
**17.12.2025  Bulletin 2025/51**

(21) Application number: **24182426.7**

(22) Date of filing: **14.06.2024**

(51) International Patent Classification (IPC):
**$G16B\ 35/20$** $^{(2019.01)}$    **$G16B\ 40/20$** $^{(2019.01)}$
**$G16C\ 20/70$** $^{(2019.01)}$

(52) Cooperative Patent Classification (CPC):
**G16B 35/20; G16B 40/20; G16C 20/30;
G16C 20/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Givaudan SA
1214 Vernier (CH)**

(72) Inventor: **The designation of the inventor has not
yet been filed**

(74) Representative: **Haseltine Lake Kempner LLP
Cheapside House
138 Cheapside
London EC2V 6BJ (GB)**

Remarks:
The complete document including Reference
Table(s) and the Sequence Listing(s) can be
downloaded from the EPO website

(54) **COMPUTER-IMPLEMENTED METHOD FOR OLFACTORY PROPERTY PREDICTION**

(57)  Described herein are computer-implemented techniques for olfactory property prediction and olfactory receptor activation data prediction and use thereof in in the creation, modification, or improvement of flavours and fragrances.

Obtain an OR activation vector for the compound or the composition — S291

Predict, using the OR activation vector and an OR activation library, olfactory properties of the compound or the composition — S292

**FIG 29**

EP 4 664 463 A1

**Description**

**Field**

Aspects and embodiments described herein relate to the fields of biotechnology and flavours and fragrances, in particular to computer-implemented methods and related storage media, data processing apparatuses, and computer programs for olfactory property and olfactory receptor activation data prediction and use thereof in the creation, modification, or improvement of flavours and fragrances. Aspects relate to modified olfactory receptors and to related nucleic acid molecules, expression vectors, recombinant host cells, libraries, and methods and uses for expressing olfactory receptors and for identifying novel olfactory receptors and novel olfactory receptor ligands, enhancers and antagonists.

**Background**

Olfactory or odorant receptors (ORs) are expressed in olfactory sensory neurons of the olfactory epithelium and are responsible for the detection of odorants. Olfactory receptors belong to the G protein-coupled receptor superfamily (GPCRs). Activation of an OR by an odorant (ligand) activates the olfactory-specific G protein which in turn promotes the production of cyclic AMP (cAMP) via a type III adenylate cyclase. The increased levels of intracellular cAMP results in the opening of cyclic nucleotide-gated ion channels which allow calcium ions to enter into the cell, depolarizing the olfactory sensory neuron and triggering an action potential which carries the information to the glomeruli of the olfactory bulb.

The human genome encodes approximately 400 different functional olfactory receptors. A specific olfactory receptor may be activated by more than one ligand molecule and a specific ligand molecule may activate multiple olfactory receptors, which creates a highly complex interaction network between the OR and ligand repertoires. Elucidation of said interactions can allow for the discovery of novel flavour and fragrance ingredients, or compounds such as odor enhancers that are more sustainable and/or easier to produce than currently used compounds. For many of the approximately 400 different olfactory receptor genes, different variants called alleles or haplotypes exist in the human populations. The protein products of different alleles or haplotypes of the same gene may have different ligand selectivity or sensitivity.

Next to their expression in olfactory sensory neurons of the olfactory epithelium, OR expression was also found in many other cells and tissues (Feldmesser et al. Widespread ectopic expression of olfactory receptor genes. BMC Genomics 2006;7: p. 121; Massberg, D. and H. Hatt. Human Olfactory Receptors: Novel Cellular Functions Outside of the Nose. Physiol Rev 2018;98(3):1739-1763). These ORs have been found to be associated with many important diseases and therefore ORs - next to their primary interest as targets for odorant ligands - have also an important interest as targets to find agonists and antagonists to treat various diseases (Lee et al. Therapeutic potential of ectopic olfactory and taste receptors. Nature Reviews Drug Discovery 2019;18(2):116-138). Particularly, OR signalling has been associated with reduced or increased cell proliferation in bladder, colon, prostate, lung and liver cancer cells. Similarly, OR expression in inflammatory cells was associated with regulation of inflammation. Thus, OR screening has multiple applications beyond olfaction.

Efficient screening of olfactory receptors requires their expression in cultured cell lines, which generally involves the introduction of an olfactory receptor gene into a cell followed by its stable or transient overexpression. In general, it has proven very difficult to functionally express olfactory receptors in host cells, as it proved difficult to obtain correct folding of the receptors and/or correct insertion of the receptors into the cell membrane. To generate olfactory receptor activation data and olfactory receptor activation vectors of a compound or composition, such olfactory receptor screening data with functionally expressed OR is desirable. Thus, several approaches have been tried to improve functional heterologous expression of olfactory receptors.

Functional heterologous olfactory receptor expression utilizing the expression systems currently available in the art generally requires co-expression of accessory proteins of the receptor transporting protein (RTP) family, such as RTP1S and RTP2 (Yu et al. Receptor-transporting protein (RTP) family members play divergent roles in the functional expression of odorant receptors. PLoS One 2017;12(6):e0179067), which are normally expressed in the olfactory sensory neurons and facilitate OR trafficking to the cell-surface membrane. Furthermore, it was found that a fusion of the olfactory receptor gene with a sequence encoding the N-terminal sequence (initiation methionine and 19 following amino acids) of rhodopsin (=rho-tag) facilitates expression of olfactory receptors (Krautwurst et al. identification of ligands for olfactory receptors by functional expression of a receptor library. Cell. 1998;95(7):917-26).

However, even when using RTP proteins and an N-terminal rho-Tag, more than half of the known olfactory receptors cannot be functionally expressed utilizing currently available nucleic acid constructs, cell lines, and methods, resulting in a presently limited coverage of the available receptor-ligand space, with multiple receptors not having identified ligands (orphan receptors), and limited industrial application of said methods. Even for receptors expressed with said methods, the expression may be low, leading to screening assays with low sensitivity. Indeed, many of those receptors that are functionally expressed in current expression systems are only strongly activated at relatively high ligand concentrations, e.g. at 10 - 300 $\mu$M, whereby many ligands already trigger a sensory experience *in vivo* at much lower concentrations. Thus, the receptors as expressed in current systems are often not activated at physiologically relevant concentrations. This indicates that the current systems often are not sufficiently sensitive to mimic the situation *in vivo.* This also leads to practical issues, as (weak) ligands which are cytotoxic or poorly soluble in cell culture media cannot trigger receptor activation in the current screening cell lines, because they are not sufficiently dissolved (most odorants are apolar molecules with limited solubility in water) or directly lead to inactivation of the cell lines by cytotoxicity at the high test concentrations applied.

Classical OR screening assays rely on approaches wherein a clonal population of cells generally receives a DNA expression construct coding for one specific receptor and/or accessory molecule at a time and is then tested for functional activation by various ligands. Said assays further generally involve the co-expression of a luciferase gene operably linked to a cAMP-inducible promoter (Saito et al. RTP family members induce functional expression of mammalian odorant receptors. Cell 2004;119(5):679-691), which is used as a reporter gene. The activation of the olfactory receptor and subsequent increase in intracellular cAMP results in expression of luciferase. Oxidation of luciferin catalysed by luciferase results in the emission of light which can then be detected and quantified. Classical OR screening assays are limited in their sensitivity, may lead to highly different expression of different receptors, and are often not compatible with high-throughput screening and selection methods such as screening of libraries of volatile flavour and fragrance compounds including ligands of moderate activity, ligands with cytotoxic properties and ligands with limited solubility in cell culture media.

**Summary**

In view of all of the above, there is a need for improved olfactory receptors and related nucleic acid molecules, expression vectors, recombinant host cells, libraries, and methods and uses for expressing olfactory receptors and for identifying novel olfactory receptor ligands, enhancers and antagonists. Specifically, such improved olfactory receptors and related nucleic acid molecules, expression vectors, recombinant host cells, libraries, and methods and uses should enhance the sensitivity of the assay to allow testing at lower concentrations and thereby describing the full ligand spectrum of the OR also in respect to poorly soluble and cytotoxic ligands. Such improvements in sensitivity should also allow to identify novel ligand-OR pairs, both to de-orphanise receptors and to better describe the full receptive space (additional ligands but also antagonists and enhancers) of already de-orphanised receptors. More specifically, there is a need for computer-implemented techniques for the creation, modification, or improvement of flavours and fragrances that make use of the acquired data indicating activation and surface expression of expressed olfactory receptors and identified novel olfactory receptor ligands, enhancers and antagonists.

Additionally, for large-scale de-orphanisation of olfactory receptors (i.e. for the identification of a ligand for all receptors with hitherto no known ligand) and for finding all active OR and especially the most sensitive OR for a given ligand of interest, a whole library of many / all human olfactory receptors needs to be expressed. In order to find the truly most important receptor for a given ligand, all receptors should be expressed at a similar level, preferably at least to the maximum extent possible. Otherwise, false-positive responses are observed, whereby a strongly expressed receptor appears as the most sensitive receptor to the ligand of interest and the truly most sensitive receptor is missed due to a lower functional expression. Thus, for such screening campaigns on multiple receptors, it is desirable to normalize the functional expression of different receptors and minimize expression differences between receptors. Hence, there is a need for libraries of human ORs that are optimized in a way to give similar functional expression of all receptors to be used in OR expression assays.

Techniques herein provide olfactory receptors and related nucleic acid molecules, expression vectors, recombinant host cells, libraries, and methods and uses, which are particularly useful in the context of ORs that are difficult to express using conventional approaches or in the context of ORs which lead to assays with limited sensitivity using conventional approaches, and in the identification of novel cognate receptor-ligand pairs. The techniques herein further provide olfactory receptor variants with improved functional expression allowing for more sensitive assays to detect OR-ligand interactions. Olfactory receptors and related nucleic acid molecules, expression vectors, recombinant host cells, libraries, and methods and uses described herein exhibit for example at least one of the following benefits in comparison with the prior art:

- Improved functional heterologous expression
- Allow deorphanisation of receptors for which no ligand is known
- Enhance sensitivity of assays to determine the complete ligand spectrum of a receptor
- Enhance sensitivity of assays to measure ligand binding at physiologically more relevant concentration
- Enhance sensitivity of assays to identify antagonists and enhancers
- Enablement of functional expression of olfactory receptors otherwise not possible using conventional approaches
- Enablement of identification of novel cognate receptor-ligand pairs
- Enhanced sensitivity of assays with olfactory receptors (as measured by reduced ligand concentration to obtain similar activity or reduced EC50 values; i.e. enhanced potency, significantly lower detection threshold or increased efficacy)

- Ability to test more cytotoxic molecules
- Ability to test poorly soluble molecules
- Ability to detect ligands in complex test mixtures and unpurified synthetic samples
- Ability to screen for a ligand with a particular odor description in complex test mixtures and unpurified synthetic samples
- Ability to identify olfactive more active isomers or enantiomers in racemic mixtures; and biodegradable compounds and compositions
- Allow generation of OR libraries having better and more uniform functional expression as compared to using wild-type OR genes
- Increased olfactory receptor and ligand screening throughput capacity

As is demonstrated in the experimental section herein, the application of the olfactory receptors and related nucleic acid molecules, expression vectors, recombinant host cells, libraries, and methods and uses described herein and the use of the acquired data indicating activation and surface expression of expressed olfactory receptors and identified novel olfactory receptor ligands, enhancers and antagonists are associated with several of the above-benefits and thus provide a highly significant improvement over conventional approaches. Accordingly, the aspects and embodiments of the present invention as described herein solve at least some of the problems and needs as discussed herein.

The disclosure herein relates to an olfactory receptor protein, wherein said protein has a modified C-terminal domain comprising the amino acid sequence motif RN[KR][EDQ][VMIL][KR]xA[LIV][KRH][KR][LI][LIF][KRG][KR][KR] (SEQ ID NO: 1). In some cases, an olfactory receptor protein of the invention is such that the modified C-terminal domain is fused to the seventh transmembrane helix (TM7) of the protein. In some cases, an olfactory receptor protein of the invention is such that the protein is a class I or class II olfactory receptor with a modified C-terminal domain, preferably a human, dog or cat class I or class II olfactory receptor with a modified C-terminal domain, more preferably a human class I or class II olfactory receptor with a modified C-terminal domain. In some cases, an olfactory receptor protein of the invention is such that the class II receptor is selected from the group consisting of OR7C1, OR9Q2, OR8K3, OR10J5, OR1C1, OR7D4, OR2T4, OR5B12, OR7A17, OR10H5, OR5A1, OR5A2, OR1N2, OR2C1, OR2T11, OR2M2, OR4S2, OR2V1, OR5P3, OR6P1, OR2L2, OR10G7, OR5AN1, OR5V1, OR2L3, OR2AG2, OR7A5, OR7E24, OR7A10, OR10H2, OR10H1, OR10D3, OR1D2, OR2A5, OR2A25, OR11G2, OR14J1, OR5M3, OR8D1, OR10G3, OR10G9, OR2L5, OR8H1, OR10K1, OR11A1, OR2AK2, OR10A3, OR10A6, OR10J1, and OR2J2, preferably wherein the class II receptor is selected from the group consisting of OR7A17, OR7C1, OR2A25, OR7E24, OR10H1, OR10K1, OR2AG2, OR10H2, OR10H5, OR10D3, OR14J1, OR7A10, OR2L5, OR2M2 and OR5A2. In some cases, an olfactory receptor protein of the invention is such that the class I receptor is selected from the group consisting of OR52A5, OR52E8, OR56A4, OR51B2, OR52K1, OR56A1, OR51B5, OR56A3, and OR51L1. In some cases, an olfactory receptor protein is such that the sequence motif is RN[KR]E[VMI][KR]xA[LIV][KR][KR]L[LIF][KR][KR][KR] (SEQ ID NO: 5).

In some cases, an olfactory receptor protein of the invention is such that x is not proline. In some cases, an olfactory receptor protein of the invention is such that x is not proline or tryptophane. In some cases, an olfactory receptor protein of the invention is such that x is selected from D, K, R, E, N, V, A, Q or G, preferably wherein x is selected from D, K, R, E, N, V, A or Q.

In some cases, an olfactory receptor protein of the invention is such that the amino acid sequence motif comprises 1 to 6 additional C-terminal amino acid residues, optionally wherein:

- the first additional amino acid residue is selected from C, R, K, E, G, H, F, P, Y, W, M and N, preferably the first additional amino acid residue is selected from C, R, K, E, G, H, F, P, Y, more preferably the first additional amino acid residue is C, R or K, most preferably C;
- the second additional amino acid residue is selected from C, R, K, N, G, I, L, F, P, T, Y and Q, preferably the second additional amino acid residue is selected from C, R, K, N, G, I, L, F, P, T and Y, more preferably the second additional amino acid residue is C, R or K, most preferably C or R;
- the third additional amino acid residue is selected from R, K, C, L, F, M, Y, A, P, S, G, H, and N, preferably the third additional amino acid residue is selected from R, K, C, L, F, M, Y, A, P, S and G, more preferably the third additional amino acid residue is R or K;
- the fourth, fifth and sixth additional amino acid residues are selected from K and R.

In some cases, the amino acid sequence motif comprises additional C-terminal amino acid residues selected from the group consisting of CC, CCR, CCRR (SEQ ID NO: 161), CCRRR (SEQ ID NO: 163), CCRRRR (SEQ ID NO: 224), CR, CRR, CRRR (SEQ ID NO: 159), CRKK (SEQ ID NO: 160), CRRRR (SEQ ID NO: 162), CRRRRR (SEQ ID NO: 164), and CRRRKK (SEQ ID NO: 165).

In some cases, an olfactory receptor protein of the invention is such that the sequence motif is selected from the group consisting of SEQ ID NOs: 1, 5-75, 86-130, 133-147, 149-151, 154, 156-158, 166, 167, 198, 219-221, 254-312, 319-326, 328-331, 740-741, 820-890.

In some cases, an olfactory receptor protein of the invention is such that it further comprises an N-terminal tag peptide, preferably wherein the N-terminal tag peptide is selected from the group consisting of a FLAG tag, a rhodopsin (rho) tag, an $SST_3$ tag, and an $M_3$ tag.

The disclosure herein relates to a nucleic acid molecule comprising a nucleotide sequence encoding an olfactory receptor protein of the invention. In some cases, a nucleic acid molecule of the invention is such that it further comprises a promoter sequence, preferably a constitutive promoter sequence. In some cases, a nucleic acid molecule of the invention is such that it further comprises a terminator sequence. In some cases, a nucleic acid molecule is such that it further comprises a nucleotide sequence encoding an N-terminal signal peptide, preferably a leucine-rich signal peptide, such as, MRPQILLLLALLTLGLA (SEQ ID NO: 76) or MSHQILLLLALLTLGLA (SEQ ID NO: 77).

The disclosure herein relates to an expression vector comprising a nucleic acid molecule of the invention. In some cases, an expression vector of the invention is a plasmid.

The disclosure herein relates to a recombinant host cell comprising a nucleic acid molecule of the invention or an expression vector of the invention, preferably wherein the cell expresses an olfactory receptor protein of the invention. In some cases, a recombinant host cell of the invention is such that the cell further expresses one or more olfactory receptor accessory proteins. In some cases, the one or more olfactory receptor accessory proteins are selected from the group consisting of RTP1, RTP1S, RTP2, REEP, β-adrenergic receptor, heat shock protein 70, Ric8b, $G\alpha_{olf}$, $Gi\alpha$, and functional variants thereof, preferably selected from the group consisting of RTP1S, RTP2 and functional variants thereof. In some cases, a recombinant host cell of the invention is such that the cell is a HEK293 cell or a HEK293T cell.

The disclosure herein relates to a library comprising a diverse repertoire of olfactory receptor proteins of the invention, nucleic acid molecules of the invention, expression vectors of the invention, or recombinant host cells of the invention. In some cases, a library of the invention is such that the diverse repertoire of olfactory receptor proteins, of olfactory receptor proteins encoded by the nucleic acid molecules or expression vectors, or of olfactory receptor proteins expressed by the recombinant host cells, shares the same modified C-terminal domain.

The disclosure herein relates to a use of an olfactory receptor protein of the invention, a nucleic acid molecule of the invention, an expression vector of the invention, a recombinant host cell of the invention, or a library of the invention, for identifying an olfactory receptor ligand, enhancer or antagonist.

The disclosure herein relates to a use of a library of the invention, for identifying an olfactory receptor that is capable of binding a target ligand.

The disclosure herein relates to a method for identifying an olfactory receptor ligand, said method comprising:

a) providing an olfactory receptor protein of the invention or a recombinant host cell expressing an olfactory receptor protein of the invention;
b) contacting said receptor or recombinant host cell with a test compound or composition; and
c) detecting activation of the olfactory receptor.

The disclosure herein relates to a method for identifying an olfactory receptor enhancer or antagonist, said method comprising:

a) providing an olfactory receptor protein of the invention or a cell expressing an olfactory receptor protein of the invention;
b) contacting said receptor or recombinant host cell with a cognate ligand and a test compound or composition; and
c) detecting increased or decreased activation of the olfactory receptor as compared to controls with ligand only.

In some cases of a method for identifying an olfactory receptor ligand, and of a method for identifying an olfactory receptor enhancer or antagonist, the olfactory receptor is selected from the group consisting of OR7C1, OR8K3 (preferably OR8K3(L122R)), OR10J5, OR7A17, OR10H5, OR5A1, OR5A2, OR1N2 (preferably OR1N2(W23R,V230G,T287M)), OR2M2, OR2V1, OR5P3, OR6P1, OR2L2 (or OR2L2(V259L)), OR10G7 (preferably OR10G7(T5S)), OR5AN1, OR5V1, OR2L3, OR2AG2 (preferably OR2AG2(Y28C)), OR7A5, OR7E24 (or OR7-E24(P242S)), OR7A10, OR10H2, OR10H1, OR10D3, OR1D2, OR2A5, OR2A25 (OR2A25(S75N,A209P)), OR11G2 (or OR11G2(I65N,V82I)), OR14J1, OR5M3, OR8D1, OR10G3 (preferably OR10G3(S73G)), OR10G9, OR2L5, OR8H1, OR10K1, OR11A1, OR2AK2 (preferably OR2AK2(S84N)), OR10A3, OR10A6 (preferably OR10-A6(A117V,V140G,L287P)), OR10J1 (preferably OR10J1(M51I,I92M)), OR2J2, and OR2AG2 (preferably OR2AG2(Y28C)).

In some cases of a method for identifying an olfactory receptor enhancer or antagonist, the method is such that it is for identifying an olfactory receptor antagonist, and the olfactory receptor is selected from the group consisting of OR52A5, OR52E8, OR56A1, OR56A3, OR56A4, OR52K1, OR51B2 (preferably OR51B2(C120R, L134F, C209S)), OR51B5, OR9Q2, OR7D4, OR2T4, OR2C1, OR2T11, OR2M2, OR2V1, OR5V1, and OR4S2, preferably selected from the group consisting of OR2M2, OR2V1, OR51B2 (preferably OR51B2(C120R,L134F,C209S)), and OR5V1, more preferably OR2M2 or OR2V1.

In some cases of a method for identifying an olfactory receptor enhancer or antagonist, the method is such that it is for identifying an olfactory receptor antagonist, the olfactory receptor is OR2M2 or OR2V1, and step b) further comprises contacting said receptor or recombinant host cell with a copper salt. In some cases of a method for identifying an OR2M2 or OR2V1 antagonist, the cognate ligand is selected from the group consisting of 3-methyl-3-sulfanyl-hexanol, 2-mercapto-2-methyl-pentanol, and 4-methoxy-2-methylpentane-2-thiol.

In some cases of a method for identifying an olfactory receptor enhancer or antagonist, the method is such that it is for identifying an olfactory receptor antagonist, the olfactory receptor is OR51B2 (preferably OR51B2(C120R,L134F,C209S)). In some cases of a method for identifying an OR51B2 antagonist, the cognate ligand is 3-methyl-2-hexenoic acid.

In some cases of a method for identifying an olfactory receptor enhancer or antagonist, the method is such that it is for identifying an olfactory receptor antagonist, the olfactory receptor is OR5V1. In some cases of a method for identifying an OR5V1 antagonist, the cognate ligand is 2,4,6-trichloroanisol.

The disclosure herein relates to a method for identifying an olfactory receptor that is capable of binding a target ligand, said method comprising:

a) providing a library of the invention;
b) optionally, obtaining a diverse repertoire of olfactory receptor proteins or recombinant host cells expressing olfactory receptor proteins from said library;
c) contacting the diverse repertoire of olfactory receptor proteins or recombinant host cells expressing olfactory receptor proteins with the target ligand; and
d) identifying an olfactory receptor that is activated by the target ligand.

The disclosure herein relates to a method for generating an objective representation of the olfactory properties of a test compound or composition, said method comprising:

a) providing a library of the invention;
b) optionally, obtaining a diverse repertoire of olfactory receptor proteins or cells expressing olfactory receptor proteins from said library;
c) contacting the diverse repertoire of olfactory receptor proteins or cells expressing olfactory receptor proteins with the test compound or composition; and
d) detecting activation of each of the olfactory receptor proteins.

The disclosure herein relates to a method for assessing the difference or similarity between two or more test compounds or compositions, said method comprising:

a) providing a library of the invention;
b) optionally, obtaining a diverse repertoire of olfactory receptor proteins or cells expressing olfactory receptor proteins from said library;
c) contacting the diverse repertoire of olfactory receptor proteins or cells expressing olfactory receptor proteins with each of the two or more test compounds or compositions;
d) detecting activation of each of the olfactory receptor proteins for each of the two or more test compounds or compositions; and
e) comparing the activated olfactory receptor proteins between each of the two or more test compounds or compositions.

An aspect of the invention relates to a computer-implemented method for predicting olfactory properties of a compound or a composition based on OR activation data. The method comprises:

a) obtaining an OR activation vector for the compound or the composition; and
b) predicting, using the OR activation vector and an OR activation vector library, olfactory properties of the compound or the composition.

In some embodiments of a computer-implemented method for predicting olfactory properties of a compound or a composition based on OR activation data, predicting olfactory properties of the compound or the composition comprises:

passing the OR activation vector through a trained machine learning model, the trained machine learning model configured to output olfactory properties.

In some embodiments of a computer-implemented method for predicting olfactory properties of a compound or a composition based on OR activation data, predicting olfactory properties of the compound or the composition comprises: calculating , from the OR activation vector, a latent space representation using a hidden layer of the trained machine learning model; and predicting, from the latent space representation, olfactory properties of the compound or the composition using an output layer of the trained machine learning model. In some embodiments of a computer-implemented method for predicting olfactory properties of a compound or a composition based on OR activation data, calculating the latent space representation uses an encoder of an auto-encoder model, optionally a variational auto-encoder model.

In some embodiments of a computer-implemented method for predicting olfactory properties of a compound or a composition based on OR activation data, the method further comprises generating a supplementary OR activation vector using a decoder of an auto-encoder model, optionally a variational auto-encoder model.

In some embodiments of a computer-implemented method for predicting olfactory properties of a compound or a composition based on OR activation data, the machine learning model is trained using the OR activation vector library including labels of olfactory properties for a plurality of OR activation vectors.

In some embodiments of a computer-implemented method for predicting olfactory properties of a compound or a composition based on OR activation data, obtaining the OR activation vector comprises measuring activations of a plurality of ORs when contacted with one or more of compounds or compositions. In some embodiments of a computer-implemented method for predicting olfactory properties of a compound or a composition based on OR activation data, such activation data being generated in an assay whereby each OR of the plurality of ORs comprises OR proteins having a modified C-terminal domain comprising an amino acid sequence motif RN[KR][EDQ][VMIL][KR]xA[LIV][KRH][KR][LI][LIF][KRG][KR][KR] (SEQ ID NO: 1). In some embodiments of a computer-implemented method for predicting olfactory properties of a compound or a composition based on OR activation data, the modified C-terminal domain is fused to a seventh transmembrane helix of the OR protein to generate such activation data. In some embodiments of a computer-implemented method for predicting olfactory properties of a compound or a composition based on OR activation data, the OR protein is a class I or class II OR with a modified C-terminal domain, preferably a human, dog, or cat class I or class II OR with a modified C-terminal domain, more preferably a human class I or class II OR with a modified C-terminal domain.

In some embodiments of a computer-implemented method for predicting olfactory properties of a composition based on OR activation data, the method further comprises outputting operating instructions adapted to control a device to fabricate the composition.

In some embodiments of a computer-implemented method for predicting olfactory properties of a compound or a composition based on OR activation data, the compound is an OR agonist or an OR antagonist, or the composition includes an OR agonist or an OR antagonist.

Another aspect of the invention relates to a computer-implemented method for predicting OR activation data for a compound or a composition. The method comprises:

a) calculating a latent space representation for the compound or the composition using an embedding algorithm; and

b) processing the latent space representation (using an OR activation library) to obtain a predicted OR activation vector for the compound or the composition.

In some embodiments of a computer-implemented method for predicting OR activation data for a compound or a composition, calculating the latent space representation for the compound or the composition uses any or all of the following: olfactory properties of the compound or the composition; unique identification details for the compound or the composition; emotion or mood portraits; any data related to customer norms or regulatory constraints, including but not limited to toxicological data; and compound or composition concentration.

In some embodiments of a computer-implemented method for predicting OR activation data for a compound or a composition, processing the latent space representation comprises use of an ensemble learning method, such as random forests or gradient-boosting.

In some embodiments of a computer-implemented method for predicting OR activation data for a compound or a composition, processing the latent space representation comprises: processing the latest space representation using a processing layer to obtain a hidden state representation; and processing the hidden state representation using an OR activation layer to obtain the predicted OR activation vector for the compound or the composition.

In some embodiments of a computer-implemented method for predicting OR activation data for a compound or a composition, the processing layer is a multi-headed attention layer of a transformer encoder. In some embodiments of a computer-implemented method for predicting OR activation data for a compound or a composition, the processing layer is a recurrent network, such as a long short-term memory, LSTM, network. In some embodiments of a computer-implemented method for predicting OR activation data for a compound or a composition, the processing layer and the OR activation layer are trained using an OR activation vector library.

In some embodiments of a computer-implemented method for predicting OR activation data for a compound or a composition, the OR activation vector library comprises a plurality of measured activations of a plurality of ORs when contacted with one or more compounds or compositions. In some embodiments of a computer-implemented method for predicting OR activation data for a compound or a composition, each OR of the plurality of ORs used to generate the activation data comprises OR proteins having a modified C-terminal domain comprising an amino acid sequence motif RN [KR][EDQ][VMIL][KR]xA[LIV][KRH][KR][LI][LIF][KRG][KR][KR] (SEQ ID NO: 1). In some embodiments of a computer-implemented method for predicting OR activation data for a compound or a composition, the modified C-terminal domain is fused to a seventh transmembrane helix of the OR protein to generate such activation data. In some embodiments of a computer-implemented method for predicting OR activation data for a compound or a composition, the OR protein is a class I or class II OR with a modified C-terminal domain, preferably a human, dog, or cat class I or class II OR with a modified C-terminal domain, more preferably a human class I or class II OR with a modified C-terminal domain.

In some embodiments of a computer-implemented method for predicting OR activation data for a compound or a composition, the method further comprises predicting olfactory properties of the predicted OR activation vector using the computer-implemented method for predicting olfactory properties of a compound or a composition based on OR activation data described above. In some embodiments of a computer-implemented method for predicting OR activation data for a compound or a composition, the method further comprises predicting a compound or a composition based on the olfactory properties for the predicted OR activation vector to design a desired fragrance or flavour.

In some embodiments of a computer-implemented method for predicting OR activation data for a compound or a composition, the method further comprises outputting operating instructions adapted to control a device to fabricate the composition.

In some embodiments of a computer-implemented method for predicting OR activation data for a compound or a composition, the compound is an OR agonist or an OR antagonist, or the composition includes one or multiple OR agonist or OR antagonist.

a) Another aspect of the invention relates to a computer-implemented method for the creation, modification, or improvement of a fragrance or flavour composition, for example by replacement of one or more compounds in a composition. The method comprises:obtaining (e.g., predicting) a first olfactory receptor, OR, activation vector for the composition using an OR activation library;

b) obtaining (e.g., predicting) a second OR activation vector for the composition in the absence of the compound using the OR activation library;

c) selecting a replacement compound based on a difference between the first OR activation vector and the second OR activation vector.

In some embodiments of a computer-implemented method for the creation, modification, or improvement of a fragrance or flavour composition, for example by replacement of one or more compounds in a composition, selecting the replacement compound comprises, for each candidate compound from a plurality of candidate compounds: obtaining a third OR activation vector for the composition in the absence of the compound and in the presence of the candidate compound; selecting the replacement compound as the candidate compound for which the difference between the first OR activation vector and the second OR activation vector most closely matches the difference between the second OR activation vector and the third OR activation vector.

In some embodiments of a computer-implemented method for the creation, modification, or improvement of a fragrance or flavour composition, for example by replacement of one or more compounds in a composition, the method further comprises selecting one or more replacement compounds for replacing the compound, or selecting a plurality of replacement compounds for replacing one or more compounds.

In some embodiments of a computer-implemented method for the creation, modification, or improvement of a fragrance or flavour composition, for example by replacement of one or more compounds in a composition, the method further comprises outputting operating instructions adapted to control a device to fabricate the composition .

In some embodiments of a computer-implemented method for the creation, modification, or improvement of a fragrance or flavour composition, for example by replacement of one or more compounds in a composition, the compound is an OR agonist or an OR antagonist, or the composition includes an OR agonist or an OR antagonist.

Another aspect of the invention relates to a computer-readable medium storing data that defines a digital representation of the compound or the composition as used in any of the above-described methods, including a representation of the OR activation vector for the compound or the composition.

In some embodiments of a computer-readable medium, the digital representation is formatted for control of a device to fabricate the composition.

In some embodiments of a computer-readable medium, the data includes operating instructions adapted to control a device to fabricate the composition using the digital representation of the compound or the composition when the data is relayed to the device.

Another aspect of the invention relates to a data processing apparatus comprising a memory storing computer-executable instructions and a processor configured to execute the instructions to perform any of the above-described methods.

Another aspect of the invention relates to a computer program comprising instructions which, when the program is executed by a computer, cause the computer to perform any of the above-described methods.

Another aspect of the invention relates to a computer-readable storage medium comprising instructions which, when executed by a computer, cause the computer to perform the method of any of the above-described methods.

**Description**

Various features of the aspects and embodiments of this disclosure are further described below. It is noted that headings used throughout this specification are to assist navigation only and should not be interpreted as definitive, and that features described in different sections may be relevant for all aspects and embodiments described herein and may thus be combined as appropriate.

Introduction

Next to the modification of the N-terminus of olfactory receptors by adding e.g. a rho-Tag, other changes to the sequences of olfactory receptors have also been made in an attempt to improve their expression.

In one attempt, a consensus sequence was derived from each different class of olfactory receptors, and it was found that for the resulting nine consensus receptors in six out of nine cases a highly expressed receptor was generated, which in general is better expressed than the individual receptors in the class (Ikegami et al. Structural instability and divergence from conserved residues underlie intracellular retention of mammalian odorant receptors. PNAS 2020;117(6):2957-2967). This consensus approach addressed the full-length sequence of the receptor, meaning that the resulting consensus receptor does not correspond to the ligand affinity of a single native receptor but rather is a fully synthetic receptor with a novel ligand spectrum and thus not of interest for screening commercially interesting odorants perceived by the human nose.

There have also been reports on the modification of the C-terminal sequence of OR genes. As shown below, changing or truncating the C-terminal sequence in most cases led to reduced activity, and only in few instances in a higher signal amplitude. So far changing the C-terminus of an OR never led to a significantly more sensitive assay, i.e. an assay which allows testing at significantly lower test concentrations.

Kotthoff *et al.* found that truncating human OR8D1 by three amino acids reduced signal amplitude, truncating by seven amino acids or by 11 or by 15 amino acids abolished the signal, but they did not find enhanced functions of a truncated C-terminus (Kotthoff et al. The FASEB Journal 2021;35:e21274.). Changing the C-terminus had negative effects in OR8D1. However, changing the parent C-terminus sequence towards the consensus sequence had a positive effect in two cases: thus, changing the C-terminus of human OR2M3 towards the consensus sequence by changing three amino acids did enhance signal amplitude three-fold, but it did not significantly enhance sensitivity / potency, i.e. it did not shift the dose-response curve towards lower concentrations. Changing mouse olfr16 towards consensus doubled signal amplitude, but it only marginally enhanced sensitivity (EC50 from 33.99 to 20.42 micromolar, Table S19 in said reference). All other changes introduced to the C terminus (> 70 variants investigated) always led to reduced activity and/or reduced surface expression, indicating overall that no large improvement of receptor expression and assay sensitivity was possible by changing the C terminus.

In a very detailed analysis in the above-mentioned reference (Ikegami *et al.* 2020), two closely related mouse receptors (mouse Olfr539 and Olfr541) were compared, whereby Olfr541 is poorly expressed and Olfr539 is well expressed. By exchanging parts of the sequence or even single amino acids of the central domain of Olfr541 with the sequence from Olfr539, strong expression could be achieved, however replacing the C-terminus of the poorly expressed Olf541 with the C-terminus of the well expressed Olfr539 did not enhance the expression of Olfr541, indicating that other parts of the sequence and not the C-terminus are critical to confer functional expression.

In another example, the sequence of the human OR5A2 was modified both at the C terminus and at the N terminus by exchanging the OR5A2 sequence with the OR2A5 sequence to generate a chimeric receptor (Example 5 in WO2019110630A1). The resulting receptor responded equally well to musk compounds as the wild-type receptor, and the dose-response curve was not changed nor was sensitivity enhanced by the changed C-terminus.

Furthermore, in a frequently cited work (Krautwurst et al. identification of ligands for olfactory receptors by functional expression of a receptor library. Cell. 1998;95(7):917-26), Krautwurst *et al.* used the N-terminal sequence and the C-terminal sequence of the well-expressed mouse receptor M4 and entered the sequence from TM2-TM7 of other tested receptors into this backbone to generate chimeric receptors. Chimeric receptors with this M4 backbone responded equally as the wild-type receptors, but no enhanced signal or better functionality was shown for the chimeric receptors and in one instance a response at an even lower concentration (1 $\mu$M instead of 10 $\mu$M) was reported for the wild-type mouse I7 receptor as compared to the chimeric receptor indicating that exchanging the C-terminus with a C-terminus of a well expressed receptor can mostly maintain, but not increase activity.

Katada *et al.* found that truncating mouse mOR-EG by three amino acids reduced signal amplitude, truncating by six amino acids reduced signal amplitude and sensitivity, truncating by 12 amino acids abolished the signal, but they did not find enhanced functions of a truncated C-terminus indicating that in the investigated cases the full-length C-terminus is required (Kadata et al. Structural determinants for membrane trafficking and G protein selectivity of a mouse olfactory receptor. Journal of Neurochemistry 2004;90(6):1453-1463).

Kato *et al.* found that mutating K296, K299, K303, K304 and K309 in the C terminus of the mouse mOR-EG to proline reduces/abolishes activity, mutations in these residues to Ala or Arg did not significantly affect the activity (with the exception of K296A which reduced sensitivity), but this study did not find enhanced functions (enhanced amplitude or enhanced sensitivity) of mutated C-terminus sequences and indicated that most of these basic residues appear to be non-essential for functional expression (Kato et al. Amino acids involved in conformational dynamics and G protein coupling of an odorant receptor: targeting gain-of-function mutation. Journal of Neurochemistry 2008;107(5):1261-1270).

Finally, Sato *et al.* investigated C-terminal olfactory receptor sequence and which residues are key, and they found that residues 299, 300, 303, and 304 in the C-terminus of mOR-S6 are important to maintain function, but they did not find enhanced functions for C-terminus modifications (Sato et al. Functional Role of the C-Terminal Amphipathic Helix 8 of Olfactory Receptors and Other G Protein-Coupled Receptors. Int. J. Mol. Sci. 2016;17(11):1930).

Taken together, despite the long-known improvement of olfactory receptor expression by changing the N-terminus by the addition of a rho-Tag, attempts at changing the C-terminus did not lead to significantly improved assays but mainly showed that the C-terminus is sensitive to changes in structure which in most cases leads to loss of function and not to gain of function modifications. Based on these teachings, it was not to be expected that a major advancement in the functional expression of olfactory receptors could be gained from changing the C-terminal sequence. Rather, this detailed analysis did not indicate that truncating the C-terminus of the receptor or changing the C-terminus towards consensus can enhance sensitivity of the assay significantly, but it indicated that the signal amplitude can be enhanced in few selected cases, while the majority of changes to the C-terminus had a negative effect.

Olfactory receptor proteins

This disclosure generally concerns olfactory receptor proteins (ORs) and activation data and activation vectors generated with such OR. In particular, such data are generated with well-expressed OR and in a particular embodiment they are generated with OR with modified C-terminal domains. Accordingly, provided herein is an olfactory receptor protein, wherein said protein has a modified C-terminal domain. Preferably, the olfactory receptor protein is a mammalian, more preferably a human olfactory receptor protein. Preferably, the olfactory receptor protein corresponds to a Class I or Class II OR, as described later herein.

The term "olfactory receptor" or "odorant receptor" (OR) as used herein has its customary meaning as ordinarily understood by the skilled person in view of this disclosure. It refers to receptors pertaining to the seven-transmembrane-domain G protein-coupled receptor superfamily (GPCRs), which are typically expressed in the cell membrane of olfactory receptor neurons. The predicted seven-transmembrane (TM) domains TM I to TM VII are connected by three predicted internal (IC) loop domains (IC I to IC III), and three predicted external (EC) loop domains (EC I to EC III). ORs typically comprise olfactory receptor-specific amino acid motifs. Examples of such motifs are a MAYDRYVAIC (SEQ ID NO: 2) motif overlapping TM III and IC II, a FSTCSSH (SEQ ID NO: 3) motif overlapping IC III and TM VI, a PMLNPFIY (SEQ ID NO: 4) motif in TM VII as well as three conserved C residues in EC II, and the presence of highly conserved GN residues in TM I, discussed in Zhang and Firestein (2002) Nature Neurosci 5(2): 124-33, and Malnic et al. (2004) PNAS 101(8):2584-9, both of which are incorporated herein by reference.

The C-terminal domain of an olfactory receptor starts right after the end of the seventh TM helix (TM7). The skilled person can determine this position without doubt on the basis of his common general knowledge. For example, the seventh transmembrane region (TM7) can easily be recognized within any OR based on sequence alignment or by using well-known and publicly available databases. For example, the "GPCR Prediction Ensemble Database (GPCR-PEnDB)" available at https://gpcr.utep.edu/database indicates the amino acid positions of TM7 and the length of the native C-terminus for many ORs.

The same information can be derived from the "HORDE" (The Human Olfactory Data Explorer) database maintained by the Weizmann Institute of Science and available at https://genome.weizmann.ac.il/horde/, described in Olender et al. (2013) Methods Mol Biol 1003:23-38, incorporated herein by reference. In HORDE, the residues of all of TM1-TM7 are annotated.

The same information can also be found in general sequence databases such as Uniprot (The UniProt Consortium, UniProt: the universal protein knowledgebase in 2021, Nucleic Acids Research, Volume 49, Issue D1, 8 January 2021, Pages D480-D489, available at www.uniprot.org).

TM7 in Class II olfactory receptors typically ends with the consensus sequence NPLIYSL (SEQ ID NO: 225), with the last of these seven amino acids usually located at residue 292-298 of the full-length receptor and the end of TM7 can thus easily be identified for any given OR. Directly after this sequence (or at the position where the native C-terminus starts as indicated under https://gpcr.utep.edu/database) the above modified C-terminus is fused to the olfactory receptor for improved functional expression.

TM7 in Class I olfactory receptors typically ends with the consensus sequence NPIIYSL (SEQ ID NO: 226) or NPIIYSGL (SEQ ID NO: 227), with the last of these seven amino acids usually located at residue 297 (290-300). Directly after this sequence (or at the position where the native C-terminus starts as indicated under https://gpcr.utep.edu/ database) the above modified C-terminus is fused to the olfactory receptor for improved functional expression.

Accordingly, in some cases, the C-terminal domain as described herein is such that it starts right after the last residue of the seventh transmembrane helix (TM7). In some cases, the C-terminal domain is fused to the seventh transmembrane helix of the olfactory receptor protein. In some cases, the C-terminal domain as described herein may also be referred to as the cytoplasmic or intracellular domain.

Thus, in some cases, olfactory receptors as described herein comprise an amino acid sequence of an olfactory receptor up to and including the last residue of the olfactory receptor's seventh transmembrane helix (TM7), followed by an amino acid sequence of a modified C-terminal domain comprising an amino acid sequence motif as described herein.

The modified C-terminal domain may comprise or consist of 10 to 40 amino acids, preferably 12 to 30 amino acids, more preferably 14 to 26 amino acids, even more preferably 16 to 22 amino acids. In some cases, the minimum length of the modified C-terminal domain of the olfactory receptors of this disclosure is 10, 11, 12, 13, 14, 15 or 16 amino acids; and the maximum length of the modified C-terminal domain of the olfactory receptors of this disclosure is 26, 25, 24, 23 or 22 amino acids.

The present inventors have found that particularly advantageous effects described herein are observed for modified C-terminal domains that are rich in basic amino acids. A basic amino acid, as used herein, is understood to refer to an amino acid having a side chain that is protonated at neutral pH. Basic amino acids include lysine (Lys, K), arginine (Arg, R), and histidine (His, H). Preferred basic amino acids in the context of this disclosure are lysine and arginine.

Accordingly, in some cases, a modified C-terminal domain as described herein is a modified C-terminal domain comprising at least 5 basic residues, preferably at least 6 basic residues, more preferably at least 7 basic residues. In some cases, a modified C-terminal domain as described herein is a modified C-terminal domain in which at least 32%, at least 33%, at least 34%, at least 35%, at least 36%, at least 37%, at least 38%, at least 39%, at least 40%, at least 41%, at least 42%, or at least 43% of the amino acids is a basic amino acid (i.e., lysine, arginine, or histidine). In preferred cases, a modified C-terminal domain as described herein is a modified C-terminal domain in which at least 32% of the amino acids is a basic amino acid (i.e., lysine, arginine, or histidine). In preferred cases, a modified C-terminal domain as described herein is a modified C-terminal domain in which at least 35% of the amino acids is a basic amino acid (i.e., lysine, arginine, or histidine). In preferred cases, a modified C-terminal domain as described herein is a modified C-terminal domain in which at least 38% of the amino acids is a basic amino acid (i.e., lysine, arginine, or histidine).

In an aspect, there is provided an olfactory receptor protein, wherein said protein has a modified C-terminal domain comprising the amino acid sequence motif:

RN[KR][EDQ][VM I L][KR]xA[LIV][KRH][KR][LI][LI F][KRG][KR][KR] (SEQ ID NO: 1).

A "sequence motif" may also be denoted as a "sequence pattern" or, simply, a "sequence". A "sequence motif" or "sequence pattern" has its customary and ordinary meaning as understood by one of skill in the art in view of this disclosure. It refers to an amino acid (or nucleotide) sequence that recurs, with a certain degree of variation, on several sites of a molecule or several different molecules and has (or is conjectured to have or is assumed to be linked to) a biological significance or exhibits a biological activity as described herein. A biological significance or biological activity of the sequence motifs described herein is preferably its ability to improve the functional heterologous expression of (nucleotide sequences encoding) an olfactory receptor protein comprising said motif as a C-terminal domain.

As used herein, the term "expression" or "heterologous expression" of a DNA molecule by a cell includes any step involved in the production of a polypeptide by a cell including, but not limited to, transcription, post-transcriptional modification, translation, post-translational modification, transport to a cellular membrane, and secretion. Expression may be assessed by any method known to a person of skill in the art. For example, expression may be assessed by measuring the levels of gene expression in a transduced cell on the level of the mRNA or the protein by standard assays known to a person of skill in the art, such as qPCR, RNA sequencing, Northern blot analysis, Western blot analysis, mass spectrometry analysis of protein-derived peptides, fluorescence activated cell sorting (FACS), immunostaining or ELISA.

As used herein, the term "functional expression" or "functional heterologous expression" refers to the production of a polypeptide by a cell wherein the polypeptide exhibits a biological activity. For example, an olfactory receptor is functionally expressed by a cell when said receptor, following its production, is transported and incorporated into the cellular membrane and is able to trigger its corresponding signalling cascade following its activation by a ligand. Conventional methods assessing the functional expression of an olfactory receptor involve the expression of said OR with the co-expression of a luciferase gene operably linked to a cAM P-inducible promoter (Saito et al. (2004) Cell 119(5): 679-691), which is used as a reporter gene. If the olfactory receptor is functionally expressed, its activation and subsequent resulting increase in intracellular cAMP results in an increase of luciferase expression. Oxidation of luciferin by luciferase in standard assays results in the emission of light which can then be detected and quantified.

The sensory potency of odorants, and hence the potency of the odorant-OR interaction *in vivo* is most commonly described as the odor detection threshold for an odorant (OTH), i.e. the lowest concentration in the gas phase which is detected by the human nose. The terms "odor detection threshold" and "odor threshold", also abbreviated herein as "OTH", are synonymous and are well-established terms in the fragrance field, see for example "The Measuring of Odors" by Neuner-Jehle, N., Etzweiler, F. (1994). In: Maller, P.M., Lamparsky, D. (eds) Perfumes. Springer, Dordrecht, incorporated herein by reference in its entirety. Odor detection thresholds can be measured by methods and means commonly available in the art, for example by making use of an olfactometer in conjunction with human subjects. Another possibility to measure the odor detection threshold is to inject a dilution series of defined quantities (measured in ng) of the ligand into a gas chromatograph (GC), whereby a human panellist is sniffing the molecule as emitted from the GC-column at a Sniff port and indicating whether it is detectable by the nose. This gives the GC-threshold (GCO) in ng.

In OR screening assays applying OR in *in vitro* systems, the ligands are dissolved in a liquid medium. Nevertheless, similar to an *in vivo* odor threshold experiment, the lowest dose can be determined at which an odorant can start to activate the receptor in the *in vitro* experiment, whereby sensitivity in the *in vitro* system is reported as the lowest concentration dissolved in the medium which starts to activate a receptor, while sensitivity in vivo is expressed as the lowest detectable concentration in the gas phase. As a practical possibility the detection threshold in vitro can be defined as the concentration leading to a two-fold response vs. the background signal in e.g. a luciferase assay as described herein.

Functional expression of an olfactory receptor polypeptide as described herein is improved (increased) relative to a baseline functional expression, leading to improved (increased) biological activity, for example relative to a non-modified corresponding receptor gene. Said functional expression may be improved (increased) by at least 10%, at least 20%, at least 30%, at least 40%, at least 50%, at least 60%, at least 70%, at least 80%, at least 100%, at least 200%, at least 300%, at least 500%, relative to a non-modified corresponding receptor gene. Such improvement of functional expression may also be manifest in an increase of the sensitivity of recombinant host cells expressing an olfactory receptor polypeptide as described herein, such that the detection threshold of the receptor in an *in vitro* assay is reduced at least a factor of 2-fold, at least 3.1-fold, at least 10-fold at least 20-fold or at least 50-fold, preferably at least 3.1-fold (i.e. increased detection sensitivity of a ligand tested). Such improvement of functional expression may further be manifest in an increase of the sensitivity of recombinant host cells expressing an olfactory receptor polypeptide as described herein (described elsewhere herein), such that the EC50, i.e. the concentration to reach 50% of the maximal signal amplitude is decreased by a factor of at least 2-fold, at least 3.1-fold, at least 10-fold at least 20-fold or at least 50-fold, preferably at least 3.1-fold (i.e. enhanced potency of a ligand tested). Such improvement of functional expression may also be manifested in an increase of the sensitivity of recombinant host cells expressing an olfactory receptor polypeptide as described herein, such that the maximal signal amplitude is enhanced by at least 30%, at least 50%, at least 80%, at least 100% at least 200% at least 500% (i.e. increased efficacy of a ligand tested). Improvement of functional expression may also be of such a magnitude that functional expression of olfactory receptors otherwise not possible using conventional approaches is achieved using the olfactory receptors, nucleic acid molecules, expression vectors, recombinant host cells, and methods and uses of the disclosure (all-or-nothing effect which cannot be quantified in numbers).

Several customary notations for describing sequence motifs are in use and are known to the skilled person, most of them being variants of standard notations for regular expressions and using at least the following conventions:

- there is an alphabet of single characters, i.e. the standard IUPAC one-letter codes for the amino acids, each denoting a specific amino acid or a set of amino acids;
- a string of characters drawn from the alphabet denotes a sequence of the corresponding amino acids;
- a string of characters between square brackets matches any one of the listed amino acids, i.e. it denotes sequence ambiguities or alternatives, e.g. the hypothetical notation [XYZ] means X or Y or Z;
- a string of characters between braces/curly brackets ("{}") means any amino acid except the listed amino acid, e.g. the hypothetical notation {X} means any amino acid except X.

Thus, the 16 amino acids in the sequence motif depicted above:
RN[KR][EDQ][VMIL][KR]xA[LIV][KRH][KR][LI][LIF][KRG][KR][KR] (SEQ ID NO: 1) are defined as follows:

- the first residue is R;
- the second residue is N;
- the third residue is K or R;
- the fourth residue is E or D or Q;

- the fifth residue is V or M or I or L;
- the sixth residue is K or R;
- the seventh residue can be any amino acid;
- the eighth residue is A;
- the ninth residue is L or I or V;
- the tenth residue is K or R or H;
- the eleventh residue is K or R;
- the twelfth residue is L or I;
- the thirteenth residue is L or I or F;
- the fourteenth residue is K or R or G;
- the fifteenth residue is K or R; and
- the sixteenth residue is K or R.

Thus, the sequence motif depicted above:
RN[KR][EDQ][VMI L][KR]xA[LIV][KRH][KR][LI][LI F][KRG][KR][KR] (SEQ ID NO: 1) may also alternatively be described as:
$RNX_1X_2X_3X_4X_5AX_6X_7X_8X_9X_{10}X_{11}X_{12}X_{13}$, wherein:

- $X_1$ is K or R;
- $X_2$ is E or D or Q;
- $X_3$ is V or M or I or L;
- $X_4$ is K or R;
- $X_5$ is any amino acid;
- $X_6$ is L or I or V;
- $X_7$ is K or R or H;
- $X_8$ is K or R;
- $X_9$ is L or I;
- $X_{10}$ is L or I or F;
- $X_{11}$ is K or R or G;
- $X_{12}$ is K or R; and
- $X_{13}$ is K or R.

Taking into account that the standard IUPAC one-letter codes for amino acids include the symbol "J" for denoting leucine (L) or isoleucine (I), the above-depicted sequence motif may alternatively be described as: $RNX_1X_2X_3X_4X_5AX_6X_7X_8JX_{10}X_{11}X_{12}X_{13}$ (SEQ ID NO: 1), wherein:

- $X_1$ is K or R;
- $X_2$ is E or D or Q;
- $X_3$ is V or M or I or L;
- $X_4$ is K or R;
- $X_5$ is any amino acid;
- $X_6$ is L or I or V;
- $X_7$ is K or R or H;
- $X_8$ is K or R;
- $X_{10}$ is L or I or F;
- $X_{11}$ is K or R or G;
- $X_{12}$ is K or R; and
- $X_{13}$ is K or R.

All of the above notations are completely equivalent and represent the same sequence (SEQ ID NO: 1); thus, a skilled person understands that they can be used interchangeably. The same holds true for any other sequence motif described herein.
Olfactory receptors may be classified as Class I or "fish-like" receptors, and Class II or "tetrapod" receptors. Class I or "fish-like" receptors are an evolutionary more ancient class of receptors known to respond especially to more soluble compounds such as carboxylic acids and it has a particular interest in the screening for antagonists to malodorants. The majority of the olfactory receptors belong to Class II or "tetrapod" receptors and these comprise the key receptors for most fragrant molecules. There is a considerable difference in the C-terminal sequence between Class I and Class II receptors, however, the present disclosure is targeted at both Class I and Class II olfactory receptors.

Accordingly, in some cases, an olfactory receptor protein as described herein is a class I or class II olfactory receptor with a modified C-terminal domain.

The olfactory receptor proteins of this disclosure have a modified C-terminal domain. Thus, the C-terminal domain of the olfactory receptor proteins of this disclosure differs from the natural or cognate C-terminal domain with which said olfactory receptor is normally associated. Therefore, the olfactory receptor proteins of this disclosure are non-naturally occurring proteins. It follows that the olfactory receptor proteins described herein can be characterized as "modified" olfactory receptor proteins, "engineered" olfactory receptor proteins, "hybrid" olfactory receptor proteins, "chimeric" olfactory receptor proteins, "non-natural" olfactory receptor proteins, or similar expressions and combinations thereof. Throughout this disclosure, the skilled person understands that the term "olfactory receptor protein" may be replaced with the term "olfactory receptor protein having a modified C-terminal domain".

This disclosure encompasses olfactory receptor proteins from any mammal. Accordingly, in some cases, an olfactory receptor protein as described herein is a mammalian olfactory receptor having a modified C-terminal domain, preferably a mammalian class I or class II olfactory receptor with a modified C-terminal domain. Preferred mammals in the context of this disclosure are pet or companion animals and humans, with humans being more preferred. Within the pet or companion animals, cats and dogs are particularly preferred. Accordingly, in some cases, an olfactory receptor protein as described herein is a human, dog or cat olfactory receptor having a modified C-terminal domain, preferably a human olfactory receptor having a modified C-terminal domain. In some cases, an olfactory receptor protein as described herein is a human, dog or cat class I or class II olfactory receptor having a modified C-terminal domain, preferably a human class I or class II olfactory receptor having a modified C-terminal domain.

Mammalian and human olfactory receptors are discussed in publications such as Mainland et al. (2015) Sci Data 2:150002, incorporated herein by reference, and in publicly available databases, such as the HORDE (The Human Olfactory Data Explorer) database maintained by the Weizmann Institute of Science and available at https://genome. weizmann.ac.il/horde/, described in Olender et al. (2013) Methods Mol Biol 1003:23-38, incorporated herein by reference. Other relevant publicly available databases exist, for example as described in Marenco et al. Database (Oxford) 2016:baw132 and Han et al. Science China. Life sciences, 10.1007/s11427-021-2081-6.

The complete list of human olfactory receptors and their alleles or haplotypes can be found in the HORDE database mentioned above. Major alleles or haplotypes are defined as those with $\geq$ 20% frequency in the human population according to the HORDE database. A listing of all the amino acid sequences of the main alleles or haplotypes of the vast majority of human olfactory receptors is available to the skilled person and can be found as supporting information to Ikegami et al. 2020 (supra), while the nucleotide sequence of 625 genes covering the vast majority of human OR genes and their major alleles or haplotypes is included as supporting information to Mainland *et al.* 2015 (supra). All known human olfactory receptors are also available from general sequence databases such as NCBI Genbank available at https://www. ncbi.nlm.nih.gov/genbank/.

In some cases, an olfactory receptor protein as described herein is selected from the group consisting of OR10A2, OR10A3, OR10A4, OR10A5, OR10A6, OR10A7, OR10AD1, OR10AG1, OR10C1, OR10D3, OR10G2, OR10G3, OR10G4, OR10G6, OR10G7, OR10G8, OR10G9, OR10H1, OR10H2, OR10H3, OR10H4, OR10H5, OR10J1, OR10J3, OR10J5, OR10K1, OR10K2, OR10P1, OR10P2, OR10Q1, OR10R2, OR10S1, OR10T2, OR10V1, OR10W1, OR10X1, OR10Z1, OR11A1, OR11G2, OR11H1, OR11H2, OR11H4, OR11H6, OR11L1, OR12D2, OR12D3, OR13C2, OR13C3, OR13C4, OR13C5, OR13C8, OR13C9, OR13D1, OR13F1, OR13G1, OR13H1, OR13J1, OR14A16, OR14A2, OR14C36, OR14I1, OR14L1P, OR1A1, OR1A2, OR1B1, OR1C1, OR1D2, OR1D4, OR1D5, OR1E1, OR1E2, OR1F1, OR1F12, OR1G1, OR1I1, OR1J1, OR1J2, OR1J4, OR1K1, OR1L1, OR1L3, OR1L4, OR1L6, OR1L8, OR1M1, OR1N1, OR1N2, OR1Q1, OR1S1, OR1S2, OR2A1, OR2A12, OR2A14, OR2A2, OR2A25, OR2A4, OR2A42, OR2A5, OR2A7, OR2A9P, ORAE1, OR2AG1, OR2AG2, OR2AJ1, OR2AK2, OR2AP1, OR2AT4, OR2B11, OR2B2, OR2B3, OR2B6, OR2B8P, OR2C1, OR2C3, OR2D2, OR2D3, OR2F1, OR2F2, OR2G2, OR2G3, OR2G6, OR2H1, OR2H2, OR2J1P, OR2J2, OR2J3, OR2K2, OR2L13, OR2L2, OR2L3, OR2L5, OR2L8, OR2M2, OR2M3, OR2M4, OR2M5, OR2M7, OR2S2, OR2T1, OR2T10, OR2T11, OR2T12, OR2T2, OR2T27, OR2T29, OR2T3, OR2T33, OR2T34, OR2T35, OR2T4OR2T5, OR2T6, OR2T7, OR2T8, OR2V1, OR2V2, OR2W1, OR2W3, OR2W5, OR2Y1, OR2Z1, OR3A1, OR3A2, OR3A3, OR3A4, OR4A15, OR4A16, OR4A4, R4A47, OR4A5, OR4B1, OR4C11, OR4C12, OR4C13, OR4C15, OR4C16, OR4C, OR4C45, OR4C46, OR4C5, OR4C6, OR4D1, OR4D10, OR4D11, OR4D2, OR4D5, OR4D6, OR4D9, OR4E2, OR4F15, OR4F16, OR4F17, OR4F21, OR4F29, OR4F3, OR4F4, OR4F5, OR4F6, OR4K1, OR4K13, OR4K14, OR4K15, OR4K17, OR4K2, OR4K3P, OR4K5, OR4L1, OR4M1, OR4M2, OR4N2, OR4N4, OR4N5, OR4P4, OR4Q3, OR4S1, OR4S2, OR4X1, OR4X2, OR51A2, OR51A4, OR51A7, OR51B2, OR51B4, OR51B5, OR51B6, OR51D1, OR51E1, OR51E2, OR51F1, OR51F2, OR51G1, OR51G2, OR51H1P, OR51I1, OR51I2, OR51J1, OR51L1, OR51M1, OR51Q1, OR51S1, OR51T1, OR51V1, OR52A1, OR52A4, OR52A5, OR52B2, OR52B4, OR52B6, OR52D1, OR52E2, OR52E4, OR52E5, OR52E6, OR52E8, OR52H1, OR52I1, OR52I2, OR52J3, OR52K1, OR52K2, OR52L1, OR52M1, OR52N1, OR52N2, OR52N4, OR52N5, OR52P1P, OR52R1, OR52W1, OR56A1, OR56A3, OR56A4, OR56A5, OR56B1, OR56B4, OR5A1, OR5A2, OR5AC2, OR5AK2, OR5AL1P, OR5AN1, OR5AP2, OR5AR1, OR5AS1, OR5AU1, OR5B12, OR5B17, OR5B2, OR5B21, OR5B3, OR5C1, OR5D13, OR5D14, OR5D16, OR5D18, OR5F1, OR5H1, OR5H14, OR5H15, OR5H2, OR5H6, OR5I1, OR5J2, OR5K1, OR5K2, OR5K3, OR5K4, OR5L1, OR5L2, OR5M1, OR5M10, OR5M11, OR5M3, OR5M8, OR5M9, OR5P2, OR5P3, OR5R1, OR5T1, OR5T2, OR5T3, OR5V1, OR5W2, OR6A2, OR6B1, OR6B2, OR6B3, OR6C1, OR6C2, OR6C3, OR6C4, OR6C6, OR6C65, OR6C68, OR6C70, OR6C74, OR6C75, OR6C76, OR6F1, OR6J1, OR6K2, OR6K3, OR6K6, OR6M1, OR6N1, OR6N2, OR6P1, OR6Q1, OR6S1, OR6T1, OR6X1, OR6Y1, OR7A10, OR7A17, OR7A5, OR7C1, OR7C2, OR7D2, OR7D4, OR7E24, OR7G1, OR7G2, OR7G3, OR8A1, OR8B12, OR8B2, OR8B3, OR8B4, OR8B8, OR8D1, OR8D2, OR8D4, OR8G1, OR8G5, OR8H1, OR8H2, OR8H3, OR8I2, OR8J1, OR8J3, OR8K1, OR8K3, OR8K5, OR8S1, OR8U1, OR8U8, OR8U9, OR9A2, OR9A4, OR9G1, OR9G4, OR9G9, OR9I1, OR9K2, OR9Q1, and OR9Q2. Variants and haplotypes of these receptors are also encompassed.

In some cases, an olfactory receptor protein as described herein is:

- a class II olfactory receptor listed as "receptor" in Table 15, or
- a class I olfactory receptor listed as "receptor" in Table 22.

The specific sequences mentioned in Table 15 (for DNA encoding the modified receptor including an N-terminal mmLucy-FLAG-rho tag (SEQ ID NO: 81) and a modified C-terminus (SEQ ID NO: 221)) and in Table 22 (for DNA encoding the modified receptor including an N-terminal mmLucy-FLAG-rho tag (SEQ ID NO: 81) and a modified C-terminus (SEQ ID NO: 741)) are specific cases which are not limiting in this context. The presence of the N-terminal tag is optional, and different N-terminal tags could be used, as described elsewhere herein. Similarly, any modified C-terminal domain disclosed herein may be used instead of the modified C-terminus of SEQ ID NO: 221 or SEQ ID NO: 741.

A modified C-terminal domain as described herein may comprise the sequence RNKEVKDALKRLLKRK (SEQ ID NO: 10). In some cases, the sequence RNKEVKDALKRLLKRK (SEQ ID NO: 10) may contain amino acid substitutions at 1, 2, 3, 4, 5, 6, 7, 8, or up to 9 positions. In some cases, the sequence may contain amino acid substitutions at 1, 2, 3, 4, or up to 5 positions but the residues at the first (R), second (N) and eighth (A) positions are fixed. In some cases, the sequence may contain amino acid substitutions at 1, 2, 3, 4, 5, 6, 7, 8, or up to 9 positions but the residues at the first (R), second (N), fourth (E) and eigth (A) positions are fixed. Amino acid substitutions can preferably be conservative amino acid substitutions, as described elsewhere herein. Examples of particularly suitable amino acid substitutions in this context include the substitution of K for R, and R for K.

In some cases, an olfactory receptor protein as described herein is a class II olfactory receptor having a modified C-terminal domain. In some cases, the class II olfactory receptor is selected from the group consisting of OR7C1, OR9Q2, OR8K3, OR10J5, OR1C1, OR7D4, OR2T4, OR5B12, OR7A17, OR10H5, OR5A1, OR5A2, OR1N2, OR2C1, OR2T11, OR2M2, and OR4S2, preferably selected from the group consisting of OR7A17, OR2M2 and OR5A2. In some cases, the class II olfactory receptor is selected from the group consisting of OR2V1, OR5P3, OR6P1, OR2L2, OR10G7, OR5AN1, OR5V1, OR2L3, OR2AG2, OR7A5, OR7E24, OR7A10, OR10H2, OR10H1, OR10D3, OR1D2, OR2A5, OR2A25, OR11G2, OR14J1, OR5A1, OR5M3, OR8D1, OR10G3, OR10G9, OR2L5, OR8H1, OR10K1, OR11A1, OR2AK2, OR10A3, OR10A6, OR10J1, and OR2J2. In some cases, the class II olfactory receptor is selected from the group consisting of OR7C1, OR9Q2, OR8K3, OR10J5, OR1C1, OR7D4, OR2T4, OR5B12, OR7A17, OR10H5, OR5A1, OR5A2, OR1N2, OR2C1, OR2T11, OR2M2, OR4S2, OR2V1, OR5P3, OR6P1, OR2L2, OR10G7, OR5AN1, OR5V1, OR2L3, OR2AG2, OR7A5, OR7E24, OR7A10, OR10H2, OR10H1, OR10D3, OR1D2, OR2A5, OR2A25, OR11G2, OR14J1, OR5M3, OR8D1, OR10G3, OR10G9, OR2L5, OR8H1, OR10K1, OR11A1, OR2AK2, OR10A3, OR10A6, OR10J1, and OR2J2, preferably selected from the group consisting of OR7A17, OR7C1, OR2A25, OR7E24, OR10H1, OR10K1, OR2AG2, OR10H2, OR10H5, OR10D3, OR14J1, OR7A10, OR2L5, OR2M2 and OR5A2.

OR7C1, OR9Q2, OR8K3, OR10J5, OR1C1, OR7D4, OR2T4, OR5B12, OR7A17, OR10H5, OR5A1, OR5A2, OR1N2, OR2C1, OR2T11, OR2M2, OR4S2, OR2L3, OR2AG2, OR7A5, OR7E24, OR7A10, OR10H2, OR10H1, OR10D3, OR1D2, OR2A5, OR2A25, OR11G2, OR14J1, OR5M3, OR8D1, OR10G3(S73G), OR10G9, OR2L5, OR8H1, OR10K1, OR11A1, OR2AK2, OR10A3, OR10A6, OR10J1, and OR2J2 are human class II olfactory receptors.

OR2A25 may also be OR2A25(S75N,A209P). OR1N2 may also be OR1N2(W23R,V230G,T287M). OR7E24 may also be OR7E24(P242S). OR2AG2 may also be OR2AG2(Y28C). OR8K3 may also be OR8K3(L122R). OR2L2 may also be OR2L2(V259L). OR11G2 may also be OR11G2(I65N,V82I). OR10G7 may also be OR10G7(T5S). OR2AK2 may also be OR2AK2(S84N). OR10A6 may also be OR10A6(A117V,V140G,L287P). OR10J1 may also be OR10J1(M51I,I92M). OR10G3 may also be OR10G3(S73G).

In some cases, (wild type) OR7C1 has an amino acid sequence encoded by the nucleotide sequence of NCBI Gene ID 26664 (NCBI Reference Sequence NP_001357414.2). In some cases, (wild type) OR9Q2 has an amino acid sequence encoded by the nucleotide sequence of NCBI Gene ID 219957 (NCBI Reference Sequence: NP_001005283.1). In some cases, (wild type) OR8K3 has an amino acid sequence encoded by the nucleotide sequence of NCBI Gene ID 219473 (NCBI Reference Sequence: NP_001005202.1). In some cases, (wild type) OR10J5 has an amino acid sequence encoded by the nucleotide sequence of NCBI Gene ID 127385 (NCBI Reference Sequence: NP_001004469.1). In some cases, (wild type) OR1C1 has an amino acid sequence encoded by the nucleotide sequence of NCBI Gene ID 26188 (NCBI Reference Sequence: NP_036485.2). In some cases, (wild type) OR7D4 has an amino acid sequence encoded by the nucleotide sequence of NCBI Gene ID 125958 (NCBI Reference Sequence: NP_001005191.1). In some cases, (wild type) OR2T4 has an amino acid sequence encoded by the nucleotide sequence of NCBI Gene ID 127074 (NCBI Reference Sequence: NP_001004696.2). In some cases, (wild type) OR5B12 has an amino acid sequence encoded by the nucleotide sequence of NCBI Gene ID 390191 (NCBI Reference Sequence: NP_001004733.1). In some cases, (wild type) OR7A17 has an amino acid sequence encoded by the nucleotide sequence of NCBI Gene ID 26333 (NCBI Reference Sequence: NP_112163.1). In some cases, (wild type) OR10H5 has an amino acid sequence encoded by the nucleotide sequence of NCBI Gene ID 284433 (NCBI Reference Sequence: NP_001004466.1). In some cases, (wild type) OR5A2 has an amino acid sequence encoded by the nucleotide sequence of NCBI Gene ID 219981 (NCBI Reference Sequence: NP_001001954.1). In some cases, (wild type) OR5A1 has an amino acid sequence encoded by the nucleotide sequence of NCBI Gene ID 219982 (NCBI Reference Sequence: NP_001004728.1). In some cases, (wild type) OR1N2 has an amino acid sequence encoded by the nucleotide sequence of NCBI Gene ID 138882 (NCBI Reference Sequence: NP_001004457.2). In some cases, (wild type) OR2C1 has an amino acid sequence encoded by the nucleotide sequence of NCBI Gene ID 4993 (NCBI Reference Sequence: NP_036500.2). In some cases, (wild type) OR2T11 has an amino acid sequence encoded by the nucleotide sequence of NCBI Gene ID 127077 (NCBI Reference Sequence: NP_001001964.1). In some cases, (wild type) OR2M2 has an amino acid sequence encoded by the nucleotide sequence of NCBI Gene ID 391194 (NCBI Reference Sequence: NP_001004688.1). In some cases, (wild type) OR4S2 has an amino acid sequence encoded by the nucleotide sequence of NCBI Gene ID 219431 (NCBI Reference Sequence: NP_001004059.2). In some cases, (wild type) OR2L3 has an amino acid sequence encoded by the nucleotide sequence of NCBI Gene ID 391192 (NCBI Reference Sequence: NP_001004687.1). In some cases, (wild type) OR2AG2 has an amino acid sequence encoded by the nucleotide sequence of NCBI Gene ID 338755 (NCBI Reference Sequence: NP_001004490.1). In some cases, (wild type) OR7A5 has an amino acid sequence encoded by the nucleotide sequence of NCBI Gene ID 26659 (NCBI Reference Sequence: NP_001357409.1). In some cases, (wild type) OR7E24 has an amino acid sequence encoded by the nucleotide sequence of NCBI Gene ID 26648 (NCBI Reference Sequence: NP_001073404.1). In some cases, (wild type) OR7A10 has an amino acid sequence encoded by the nucleotide sequence of NCBI Gene ID 390892 (NCBI Reference Sequence: NP_001005190.1). In some cases, (wild type) OR10H2 has an amino acid sequence encoded by the nucleotide sequence of NCBI Gene ID 26538 (NCBI Reference Sequence: NP_039227.1). In some cases, (wild type) OR10H1 has an amino acid sequence encoded by the nucleotide sequence of NCBI Gene ID 26539 (NCBI Reference Sequence: NP_039228.1), In some cases, (wild type) OR10D3 has an amino acid sequence encoded by the nucleotide sequence of NCBI Gene ID 26497 (NCBI Reference Sequence: NP_001342142.1). In some cases, (wild type) OR1D2 has an amino acid sequence encoded by the nucleotide sequence of NCBI Gene ID 4991 (NCBI Reference Sequence: NP_001373017.1). In some cases, (wild type) OR2A5 has an amino acid sequence encoded by the nucleotide sequence of NCBI Gene ID 393046 (NCBI Reference Sequence: NP_036497.1). In some cases, (wild type) OR2A25 has an amino acid sequence encoded by the nucleotide sequence of NCBI Gene ID 392138 (NCBI Reference Sequence: NP_001004488.1). In some cases, (wild type) OR1 1G2 has an amino acid sequence encoded by the nucleotide sequence of NCBI Gene ID 390439 (NCBI Reference Sequence: NP_001005503.2). In some cases, (wild type) OR14J1 has an amino acid sequence encoded by the nucleotide sequence of NCBI Gene ID 442191 (NCBI Reference Sequence: NP_112208.1). In some cases, (wild type) OR5M3 has an amino acid sequence encoded by the nucleotide sequence of NCBI Gene ID 219482 (NCBI Reference Sequence: NP_001004742.2). In some cases, (wild type) OR8D1 has an amino acid sequence encoded by the nucleotide sequence of NCBI Gene ID 283159 (NCBI Reference Sequence: NP_001002917.1). In some cases, (wild type) OR10G3 has an amino acid sequence encoded by the nucleotide sequence of NCBI Gene ID 26533 (NCBI Reference Sequence: NP_001005465.1). In some cases, (wild type) OR10G9 has an amino acid sequence encoded by the nucleotide sequence of NCBI Gene ID 219870 (NCBI Reference Sequence: NP_001001953.1). In some cases, (wild type) OR2L5 has an amino acid sequence encoded by the nucleotide sequence of NCBI Gene ID 81466 (NCBI Reference Sequence: NP_001245213.1). In some cases, (wild type) OR8H1 has an amino acid sequence encoded by the nucleotide sequence of NCBI Gene ID 219469 (NCBI Reference Sequence: NP_001005199.1). In some cases, (wild type) OR10K1 has an amino acid sequence encoded by the nucleotide sequence of NCBI Gene ID 391109 (NCBI Reference Sequence: NP_001004473.1). In some cases, (wild type) OR11A1 has an amino acid sequence encoded by the nucleotide sequence of NCBI Gene ID 26531 (NCBI Reference Sequence: NP_001381757.1). In some cases, (wild type) OR2V1 has an amino acid sequence encoded by the nucleotide sequence of NCBI Gene ID 26693 (NCBI Reference Sequence: NP_001245212.1). In some cases, (wild type) OR5P3 has an amino acid sequence encoded by the nucleotide sequence of NCBI Gene ID 120066 (NCBI Reference Sequence: NP_703146.1). In some cases, (wild type) OR6P1 has an amino acid sequence encoded by the nucleotide sequence of NCBI Gene ID 128366 (NCBI Reference Sequence: NP_001153797.1). In some cases, (wild type) OR2L2 has an amino acid sequence encoded by the nucleotide sequence of NCBI Gene ID 26246 (NCBI Reference Sequence: NP_001004686.1). In some cases, (wild type) OR10G7 has an amino acid sequence encoded by the nucleotide sequence of NCBI Gene ID 390265 (NCBI Reference Sequence: NP_001004463.1). In some cases, (wild type) OR5AN1 has an amino acid sequence encoded by the nucleotide sequence of NCBI Gene ID 390195 (NCBI Reference Sequence: NP_001004729.1). In some cases, (wild type) OR5V1 has an amino acid sequence encoded by the nucleotide sequence of NCBI Gene ID 81696 (NCBI Reference Sequence: NP_110503.3). In some cases, (wild type) OR2AK2 has an amino acid sequence encoded by the nucleotide sequence of NCBI Gene ID 391191 (NCBI Reference Sequence: NP_001004491.2). In some cases, (wild type) OR10A3 has an amino acid sequence encoded by the nucleotide sequence of NCBI Gene ID 26496 (NCBI Reference Sequence: NP_001003745.1). In some cases, (wild type) OR10A6 has an amino acid sequence encoded by the nucleotide sequence of NCBI Gene ID 390093 (NCBI Reference Sequence: NP_001004461.1). In some cases, (wild type) OR10J1 has an amino acid sequence encoded by the nucleotide sequence of NCBI Gene ID 26476 (NCBI Reference Sequence: NP_001350486.1). In some cases, (wild type) OR2J2 has an amino acid sequence encoded by the nucleotide sequence of NCBI Gene ID 26707 (NCBI Reference Sequence: NP_112167.2). Major and functional alleles or haplotypes of these ORs were used herein.

In some cases, an olfactory receptor protein as described herein is a class I olfactory receptor having a modified C-terminal domain. In some cases, the class I receptor is selected from the group consisting of OR52A5, OR52E8, OR56A4, OR51B2, OR52K1, OR56A1, OR51B5, OR56A3, and OR51L1. OR51B2 may preferably be OR51B2(C120R,L134F,C209S). OR52K1 may also be OR52K1(Q52R). OR51B5 may also be OR51B5(G5S). OR56A3 may also be OR56A3(M51T).

OR52A5, OR52E8, OR56A4 , OR51B2, OR52K1, OR56A1, OR51B5, OR56A3, and OR51L1 are human class I olfactory receptors. In some cases, (wild type) OR52A5 has an amino acid sequence encoded by the nucleotide sequence of NCBI Gene ID 390054 (NCBI Reference Sequence: NP_001005160.1). In some cases, (wild type) OR52E8 has an amino acid sequence encoded by the nucleotide sequence of NCBI Gene ID 390079 (NCBI Reference Sequence: NP_001005168.2). In some cases, (wild type) OR56A4 has an amino acid sequence encoded by the nucleotide sequence of NCBI Gene ID 120793 (NCBI Reference Sequence: NP_001005179.3). In some cases, (wild type) OR51B2 has an amino acid sequence encoded by the nucleotide sequence of NCBI Gene ID 79345 (NCBI Reference Sequence: NP_149420.4). In some cases, (wild type) OR52K1 has an amino acid sequence encoded by the nucleotide sequence of NCBI Gene ID 390036 (NCBI Reference Sequence: NP_001005171.2). In some cases, (wild type) OR56A1 has an amino acid sequence encoded by the nucleotide sequence of NCBI Gene ID 120796 (NCBI Reference Sequence: NP_001001917.3). In some cases, (wild type) OR51B5 has an amino acid sequence encoded by the nucleotide sequence of NCBI Gene ID 282763 (NCBI Reference Sequence: NP_001005567.2). In some cases, (wild type) OR56A3 has an amino acid sequence encoded by the nucleotide sequence of NCBI Gene ID 390083 (NCBI Reference Sequence: NP_001003443.2). In some cases, (wild type) OR51L1 has an amino acid sequence encoded by the nucleotide sequence of NCBI Gene ID 119682 (NCBI Reference Sequence: NP_001004755.1).

As elaborated in the experimental section, the inventors have found that certain sequences corresponding with the sequence motif of SEQ ID NO: 1 are particularly advantageous. On that basis, a more preferred sequence motif has been identified. Accordingly, in preferred cases, an olfactory receptor protein as described herein is such that the sequence motif is:

RN[KR]E[VM!][KR]xA[L!V][KR][KR]L[L!F][KR][KR][KR] (SEQ ID NO: 5).

The sequence motif:

RN[KR]E[VM!][KR]xA[L!V][KR][KR]L[L!F][KR][KR][KR] (SEQ ID NO: 5) may alternatively be described as:

$RNX_1EX_3X_4X_5AX_6X_7X_8LX_{10}X_{11'}X_{12}X_{13}$ (SEQ ID NO: 5), wherein:

- $X_1$ is K or R;
- $X_{3'}$ is V or M or I;
- $X_4$ is K or R;
- $X_5$ is any amino acid;
- $X_6$ is L or I or V;
- $X_{7'}$ is K or R;
- $X_8$ is K or R;
- $X_{10}$ is L or I or F;
- $X_{11'}$ is K or R;
- $X_{12}$ is K or R; and
- $X_{13}$ is K or R.

In some cases, an olfactory receptor protein as described herein is such that the sequence motif is:

RN[KR]QIRxA[LIV][KRH][KR][LI][LIF][KRG][KR][KR] (SEQ ID NO: 824). The sequence motif RN[KR]QIRxA[LIV][KRH][KR][LI][LIF][KRG][KR][KR] (SEQ ID NO: 824) may alternatively be described as:

$RNX_1QIRX_5AX_6X_7X_8JX_{10}X_{11}X_{12}X_{13}$ (SEQ ID NO: 824), wherein:

- $X_1$ is K or R;

- $X_5$ is any amino acid;
- $X_6$ is L or I or V;
- $X_7$ is K or R or H;
- $X_8$ is K or R;
- $X_{10}$ is L or I or F;
- $X_{11}$ is K or R or G;
- $X_{12}$ is K or R; and
- $X_{13}$ is K or R.

A modified C-terminal domain comprising the motif of SEQ ID NO: 824 may be particularly advantageous in the case of Class I olfactory receptors.

The sequence motifs of SEQ ID NO: 1, SEQ ID NO: 5, and SEQ ID NO: 824 contain one residue which may be any amino acid, denoted as "x" or "$X_5$" herein above. The inventors have found that certain residues at this position yield particularly advantageous sequences. Accordingly, in further preferred cases, an olfactory receptor protein as described herein and having a modified C-terminal domain comprising any of the amino acid sequence motifs described above, is such that "x" or "$X_5$" is selected from any amino acid except proline (Pro, P).

In line with the above, in preferred cases, an olfactory receptor protein as described herein is such that the sequence motif is RN[KR][EDQ][VMIL][KR]{P}A[LIV][KRH][KR][LI][LIF][KRG][KR][KR] (SEQ ID NO: 311). The sequence motif RN[KR][EDQ][VMIL][KR]{P}A[LIV][KRH][KR][LI][LIF][KRG][KR][KR] (SEQ ID NO: 311) may be alternatively described as:

$RNX_1X_2X_3X_4X_{5''''}AX_6X_7X_8JX_{10}X_{11}X_{12}X_{13}$ (SEQ ID NO: 311), wherein:

- $X_1$ is K or R;
- $X_2$ is E or D or Q
- $X_3$ is V or M or I or L;
- $X_4$ is K or R;
- $X_{5''''}$ is D or K or E or N or R or V or A or Q or G or C or F or H or I or L or M or S or T or W or Y (any amino acid except P);
- $X_6$ is L or I or V;
- $X_7$ is K or R or H;
- $X_8$ is K or R;
- $X_{10}$ is L or I or F;
- $X_{11}$ is K or R or G;
- $X_{12}$ is K or R; and
- $X_{13}$ is K or R.

In other further preferred cases, an olfactory receptor as described herein is such that the sequence motif is:
$RNX_1EX_{3'}X_4X_{5''''}AX_6X_{7'}X_8LX_{10}X_{11'}X_{12}X_{13}$ (SEQ ID NO: 312), wherein:

- $X_1$ is K or R;
- $X_{3'}$ is V or M or I;
- $X_4$ is K or R;
- $X_{5''''}$ is D or K or E or N or R or V or A or Q or G or C or F or H or I or L or M or S or T or W or Y (any amino acid except P);
- $X_6$ is L or I or V;
- $X_{7'}$ is K or R;
- $X_8$ is K or R;
- $X_{10}$ is L or I or F;
- $X_{11'}$ is K or R;
- $X_{12}$ is K or R; and
- $X_{13}$ is K or R.

In some cases, an olfactory receptor protein as described herein and having a modified C-terminal domain comprising any of the amino acid sequence motifs described above, is such that "x" or "$X_5$" is selected from any amino acid except proline (Pro, P) and tryptophane (Trp, W).

In some cases, an olfactory receptor protein as described herein and having a modified C-terminal domain comprising any of the amino acid sequence motifs described above, is such that "x" or "$X_5$" is selected from D, K, R, E, N, V, A, Q, or G, preferably such that "x" or "$X_5$" is selected from D, K, R, E, N, V, A, or Q, more preferably such that "x" or "$X_5$" is selected from D, K, or R. In other words, in further preferred cases, an olfactory receptor protein as described herein is such that the sequence motif is:

RN[KR][EDQ][VMIL][KR][DKRENVAQG]A[LIV][KRH][KR][LI][LIF][KRG][KR][KR] (SEQ ID NO: 6), preferably: RN[KR][EDQ][VMIL][KR][DKREN VAQ]A[LI V][KRH][KR][LI][LI F][KRG][KR][KR] (SEQ ID NO: 7), more preferably: RN[KR][EDQ][VMIL][KR][DKR]A[LIV][KRH][KR][LI][LIF][KRG][KR][KR] (SEQ ID NO: 166); or the sequence motif is: RN[KR]E[VMI][KR][DKRENVAQG]A[LIV][KR][KR]L[LIF][KR][KR][KR] (SEQ ID NO: 8), preferably: RN[KR]E[VMI][KR][DKRENVAQ]A[LIV][KR][KR]L[LIF][KR][KR][KR] (SEQ ID NO: 9), more preferably: RN[KR]E[VM!][KR][DKR]A[L!V][KR][KR]L[L!F][KR][KR][KR] (SEQ ID NO: 167).

In some cases, an olfactory receptor protein as described herein is such that the sequence motif is: RN[KR][EDQ][VMI L][KR]KA[LIV][KRH][KR][LI][LI F][KRG][KR][KR] (SEQ ID NO: 820) The sequence motif RN[KR][EDQ][VMIL][KR]KA[LIV][KRH][KR][LI][LIF][KRG][KR][KR] (SEQ ID NO: 820) may alternatively be described as $RNX_1X_2X_3X_4KAX_6X_7X_8JX_{10}X_{11}X_{12}X_{13}$ (SEQ ID NO: 820), wherein:

- $X_1$ is K or R;
- $X_2$ is E or D or Q;
- $X_3$ is V or M or I or L;
- $X_4$ is K or R;
- $X_6$ is L or I or V;
- $X_7$ is K or R or H;
- $X_8$ is K or R;
- $X_{10}$ is L or I or F;
- $X_{11}$ is K or R or G;
- $X_{12}$ is K or R; and
- $X_{13}$ is K or R.

In some cases, an olfactory receptor protein as described herein is such that the sequence motif is: RN[KR]E[VM I][KR]KA[LIV][KR][KR]L[LI F][KR][KR][KR] (SEQ ID NO: 821).

The sequence motif:
RN[KR]E[VM I][KR]KA[LIV][KR][KR]L[LI F][KR][KR][KR] (SEQ ID NO: 821) may alternatively be described as:
$RNX_1EX_{3'}X_4KAX_6X_{7'}X_8LX_{10}X_{11'}X_{12}X_{13}$ (SEQ ID NO: 821), wherein:

- $X_1$ is K or R;
- $X_{3'}$ is V or M or I;
- $X_4$ is K or R;
- $X_5$ is any amino acid;
- $X_6$ is L or I or V;
- $X_{7'}$ is K or R;
- $X_8$ is K or R;
- $X_{10}$ is L or I or F;
- $X_{11'}$ is K or R;
- $X_{12}$ is K or R; and
- $X_{13}$ is K or R.

In some cases, an olfactory receptor protein as described herein is such that the sequence motif is: RNKEVK-$X_{5''''}$ALKRLLKRK (SEQ ID NO: 319), wherein:

- $X_{5''''}$ is D or K or E or N or R or V or A or Q or G or C or F or H or I or L or M or S or T or W or Y.

Several specific and particularly advantageous sequences corresponding with the sequence motifs described herein have been identified. Accordingly, in some cases, an olfactory receptor protein as described herein is such that the sequence motif is selected from the group consisting of:

RNKEVKDALKRLLKRK (SEQ ID NO: 10),
RNREVKDALKRLLKRK (SEQ ID NO: 11),
RNKEIKDALKRLLKRK (SEQ ID NO: 12),
RNKEMKDALKRLLKRK (SEQ ID NO: 13),
RNKEVRDALKRLLKRK (SEQ ID NO: 14),
RNKEVKKALKRLLKRK (SEQ ID NO: 15),
RNKEVKEALKRLLKRK (SEQ ID NO: 16),
RNKEVKNALKRLLKRK (SEQ ID NO: 17),

RNKEVKRALKRLLKRK (SEQ ID NO: 18),
RNKEVKVALKRLLKRK (SEQ ID NO: 19),
RNKEVKAALKRLLKRK (SEQ ID NO: 20),
RNKEVKQALKRLLKRK (SEQ ID NO: 21),
RNKEVKDAVKRLLKRK (SEQ ID NO: 22),
RNKEVKDAIKRLLKRK (SEQ ID NO: 23),
RNKEVKDALRRLLKRK (SEQ ID NO: 24),
RNKEVKDALKKLLKRK (SEQ ID NO: 25),
RNKEVKDALKRLIKRK (SEQ ID NO: 26),
RNKEVKDALKRLFKRK (SEQ ID NO: 27),
RNKEVKDALKRLLRRK (SEQ ID NO: 28),
RNKEVKDALKRLLKKK (SEQ ID NO: 29),
RNKEVKDALKRLLKRR (SEQ ID NO: 30),
RNKDVKDALKRLLKRK (SEQ ID NO: 31),
RNKQVKDALKRLLKRK (SEQ ID NO: 32),
RNKELKDALKRLLKRK (SEQ ID NO: 33),
RNKEVKGALKRLLKRK (SEQ ID NO: 34),
RNKEVKDALHRLLKRK (SEQ ID NO: 35),
RNKEVKDALKRILKRK (SEQ ID NO: 36),
RNKEVKDALKRLLGRK (SEQ ID NO: 37),
RNKEVKRAIKRLLKRK (SEQ ID NO: 38),
RNKEVKKAIKRLLKRK (SEQ ID NO: 39),
RNKEVKRAIKRLFKRK (SEQ ID NO: 40),
RNKEVKKAIKRLFKRK (SEQ ID NO: 41),
RNKEVKRAIRKLLKRK (SEQ ID NO: 42),
RNKEVKDALRKLLKRK (SEQ ID NO: 43),
RNKEVKDALKRLLRRR (SEQ ID NO: 44),
RNREMRKALHRLLGKK (SEQ ID NO: 827),
RNREVKKAIHKLIGRK (SEQ ID NO: 828),
RNREVRKAVHRLFKRK (SEQ ID NO: 829),
RNKEMKKA!HKLFGKK (SEQ ID NO: 830),
RNRDVKKAVHKLFRRK (SEQ ID NO: 831),
RNRDMKKAVHKLFGKR (SEQ ID NO: 832),
RNKELRKALHKLLGRK (SEQ ID NO: 833),
RNRDVRKALRRILRRR (SEQ ID NO: 834),
RNKDVRKAVRKLIRRR (SEQ ID NO: 835),
RNRDVRKAVRRLFRKR (SEQ ID NO: 836),
RNKDIKKAVKKLIKKK (SEQ ID NO: 837),
RNRELRKAVRRLFKRR (SEQ ID NO: 838),
RNKELRKAVRKIIKKK (SEQ ID NO: 839),
RNRDVKKAVRRLFRRK (SEQ ID NO: 840),
RNREVRKALRRIIRKR (SEQ ID NO: 841),
RNKDIRKAVKKIFRRK (SEQ ID NO: 842),
RNKDVRKAVRRLIKRK (SEQ ID NO: 843),
RNRDLRKAVRKLFKKK (SEQ ID NO: 844),
RNRDLRKALRRIFKRR (SEQ ID NO: 845),
RNRDVRKAIKKLIRKR (SEQ ID NO: 846),
RNKELKKAIKRILKKK (SEQ ID NO: 847),
RNRDVRKAIRKLLKRK (SEQ ID NO: 848),
RNRDLRKAVRRIFKKR (SEQ ID NO: 849),
RNRDVRKAVRKLFKRR (SEQ ID NO: 850),
RNRDVRKALRRLFKKR (SEQ ID NO: 851),
RNKELKKALRKLIGKK (SEQ ID NO: 852),
RNREMRKAIKKIIKKK (SEQ ID NO: 853),
RNKEIKKAIKKIIKKR (SEQ ID NO: 854),
RNRDVKKAIRRLFRRR (SEQ ID NO: 855),
RNREVKKAVKKLIGKR (SEQ ID NO: 856),
RNREMRKALRRLFRKR (SEQ ID NO: 857),

RNKELKKALRRLIGRR (SEQ ID NO: 858),
RNRDVKKALRKLIGKR (SEQ ID NO: 859),
RNREVKKAVKKLIRRK (SEQ ID NO: 860),
RNKEVRKALKKLFGKK (SEQ ID NO: 861),
RNKEIRKALRRLFGKK (SEQ ID NO: 862),
RNKDVKKALRRLFGKK (SEQ ID NO: 863),
RNKELKKAIKRLIRRK (SEQ ID NO: 864),
RNKDVRKAVKRLLKKR (SEQ ID NO: 865),
RNKELRKA!RRLLRRR (SEQ ID NO: 866),
RNRDIRKALRKLFKKK (SEQ ID NO: 867),
RNRELKKALRRLLRRR (SEQ ID NO: 868),
RNREVKKALRRLFGKK (SEQ ID NO: 869),
RNRDVRKALKRLLKRK (SEQ ID NO: 870),
RNRDMRKAIRKLFGRK (SEQ ID NO: 871),
RNRELKKAIRKLLKRK (SEQ ID NO: 872),
RNRDIRKAVKKLFGKK (SEQ ID NO: 873),
RNKEVKKAIRKLFGRR (SEQ ID NO: 874),
RNREVRKAVRKLFRRK (SEQ ID NO: 875),
RNRDMKKALKKLFRRR (SEQ ID NO: 876),
RNRDVRKALKRLLGRR (SEQ ID NO: 877),
RNKDLKKAVKKLFGRK (SEQ ID NO: 878),
RNKDVRKAVRRLFGRR (SEQ ID NO: 879),
RNKEVKCALKRLLKRK (SEQ ID NO: 880),
RNKEVKFALKRLLKRK (SEQ ID NO: 881),
RNKEVKHALKRLLKRK (SEQ ID NO: 882),
RNKEVKIALKRLLKRK (SEQ ID NO: 883),
RNKEVKLALKRLLKRK (SEQ ID NO: 884),
RNKEVKMALKRLLKRK (SEQ ID NO: 885),
RNKEVKSALKRLLKRK (SEQ ID NO: 886),
RNKEVKTALKRLLKRK (SEQ ID NO: 887),
RNKEVKWALKRLLKRK (SEQ ID NO: 888), and
RNKEVKYALKRLLKRK (SEQ ID NO: 889).

Olfactory receptors having a modified C-terminal domain comprising any of these specific sequences listed above have been shown to display advantageous and surprising technical effects, as described in detail in the experimental section of this disclosure. Based on these insights, the skilled person can design further advantageous sequences correspondingly fitting within the sequence motifs described herein. Some illustrative and non-limiting examples of such sequences are the following:

RNKEVKRALKRLLRRR (SEQ ID NO: 45)
RNKEVKKALKRLLRRR (SEQ ID NO: 46)
RNREVKRAIKRLLKRK (SEQ ID NO: 47)
RNREVKKAIKRLLKRK (SEQ ID NO: 48)
RNREVKRAIKRLFKRK (SEQ ID NO: 49)
RNREVKKAIKRLFKRK (SEQ ID NO: 50)
RNREVKRAIRKLLKRK (SEQ ID NO: 51)
RNREVKDALRKLLKRK (SEQ ID NO: 52)
RNREVKDALKRLLRRR (SEQ ID NO: 53)
RNKEVKKAIKRLLRRK (SEQ ID NO: 54)
RNKEVKKAIKRLLKKK (SEQ ID NO: 55)
RNKEVKKAIKRLLKRR (SEQ ID NO: 56)
RNKEVKRAIKRLLRRK (SEQ ID NO: 57)
RNKEVKRAIKRLLKKK (SEQ ID NO: 58)
RNKEVKRAIKRLLKRR (SEQ ID NO: 59)
RNKEVKKAIKRLFRRK (SEQ ID NO: 60)
RNKEVKKAIKRLFKKK (SEQ ID NO: 61)
RNKEVKKAIKRLFKRR (SEQ ID NO: 62)
RNKEVKRAIKRLFRRK (SEQ ID NO: 63)

RNKEVKRAIKRLFKKK (SEQ ID NO: 64)
RNKEVKRAIKRLFKRR (SEQ ID NO: 65)
RNREVKRAIKRLLRKK (SEQ ID NO: 66)
RNREVKKAIKRLLRKK (SEQ ID NO: 67)
RNREVKRA!KRLFRRR (SEQ ID NO: 68)
RNREVKKAIKRLFRRR (SEQ ID NO: 69)
RNREVKKAIKRLFRRK (SEQ ID NO: 70)
RNREVKKAIKRLFKKK (SEQ ID NO: 71)
RNREVKKAIKRLFKRR (SEQ ID NO: 72)
RNREVKRAIKRLFRRK (SEQ ID NO: 73)
RNREVKRAIKRLFKKK (SEQ ID NO: 74)
RNREVKRAIKRLFKRR (SEQ ID NO: 75)

In some cases, an olfactory receptor protein as described herein is such that the sequence motif is RNRDVRKALRRLFRKK (SEQ ID NO: 307) or RNRDVRRALRRLFRKK (SEQ ID NO: 308). Such C-terminal motifs may be particularly advantageous as they comprise statistically optimal amino acids at each individual position.

In some cases, an olfactory receptor protein as described herein is such that the sequence motif is RNKQIRDALKRLLKRK (SEQ ID NO: 890). Such a C-terminal motif may be particularly advantageous in the case of class I olfactory receptors.

The 16 amino acid long sequence motifs described herein may optionally comprise additional C-terminal residues. Indeed, the inventors have found that, while not being essential, such additional C-terminal residues may lead to further advantageous effects, as described in detail in the Experimental section. The number of additional C-terminal residues, if present, is not particularly limited, but it is preferably less than 10 More preferably, the number of additional C-terminal residues, if present, is from 1 to 6. Thus, depending on the number of additional C-terminal residues that are added to the 16 amino acid long sequence motifs described herein, the total length of the sequence motif described herein may be 17, 18, 19, 20, 21, 22, 23, 24, 25, or 26 amino acids, preferably 17, 18, 19, 20, 21, or 22 amino acids, when optional additional C-terminal residues are present.

Accordingly, in some cases, an olfactory receptor protein as described herein is such that the amino acid sequence motif comprises 1 to 10, preferably 1 to 6, additional C-terminal amino acid residues. In some cases, the additional C-terminal amino acid residues are as follows:

- the first additional amino acid residue is selected from C, R, K, E, G, H, F, P, Y, W, M, and N, preferably the first additional amino acid residue is selected from C, R, K, E, G, H, F, P, and Y, more preferably the first additional amino acid residue is C, R, or K, most preferably the first additional amino acid residue is C;
- the second additional amino acid residue is selected from C, R, K, N, G, I, L, F, P, T, Y, and Q, preferably the second additional amino acid residue is selected from C, R, K, N, G, I, L, F, P, T, and Y, more preferably the second additional amino acid residue is C, R, or K, most preferably the second additional amino acid residue is C or R;
- the third additional amino acid residue is selected from R, K, C, L, F, M, Y, A, P, S, G, H, and N, preferably the third additional amino acid residue is selected from R, K, C, L, F, M, Y, A, P, S, and G, more preferably the third additional amino acid residue is R or K;
- the fourth to tenth, or the fourth, fifth and sixth, preferably the fourth, fifth, and sixth, additional amino acid residues are selected from K and R.

In some cases, the amino acid sequence motif comprises 1 additional C-terminal residue. The additional amino acid residue preferably corresponds to the first additional amino acid residue as defined above, more preferably is selected from C, R, P, L, K, G, Y, F, M, or W.

In some cases, the amino acid sequence motif comprises 2 additional C-terminal residues. The first and second additional amino acid residue are preferably as defined above. Specific advantageous examples of combinations of a first and second additional amino acid residue include CC, SI, YP, PQ, FR, CR, RR, EK, PR, CG, FK, RG, RC, RT, RF, GG, YR, GC, TG, PC, HP, PG, KY, CP, YY, FF, CF, NP, YL, IC, HC, CL, YC, ER, RP, PA, FC, and RY. Particularly preferred in this context is a CC sequence.

Therefore, in some cases, an olfactory receptor protein as described herein is such that the sequence motif is selected from the group consisting of:

$RNX_1X_2X_3X_4KAX_6X_7X_8JX_{10}X_{11}X_{12}X_{13}CC$ (SEQ ID NO: 822),
$RNX_1EX_{3'}X_4KAX_6X_{7'}X_8LX_{10}X_{11'}X_{12}X_{13}CC$ (SEQ ID NO: 823), and;
$RNKEVKX_{5''''}ALKRLLKRKCC$ (SEQ ID NO: 320), wherein:

- $X_1$ is K or R;
- $X_2$ is E or D or Q;
- $X_3$ is V or M or I or L;
- $X_{3'}$ is V or M or I;
- $X_4$ is K or R;
- $X_{5''''}$ is D or K or E or N or R or V or A or Q or G or C or F or H or I or L or M or S or T or W or Y;
- $X_6$ is L or I or V;
- $X_7$ is K or R or H;
- $X_{7'}$ is K or R;
- $X_8$ is K or R;
- $X_{10}$ is L or I or F;
- $X_{11}$ is K or R or G;
- $X_{11'}$ is K or R;
- $X_{12}$ is K or R; and
- $X_{13}$ is K or R.

In some cases, an olfactory receptor protein as described herein is such that the sequence motif is selected from the group consisting of:

RNKEVKDALKRLLKRKCC (SEQ ID NO: 86),
RNREVKDALKRLLKRKCC (SEQ ID NO: 87),
RNKEIKDALKRLLKRKCC (SEQ ID NO: 88),
RNKEMKDALKRLLKRKCC (SEQ ID NO: 89),
RNKEVRDALKRLLKRKCC (SEQ ID NO: 90),
RNKEVKKALKRLLKRKCC (SEQ ID NO: 91),
RNKEVKEALKRLLKRKCC (SEQ ID NO: 92),
RNKEVKNALKRLLKRKCC (SEQ ID NO: 93),
RNKEVKRALKRLLKRKCC (SEQ ID NO: 94),
RNKEVKVALKRLLKRKCC (SEQ ID NO: 95),
RNKEVKAALKRLLKRKCC (SEQ ID NO: 96),
RNKEVKQALKRLLKRKCC (SEQ ID NO: 97),
RNKEVKDAVKRLLKRKCC (SEQ ID NO: 98),
RNKEVKDAIKRLLKRKCC (SEQ ID NO: 99),
RNKEVKDALRRLLKRKCC (SEQ ID NO: 100),
RNKEVKDALKKLLKRKCC (SEQ ID NO: 101),
RNKEVKDALKRLIKRKCC (SEQ ID NO: 102),
RNKEVKDALKRLFKRKCC (SEQ ID NO: 103),
RNKEVKDALKRLLRRKCC (SEQ ID NO: 104),
RNKEVKDALKRLLKKKCC (SEQ ID NO: 105),
RNKEVKDALKRLLKRRCC (SEQ ID NO: 106),
RNKDVKDALKRLLKRKCC (SEQ ID NO: 107),
RNKQVKDALKRLLKRKCC (SEQ ID NO: 108),
RNKELKDALKRLLKRKCC (SEQ ID NO: 109),
RNKEVKGALKRLLKRKCC (SEQ ID NO: 110),
RNKEVKDALHRLLKRKCC (SEQ ID NO: 111),
RNKEVKDALKRILKRKCC (SEQ ID NO: 112),
RNKEVKDALKRLLGRKCC (SEQ ID NO: 113),
RNKEVKRAIKRLLKRKCC (SEQ ID NO: 114),
RNKEVKKAIKRLLKRKCC (SEQ ID NO: 115),
RNKEVKRAIKRLFKRKCC (SEQ ID NO: 116),
RNKEVKKAIKRLFKRKCC (SEQ ID NO: 117),
RNKEVKRAIRKLLKRKCC (SEQ ID NO: 118),
RNKEVKDALRKLLKRKCC (SEQ ID NO: 119),
RNKEVKDALKRLLRRRCC (SEQ ID NO: 120),
RNREMRKALHRLLGKKCC (SEQ ID NO: 254),
RNREVKKAIHKLIGRKCC (SEQ ID NO: 255),
RNREVRKAVHRLFKRKCC (SEQ ID NO: 256),
RNKEMKKAIHKLFGKKCC (SEQ ID NO: 257),

RNRDVKKAVHKLFRRKCC (SEQ ID NO: 258),
RNRDMKKAVHKLFGKRCC (SEQ ID NO: 259),
RNKELRKALHKLLGRKCC (SEQ ID NO: 260),
RNRDVRKALRRILRRRCC (SEQ ID NO: 261),
RNKDVRKAVRKLIRRRCC (SEQ ID NO: 262),
RNRDVRKAVRRLFRKRCC (SEQ ID NO: 263),
RNKDIKKAVKKLIKKKCC (SEQ ID NO: 264),
RNRELRKAVRRLFKRRCC (SEQ ID NO: 265),
RNKELRKAVRKIIKKKCC (SEQ ID NO: 266),
RNRDVKKAVRRLFRRKCC (SEQ ID NO: 267),
RNREVRKALRRIIRKRCC (SEQ ID NO: 268),
RNKDIRKAVKKIFRRKCC (SEQ ID NO: 269),
RNKDVRKAVRRLIKRKCC (SEQ ID NO: 270),
RNRDLRKAVRKLFKKKCC (SEQ ID NO: 271),
RNRDLRKALRRIFKRRCC (SEQ ID NO: 272),
RNRDVRKAIKKLIRKRCC (SEQ ID NO: 273),
RNKELKKAIKRILKKKCC (SEQ ID NO: 274),
RNRDVRKAIRKLLKRKCC (SEQ ID NO: 275),
RNRDLRKAVRRIFKKRCC (SEQ ID NO: 276),
RNRDVRKAVRKLFKRRCC (SEQ ID NO: 277),
RNRDVRKALRRLFKKRCC (SEQ ID NO: 278),
RNKELKKALRKLIGKKCC (SEQ ID NO: 279),
RNREMRKAIKKIIKKKCC (SEQ ID NO: 280),
RNKEIKKAIKKIIKKRCC (SEQ ID NO: 281),
RNRDVKKAIRRLFRRRCC (SEQ ID NO: 282),
RNREVKKAVKKLIGKRCC (SEQ ID NO: 283),
RNREMRKALRRLFRKRCC (SEQ ID NO: 284),
RNKELKKALRRLIGRRCC (SEQ ID NO: 285),
RNRDVKKALRKLIGKRCC (SEQ ID NO: 286),
RNREVKKAVKKLIRRKCC (SEQ ID NO: 287),
RNKEVRKALKKLFGKKCC (SEQ ID NO: 288),
RNKEIRKALRRLFGKKCC (SEQ ID NO: 289),
RNKDVKKALRRLFGKKCC (SEQ ID NO: 290),
RNKELKKAIKRLIRRKCC (SEQ ID NO: 291),
RNKDVRKAVKRLLKKRCC (SEQ ID NO: 292),
RNKELRKAIRRLLRRRCC (SEQ ID NO: 293),
RNRDIRKALRKLFKKKCC (SEQ ID NO: 294),
RNRELKKALRRLLRRRCC (SEQ ID NO: 295),
RNREVKKALRRLFGKKCC (SEQ ID NO: 296),
RNRDVRKALKRLLKRKCC (SEQ ID NO: 297),
RNRDMRKAIRKLFGRKCC (SEQ ID NO: 298),
RNRELKKAIRKLLKRKCC (SEQ ID NO: 299),
RNRDIRKAVKKLFGKKCC (SEQ ID NO: 300),
RNKEVKKAIRKLFGRRCC (SEQ ID NO: 301),
RNREVRKAVRKLFRRKCC (SEQ ID NO: 302),
RNRDMKKALKKLFRRRCC (SEQ ID NO: 303),
RNRDVRKALKRLLGRRCC (SEQ ID NO: 304),
RNKDLKKAVKKLFGRKCC (SEQ ID NO: 305),
RNKDVRKAVRRLFGRRCC (SEQ ID NO: 306),
RNRDVRKALRRLFRKKCC (SEQ ID NO: 309),
RNRDVRRALRRLFRKKCC (SEQ ID NO: 310),
RNKEVKCALKRLLKRKCC (SEQ ID NO: 321),
RNKEVKFALKRLLKRKCC (SEQ ID NO: 322),
RNKEVKHALKRLLKRKCC (SEQ ID NO: 323),
RNKEVKIALKRLLKRKCC (SEQ ID NO: 324),
RNKEVKLALKRLLKRKCC (SEQ ID NO: 325),
RNKEVKMALKRLLKRKCC (SEQ ID NO: 326),
RNKEVKSALKRLLKRKCC (SEQ ID NO: 328),

RNKEVKTALKRLLKRKCC (SEQ ID NO: 329),
RNKEVKWALKRLLKRKCC (SEQ ID NO: 330),
RNKEVKYALKRLLKRKCC (SEQ ID NO: 331), and
RNKQIRDALKRLLKRKCC (SEQ ID NO: 740).

C-terminal motifs having the sequence RNRDVRKALRRLFRKKCC (SEQ ID NO: 309) or RNRDVRRALRRLFRKKCC (SEQ ID NO: 310), may be particularly advantageous as they comprise statistically optimal amino acids at each individual position. A C-terminal motif having the sequence_RNKQIRDALKRLLKRKCC (SEQ ID NO: 740) may be particularly advantageous in the case of class I olfactory receptors.

In addition to a CC sequence, other preferred combinations of a first and second additional amino acid residue are CR, RR, YP, RF and FK. Accordingly, in some cases, an olfactory receptor protein as described herein is such that the sequence motif is selected from the group consisting of:

RNKEVKRAIKRLLKRKCR (SEQ ID NO: 121),
RNKEVKKAIKRLLKRKCR (SEQ ID NO: 122),
RNKEVKRALKRLLKRKRR (SEQ ID NO: 123),
RNKEVKRALKRLLKRKYP (SEQ ID NO: 124),
RNKEVKRALKRLLKRKRF (SEQ ID NO: 125),
RNKEVKRALKRLLKRKFK (SEQ ID NO: 126),
RNKEVKKALKRLLKRKRR (SEQ ID NO: 127),
RNKEVKKALKRLLKRKYP (SEQ ID NO: 128),
RNKEVKKALKRLLKRKRF (SEQ ID NO: 129), and
RNKEVKKALKRLLKRKFK (SEQ ID NO: 130).

In some cases, the amino acid sequence motif comprises 3 additional C-terminal residues. The first and second and third additional amino acid residue are preferably as defined above. In some cases, the first and second additional amino acid residues are CC and the third additional amino acid residue is as defined above. Specific advantageous examples of combinations of a first and second and third additional amino acid residue include CRR, CCC, CCF, CCL, CCM, CCS, CCP, CCA, CCY, CCH, CCN, CCD, CCK, CCR and CCG.

Accordingly, in some cases, an olfactory receptor protein as described herein is such that the sequence motif is selected from the group consisting of:

RNKEVKDALKRLLKRKCRR (SEQ ID NO: 133),
RNKEVKDALKRLLKRKCCC (SEQ ID NO: 134),
RNKEVKDALKRLLKRKCCF (SEQ ID NO: 135),
RNKEVKDALKRLLKRKCCL (SEQ ID NO: 136),
RNKEVKDALKRLLKRKCCM (SEQ ID NO: 137),
RNKEVKDALKRLLKRKCCS (SEQ ID NO: 138),
RNKEVKDALKRLLKRKCCP (SEQ ID NO: 139),
RNKEVKDALKRLLKRKCCA (SEQ ID NO: 140),
RNKEVKDALKRLLKRKCCY (SEQ ID NO: 141),
RNKEVKDALKRLLKRKCCH (SEQ ID NO: 142),
RNKEVKDALKRLLKRKCCN (SEQ ID NO: 143),
RNKEVKDALKRLLKRKCCD (SEQ ID NO: 144),
RNKEVKDALKRLLKRKCCK (SEQ ID NO: 145),
RNKEVKDALKRLLKRKCCR (SEQ ID NO: 146), and
RNKEVKDALKRLLKRKCCG (SEQ ID NO: 147).

In some cases, the amino acid sequence motif comprises 4 additional C-terminal residues. The first and second and third and fourth additional amino acid residue are preferably as defined above. Specific advantageous examples of combinations of a first and second and third and fourth additional amino acid residue include CRRR (SEQ ID NO: 159), CRKK (SEQ ID NO: 160), CCRR (SEQ ID NO: 161), CCRK (SEQ ID NO: 228), and CCKR (SEQ ID NO: 229), among which CRRR (SEQ ID NO: 159), CRKK (SEQ ID NO: 160), and CCRR (SEQ ID NO: 161) are preferred. Accordingly, in some cases, an olfactory receptor protein as described herein is such that the sequence motif is selected from the group consisting of:

RNKEVKDALKRLLKRKCRRR (SEQ ID NO: 149),
RNKEVKDALKRLLKRKCRKK (SEQ ID NO: 150), and

RNKEVKDALKRLLKRKCCRR (SEQ ID NO: 151).

In some cases, the amino acid sequence motif comprises 5 additional C-terminal residues. The first and second and third and fourth and fifth additional amino acid residue are preferably as defined above. Specific advantageous examples of a combination of first and second and third and fourth and fifth additional amino acid residue include CRRRR (SEQ ID NO: 162), CCRRR (SEQ ID NO: 163). CCKRR (SEQ ID NO: 230), CCRKR (SEQ ID NO: 231), CCRRK (SEQ ID NO: 232), CCRKK (SEQ ID NO: 233), CCKRK (SEQ ID NO: 234), CCKKR (SEQ ID NO: 235), and CCKKK (SEQ ID NO: 236), among which CRRRR (SEQ ID NO: 162) and CCRRR (SEQ ID NO: 163) are preferred.

In some cases, an olfactory receptor protein as described herein is such that the sequence motif is selected from the group consisting of:

$RNX_1X_2X_3X_4X_5AX_6X_7X_8JX_{10}X_{11}X_{12}X_{13}CCRRR$ (SEQ ID NO: 825), and
$RNX_1QIRX_5AX_6X_7X_8JX_{10}X_{11}X_{12}X_{13}CCRRR$ (SEQ ID NO: 826), wherein:

- $X_1$ is K or R;
- $X_2$ is E or D or Q;
- $X_3$ is V or M or I or L;
- $X_4$ is K or R;
- $X_5$ is any amino acid;
- $X_6$ is L or I or V;
- $X_7$ is K or R or H;
- $X_8$ is K or R;
- $X_{10}$ is L or I or F;
- $X_{11}$ is K or R or G;
- $X_{12}$ is K or R; and
- $X_{13}$ is K or R.

Preferably, $X_5$ is any amino acid except proline (Pro, P).

In some cases, an olfactory receptor protein as described herein is such that the sequence motif is selected from the group consisting of:

RNKEVKDALKRLLKRKCRRRR (SEQ ID NO: 154),
RNKEVKDALKRLLKRKCCRRR (SEQ ID NO: 156),
RNKEVKKAIKRLLKRKCCRRR (SEQ ID NO: 220),
RNKEVKRAIKRLLKRKCCRRR (SEQ ID NO: 237),
RNKEVKKAIKRLFKRKCCRRR (SEQ ID NO: 221),
RNKEVKRAIKRLFKRKCCRRR (SEQ ID NO: 238), and
RNKQIRDALKRLLKRKCCRRR (SEQ ID NO: 741),
preferably the sequence motif is RNKEVKKAIKRLFKRKCCRRR (SEQ ID NO: 221).
A C-terminal motif having the sequence RNKQIRDALKRLLKRKCCRRR (SEQ ID NO: 741) may be particularly advantageous in the case of class I olfactory receptors.

In some cases, the amino acid sequence motif comprises 6 additional C-terminal residues. The first and second and third and fourth and fifth and sixth additional amino acid residue are preferably as defined above. Specific advantageous examples of combinations of a first and second and third and fourth and fifth and sixth additional amino acid residue include CRRRRR (SEQ ID NO: 164), CRRRKK (SEQ ID NO: 165), and CCRRRR (SEQ ID NO: 224).

Accordingly, in some cases, an olfactory receptor protein as described herein is such that the sequence motif is selected from the group consisting of:

RNKEVKDALKRLLKRKCRRRRR (SEQ ID NO: 157),
RNKEVKDALKRLLKRKCRRRKK (SEQ ID NO: 158), and
RNKEVKDALKRLLKRKCCRRRR (SEQ ID NO: 219).

Olfactory receptors having a modified C-terminal domain comprising any of these specific sequences of listed above have been shown to display advantageous and surprising technical effects, as described in detail in the experimental section of this disclosure.

In some cases, an olfactory receptor protein as described herein may be such that the amino acid sequence motif comprises additional C-terminal amino acid residues selected from the group consisting of CC, CCR, CCRR (SEQ ID NO:

161), CCRRR (SEQ ID NO: 163), CCRRRR (SEQ ID NO: 224), CR, CRR, CRRR (SEQ ID NO: 159), CRKK (SEQ ID NO: 160), CRRRR (SEQ ID NO: 162), CRRRRR (SEQ ID NO: 164), CRRRKK (SEQ ID NO: 165).

In some cases, an olfactory receptor as described herein further comprises an N-terminal signal peptide. Typically, the N-terminal signal peptide is a cleavable peptide. This means that it is cleaved from the mature protein and cannot alter OR-ligand binding and signalling. Thus, it is understood by the skilled person that the N-terminal signal peptides described herein are usually not a part of the mature olfactory receptor. In some cases, the N-terminal signal peptide is a leucine-rich signal peptide, preferably MRPQILLLLALLTLGLA (SEQ ID NO: 76) or MSHQILLLLALLTLGLA (SEQ ID NO: 77). MRPQILLLLALLTLGLA (SEQ ID NO: 76) and MSHQILLLLALLTLGLA (SEQ ID NO: 77) are known as a so-called Lucy-tag. MRPQILLLLALLTLGLA (SEQ ID NO: 76) is a human Lucy tag while MSHQILLLLALLTLGLA (SEQ ID NO: 77) is a mouse Lucy tag. Also encompassed are the sequences of SEQ ID NO: 76 and 77 wherein 1, 2, 3, 4, or up to 5 amino acids are substituted, deleted, added, or inserted. Substitutions, and in particular conservative substitutions, are preferred.

In some cases, an olfactory receptor as described herein further comprises an N-terminal tag peptide. Typically, the N-terminal tag peptide is a non-cleavable peptide.

N-terminal tag peptides may be epitope tags used to purify or capture the proteins. An example of such an epitope tag is a FLAG tag, further described below.

N-terminal tag peptides may also be peptides that facilitate expression. Examples of such tag peptides facilitating expression are a rhodopsin (rho) tag, an $SST_3$ tag and an $M_3$-Tag, further described below.

In some cases, the N-terminal tag peptide is selected from the group consisting of a FLAG tag, a rhodopsin (rho) tag, an IL-6 tag, an $SST_3$ tag (45 -N-terminal amino acids of the Somatostatin 3 receptor; an example of which is SEQ ID NO: 223), an $M_3$-Tag (61-N-terminal amino acids of the muscarinic acetylcholine receptor Ms; an example of which is SEQ ID NO: 222), a c-myc tag, and a HA tag, preferably from the group consisting of a FLAG tag, a rhodopsin (rho) tag, an $SST_3$ tag, and an $M_3$ tag, even more preferably from the group consisting of a FLAG tag and a rhodopsin (rho) tag, most preferably a rhodopsin (rho) tag. Accordingly, in some cases, an olfactory receptor protein as described herein further comprises an N-terminal tag peptide, preferably wherein the N-terminal tag peptide is selected from the group consisting of a FLAG tag, a rhodopsin (rho) tag, an IL-6 tag, an $SST_3$ tag, an M3-Tag, a c-myc tag, and a HA tag, more preferably from the group consisting of a FLAG tag, a rhodopsin (rho) tag, an $SST_3$ tag, and an $M_3$ tag, even more preferably from the group consisting of a FLAG tag and a rhodopsin (rho) tag, most preferably is a rhodopsin (rho) tag.

In preferred cases, the N-terminal tag peptide comprises at least a tag peptide selected from the group consisting of a rhodopsin (rho) tag, an SSTstag, and an Mstag, preferably a rho tag. Optionally, a FLAG peptide may be further present.

Combinations of the above-described tag peptides may also be used. Typically, such combination will comprise at least one of a rho tag, an $SST_3$ tag, and an M3-Tag, preferably at least a rho tag. For example, in some cases, the N-terminal tag peptide is a combination of a Rho tag and a FLAG tag. In other words, in some cases, an olfactory receptor protein as described herein further comprises an N-terminal tag peptide, wherein the N-terminal tag peptide comprises a Rho tag and a FLAG tag. Preferably, in that situation, the FLAG tag is positioned N-terminally from the Rho tag, for example as shown in SEQ ID NO: 81.

FLAG-tags and rho-tags are described in Shepard et al. (2013) PloS One 8(7): e68758, in Zhuang and Matsunami (2007) J Biol Chem 282(20): 15284-15293, and in WO2014/037800, each of which is incorporated herein by reference. IL-6 tags are described in Noe et al. A bi-functional IL-6-HaloTag® as a tool to measure the cell-surface expression of recombinant odorant receptors and to facilitate their activity quantification. J Biol Methods. 2017, 4(4):e82, incorporated herein by reference. $SST_3$ tags and $M_3$ tags are described in Tan et al. (2022) Scientific reports 12:17658, incorporated herein by reference.

In some cases, a FLAG tag as described herein has the sequence of SEQ ID NO: 78. In some cases, a rho tag as described herein has the sequence of SEQ ID NO: 79. In some cases, an IL-6 tag as described herein is an IL-6-HaloTag® as described in Noe et al. (supra). A bi-functional IL-6-HaloTag® as a tool to measure the cell-surface expression of recombinant odorant receptors and to facilitate their activity quantification. J Biol Methods. 2017, 4(4):e82, incorporated herein by reference. In some cases, an $SST_3$ tag as described herein has the sequence of SEQ ID NO: 223. In some cases, an $M_3$ tag as described herein has the sequence of SEQ ID NO: 222. Also encompassed are the sequences of SEQ ID NO: 78, 79, 222, and 223, wherein 1, 2, 3, 4, or up to 5 amino acids are substituted, deleted, added, or inserted. Substitutions, and in particular conservative substitutions, are preferred.

N-terminal signal peptides as described herein and N-terminal tag peptides as described herein can, advantageously, be used in combination with each other. Typically, the N-terminal signal peptide will be positioned upstream (N-terminally) from the N-terminal tag. For example, an olfactory receptor as described herein may further comprise an N-terminal signal peptide and one or more N-terminal tag peptides. In some cases, an olfactory receptor as described herein may further comprise:

- a human or mouse Lucy signal peptide (such as, SEQ ID NO: 76 or 77);

- a FLAG tag (such as, SEQ ID NO: 78) or an IL-6 tag, preferably a FLAG tag (such as, SEQ ID NO: 78); and
- a rho tag (such as, SEQ ID NO: 79), an $SST_3$ tag, or an M3-Tag, preferably a rho tag (such as, SEQ ID NO: 79).

SEQ ID NO: 80 is an example of a nucleotide sequence encoding a combination of a mouse Lucy signal peptide, a FLAG tag peptide and a rho tag peptide (SEQ ID NO: 81).

In some cases, an olfactory receptor as described herein is modified to comprise one or more additional N-terminal glycosylation sites. Such glycosylation sites are for example present in the $M_3$ and $SST_3$ tags (Tan et al., Scientific Reports (2022) 12:17658) and in the N-terminal rho-tag (Kaushal et al., 1998, Proc Natl Acad Sci U S A 91(9):4024-4028), described elsewhere herein.

In some cases, an olfactory receptor protein as described herein is such that the sequence motif is selected from the group consisting of SEQ ID NOs: 1, 5-75, 86-130, 133-147, 149-151, 154, 156-158, 166, 167, 198, 219-221, 254-312, 319-326, 328-331, 740-741, 820-890 . Also encompassed in this context are the sequences of SEQ ID NOs: 1, 5-75, 86-130, 133-147, 149-151, 154, 156-158, 166, 167, 198, 219-221, 254-312, 319-326, 328-331, 740-741, and 820-890, wherein 1, 2, 3, 4, 5, 6, 7, 8, or up to 9 amino acids are substituted, deleted, added, or inserted. Preferably the sequence including substitutions, deletions, additions and/or insertions still corresponds to the general sequence motif of SEQ ID NO: 1. Substitutions, and in particular conservative substitutions, are preferred. Examples of particularly suitable amino acid substitutions in this context include the substitution of K for R, and R for K.

In some cases, an olfactory receptor protein as described herein is such that the sequence motif is selected from the group consisting of SEQ ID NOs: 1, 10-75, 86-130, 133-147, 149-151, 154, 156-158, 198, 219-221, 254-310, 321-326, 328-331, 740-741, and 827-890. Also encompassed in this context are the sequences of SEQ ID NOs: 1, 10-75, 86-130, 133-147, 149-151, 154, 156-158, 198, 219-221, 254-310, 321-326, 328-331, 740-741, and 827-890 wherein 1, 2, 3, 4, 5, 6, 7, 8, or up to 9 amino acids are substituted, deleted, added, or inserted. Substitutions, and in particular conservative substitutions, are preferred. Examples of particularly suitable amino acid substitutions in this context include the substitution of K for R, and R for K.

It is understood that, in the context of any of the olfactory receptors described throughout this disclosure, the term "comprising" may be replaced with the term "consisting essentially of" or "consisting". In other words, in some cases, the olfactory receptors described herein have a modified C-terminal domain that consists essentially of the amino acid sequence motifs disclosed herein, or that consists of the amino acid sequence motifs disclosed herein.

Nucleic acid molecules

In another aspect, this disclosure relates to nucleic acid molecules comprising a nucleotide sequence encoding any of the olfactory receptor proteins as described herein. A nucleotide sequence encoding an olfactory receptor may also be denoted as a "gene" encoding an olfactory receptor or a "coding sequence" for an olfactory receptor. Nucleotide sequences encoding olfactory receptors are part of the common general knowledge and can be obtained from well-known general and specific sequence databases by the person skilled in the art, as described elsewhere herein.

The nucleic acid molecules of this disclosure do not encode wild type olfactory receptors, instead, they encode the olfactory receptors having a modified C-terminal domain as described in detail in the preceding section. The nucleic acid molecules of this disclosure are therefore non-naturally occurring. It follows that the nucleic acid molecules described herein can, similarly, be characterized as "modified" nucleic acid molecules, "engineered" nucleic acid molecules, "hybrid" nucleic acid molecules, "chimeric" nucleic acid molecules, "non-natural" nucleic acid molecules, or similar expressions and combinations thereof.

Exemplary nucleic acid molecules of this disclosure are provided as SEQ ID NOs: 331-739 and SEQ ID NOs: 742-819. Accordingly, in some cases, an olfactory receptor described herein is encoded by a nucleic acid molecule comprising a nucleotide sequence comprising at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% sequence identity with a sequence selected from the group consisting of SEQ ID NOs: 331-739 and SEQ ID NOs: 742-819.

SEQ ID NOs 331-739 represent DNA encoding a modified receptor including an N-terminal mmLucy-FLAG-rho tag (SEQ ID NO: 81) and a modified C-terminus (SEQ ID NO: 221). SEQ ID NOs 742-819 represent DNA encoding a modified receptor including an N-terminal mmLucy-FLAG-rho tag (SEQ ID NO: 81) and a modified C-terminus (SEQ ID NO: 741). As explained in this disclosure, the presence of the N-terminal tag is optional, and different N-terminal tags could be used, as described elsewhere herein. Similarly, any modified C-terminal domain disclosed herein may be used instead of the modified C-terminus of SEQ ID NO: 221 or SEQ ID NO: 741. SEQ ID NOs 331-739 and SEQ ID NOs 742-819 also include a 5' BamHI restriction site (GGATCC) and Kozak sequence (GCCACC), and a 3' NotI restriction site (GCGGCCGC) for cloning and expression purposes. The presence of these sequences is entirely optional.

The nucleic acid molecules of this disclosure may comprise further sequence elements. Typically, further sequence elements may be sequence elements that are commonly used to aid in expressing a nucleotide sequence such as, promoters, nuclear localization signals, kozak sequences, polyA-tails, transcription terminators, and the like. When one or more of such further sequence elements are present, the nucleic acid molecules described herein may also be referred to as "nucleic acid constructs" or "gene constructs". It is understood that the different sequence elements may be "operably linked" with each other to achieve functional nucleic acid molecules. A description of "operably linked" is provided elsewhere herein in the section entitled "general information".

As used herein, a "nucleic acid construct" refers to a DNA molecule comprising a region (coding region or ORF), which is transcribed into an RNA molecule (e.g. an mRNA molecule) in a cell, operably linked to a suitable regulatory region such as, but not limited to, a promoter and/or enhancer sequence. A nucleic acid construct will generally comprise multiple operably linked fragments, such as, a promoter, an enhancer, a 5' leader sequence, a coding region, and/or a 3' untranslated region (3'-end) e.g. comprising a polyadenylation and/or transcription termination site. A nucleic acid construct may be recombinant, i.e. not normally found in nature, such as, a nucleic acid construct wherein the promoter is not associated in nature with part or all of the coding region. Molecular toolbox techniques for preparation of nucleic acid constructs are well-known in the art and are discussed in standard handbooks such as Ausubel et al., Current Protocols in Molecular Biology, 3rd edition (2003), John Wiley & Sons Inc and Sambrook and Green, Molecular Cloning: A Laboratory Manual, 4th Edition (2012), Cold Spring Harbor Laboratory Press; both of which are incorporated herein by reference in their entireties. Non-limiting examples of such techniques, some of which are demonstrated in the experimental section herein, are fusion PCR, restriction digestion, Golden-gate cloning, and the like.

In some cases, a nucleic acid molecule as described herein further comprises a promoter sequence. Put differently, this disclosure encompasses nucleic acid molecules as described herein, wherein said nucleotide sequence is operably linked to a promoter sequence. In some cases, a promoter sequence as described herein is a constitutive promoter sequence.

As used herein, the term "promoter" or "transcription regulatory sequence" refers to a nucleic acid sequence that functions to control the transcription of one or more coding sequences (i.e. expression), is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter.

In some cases, a promoter sequence as described herein, which is a constitutive promoter sequence as described herein, is a CMV promoter. In some cases, a CMV promoter may have the nucleotide sequence of SEQ ID NO: 82, or a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO: 82.

In some cases, a nucleic acid molecule as described herein further comprises an enhancer sequence.

As used herein, the term "enhancer" refers to a nucleic acid sequence that can stimulate the transcription of a sequence it is operably linked to. An operably linked enhancer does not necessarily need to be contiguous with a coding sequence whose transcription it controls. An enhancer may be used as single sequence or may be comprised in a fusion nucleotide sequence with other enhancers and/or a promoter as described herein.

In some cases, a nucleic acid molecule as described herein further comprises a terminator sequence. Put differently, this disclosure encompasses nucleic acid molecules as described herein, wherein said nucleotide sequence is operably linked to a terminator sequence. A "terminator sequence" may alternatively be denoted herein as a "transcription terminator", a "transcription terminator sequence" or simply a "terminator". In some cases, a terminator sequence is a bovine growth hormone (bgh) terminator sequence. In some cases, a bgh terminator sequence may have the nucleotide sequence of SEQ ID NO: 83, or a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO: 83.

In some cases, a nucleic acid molecule as described herein further comprises a nucleotide sequence encoding an N-terminal signal peptide. Suitable N-terminal signal peptides are discussed earlier herein. In some cases, the N-terminal signal peptide is a leucine-rich signal peptide, preferably a human Lucy tag or a mouse Lucy tag, more preferably is a human Lucy tag or a mouse Lucy tag represented by the amino acid sequence MRPQILLLLALLTLGLA (SEQ ID NO: 76) or MSHQILLLLALLTLGLA (SEQ ID NO: 77). Also encompassed are the sequences of SEQ ID NO: 76 and 77 wherein 1, 2, 3, 4, or up to 5 amino acids are substituted, deleted, added, or inserted. Substitutions, and in particular conservative substitutions, are preferred.

In some cases, a nucleic acid molecule as described herein further comprises a nucleotide sequence encoding an N-terminal tag peptide. Suitable N-terminal tag peptides are discussed earlier herein.

In some cases, the N-terminal tag peptide is selected from the group consisting of a FLAG tag, a rhodopsin (rho) tag, an IL-6 tag, an $SST_3$ tag (45 -N-terminal amino acids of the Somatostatin 3 receptor; an example of which is SEQ ID NO: 223), an $M_3$-Tag (61-N-terminal amino acids of the muscarinic acetylcholine receptor $M_3$; an example of which is SEQ ID NO: 222), a c-myc tag, and a HA tag, preferably from the group consisting of a FLAG tag, a rhodopsin (rho) tag, an $SST_3$ tag, and an $M_3$ tag, even more preferably from the group consisting of a FLAG tag and a rhodopsin (rho) tag, most preferably is a rhodopsin (rho) tag.

Accordingly, in some cases, a nucleic acid molecule as described herein further comprises a nucleotide sequence encoding an N-terminal tag peptide, preferably wherein the N-terminal tag peptide is selected from the group consisting of a FLAG tag, a rhodopsin (rho) tag, an IL-6 tag, an $SST_3$ tag, an M3-Tag, a c-myc tag, and a HA tag, more preferably from the group consisting of a FLAG tag, a rhodopsin (rho) tag, an $SST_3$ tag, and an $M_3$ tag, even more preferably from the group consisting of a FLAG tag and a rhodopsin (rho) tag, most preferably is a rhodopsin (rho) tag.

In preferred cases, the N-terminal tag peptide comprises at least a tag peptide selected from the group consisting of a rhodopsin (rho) tag, an SSTstag, and an Mstag, preferably a rho tag. Optionally, a FLAG peptide may be further present.

Combinations of the above-described tag peptides may also be used. Typically, such combination will comprise at least one of a rho tag, an $SST_3$ tag, and an M3-Tag, preferably at least a rho tag. For example, in some cases, the N-terminal tag peptide is a combination of a rho tag and a FLAG tag. In other words, in some cases, nucleic acid molecule as described herein further comprises a nucleotide sequence encoding an N-terminal tag peptide, wherein the N-terminal tag peptide comprises a rho tag and a FLAG tag. Preferably, in that situation, the FLAG tag is positioned N-terminally from the rho tag, for example as shown in SEQ ID NO: 81.

In some cases, an encoded FLAG tag by a nucleic acid molecule as described herein has the sequence of SEQ ID NO: 78. In some cases, an encoded rho tag by a nucleic acid molecule as described herein has the sequence of SEQ ID NO: 79. In some cases, an encoded IL-6 tag by a nucleic acid molecule as described herein is an IL-6-HaloTag® as described in Noe et al. In some cases, an encoded $SST_3$ tag by a nucleic acid molecule as described herein has the sequence of SEQ ID NO: 223. In some cases, an encoded $M_3$ tag by a nucleic acid molecule as described herein has the sequence of SEQ ID NO: 222. Also encompassed are the sequences of SEQ ID NO: 78, 79, 222, and 223 wherein 1, 2, 3, 4, or up to 5 amino acids are substituted, deleted, added, or inserted. Substitutions, and in particular conservative substitutions, are preferred.

Nucleotide sequences encoding N-terminal signal peptides as described herein and N-terminal tag peptides as described herein can, advantageously, be used in combination with each other. Typically, the nucleotide sequence encoding an N-terminal signal peptide will be positioned upstream (N-terminally) from the nucleotide sequence encoding the N-terminal tag. For example, a nucleic acid molecule as described herein may further comprise a nucleotide sequence encoding an N-terminal signal peptide and one or more N-terminal tag peptides. In some cases, a nucleic acid molecule as described herein may further comprise:

- a nucleotide sequence encoding a human or mouse Lucy signal peptide (such as, SEQ ID NO: 76 or 77);
- a FLAG tag (such as, SEQ ID NO: 78) or an IL-6 tag, preferably a FLAG tag (such as, SEQ ID NO: 78); and
- a rho tag (such as, SEQ ID NO: 79), an $SST_3$ tag, or an M3-Tag, preferably a rho tag (such as, SEQ ID NO: 79).

SEQ ID NO: 80 is an example of a nucleotide sequence encoding a combination of a mouse Lucy signal peptide, a FLAG tag peptide and a rho tag peptide (SEQ ID NO: 81).

In some cases, a nucleic acid molecule as described herein is modified to comprise a nucleotide sequence encoding one or more additional N-terminal glycosylation sites.

In some cases, a nucleic acid molecule as described herein further comprises a nucleotide sequence encoding one or more olfactory receptor accessory proteins. Olfactory receptor "accessory proteins" or "chaperones" are proteins or peptides that may assist in the expression, trafficking, and/or signalling of an olfactory receptor to the surface of a cell expressing said olfactory receptor.

Non-limiting examples of accessory proteins encompassed by this disclosure include RTP1, RTP1S, RTP2, REEP, β-adrenergic receptor, heat shock protein 70, Ric8b, $G\alpha_{olf}$, $Gi\alpha$, or functional variants thereof, and the like, and are further described in WO2006/002161 and WO2014/037800, incorporated herein by reference in their entireties. Preferred accessory proteins are RTP1S and/or RTP2, preferably human RTP1S and/or human RTP2.

Accessory proteins described herein also encompass functional variants of their wildtype counterparts, i.e., accessory molecules that have been modified as compared to the corresponding naturally-occurring or wildtype sequence. In that context, RTP1S as used herein includes the RTP1S V227I variant, and RTP2 as used herein includes the RTP2 L220R variant. Preferred accessory proteins are the human RTP1S V227I variant (SEQ ID NO: 84) and the human RTP2 L220R variant (SEQ ID NO: 85).

Thus, in some cases, the one or more olfactory receptor "accessory proteins" as described herein are selected from the group consisting of RTP1, RTP1S, RTP2, REEP, β-adrenergic receptor, heat shock protein 70, Ric8b, $G\alpha_{olf}$, $Gi\alpha$, and functional variants thereof, preferably selected from the group consisting of RTP1S, RTP2 and functional variants thereof. In some cases, the one or more olfactory receptor "accessory proteins" as described herein are the RTP1S V227I variant and the RTP2 L220R variant. In some cases, a nucleotide sequence encoding one or more olfactory receptor accessory proteins comprises a nucleotide sequence encoding a polypeptide as represented by SEQ ID NO: 84 and/or 85, or a nucleotide sequence encoding a polypeptide having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity or similarity with SEQ ID NO: 84 and/or 85.

Nucleotide sequences described herein may be codon optimized for expression in a host cell, preferably in a eukaryotic cell, more preferably in a human cell. Suitable host cells are also described elsewhere herein, see e.g. the section "cells". "Codon optimization", as used herein, refers to the processes employed to modify an existing coding sequence, or to design a coding sequence, for example, to improve translation in an expression host cell or organism of a transcript RNA molecule transcribed from the coding sequence, or to improve transcription of a coding sequence. Codon optimization includes, but is not limited to, processes including selecting codons for the coding sequence to suit the codon preference of the expression host cell or organism. Codon optimization also eliminates elements that potentially impact negatively RNA stability and/or translation (e. g. termination sequences, TATA boxes, splice sites, ribosomal entry sites, repetitive and/or GC rich sequences and RNA secondary structures or instability motifs). In some cases, codon-optimized sequences show at least 3%, 5%, 10%, 15%, 20%, 25%, 30%, 40%, 50%, 60%, 70%, 80%, 90%, 100% or more increase in gene expression, transcription, RNA stability and/or translation compared to the original, non-codon-optimized sequence.

Expression vectors

Nucleic acid molecules as described herein can be placed in expression vectors. Thus, in another aspect there is provided an expression vector comprising any of the nucleic acid molecules as described herein.

An "expression vector", alternatively referred to herein as "vector" or "delivery vector" (not to be confused with "OR activation vector" or "feature vector" or the like used elsewhere herein), refers to a molecular biology tool used to obtain expression of a coding region (such as a gene) in a host cell, for example by introducing a nucleotide sequence that is capable of effecting expression of a gene or a coding sequence in a host cell compatible with said sequence. An expression vector may be able to stabilize and remain episomal in a host cell. Alternatively, a vector may be able to integrate into a host cell's genome, for example through homologous recombination, non-homologous end-joining, or otherwise. A description of suitable "host cells" in the context of this disclosure is provided elsewhere herein.

Suitable expression vectors may be selected from any genetic element known in the art which can facilitate transfer of nucleic acids between cells, such as, but not limited to, plasmids, phages, transposons, cosmids, chromosomes, artificial chromosomes, viruses (such as, but not limited to, retroviruses, lentiviruses, and the like), virions, and the like. An expression vector may also be a chemical vector, such as, a lipid complex or naked DNA. "Naked DNA" or "naked nucleic acid" refers to a nucleic acid molecule that is not contained in encapsulating means that facilitates delivery of a nucleic acid into the cytoplasm of a target host cell. Naked DNA may be circular or linear (linearized DNA sequence). Optionally, a naked nucleic acid can be associated with standard means used in the art for facilitating its delivery of the nucleic acid to the target host cell, for example to facilitate the transport of the nucleic acid through the cell membrane.

A preferred expression vector is a plasmid. Suitable plasmids are known in the art and described in standard handbooks such as Ausubel et al. and Sambrook and Green (supra). Suitable plasmids may also be selected from commercially available vectors, such as, the pcDNA3.1(+) series (Invitrogen, MA, USA) or the pGL4.29 series of vectors (Promega, WI, USA).

Cells

The nucleic acid molecules and expression vectors described herein are particularly useful for introduction into a host cell. Accordingly, in another aspect, there is provided a recombinant host cell comprising a nucleic acid molecule or an expression vector as described earlier herein. Preferably, the recombinant host cells described herein express or are capable of expressing an olfactory receptor protein as described herein.

In some cases, host cells of this disclosure comprise multiple, i.e. two or more, nucleic acid molecules and/or expression vectors as described herein. Accordingly, in that case, the recombinant host cells express or are capable of expressing multiple, i.e. two or more, olfactory receptor proteins as described herein. In the context of such host cells, the two or more olfactory receptors are preferably activated by odorants having similar odors. Odorants having a similar odor will typically be described by the same odor descriptors. An "odor descriptor" is a common term used by trained perfumers and evaluators to describe a particular odor sensation common to a group of ligands or mixture of ligands. Odor descriptors are for example 'green' for odors reminiscent of fresh crushed leaves or 'floral' for scents of flowers. They can then also be more specific such as 'floral-rosy' for notes reminding of rose odors, or 'white-floral' for odors reminding of Ylang-ylang or jasmine and so on.

In some cases, combinations of two or more olfactory receptor proteins in this context may be selected from the group consisting of:

- OR7A17 and OR7C1. These ORs are activated by molecules with the odor descriptor 'woody-ambery' and could be co-expressed in a cell to detect woody-ambery notes.
- OR5A2, OR5AN1, and OR1N2. These ORs are activated by molecules with the odor descriptor 'musky' and two or more olfactory receptor proteins of this group could be co-expressed in a cell to detect musky notes.
- OR2L2, OR2L3, OR2L5, OR2AK2, and OR11G2. These ORs are activated by molecules with the odor descriptor 'fruity-ester' and two or more olfactory receptor proteins of this group could be co-expressed in a cell to detect fruity-ester notes.
- OR10A3, OR10A6, and OR10J1. These ORs are activated by molecules with the odor descriptor 'fruity-lactonic' and two or more olfactory receptor proteins of this group could be co-expressed in a cell to detect fruity-lactonic notes.
- OR10H1, OR10H2, OR10H5, and OR10K1. These ORs are activated by molecules with the odor descriptor 'marine' and two or more olfactory receptor proteins of this group could be co-expressed in a cell to detect marine notes.
- OR10G7 and OR10D3. These ORs are activated by molecules with the odor descriptor 'spicy' and could be co-expressed in a cell to detect spicy notes.

A "host cell", alternatively referred to herein as a "recombinant host cell", "engineered cell", or simply "cell" refers to a cell that has been engineered by the introduction of a nucleic acid molecule and/or an expression vector as defined herein. A host cell may refer to a cell in isolation or in culture. Host cells may be "transduced cells", wherein the cells have been infected with e.g. a modified virus. As a non-limiting example a lentivirus may be used, but other suitable viruses such as retroviruses or others may be contemplated as well. Introduction of a nucleic acid construct may also be performed by non-viral methods, e.g. by transfection. "Transfection" refers to non-viral methods of DNA (or RNA) transfer to cells such that the transferred nucleic acid sequence is expressed. Transfection methods and protocols are well-known in the art, with non-limiting examples being calcium phosphate transfection, PEG transfection, and liposomal or lipoplex transfection, and discussed in standard handbooks such as Ausubel et al. and Sambrook and Green (supra). A further example of a transfection method is provided in the exemplary section herein. A transfection may be transient or stable, the latter referring to cases wherein cells have the nucleic acid construct integrated in their genome. Host cells comprising a nucleic acid construct as described herein may thus also be "stably transfected cells" or "transiently transfected cells".

A host cell may be further genetically modified, for example by the introduction of one or more genetic modifications including, but not limited to, nucleotide mutations, substitutions, insertions, and/or deletions in its genome, and/or introduction of additional nucleic acid constructs. Said modifications may be comprised in a nucleotide sequence encoding an olfactory receptor, an accessory molecule, and/or another genomic region and may result in functional expression or improved functional expression of said olfactory receptor and/or said accessory molecule. A definition of functional expression is provided elsewhere herein.

Modification of a nucleic acid sequence may be performed using any recombinant DNA technique as known in the art, such as for example described in standard handbooks such as Ausubel et al. and Sambrook and Green (supra). Also see, Kunkel (1985) Proc. Natl. Acad. Sci. 82:488 (describing site directed mutagenesis) and Roberts et al. (1987) Nature 328:731 734 or Wells, J.A., et al. (1985) Gene 34: 315 (describing cassette mutagenesis).

A host cell may comprise epigenetic modifications in a nucleic acid molecule encoding an olfactory receptor, an accessory protein, and/or another genomic region which may result in functional expression or improved functional expression of said olfactory receptor and/or said accessory protein. As used herein, the term "epigenetic modification" has its customary meaning as ordinarily understood by the skilled person in view of this disclosure. It refers to chemical modifications of DNA or histone proteins that do not alter a nucleotide sequence itself. Non-limiting examples of epigenetic modifications include nucleic acid methylation, acetylation, phosphorylation, serotonylation, citrullination, ubiquitination, sumoylation, and ribosylation.

Advantageously, in some cases, a recombinant host cell as described herein further expresses one or more olfactory receptor accessory proteins as described herein. To achieve this, additional nucleic acid molecules or expression vectors encoding one or more olfactory receptor accessory proteins may be comprised in the recombinant host cells. These additional nucleic acid molecules or expression vectors may be stably integrated into the chromosome or they may be introduced for transient expression, e.g. by transfection. Suitable olfactory receptor accessory proteins have already been described elsewhere herein. Thus, in some cases, the one or more olfactory receptor "accessory proteins" as described herein are selected from the group consisting of RTP1, RTP1S, RTP2, REEP, β-adrenergic receptor, heat shock protein 70, Ric8b, $G\alpha_{olf}$, $G\alpha$, and functional variants thereof, preferably selected from the group consisting of RTP1S, RTP2 and functional variants thereof. In some cases, the one or more olfactory receptor "accessory proteins" as described herein are the RTP1S V227I variant and the RTP2 L220R variant. In some cases, a nucleotide sequence encoding one or more olfactory receptor accessory proteins comprises a nucleotide sequence encoding a polypeptide as represented by SEQ ID NO: 84 and/or 85, or a nucleotide sequence encoding a polypeptide having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity or similarity with SEQ ID NO: 84 and/or 85.

In some cases, a recombinant host cell as described herein further expresses one or more reporter genes, such as a luciferase gene. Reporter genes are described in more detail elsewhere herein.

Recombinant host cells as described herein may be prokaryotic or eukaryotic cells, preferably they are eukaryotic cells. Suitable prokaryotic cells may be selected from bacteria and archaea. Suitable eukaryotic cells may be selected from insect, plant, yeast, fungal, algal, mammalian, and human cells, of which human cells are preferred.

Suitable host cells include, but are not limited to, HEK293, HEK293T, HeLa, CHO, OP6, HeLa-S3, HEKn, HEKa, PC-3, Calul, Hep G2, HeLa B, HeLa T4, COS, COS-1, COS-6, C0S-M6A, BS-C-1 monkey kidney epithelial cells, BALB/3T3 mouse embryo fibroblasts, 3T3 Swiss, 3T3-L1, 132-d5 human fetal fibroblasts, 10.1 mouse fibroblasts, 293-T, 3T3, BHK, BHK-21, BR 293, BxPC3, C3H-10T1/2, C6/36, Cal-27, CHO-7, CHO-IR, CHO-K1, CHO-K2, CHO-T, CHO Dhfr-/- COS-7, HL-60, LNCap, MCF-7, MCF-IOA, MDCK II, SkBr3, Vero cells, primary olfactory cells, immortalized olfactory cells, immortalized taste cells, and transgenic varieties thereof, of which HEK293 and HEK293T are preferred. Accordingly, in some cases, a recombinant host cell as described herein is a HEK293 or HEK293T cell. Among HEK293 and HEK293T, HEK293T is more preferred. Cell lines are available from a variety of publicly available culture collections, e.g. the American Type Culture Collection (VA, USA).

Libraries

In another aspect, this disclosure relates to a library comprising a diverse repertoire of olfactory receptor proteins as described herein, nucleic acid molecules as described herein, expression vectors as described herein, or recombinant host cells as described herein.

In some cases of a library as described herein, the diverse repertoire of olfactory receptor proteins, of olfactory receptor proteins encoded by the nucleic acid molecules or expression vectors, or of olfactory receptor proteins expressed by the recombinant host cells, shares the same modified C-terminal domain. In a specific but non-limiting example, they share the modified C-terminal domain represented by SEQ ID NO: 221 (particularly in the context of class II olfactory receptors) or SEQ ID NO: 741 (particularly in the context of class I olfactory receptors).

Such a library of olfactory receptors with an identical C-terminal domain advantageously provides for a more homogenous functional activity of all receptors. While a library of the majority of receptors with their wild-type C-terminus has vastly different functional expression levels, functional expression between receptors is better comparable in a library with identical C-terminal sequences. Thus testing a ligand vs. such a normalized library with identical C-terminus allows to find the most sensitive receptor(s) activated by a given ligand, which is crucial to later screen novel ligands within a given odor description. If on the other hand the target receptor would have been identified from a library with vastly divergent functional expression, the selection of the receptor may be skewed by the arbitrary functional expression in the *in vitro* system rather than by the true ligand affinity.

A library as described herein is not particularly limited with respect to the number of distinct olfactory receptor proteins, nucleic acid molecules or expression vectors encoding distinct olfactory receptor proteins, or recombinant host cells expressing distinct olfactory receptor proteins. However, in some cases, a library as described herein comprises a diverse repertoire of at least 10, at least 25, at least 50, at least 75, at least 100, at least 150, at least 200, at least 250, at least 300, at least 350, or at least 400 distinct olfactory receptor proteins, nucleic acid molecules or expression vectors encoding distinct olfactory receptor proteins, or recombinant host cells expressing distinct olfactory receptor proteins.

In some cases, a library as described herein comprises a diverse repertoire of about 250 to about 800 distinct olfactory receptor proteins, nucleic acid molecules or expression vectors encoding distinct olfactory receptor proteins, or recombinant host cells expressing distinct olfactory receptor proteins. Such library size allows coverage of the majority of dog olfactory receptors.

In some cases, a library as described herein comprises a diverse repertoire of about 250 to about 670 distinct olfactory receptor proteins, nucleic acid molecules or expression vectors encoding distinct olfactory receptor proteins, or recombinant host cells expressing distinct olfactory receptor proteins. Such library size allows coverage of the majority of cat olfactory receptors.

In some cases, a library as described herein comprises a diverse repertoire of about 250 to about 500 or 250 to about 400 distinct olfactory receptor proteins, nucleic acid molecules or expression vectors encoding distinct olfactory receptor proteins, or recombinant host cells expressing distinct olfactory receptor proteins. Such library size allows coverage of the majority of human olfactory receptors.

In some cases, a library as described herein comprises a diverse repertoire of about 400 to about 800 distinct olfactory receptor proteins, nucleic acid molecules or expression vectors encoding distinct olfactory receptor proteins, or recombinant host cells expressing distinct olfactory receptor proteins. Such library size allows coverage of the majority of human olfactory receptors including major alternative alleles or haplotypes.

Libraries may contain both class I and class II ORs, or libraries may be focused on Class I ORs or on Class II ORs.

Accordingly, in some cases, a library as described herein comprises distinct class II olfactory receptor proteins, preferably human class II olfactory receptor proteins, nucleic acid molecules or expression vectors encoding said olfactory receptor proteins, or recombinant host cells expressing said olfactory receptor proteins. A library of Class II ORs as described herein may comprises a diverse repertoire of at least 10, at least 25, at least 50, at least 75, at least 100, at least 150, at least 200, at least 250, at least 300, at least 350, or at least 400 distinct olfactory receptor proteins, nucleic acid molecules or expression vectors encoding distinct olfactory receptor proteins, or recombinant host cells expressing distinct olfactory receptor proteins. A preferred number in the context of libraries of human Class II ORs is 400 to 450. Preferably, such a library comprises at least one, preferably all of the class II olfactory receptors listed as "receptor" in Table 15. It is understood that in such a library, a class II olfactory receptor may differ from the specific SEQ ID NOs in Table 15 in its N-terminal tag or its modified C-terminal domain, for example a different modified C-terminal domain as described herein may be comprised by the receptor instead of the modified C-terminal domain comprised by the olfactory receptors encoded by the nucleic acid molecules listed in Table 15. SEQ ID NOs 331-739 also include a 5' BamHI restriction site (GGATCC) and Kozak sequence (GCCACC), and a 3' NotI restriction site (GCGGCCGC) for cloning and expression purposes. The presence of these sequences is entirely optional.

In some cases, a library as described herein comprises distinct class I olfactory receptor proteins, preferably human class I olfactory receptor proteins, nucleic acid molecules or expression vectors encoding said olfactory receptor proteins, or recombinant host cells expressing said olfactory receptor proteins. A library of Class I ORs as described herein may comprises a diverse repertoire of at least 10, at least 25, at least 50, or at least 75 distinct olfactory receptor proteins, nucleic acid molecules or expression vectors encoding distinct olfactory receptor proteins, or recombinant host cells expressing distinct olfactory receptor proteins. A preferred number in the context of libraries of human Class II ORs is 70 to 100. Preferably, such a library comprises at least one, preferably all of the class I olfactory receptors listed as "receptor" in Table 22. It is understood that in such a library, a class I olfactory receptor may differ from the specific SEQ ID NOs in Table 22 in its N-terminal tag or its modified C-terminal domain, for example a different modified C-terminal domain as described herein may be comprised by the receptor instead of the modified C-terminal domain comprised by the olfactory receptors encoded by the nucleic acid molecules listed in Table 22. SEQ ID NOs 742-819 also include a 5' BamHI restriction site (GGATCC) and Kozak sequence (GCCACC), and a 3' NotI restriction site (GCGGCCGC) for cloning and expression purposes. The presence of these sequences is entirely optional.

In some cases, a library as described herein comprises distinct class I and class II olfactory receptor proteins, preferably human class I and class II olfactory receptor proteins, nucleic acid molecules or expression vectors encoding said olfactory receptor proteins, or recombinant host cells expressing said olfactory receptor proteins. In this context, a library may preferably comprise a diverse repertoire of 470 to 550 distinct olfactory receptor proteins, nucleic acid molecules or expression vectors encoding distinct olfactory receptor proteins, or recombinant host cells expressing distinct olfactory receptor proteins.

Olfactory receptor activation data, OR activation vectors, and OR activation library

Known databases including data concerning single fragrance compounds may typically have the structure of Table 27. A fragrance composition may then be composed of a selection of compounds of the database, in a given proportion. The complete composition may then be described by, for example, a profile of evaporation and substantivity based on physicochemical properties and odor descriptions of the complex formulation, using subjective odor descriptors acquired after smelling the composition by experts. The composition may be required to comply with specific regulatory requirements based on the target application or the target jurisdiction. Compliance with these requirements may be calculated based on regulatory information concerning the compounds and the concentration thereof within in the composition.

Table 27. Example known compound database, including physiochemical parameters, odor descriptors, and regulatory information

| Identifier | 2 D-Chemical structure | Physicochemical parameter a (e.g. Log P) | Physicochemical parameter b (e.g. Vapor pressure) | Odor descriptor 1 | Odor descriptor 2 | Odor descriptor 3 | Regulatory information I | Regulatory information N |
|---|---|---|---|---|---|---|---|---|
| CAS 1 | [graph 1] | 5 | 56 | Floral | Sweet | Honey | H317 | H319 |
| CAS 2 | [graph 2] | 3 | 89 | Woody | Earthy | Ambery | H400 | H410 |
| CAS 3 | [graph 3] | 2.5 | 38 | Floral | NA | NA | H331 | NA |
| CAS 4 | [graph 4] | 2.2 | 23 | Musky | Sweet | NA | H330 | H340 |
| ... | ... | ... | ... | ... | ... | ... | ... | ... |
| CAS n | [graph n] | an | $b_n$ | $desc_{1,n}$ | $desc_{2,n}$ | $desc_{3,n}$ | In | $N_n$ |

Table 28. Example OR activation data (OR activation library), including physiochemical parameters, odor descriptors, regulatory information, and OR activation expressions

| Identifier | 2 D - Chemical structure | Physicochemical parameter a (e.g. Log P) | Physicochemical parameter b (e.g. Vapor pressure) | EC50 OR1A1 | EC50 OR1A2 | EC50 ORm | Odor descriptor 1 | Odor descriptor 2 | Odor descriptor 3 | Regulatory information I | Regulatory information N |
|---|---|---|---|---|---|---|---|---|---|---|---|
| CAS 1 | [graph 1] | 5 | 56 | 86.2 | 4.5 | $OR_{m,1}$ | Floral | Sweet | Honey | H317 | H319 |
| CAS 2 | [graph 2] | 3 | 89 | 5.6 | 0.16 | $OR_{m,2}$ | Woody | Earthy | Ambery | H400 | H410 |
| CAS 3 | [graph 3] | 2.5 | 38 | 24.5 | 2.2 | $OR_{m,3}$ | Floral | NA | NA | H331 | NA |
| CAS 4 | [graph 4] | 2.2 | 23 | 4.4 | 3.8 | $OR_{m,4}$ | Musky | Sweet | NA | H330 | H340 |
| ... | ... | ... | ... | ... | ... | ... | ... | ... | ... | ... | ... |
| CAS n | [graph n] | an | $b_n$ | $OR_{1A1,n}$ | $OR_{1A2,n}$ | $OR_{m,n}$ | $desc_{1,n}$ | $desc_{2,n}$ | $desc_{3,n}$ | $I_n$ | $N_n$ |

Known computer-implemented learning or inference techniques based on the database with the structure of Table 27 may be based on assumptions, in particular that the chemical structure is an accurate predictor of odor descriptor and that there is a causal link between odor descriptors and chemical structure.

This approach of learning from subjective odor descriptors and chemical structure only is subject to uncertainty, as subjective odour descriptors for individual molecules are often not fully accurate and the inherent noise in the subjective assessment of individual molecules may appreciably influence any model. This is especially the case if an individual compound has several odor descriptors that are difficult to separate by a verbal description alone.

Moreover, recent studies show that vastly different structures may have similar odor descriptors because they bind the same OR - while very closely related chemical structures have very different odor descriptors because the bind to different ORs.

Thus for example as shown in the Examples, OR2M2, which was hitherto an orphan receptor, could be functionally expressed when applying a modified C-terminal sequence of SEQ ID 221. This OR is activated by 1-p-Menthene-8-thiol (CAS Nr. 83108-07-0), Nootkatone (5,6-Dimethyl-8-iso-propenylbicyclo-(4,4,0)-dec-1-en-3-one; CAS 4674-50-4) and Paradisamide (CAS 406488-30-0), three completely different chemical structures - not sharing any common structural motif, but all smelling grapefruit and surprisingly activating the same OR. Similarly, Patchoulol ((4S)-4,8a,9,9-tetramethyloctahydro-1,6-methanonaphthalen-1(2H)-ol) and 2-(tricyclopropylsilyl)propan-2-ol (Sunderkotter, A., et al., Chemistry, 2010. 16(25): p. 7404-21) both smell of Patchouli oil and activate OR14J1, which was hitherto an orphan receptor, and could only be functionally expressed when applying a modified C-terminal sequence of SEQ ID 221. These two structures do not share common 2D structural features except an alcohol group. These examples show that similarity in odour can best be captured by activation of the same OR or OR activation vector, but not by their 2D-chemical structure.

On the other hand, Eugenol (4-allyl-2-methoxyphenol) and Isoeugenol ((E)-2-methoxy-4-(prop-1-en-1-yl)phenol) are different only by the shift of one double bond in the side chain by one carbon atom. They share a common spicy character by their activation of OR10G7, yet Isoeugenol has an important floral note in addition, and it uniquely activates a second OR (OR10D3), and hence has a distinct OR activation vector despite their very high structural similarity. This could not have been predicted from the 2D structure.

These findings highlight the limitation in linking the odor descriptors to compound structure only. While in some cases structure and odor positively correlate, the structure alone is not a unique and reliable indicator of compound and odor descriptor link.

Thus, databases of composition compounds preferably include an objective measure of olfactory activation, which may be generated through measurement of OR activation using the techniques described herein. Example OR activation data (including OR activation libraries thereof) may then have the structure of Table 28.

The tabular representation of OR activation may be referred to as one form "OR activation data" (of course, the data does not necessarily need to be presented in tabular form). From OR activation data, one may obtain an "OR activation (feature) vector" through transformation of the numeric and categorical data. Depending on the presence of categorical data (e.g., the number of distinct odor descriptors at hand), feature indexing may be used (allocating a distinct numerical index for each distinct odor descriptor), thereby forming a vocabulary. ML models, for instance, may then look up the index from input strings, assigning 1.0 to the corresponding slot in a feature vector and 0.0 to all the other slots in the feature vector (one-hot encoding). Alternatively, hashing techniques may be used or hybrid hashing-vocabulary techniques may be used, all of which will be known the skilled reader. Similarly, numeric data (such as EC50 values) may be transformed using such known techniques as normalising and bucketing. For example, averaging of compound data over varying concentrations may be used to provide a single value representative of that compound.

OR activation data may include data for OR agonist and/or OR antagonists, as described elsewhere herein.

Optionally, the OR activation data may also contain relative surface expression of each tested OR the EC50 value for each OR may be normalized to this surface expression.

The activation of all human ORs, including all key haplotypes of human ORs (n = 500 - 600), generates an OR activation vector, $V_{act}$, with n= 500 - 600 dimensions for a given compound.

By having all OR activation vectors linked with the odor descriptors, it is possible to implement computing techniques to learn stable associations between odor descriptors and specific dimensions of the OR activation vector. By linking the odor descriptors to OR activation dimensions, more stable predictions are generated, as the noise from the subjective evaluation of single compounds is diminished by the learning on association of OR activation on multiple compounds with verbal descriptors for multiple compounds.

Thus far, generation of OR activation vectors using existing techniques is fragmentary, as a majority of ORs have not been successfully expressed in cell systems. Techniques herein, however, allow for OR activation vector measurement on a library of all ORs, including all key haplotypes of human OR, in which any or all ORs have a changed C-terminal domain to facilitate their surface expression, thereby forming an "OR activation library".

Of course, not all columns in Table 28 are essential to all instances of OR activation data; OR activation data may be formed, for example, using only a digital identifier for a compound (e.g., CAS) and an OR activation vector for a single OR (e.g., a single, unnormalized EC50 value). Tabular data and associated data may be expressed in any suitable data structure format, for example as XML data or within a SQL database.

The C-terminal sequence modification mentioned throughout is only needed to generate the assay OR activation data.

As discussed herein, the C-terminal modification does not affect the OR-binding site. Thus, OR activation data generated with these C-terminal modified ORs are representative of the true Odor perception, in that they predict the true OR activation data in vivo - this can be contrasted to teachings in Yoshikawa, K., et al., An odorant receptor that senses four classes of musk compounds. Curr Biol, 2022. 32(23): p. 5172-5179 e5. and Ikegami, K., et al., Structural instability and divergence from conserved residues underlie intracellular retention of mammalian odorant receptors. Proc Natl Acad Sci USA, 2020. 117(6): p. 2957-2967. in these state of the art references, a consensus sequence of an OR was made - whereby functional expression is achieved, but then there is a change in the OR binding site of the human OR and hence it is unclear whether the OR gives a true representation of the odorant binding profile perceived by human panellists.

Methods and uses

The olfactory receptors, nucleic acid molecules, recombinant host cells, and libraries described herein enable the functional expression of olfactory receptors otherwise not possible to express using conventional approaches or which lead to assays with limited sensitivity using conventional approaches and in the identification of novel cognate receptor-ligand pairs. Thus, they are particularly useful for application in methods and uses for expressing olfactory receptors and for identifying novel olfactory receptors and novel olfactory receptor ligands, enhancers and antagonists.

in an aspect, there is provided a use of an olfactory receptor protein as described herein, a nucleic acid molecule as described herein, an expression vector as described herein, a cell as described herein, or a library as described herein, for identifying an olfactory receptor ligand, enhancer or antagonist.

in an aspect, there is provided a use of a library as described herein, for identifying an olfactory receptor that is capable of binding a target ligand.

in an aspect, there is provided a method for identifying an olfactory receptor ligand, said method comprising:

a) providing an olfactory receptor protein as described herein or a cell expressing an olfactory receptor protein as described herein;

b) contacting said receptor or cell with a test compound or composition; and

c) detecting activation of the olfactory receptor.

in an aspect, there is provided a method for identifying an olfactory receptor enhancer or antagonist, said method comprising:

a) providing an olfactory receptor protein as described herein or a cell expressing an olfactory receptor protein as described herein;

b) contacting said receptor or cell with a cognate ligand and a test compound or composition; and

c) detecting increased or decreased activation of the olfactory receptor as compared to controls with ligand only.

An olfactory receptor "antagonist" as used herein is a compound that decreases the activation of a given olfactory receptor by an OR ligand. An olfactory receptor "enhancer" as used herein is a compound that increases the activation of a given olfactory receptor by an OR ligand.

in some cases of a method for identifying an olfactory receptor ligand, and of a method for identifying an olfactory receptor enhancer or antagonist, the olfactory receptor is selected from the group consisting of OR7C1, OR8K3 (preferably OR8K3(L122R)), OR10J5, OR7A17, OR10H5, OR5A1, OR5A2, OR1N2 (preferably OR1N2(W23R,V230G,T287M)), OR2M2, OR2V1, OR5P3, OR6P1, OR2L2 (or OR2L2(V259L)), OR10G7 (preferably OR10G7(T5S)), OR5AN1, OR5V1, OR2L3, OR2AG2 (preferably OR2AG2(Y28C)), OR7A5, OR7E24 (or OR7-E24(P242S)), OR7A10, OR10H2, OR10H1, OR10D3, OR1D2, OR2A5, OR2A25 (OR2A25(S75N,A209P)), OR11G2 (or OR11G2(I65N,V82I)), OR14J1, OR5M3, OR8D1, OR10G3 (preferably OR10G3(S73G)), OR10G9, OR2L5, OR8H1, OR10K1, OR11A1, OR2AK2 (preferably OR2AK2(S84N)), OR10A3, OR10A6 (preferably OR10-A6(A117V,V140G,L287P)), OR10J1 (preferably OR10J1(M51I,I92M)), OR2J2, and OR2AG2 (preferably OR2AG2(Y28C)). For these receptors, identifying olfactory receptor ligands and identifying olfactory receptor enhancers is of particular interest. In some cases of a method for identifying an olfactory receptor ligand, and of a method for identifying an olfactory receptor enhancer or antagonist, the olfactory receptor is OR5A2, OR5A1, OR7A17, OR7C1, OR8K3, OR1N2, OR10J5, OR5B12 or OR10H5.

in some cases of a method for identifying an olfactory receptor enhancer or antagonist, the method is such that it is for identifying an olfactory receptor antagonist, and the olfactory receptor is selected from the group consisting of OR52A5, OR52E8, OR56A1, OR56A3, OR56A4, OR52K1, OR51B2 (preferably OR51B2(C120R, L134F, C209S)), OR51B5, OR9Q2, OR7D4, OR2T4, OR2C1, OR2T11, OR2M2, OR2V1, OR5V1, and OR4S2, preferably selected from the group consisting of OR2M2, OR2V1, OR51B2 (preferably OR51B2(C120R,L134F,C209S)), and OR5V1. OR2M2 and OR2V1 are more preferred. Among these two, OR2M2 is more preferred.

in some cases of a method for identifying an olfactory receptor enhancer or antagonist, the method is such that it is for identifying an olfactory receptor antagonist, and the olfactory receptor is OR2M2 or OR2V1, preferably OR2M2. in this situation, optionally, step b) further comprises contacting said receptor or recombinant host cell with a copper salt. Accordingly, in some cases, this disclosure provides a method for identifying an olfactory receptor antagonist, said method comprising:

> a) providing an OR2M2 or OR2V1 olfactory receptor protein as described herein or a cell expressing an OR2M2 or OR2V1 olfactory receptor protein as described herein;
> b) contacting said receptor or cell with a cognate ligand, a test compound or composition, and a copper salt; and
> c) detecting increased or decreased activation of the olfactory receptor as compared to controls with ligand only.

in some cases, the copper salt may be used in a concentration between 1 and 100 $\mu$M, preferably between 10 and 100 $\mu$m, for example 30 $\mu$M. Suitable copper salts include copper(II) salts such as $CuCl_2$, $CuSO_4$, $Cu(OH)_2$ and copper acetate.

In the context of such methods for identifying an OR2M2 or an OR2V1 antagonist, the cognate ligand is preferably selected from the group consisting of 3-methyl-3-sulfanyl-hexanol, 2-mercapto-2-methyl-pentanol, and 4-methoxy-2-methylpentane-2-thiol.

In some cases of a method for identifying an olfactory receptor enhancer or antagonist, the method is such that it is for identifying an olfactory receptor antagonist, and the olfactory receptor is OR51B2, preferably OR51B2(C120R,L134F,C209S). In the context of such methods for identifying an OR51B2 antagonist, the cognate ligand is preferably 3-methyl-2-hexenoic acid.

In some cases of a method for identifying an olfactory receptor enhancer or antagonist, the method is such that it is for identifying an olfactory receptor antagonist, and the olfactory receptor is OR5V1. in the context of such methods for identifying an OR5V1 antagonist, the cognate ligand is preferably 2,4,6-trichloroanisol.

As explained earlier herein, this disclosure encompasses libraries comprising a diverse repertoire of olfactory receptor proteins, of nucleic acid molecules or expression vectors expressing olfactory receptor proteins, and of recombinant host cells expressing olfactory receptor proteins, preferably wherein each of the olfactory receptor proteins shares the same modified C-terminal domain. Advantageously, such a library of olfactory receptors with an identical C-terminal domain provides for a homogenous and uniform functional expression of all receptors when detecting activation of olfactory receptors.

Accordingly, in an aspect there is provided a method for generating an objective representation of the olfactory properties of a test compound or composition, said method comprising:

> a) providing a library as described herein;
> b) optionally, obtaining a diverse repertoire of olfactory receptor proteins or cells expressing olfactory receptor proteins from said library;
> c) contacting the diverse repertoire of olfactory receptor proteins or cells expressing olfactory receptor proteins with the test compound or composition; and
> d) detecting activation of each of the olfactory receptor proteins.

Also encompassed is the use of a library as described herein, for generating an objective representation of the olfactory properties of a test compound or composition.

Such objective representation of olfactory properties can be referred to as an OR activation 'fingerprint'. It is possible to represent the level of activation of each olfactory receptor as an n-dimensional vector, wherein n is the number of distinct olfactory receptors that is included in the library. More information about ways to measure and express the level of activation of olfactory receptors is provided elsewhere herein.

This type of objective representation of olfactory properties also allows to compare, in an objective manner, the olfactory properties between two or more test compounds or compositions.

Accordingly, in an aspect there is provided a method for assessing the difference or similarity between two or more test compounds or compositions, said method comprising:

> 1) generating an objective representation of the olfactory properties of the two or more test compounds or compositions, as described herein; and
> 2) comparing the objective representation of the olfactory properties between the two or more test compounds or compositions.

In some cases, there is provided a method for assessing the difference or similarity between two or more test compounds or compositions, said method comprising:

a) providing a library as described herein;
b) optionally, obtaining a diverse repertoire of olfactory receptor proteins or cells expressing olfactory receptor proteins from said library;
c) contacting the diverse repertoire of olfactory receptor proteins or cells expressing olfactory receptor proteins with each of the two or more test compounds or compositions;
d) detecting activation of each of the olfactory receptor proteins for each of the two or more test compounds or compositions; and
e) comparing the activated olfactory receptor proteins between each of the two or more test compounds or compositions.

Also encompassed is the use of a library as described herein, for assessing the difference or similarity between two or more test compounds or compositions.

In some cases, the two or more test compounds or compositions may involve a first test composition and a second test composition. In some cases, the second composition lacks one or more compounds present in the first composition but otherwise comprises the same compounds as the first composition; or the second composition comprises one or more alternative compounds for one or more compounds present in the first composition, but otherwise comprises the same compounds as the first composition.

in some cases, the last step of comparing the objective representation of the olfactory properties the activated olfactory receptor proteins between each of the two or more test compounds or compositions, includes the calculation of a distance measure. Suitable distance measures are known to the skilled person. As an example, the level of activation of each olfactory receptor may be represented as an n-dimensional vector, and the distance measure may be a measure of the distance between two vectors. In some cases, the distance between two vectors may be based on the the so-called 1-norm or L1 norm. The L1 norm is a standard measure in mathematics and is calculated as the sum of the absolute values of the vector. The distance between two vectors can then be calculated based on the norm of their difference. Thus, the distance between a first test compound or composition and a second test compound or composition may be calculated according to the following formula:

$$Distance = \sum_{OR_1}^{OR_n} |level\ of\ activation\ for\ test\ compound\ or\ composition\ 1$$
$$-\ level\ of\ activation\ for\ test\ compound\ or\ composition\ 2|$$

This distance is also known as the Euclidean distance. More information about ways to measure and express the level of activation of olfactory receptors is provided elsewhere herein.

In some cases, the test compound or composition involved in the methods of this disclosure is a mixture of odorants. Indeed, the increased sensitivity of the methods disclosed herein enables the detection of ligands, enhancers, and antagonists from complex samples against a matrix of strong odorants. in some cases, the test compound or composition is a perfume composition. In some cases, the test compound or composition is a composition, such as a perfume composition, comprising at least 5, at least 10, at least 15, at least 20, at least 25, at least 30, at least 35, at least 40, at least 45, or at least 50 odorants.

in some cases, the test compound or composition involved in the methods of this disclosure is an unpurified synthetic compound. Automated parallel synthesis of single compounds has dramatically increased the numbers of novel organic compounds available for biological assays. However, a disadvantage of automated parallel synthesis is that difficult and expensive subsequent purification steps are usually required before they can be employed in biological assays. This is especially true for sensory assays, where the olfactive assessment of a new perfume or flavour ingredient is difficult in presence of smelling impurities contributing off-odors or masking the scent of the desired molecule. Advantageously, the increased sensitivity of the methods disclosed herein enables the use of unpurified synthetic compounds. Accordingly, in some cases, the test compound or composition involved in the methods of this disclosure comprises an unpurified synthetic molecule. In this case, the synthetic molecule may have a purity level of less than 90%, less than 85%, less than 80%, less than 75% or less than 70% (the % purity level being the ratio of the desired product to that of the combined impurities, typically measured by liquid chromatography, gas chromatography or quantitative NMR).

In accordance with the above, in some cases, the test compound or composition involved in the methods of this disclosure is a mixture of odorants (as described above) or an unpurified synthetic compound (as described above).

In some cases, the test compound or composition involved in the methods of this disclosure is a racemic mixture of odorants. In some cases, the test compound or composition involved in the methods of this disclosure are isolated or synthetic isomers or enantiomers of such a racemic mixture.

As explained elsewhere, olfactory receptors have also been found to be associated with various disease. Accordingly, in some cases, the test compound or composition may comprise a candidate therapeutic agent, such as a candidate anti-cancer agent. In some cases, the test compound or composition may comprise a pharmacologically active agent.

Ligands, enhancers and antagonists as described herein are preferably biodegradable. Indeed, there is a growing interest in the field to move towards ingredients that are biodegradable. Accordingly, in some cases, the test compound or composition involved in the methods of this disclosure is a biodegradable compound or composition. As used herein, a compound or composition is considered to be biodegradable if it meets the pass criteria in accordance with OECD manometric respirometry methods, and in particular the OECD 301F method which methods are well known in the art. in this method the pass level for a compound to be considered as having "ready biodegradability" or being "readily biodegradable" is to reach 60% of theoretical oxygen demand and/or chemical oxygen demand. This pass value has to be reached in a 10-day window within the 28-day period of the test. The 10-day window begins when the degree of biodegradation has reached 10% of theoretical oxygen demand and/or chemical oxygen demand and must end before day 28 of the test. Given a positive result in a test of ready biodegradability, it may be assumed that a compound will undergo rapid and ultimate biodegradation in the environment (introduction to the OECD 25 Guidelines for the Testing of Chemicals, Section 3, Part 1: Principles and Strategies Related to the Testing of Degradation of Organic Chemicals; Adopted: July 2003). An assessment of "inherently biodegradable" can also be made using the OECD Method 301F, although with a different pass criterion. More specifically, the pass criterion is 60 % of theoretical oxygen demand and/or chemical oxygen demand. This pass value can be reached after the 28-day period of the test, which is usually extended to 60 days. No 10-day window applies.

in some cases, the test compound or composition involved in the methods of this disclosure is a readily biodegradable compound or composition. In some cases, the test compound or composition involved in the methods of this disclosure is an inherently biodegradable compound or composition.

In an aspect, there is provided a method for identifying an olfactory receptor that is capable of binding a target ligand, said method comprising:

a) providing a library as described herein;
b) optionally, obtaining a diverse repertoire of olfactory receptor proteins or cells expressing olfactory receptor proteins from said library;
c) contacting the diverse repertoire of olfactory receptor proteins or cells expressing olfactory receptor proteins with the target ligand; and
d) identifying an olfactory receptor that is activated by the target ligand.

All of the methods described herein involve detection of activation of an olfactory receptor. It is understood that detecting activation of an olfactory receptor may mean to measure the level of activation of an olfactory receptor. Hence, throughout this disclosure, "detecting activation" may be replaced with "measuring the level of activation" or similar expressions. Means and methods for detecting activation of olfactory receptors are commonly available in the art.

For example, and as described in detail in the experimental section herein, detecting activation of olfactory receptors may involve the co-transfection or use of a luciferase gene operably linked to a cAMP-responsive promoter/-element (Saito et al. (2004) Cell 119(5): 679-691, incorporated herein by reference in its entirety), which is used as a reporter gene. The activation of the olfactory receptor and subsequent increase in intracellular cAMP results in expression of luciferase. Cleavage of luciferin by luciferase results in the emission of light which can then be detected and quantified.

To measure and report the (level of) activation of olfactory receptors, for example, the fold-induction of luciferase can be calculated. Typically, the fold-induction is taken relative to a solvent-only control. Usually, a background control without cells but with all other reagents is also taken along, and the value of this control will be subtracted from all measured values to account for background luminescence. For the solvent-only control, cells expressing the OR and the luciferase gene are treated with solvent only (excluding the test compound or composition). All the values of experiments with test compounds and compositions are then divided by the average of these solvent-only control measurements to calculate fold-luciferase induction. Solvent controls and test compounds and compositions that do not cause OR activation will thus obtain a value of 1, indicating no luciferase induction. Values which are significantly above 1 indicate activation of the luciferase gene and thus enhanced cAMP production due to OR activation.

Once a strong ligand is known for a given OR and the concentration of that ligand for maximal OR activation, this ligand (tested at its maximal inducing concentration) can be introduced as a positive control into the experiment, and fold-luciferase can be reported as % activation of the positive control according to the following formula:

$$\% \ activation = \frac{Fold \ luciferase \ activation \ test \ compound - 1}{Fold \ luciferase \ activation \ positive \ control - 1} \times 100$$

Based on this calculation, potency of ligands can then be compared by applying sigmoidal curve fit with the Hill-equation and calculating e.g. EC20% or EC50% values, i.e. concentrations leading to 20% or 50% activation of the OR compared to the positive control.

In experiments without positive controls, e.g. in experiments with a library of receptors, other types of normalisation can be used. Thus, as the dynamic range (maximal efficacy) of different receptors can be quite different, it may be appropriate to use logarithmic values of fold-induction to report the data. Other options are normalisation to the highest fold-induction for each receptor when multiple samples are tested or normalisation to historical values of maximal efficacy for a given OR.

Other reporter genes which can also be coupled to a cAMP-responsive promoter/element include green fluorescent proteins. There are also multiple methods to directly measure the change in intracellular cAMP concentrations based on antibody binding to cAMP. Other methods to detect OR-activation include the coupling of a hybrid G-protein to the OR, whereby the hybrid G-protein activates the release of calcium from intracellular stores. Changes in calcium concentration are then measured either by chemical fluorescent probes sensitive to changed calcium concentrations, or by recombinant fluorescent or luminescent proteins which can sense differences in calcium concentrations.

Further means and methods for detection of activation of an olfactory receptor are known to the skilled person, including: GTPase/GTP binding assays, aequorin-based assays, fluorescence-based assays, membrane depolarization assays, melanophore assays, PKC activation assays, PKA activation assays, kinase assays, among others, for example as described in WO2019/110630 incorporated herein by reference in its entirety. An overview of some methods to detect activation of olfactory receptors in heterologous cells by odorants is given in the review by Veithen et al., 2017, Springer Handbook of Odor Chapter 22.2, Springer International publishing (CH) incorporated herein by reference in its entirety.

The ligand and/or test compound or composition as described herein may be added in an existing culture, or alternatively the culture medium of an existing culture may be replaced by fresh culture medium comprising said ligand. Suitable ligands may be selected from any chemical compound known in the art that is able to activate an olfactory receptor (alternatively referred to as "aroma compounds" or "odorants"), which are discussed in standard handbooks such as Buettner (2017), Springer Handbook of Odor, Springer International publishing (CH), incorporated herein by reference in its entirety. Suitable compounds may also be found in publicly available databases such as "OlfactionBase", available at https://olfab.iiita.ac.in/olfactionbase/ and discussed in Sharma et al. OlfactionBase: a repository to explore odors, odorants, olfactory receptors and odorant-receptor interactions. Nucleic Acids Res. 2022 Jan 7;50(D1):D678-D686.

Non-limiting examples of suitable ligands include esters (e.g. geranyl acetate, methyl formate, methyl acetate, methyl propionate, methyl butyrate, ethyl acetate, ethyl butyrate, isoamyl acetate, pentyl butyrate, pentyl penthanoate, octyl acetate, benzyl acetate, methyl anthranilate, hexyl acetate), linear terpenes (e.g. myrcene, geraniol, nerol, citral, citronellal, citronellol, linalool, nerolidol, ocimene), cyclic terpenes (limonene, camphor, methol, carvone, terpineol, alpha-Ionone, thujone, eucalyptol, jasmine), aromatic compounds (e.g. benzaldehyde, eugenol, isoeugenol, cinnamaldehyde, ethyl maltol, ethyl vanillin, anisole, anethole, estragole, thymol), amines (e.g. trimethylamine, putrescine, cadaverine, pyridine, indole, skatole), alcohols (e.g. furaneol, 1-hexanol, ethanol), aldehydes (e.g. acetaldehyde, hexanal, furfural, hexyl cinnamaldehyde, isovaleraldehyde, anisic aldehyde, cuminaldehyde), ketones (e.g. dihydrojasmone, 2-acetyl-1-pyrroline, 6-acetyl-2,3,4,5-tetrahydropyridine), lactones (e.g. gamma-decalactone, gamma-nonalactone, delta-octalactone, jasmine lactone, massoia lactone, wine lactone, sotolon), thiols (e.g. thioacetone, allyl thiol, ethanethiol, 2-methyl-2-propanethiol, butane-1-thiol, mercaptan, methanethiol, furan-2-ylmethanethiol, benzyl mercaptan), musks (e.g. nitromusks, polycyclic musks, macrocyclic musks, linear/alicyclic musks, musk ketone, musk ambrette, musk moskene, musk tibetene, musk xylene), cresols (e.g. vanilla cresol (ultravanil)), propenyl guaethol (vanitrope), carboxylic acids, and the like.

Preferred ligands include ligands with a musky, woody, lilly-of-the valley, floral, green, balsamic, spicy or fruity note. "Musky", "Woody", "lilly-of-the valley", "floral", "green", "balsamic", "spicy" and "fruity" are accepted terms of art in the context of odorant molecules. Ligands having musky, woody, lilly-of-the valley, floral, green, balsamic, spicy and/or fruity notes can be identified by the skilled person in publicly available databases such as "OlfactionBase", available at https://olfab.iiita.ac.in/olfactionbase/ and discussed in Sharma et al. OlfactionBase: a repository to explore odors, odorants, olfactory receptors and odorant-receptor interactions. Nucleic Acids Res. 2022 Jan 7;50(D1):D678-D686, incorporated herein by reference in its entirety. Relevant musk compounds are also described in WO2019/11630, incorporated herein by reference in its entirety.

Further examples of preferred ligands are ambermax, para-cresol, menthol, (S)-menthol, menthone, mahonial, nympheal, linalool, androstenone, androstenol, cyclopentanethiol, hedione, hedione HC, ambrofix, calone, 4-ethyloctanoic acid, galaxolide, galaxolide S, ethyl vanillin, beta-ionone, ambrettolide, 3-methyl-3-hydroxy-hexanoic acid, 3-methyl-2-hexenoic acid, nonanoic acid, decanoic acid, undecanoic acid, ethyl 3-mercaptopropionate, diallyl disulfide, benzothiazole, 2-methyl-3-tetrahydrofuranethiol, muscone, dipropyl disulfide, musk ketone, arborone, georgywood, iso E super, cedrol, mercapto-8-p-menthane-3-one, 2-napthalenethiol, 3-(methylthio)propionaldehyde, 1,3-propanedithiol, benzothiazole, allyl sulfide, allyl mercaptan, hydroxy ethyl methyl thiazol, dimethyl trisulfide, thioglycolic acid, 3-mercapto-2-pentanone, 2-((methyldisulfanyl)methyl furan, bis(methylthio)methane, ethyl- 2-mercaptopropionate, methyl thio butyrate, 3-mercapto-3-methylbutyl formate, methyl 3-mercaptopropionate, butyl 3-mercaptopropionate, 3-mercaptopropionic acid, dimethyl disulfide, 2-mercaptopropionic acid, 2-methyl-3-furanthiol, benzyl mercaptan, 2-mercapto-2-methyl-1 pentanol, allyl isothiocyanate, 2-mercapto butanone, 2-heptane-thiol, 2-methyl-3-tetrahydrofuranthiol, cis-2-isobutyl-4,5-dimethyl-2,5-dihydrothiazole, 3-methyl-3-sulfanylhexan-1-ol, (rac)-3-mercapto-2-methyl-1-pentanol, 1-hexanthiol, cyclolpentanethiol, 2-methyl-2-propanethiol, 2-methyl-3-(methyldithio)furan, 2-methyl-3-buten-1-ol, sodium methanethiolate, sodium hydrosulfide hydrate, 4-methoxy-2-methylpetane-2-thiol, blackcurrant body, furfuryl mercaptan, anjeruk, 3-mercaptohexyl acetate, methoxy methyl butanethiol, 3-mercaptohexanol, dimethyl sulfide, acetyl thiazole, mercapto-8 methene-1-para, 2-methyl-3-sulfanyl-pentanol, (E,S)-3,7-dimethylnon-6-en-1-ol, 3-Mercapto-3-methylhexan-1-ol, 7-(3-Methylbutyl)-benzo[b][1,4]dioxepin-3-one, benzyl salicylate, delta-damascone, ethyl cyclohexanecarboxylate, geosmin, 3-(4-isobutyl-2-methylphenyl)propanal, patchoulol, peonile, rotundone, heliotropine, methyl salicylate, galbanone, javanol, timberol, trans 2, cis 6-nonadienal, esterly, cascalone, azurone, rosabloom, rosyfolia, β-Ionone, sotolone, 3-methyl-3-hydroxyhexanoic acid, 2-methylundecanoic acid.

The skilled person understands that the amount of ligand required for the activation of an olfactory receptor may vary depending on the olfactory receptor and the ligand's ability to physically associate with said olfactory receptor. A ligand may be considered to be "of' a given olfactory receptor (i.e. specific for that receptor) if it can physically associate (i.e. bind to) with said receptor at an $EC_{50}$ value of 1 mM or less, typically at an $EC_{50}$ value between 1 nM and 1 mM. $EC_{50}$ in the context of ligands of olfactory receptors refers to that concentration of a ligand at which a given activation of an olfactory receptor is 50% of the maximum for that olfactory receptor, measurable using methods as described elsewhere herein.


General information

Unless stated otherwise, all technical and scientific terms used herein have the same meaning as customarily and ordinarily understood by a person of ordinary skill in the art to which this invention belongs, and read in view of this disclosure.


*Sequence identity*

it is to be understood that each nucleic acid molecule or protein fragment or polypeptide or peptide or derived peptide or construct as identified herein by a given sequence identity number (SEQ ID NO) is not limited to this specific sequence as disclosed. Each coding sequence as identified herein encodes a given protein fragment or polypeptide or peptide or derived peptide or construct or is itself a protein fragment or polypeptide or construct or peptide or derived peptide.

Throughout this application, each time one refers to a specific nucleotide sequence SEQ ID NO (take SEQ ID NO: X as example) encoding a given protein fragment or polypeptide or peptide or derived peptide, one may replace it by:

> i. a nucleotide sequence that has at least 50%, 55%, 60%, 65%, 70%, 75%, 80%, 85%, 90%, 95%, 97% or 99% sequence identity with SEQ ID NO: X;
> ii. a nucleotide sequence the sequence of which differs from the sequence of a nucleic acid molecule of (i) due to the degeneracy of the genetic code; or
> iii. a nucleotide sequence that encodes an amino acid sequence that has at least 50%, 55%, 60%, 65%, 70%, 75%, 80%, 85%, 90%, 95%, 97% or 99% amino acid identity or similarity with an amino acid sequence encoded by a nucleotide sequence SEQ ID NO: X.

A preferred level of sequence identity is 70%. Another preferred level of sequence identity or similarity is 75%. Another preferred level of sequence identity or similarity is 80%. Another preferred level of sequence identity or similarity is 85%. Another preferred level of sequence identity or similarity is 90%. Another preferred level of sequence identity or similarity is 95%. Another preferred level of sequence identity or similarity is 99%.

Throughout this application, each time one refers to a specific amino acid sequence SEQ ID NO (take SEQ ID NO: Y as example), one may replace it by: a polypeptide represented by an amino acid sequence comprising a sequence that has at least 50%, 55%, 60%, 65%, 70%, 75%, 80%, 85%, 90%, 95%, 97% or 99% sequence identity or similarity with amino acid sequence SEQ ID NO: Y. A preferred level of sequence identity is 70%. Another preferred level of sequence identity or similarity is 75%. Another preferred level of sequence identity or similarity is 80%. Another preferred level of sequence identity or similarity is 85%. Another preferred level of sequence identity or similarity is 90%. Another preferred level of sequence identity or similarity is 95%. Another preferred level of sequence identity or similarity is 99%.

Each nucleotide sequence or amino acid sequence described herein by virtue of its identity or similarity percentage with a given nucleotide sequence or amino acid sequence respectively has in a further preferred embodiment an identity or a similarity of at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% with the given nucleotide or amino acid sequence, respectively.

Each non-coding nucleotide sequence (i.e. of a promoter or of another regulatory region) could be replaced by a nucleotide sequence comprising a nucleotide sequence that has at least 60% sequence identity or similarity with a specific nucleotide sequence SEQ ID NO (take SEQ ID NO: A as example). A preferred nucleotide sequence has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO: A. in a preferred embodiment, such non-coding nucleotide sequence such as a promoter exhibits or exerts at least an activity of such a non-coding nucleotide sequence such as an activity of a promoter as known to a person of skill in the art.

The terms "homology", "sequence identity" and the like are used interchangeably herein. Sequence identity is described herein as a relationship between two or more amino acid (polypeptide or protein) sequences or two or more nucleic acid (polynucleotide) sequences, as determined by comparing the sequences. In a preferred embodiment, sequence identity is calculated based on the full length of two given sequences or on a part thereof, more preferably based on the full length of two given sequences. Part thereof preferably means at least 50%, 60%, 70%, 80%, 90%, or 100% of both sequences. In the art, "identity" also refers to the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in Bioinformatics and the Cell: Modern Computational Approaches in Genomics, Proteomics and transcriptomics, Xia X., Springer International Publishing, New York, 2018; and Bioinformatics: Sequence and Genome Analysis, Mount D., Cold Spring Harbor Laboratory Press, New York, 2004, each incorporated herein by reference.

"Sequence identity" and "sequence similarity" can be determined by alignment of two peptide or two nucleotide sequences using global or local alignment algorithms, depending on the length of the two sequences. Sequences of similar lengths are preferably aligned using a global alignment algorithm (e.g. Needleman-Wunsch) which aligns the sequences optimally over the entire length, while sequences of substantially different lengths are preferably aligned using a local alignment algorithm (e.g. Smith-Waterman). Sequences may then be referred to as "substantially identical" or "essentially similar" when they (when optimally aligned by for example the program EMBOSS needle or EMBOSS water using default parameters) share at least a certain minimal percentage of sequence identity (as described below).

A global alignment is suitably used to determine sequence identity when the two sequences have similar lengths. When sequences have a substantially different overall length, local alignments, such as those using the Smith-Waterman algorithm, are preferred. EMBOSS needle uses the Needleman-Wunsch global alignment algorithm to align two sequences over their entire length (full length), maximizing the number of matches and minimizing the number of gaps. EMBOSS water uses the Smith-Waterman local alignment algorithm. Generally, the EMBOSS needle and EMBOSS water default parameters are used, with a gap open penalty = 10 (nucleotide sequences) / 10 (proteins) and gap extension penalty = 0.5 (nucleotide sequences) / 0.5 (proteins). For nucleotide sequences the default scoring matrix used is DNAfull and for proteins the default scoring matrix is Blosum62 (Henikoff & Henikoff, 1992, PNAS 89, 915-919, incorporated herein by reference).

Alternatively percentage similarity or identity may be determined by searching against public databases, using algorithms such as FASTA, BLAST, etc. Thus, the nucleic acid and protein sequences of some cases of the present disclosure can further be used as a "query sequence" to perform a search against public databases to, for example, identify other family members or related sequences. Such searches can be performed using the BLASTn and BLASTx programs (version 2.0) of Altschul, et al. (1990) J. Mol. Biol. 215:403-10, incorporated herein by reference. BLAST nucleotide searches can be performed with the NBLAST program, score = 100, wordlength = 12 to obtain nucleotide sequences homologous to nucleic acid molecules of the disclosure. BLAST protein searches can be performed with the BLASTx program, score = 50, wordlength = 3 to obtain amino acid sequences homologous to protein molecules of the disclosure. To obtain gapped alignments for comparison purposes, Gapped BLAST can be utilized as described in Altschul et al., (1997) Nucleic Acids Res. 25(17): 3389-3402, incorporated herein by reference. When utilizing BLAST and Gapped BLAST programs, the default parameters of the respective programs (e.g., BLASTx and BLASTn) can be used. See the homepage of the National Center for Biotechnology information accessible on the world wide web at www.ncbi.nlm.nih.gov/.

Optionally, in determining the degree of amino acid similarity, the skilled person may also take into account so-called conservative amino acid substitutions. As used herein, "conservative" amino acid substitutions refer to the interchangeability of residues having similar side chains. Examples of classes of amino acid residues for conservative substitutions are given in the Tables below.

| Acidic Residues | Asp (D) and Glu (E) |
|---|---|
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

Alternative conservative amino acid residue substitution classes :

| 1 | A | S | T |
|---|---|---|---|
| 2 | D | E | |
| 3 | N | Q | |
| 4 | R | K | |
| 5 | I | L | M |
| 6 | F | Y | W |

Alternative physical and functional classifications of amino acid residues:

| Alcohol group-containing residues | S and T |
|---|---|
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P and T |
| Flexible residues | Q, T, K, S, G, P, D, E, and R |

For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to Ser; Arg to Lys; Asn to Gln or His; Asp to Glu; Cys to Ser or Ala; Gln to Asn; Glu to Asp; Gly to Pro; His to Asn or Gln; Ile to Leu or Val; Leu to Ile or Val; Lys to Arg; Gln or Glu; Met to Leu or Ile; Phe to Met, Leu or Tyr; Ser to Thr; Thr to Ser; Trp to Tyr; Tyr to Trp or Phe; and, Val to Ile or Leu. Particularly suitable amino acid substitutions in this disclosure include the substitutioLys for Arg and Arg for Lys.

*Gene or coding nucleotide sequence*

The term "gene" refers to a DNA fragment comprising a region (transcribed region), which is transcribed into an RNA molecule (e.g. an mRNA) in a cell, operably linked to suitable regulatory regions (e.g. a promoter). Coding nucleotide sequences may comprise sequences that are native to the cell, sequences that naturally do not occur in the cell and it may comprise combinations of both.

*Operably linked*

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame. Linking can be accomplished by ligation at convenient restriction sites or at adapters or linkers inserted in lieu thereof, or by gene synthesis.

*Proteins and amino acids*

The terms "protein" or "peptide" or "polypeptide" or "amino acid sequence" are used interchangeably and refer to molecules consisting of a chain of amino acids, without reference to a specific mode of action, size, 3-dimensional structure or origin. In amino acid sequences as described herein, amino acids or "residues" are denoted by three-letter symbols. These three-letter symbols as well as the corresponding one-letter symbols are well known to a person of skill in the art and have the following meaning: A (Ala) is alanine, C (Cys) is cysteine, D (Asp) is aspartic acid, E (Glu) is glutamic acid, F (Phe) is phenylalanine, G (Gly) is glycine, H (His) is histidine, I (Ile) is isoleucine, K (Lys) is lysine, L (Leu) is leucine, M (Met) is methionine, N (Asn) is asparagine, P (Pro) is proline, Q (Gln) is glutamine, R (Arg) is arginine, S (Ser) is serine, T (Thr) is threonine, V (Val) is valine, W (Trp) is tryptophan, Y (Tyr) is tyrosine. A residue may be any proteinogenic amino acid, but also any non-proteinogenic amino acid such as D-amino acids and modified amino acids formed by post-translational modifications, and also any non-natural amino acid. In a preferred embodiment, an amino acid in this disclosure may refer to any one of the 20 standard proteinogenic amino acids (A, C, D, E, F, G, H, I, K, L, M, N, P, Q, R, S, T, V, W, Y).

in this document and in its claims, the verb "to comprise" and its conjugations is used in its non-limiting sense to mean that items following the word are included or contained, but items not specifically mentioned are not excluded. Thus, the terms 'comprising', 'comprises', 'comprised of' and the like as used herein are synonymous with 'including', 'includes' or 'containing', 'contains', and are inclusive or open-ended and do not exclude additional, non-recited members, elements or method steps.

In addition, the verb "to consist" may be replaced by "to consist essentially of" meaning that subject matter as described herein may comprise additional component(s) than the ones specifically identified, said additional component(s) not altering the unique characteristic of this disclosure. In addition, the verb "to consist" may be replaced by "to consist essentially of" meaning that a method as described herein may comprise additional step(s) than the ones specifically identified, said additional step(s) not altering the unique characteristic of this disclosure.

Throughout this disclosure, the term "comprising" may be replaced with the term "consisting essentially of" or "consisting of".

As used herein, the singular forms 'a', 'an', and 'the' include both singular and plural referents unless the context clearly dictates otherwise. As such, the terms "a" (or "an"), "one or more," and "at least one" can be used interchangeably herein.

As used herein, with "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments described herein are capable of operation in other sequences than described or illustrated herein.

The word "about" or "approximately" when used in association with a numerical value (e.g. about 10) preferably means that the value may be the given value (of 10) more or less 1% of the value.

As used herein, the term "and/or" indicates that one or more of the stated cases may occur, alone or in combination with at least one of the stated cases, up to with all of the stated cases.

Various embodiments are described herein. Each embodiment as identified herein may be combined together unless otherwise indicated. Titles, subtitles, headings and the likes are used herein solely for ease of reading and are not intended to limit or restrict the disclosure in any way.

All patent applications, patents, and printed publications cited herein are incorporated herein by reference in the entireties, except for any definitions, subject matter disclaimers or disavowals, and except to the extent that the incorporated material is inconsistent with the express disclosure herein, in which case the language in this disclosure controls.

One skilled in the art will recognize many methods and materials similar or equivalent to those described herein, which could be used in the practice of the present invention. Indeed, the present invention is in no way limited to the methods and materials described.

The present invention is further described by the following examples which should not be construed as limiting the scope of the invention. The generalization of certain aspects and features disclosed in the below examples to the foregoing description is part of this disclosure.

Description of the figures

**Figure 1.** Comparison of wild-type OR5A2 with wild-type C-terminal sequence (SEQ ID NO: 239) and chimeric OR5A2 with the optimized C-terminal sequence RNKEVKDALKRLLKRKCC (SEQ ID NO: 86). Shown is dose-dependent luciferase induction by different ligands.

**Figure 2.** Examples of dose-response analysis of wild-type and chimeric OR7C1 and OR9Q2 with the optimized C-terminal sequence (SEQ ID NO: 86) used to derive detection thresholds and EC50 values as summarized in Table 1.

**Figure 3.** Dose-response analysis of wild-type OR5B12 with wild-type C-terminal sequence (SEQ ID NO: 241) and chimeric OR5B12 with the optimized C-terminal sequence (SEQ ID NO: 86) compared to a variant whereby the wild-type C-terminal sequence was changed towards consensus (SEQ ID NO: 242) by changing the amino acid sequence at two positions to a leucine residue thereby rendering all conserved residues identical to the consensus sequence.

**Figure 4.** Dose-response analysis of wild-type OR5A2 with wild-type C-terminal sequence (SEQ ID NO: 239) and chimeric OR5A2 with the optimized C-terminal sequence (SEQ ID NO: 86) compared to a variant whereby the wild-type C-terminal sequence was changed towards consensus (SEQ ID NO: 240) by changing the amino acid sequence at three positions thereby rendering all conserved residues identical to the consensus sequence.

**Figure 5.** Dose-response analysis of wild-type OR7A17 with wild-type C-terminal sequence (SEQ ID NO: 243) and chimeric OR7A17 with the optimized C-terminal sequence (SEQ ID NO: 86) compared to a variant whereby the wild-type C-terminal sequence was changed towards consensus (SEQ ID NO: 244) by changing the amino acid sequence at five positions thereby rendering all conserved residues identical to the consensus sequence.

**Figure 6.** Dose-response analysis of wild-type and chimeric O8K3 with different ligands. The weaker agonist menthone can only be identified with the more sensitive variant with the optimized C-terminus (SEQ ID NO: 86).

**Figure 7.** Dose-response analysis of wild-type and chimeric O7D4 with different ligands. The weaker agonist androstenol can only be identified with the more sensitive chimeric variant with the optimized C-terminus (SEQ ID NO: 86).

**Figure 8.** Screening of OR2T4 wild-type and OR2T4 with the optimized C-terminus sequence RNKEVKDALKRLLKRKCC (SEQ ID NO: 86) against 52 sulfur compounds

**Figure 9.** Dose-response analysis of wild-type and chimeric OR2T4 with different ligands. No ligand was known for OR2T4 and screening of this wild-type receptor with potential ligands did not lead to de-orphanisation, while testing the chimeric variant with the optimized C-terminus (SEQ ID NO: 86) revealed this receptor to be specifically activated by specific sulfur-containing compounds such as cyclopentanethiol.

**Figure 10.** Screening of OR2T11 wild-type and OR2T11 with the optimized C-terminus sequence RNKEVKDALKRLLKRKCC (SEQ ID NO: 86) against 52 sulfur compounds.

**Figure 11.** Examples of dose-response analysis of wild-type and chimeric OR7C1 with C-terminal sequence (SEQ ID NO: 86) with variants of the optimized C-terminal sequences RNKEVKRAIRKLLKRKCC (SEQ ID NO: 118) and RNKEVKRAIKRLLKRKCR (SEQ ID NO: 121) combining multiple sequence variations described in Example 6 and 7.

**Figure 12.** Activation of OR52E8 by odorant acids present in human sweat. The wild-type was not activated by the acids. Replacing the wild-type with the C-terminal sequence of the functional OR51E1 did not improve expression. However, using the optimized sequence from Example 1 - 5 (SEQ ID NO: 86) led to a strong signal upon addition of 3-methyl-3-hydroxy hexanoic acid.

**Figure 13.** Activation of OR56A4 by acids of different chain length. The wild-type was activated at high concentration by decanoic acid and undecanoic acid, but not by nonanoic acid. Replacing the wild-type with the C-terminal sequence of the functional OR51E1 did reduce activity. However, using the optimized sequence form example 1 - 5 (SEQ ID NO: 86) led to a strong signal and much lower detection threshold upon addition of all three acids.

**Figure 14.** Different chimeric variants of OR8K3: The wild-type C-terminal sequence was replaced by either the optimized sequence of Examples 1 - 5 (SEQ ID NO: 86) or the C-terminal sequence of two functional receptors, namely OR1N2 and OR5AN1. Chimeric receptors with C-terminal sequences from other functional receptors did not provide functional expression. Shown is dose-dependent luciferase induction by different ligands.

**Figure 15.** Chimeric variant of OR5AN1: The wild-type C-terminal sequence was replaced by the C-terminal sequence of the functional receptor OR1N2. The chimeric receptors with C-terminal sequences from OR1N2 did not provide functional expression. Shown is dose-dependent luciferase induction by different ligands.

**Figure 16.** Chimeric variant of OR5A2: The wild-type C-terminal sequence was replaced by the C-terminal sequence of the functional receptor OR1 N2. The chimeric receptors with C-terminal sequences from OR1 N2 did provide functional expression, which is around 100-fold weaker as compared to OR5A2 with the optimized C-terminus sequence RNKEVKDALKRLLKRKCC (SEQ ID NO: 86). Shown is dose-dependent luciferase induction by different ligands.

**Figure 17.** Arborone (left) compared to Iso E Super (OTNE, right) for activation of OR7A17 with the optimized C-terminus RNKEVKDALKRLLKRKCC (SEQ ID NO: 86). Shown is dose-dependent luciferase induction by the mentioned ligands.

**Figure 18.** Chemicals tested for odor threshold in vivo (OTH_mean, ng/L) vs. the EC50% (in $\mu$M) as determined for activation of OR7A17 with the optimized C-terminus RNKEVKDALKRLLKRKCC (SEQ ID NO: 86) in vitro.

**Figure 19.** Screening of OR2M2 with the optimized C-terminus sequence RNKEVKDALKRLLKRKCC (SEQ ID NO: 86) against 52 sulfur compounds in the presence of 30 $\mu$M copper

**Figure 20.** Dose-response analysis of OR2M2 with the optimized C-terminus sequence RNKEVKDALKRLLKRKCC (SEQ ID NO: 86) against the key human malodorant sulfur compound 3-methyl-3-sulfanyl-hexanol and related chemicals in presence and absence of copper.

**Figure 21.** Screening of the full library of class II OR variants (n=408) with 50 $\mu$M Patchoulol. Y-axis shows fold luciferase induction. OR numbering on the X-axis corresponds to the numbering in Table 15.

**Figure 22.** Dose-response analysis of the library hit Patchoulol - OR14J1, comparing the improved OR sequence with the wild-type. The wild-type is inactive and the de-orphanisation of the OR was only possible with the modified sequence.

**Figure 23.** Screening of the library of class I OR variants (n=77) with 500 $\mu$M 3-methyl-2-hexenoic acid. Y-axis shows fold luciferase induction. OR numbering on the X-axis corresponds to the numbering in Table 22.

**Figure 24.** Screening of the full library of class II OR variants (n=408) with three different perfume oils tested at 10 ppm. Y-axis shows fold luciferase induction. On the X-axis OR are depicted which led to an at least 2-fold luciferase induction by at least one of the perfumes. Only activated OR are shown.

**Figure 25.** Geranium oil spiked with different levels of Ambrofix (0.1% - 10%) and tested in a dose-response analysis on OR7A17 with a C-terminal domain of SEQ ID 221 (The full DNA sequences encoding the modified receptor is SEQ ID NO: 611). Y-axis shows fold luciferase induction. On the X-axis concentration of Geranium oil in ppm is indicated.

**Figure 26.** Screening of Iso E super and Ambermax on cells expressing either one or both of OR7A17 or OR7C1 with a C-terminal domain of SEQ ID 221. Y-axis shows fold luciferase induction. On the X-axis concentration in $\mu$M is indicated.

**Figure 27.** Activation of OR10J5 with a C-terminal domain of SEQ ID 86 by the two structural isomer (S,E)-10-hydroxy-4,8-dimethyldec-4-enal and (R,E)-10-hydroxy-4,8-dimethyldec-4-enal. Y-axis shows % luciferase induction of the positive control (Mahonial). On the X-axis concentration of test compounds in micromolar is indicated.

**Figure 28.** Computational principal component analysis schematic for characterization of OR activation vectors.

**Figure 29.** A general computer-implemented method of predicting olfactory properties of a compound or a composition based on OR activation data.

**Figure 30.** Use of a trained autoencoder for prediction of olfactory properties of a compound or a composition based on OR activation data.

**Figure 31.** A general computer-implemented method of predicting OR activation data for a compound or a composition.

**Figure 32.** Use of a Transformer-based model for prediction of OR activation data.

**Figure 33.** A general computer-implemented method of replacing a compound in a composition based on OR activation data.

**Figure 34.** An OR prediction system for executing methods for use of acquired OR activation data and for prediction of OR activation data.

**Examples**

General approach for OR expression

To create an expression plasmid, an OR coding sequence fused at the end of TM7 to the desired C-terminus sequence was synthesized by a DNA synthesis service provider (BioCat GmbH, Germany) and inserted into pcDNA3.1(+) (Invitrogen, MA, USA) downstream of the CMV promoter sequence (SEQ ID NO: 82) using BamHI and NotI restriction sites. All the synthetic OR nucleotide sequences described below in Examples 1-15 further contained at their N-terminus a nucleotide sequence encoding a signal peptide (mmLucy-FLAG-rho, SeQ ID NOs: 80, 81) and at the C-terminus the bgh terminator sequence (SEQ ID NO: 83). All the OR expression plasmids also contain a Kozak sequence (GCCACC) between the BamHI restriction site and the start codon of the signal peptide. These plasmids thus contain a constitutively expressed OR gene.

Expression of the OR genes was in general performed in HEK293T cells which had been stably transfected with a DNA sequence coding for functional variants of the human RTP1S (V227I, SEQ ID NO: 84) and RTP2 (L220R, SEQ ID NO: 85). These cells were seeded into polyethyleneimine coated 96-well plates (100μl/well) at a density of 10,000 cells/well and grown at 37°C in presence of 5% $CO_2$ for 24 h.

0.625 μg of the OR expression plasmids, 1μg of the empty pcDNA3.1(+) vector and 1 μg of pGL4.29 (Promega) harbouring the CRE-inducible luciferase were diluted in 0.25 ml OptiMEM medium (Gibco™, ThermoFisher Scientific, MA, USA). In parallel 15 μl Lipofectamine 2000 (Invitrogen) was diluted in 0.25 ml OptiMEM medium and after 5 min pre-incubation, the two mixtures were combined to prepare the transfection mixture which was incubated for further 25 min.

50 μl of growth medium was replaced with fresh DMEM containing 9% foetal bovine serum (FBS). The pre-incubated transfection mixture was diluted to 5 ml in OptiMEM medium and 50 μl of the diluted mixture was added per well (total final volume 150 μl). Cells were further incubated for 24 h at 37°C in presence of 5% CO2 to allow for DNA uptake and expression of the OR.

Functional expression and response to ligands was tested by removal of 100 μl growth medium and addition of 50 μl of DMEM containing 9% FBS and containing ligands and a maximal amount of 1% DMSO. Cells were stimulated for 4.5 h and then the luciferase signal, which is induced based on OR-dependent cAMP production, was measured.

Example 1: Improved functional expression of modified OR5A2 using the optimized C-terminal sequence RNKEVKDALKRLLKRKCC (SEQ ID NO: 86)

The wild-type OR5A2 and the OR5A2 gene modified with the optimized C-terminal sequence RNKEVKDALKRLLKRKCC (SEQ ID NO: 86) were transfected into HEK293T cells which had a stably integrated DNA sequence coding for functional variants of the human RTP1S (V227I, SEQ ID NO: 84) and RTP2 (L220R, SEQ ID NO: 85).

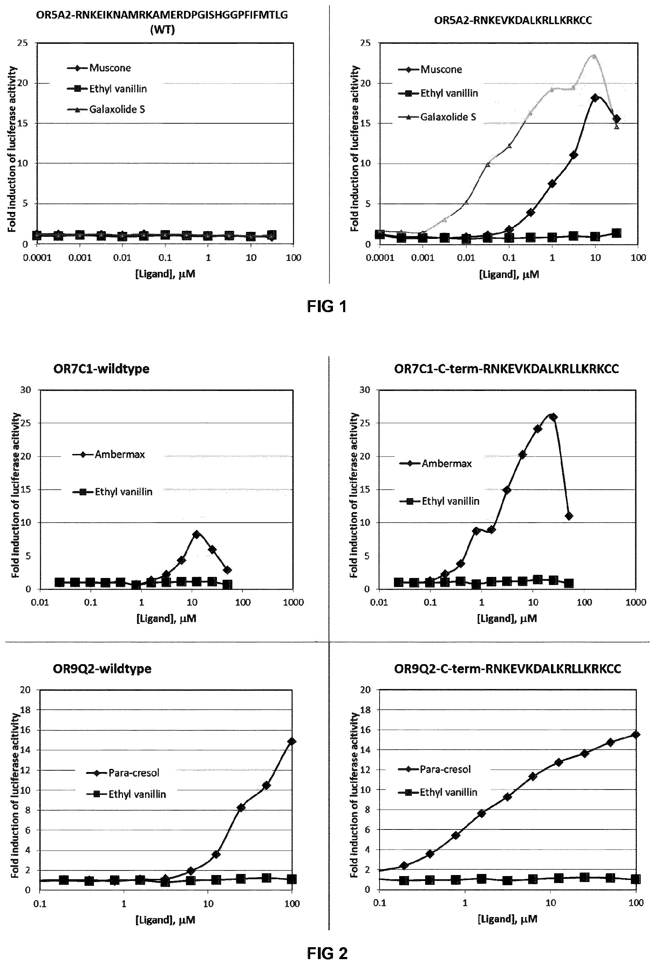

No activation of the wild-type receptor by the polycyclic musk Galaxolide nor by the macrocyclic musk Muscone was detected (Figure 1, left panel). The modified receptor, however, was activated by both musk compounds. As shown in Figure 1 (right panel), activation occurs at low concentration (<0.01 μM for galaxolide and at 1 μM for muscone) and it is specific to musk compounds with no response recorded for ethyl vanillin.

Thus, only by using the modified variant of OR5A2 with the modified C-terminal sequence but not with the wild-type C-terminal sequence, potentially musky-smelling compounds can efficiently be screened against OR5A2 expressed in a cell line along with RTP1S and RTP2 and a Lucy-FLAG-rho-Tag.

Example 2: improved sensitivity of a wide range of modified OR using the optimized C-terminal sequence RNKEVKDALKRLLKRKCC (SEQ ID NO: 86)

Modified versions of different OR genes were generated by exchanging the C-terminal sequence after TM7 by the optimized sequence RNKEVKDALKRLLKRKCC (SEQ ID NO: 86) as shown in example 1 for OR5A2. Cells were transfected either with the wild-type OR gene or with the respective modified version. Transfected cells were stimulated with a cognate ligand of these receptors in a dose response analysis and from the dose-response curve the lowest concentration, at which the ligand triggers a 2-fold induction of the luciferase signal over the background was determined as a measure of the lower detection threshold. In parallel the EC50, i.e. the concentration to reach 50% of the maximal activation (potency) was calculated. Finally, the maximal fold-induction of luciferase over the solvent control (efficacy) was determined and compared between wild-type and modified version.

As can be observed in Table 1 and in the examples shown in Figure 2, for all tested receptors, using the optimized sequence SEQ ID NO: 86, the detection threshold (concentration for 2-fold induction in μM) was clearly reduced, and for many receptors this increase in sensitivity was 10 to more than 100-fold. This increased sensitivity was also observed by comparing the EC50 values, however, EC50 values are also influenced by the maximal induction observed (efficacy), which for several receptors is lower for the wild-type than for the modified version. Thus, for OR52E8, OR2T4 and OR5A2, no induction was observed for the wild-type, while functional expression was achieved with the modified version (all-or-nothing effect). Other receptors (e.g. OR8K3, OR5B12, OR7C1, OR7D4) have (next to much higher detection threshold for the wild-type) also much lower efficacy for the wild-type as compared to the modified version.

Table 1. Improved functional expression of modified OR-genes containing the truncated, optimized C-terminus RNKEVKDALKRLLKRKCC (SEQ ID NO: 86)

| Receptor | | | | Detection threshold; concentration for 2-fold induction (μM) | | | Potency (μM) | | | |
|---|---|---|---|---|---|---|---|---|---|---|
| Gene | NCBI Gene ID / GenBank accession number (sequence source wild-type with nucleotide position ORF) | SEQ ID NO modified sequence | Ligand | wild-type | changed C-term. | Fold-improvement threshold | EC50 wild-type | EC50 changed C-term | Fold-improvement potency | Comments on efficacy |
| OR7C1 | 26664 / NM_001370485.4 (735-1697) | 168 | Ambermax | 2.76 | 0.17 | **16.2** | 6.14 | 2.3 | **2.7** | 3.5-fold lower maximal induction for wild-type |
| OR9Q2 | 219957/ KP290525.1 (1-945) | 169 | Para-Cresol | 6.6 | 0.12 | **53** | 30.7 | 2.8 | **11** | |
| OR8K3 | 219473/ KP290269.1 (1-939) | 170 | Menthol | 160 | 11 | **14.4** | 236 | 115 | **2** | 3-fold lower maximal induction for wild-type |
| OR10J5 | 127285 / NM_001004469.1 (1-930) | 171 | Mahonial | 7.42 | 0.65 | **11.5** | 46 | 20 | **2.3** | |
| OR10J5 | 127285 / NM_001004469.1 (1-930) | 171 | Nympheal | n.i. (>31 μM, at cytotoxic dose) | 5.64 | **>>6** | n.i. (>31 μM, at cytotoxic dose) | 6.2 | **>>6** | Only 1.8-fold induction for WT, but 5.5-fold for changed C-terminus |
| OR1C1 | 26188 / KP290137.1 (1-945) | 172 | Linalool | 34.7 | 16.2 | **2.1** | 379 | 202 | **1.9** | |

| Receptor | | | | Detection threshold; concentration for 2-fold induction ($\mu$M) | | | Potency ($\mu$M) | | | |
|---|---|---|---|---|---|---|---|---|---|---|
| Gene | NCBI Gene ID / GenBank accession number (sequence source wild-type with nucleotide position ORF) | SEQ ID NO modified sequence | Ligand | wild-type | changed C-term. | Fold-improvement threshold | EC50 wild-type | EC50 changed C-term | Fold-improvement potency | Comments on efficacy |
| OR7D4 | 125958 / NM_001005191.3 (404-1342) | 173 | Androstenone | 3.6 | 0.014 | **265** | 8.44 | 0.13 | **66** | 3-fold lower maximal induction for wild-type |
| OR2T4 | 127047 / NM_001004696.2 (19-963) | 174 | Cyclopentanethiol | n.i. (>> 316) | 3.89 | **>>100** | n.i. (>> 316) | 14.6 | **>>2** | No induction wild-type |
| OR5B12 | 390191 / NM_001004733.3 (211-1155) | 175 | Hedione | n.i. >>( 250) | 29 | **>9** | n.i. (>> 250) | 58.3 | **>5** | Induction wild-type only 1.75-fold |
| OR5B12 | 390191 / NM_001004733.3 (211-1155) | 175 | Hedione HC | 96 | 8.8 | **11** | 69 | 18.3 | **3.8** | 4-fold lower maximal induction for wild-type |
| OR7A17 | 26333 / NM_030901.2 (487-1416) | 176 | Ambrofix | 1.9 | 0.1 | **18.4** | 5.6 | 1.3 | **4.2** | 2-fold lower maximal induction for wild-type |
| OR10H5 | 284433 / NM_001004466.2 (68-1015) | 177 | Calone | 60.1 | 24.3 | **2.5** | 41 | 60 | **0.7 *** | 3-fold lower maximal induction for wild-type |

52

| Receptor | | | | Detection threshold; concentration for 2-fold induction ($\mu$M) | | | Potency ($\mu$M) | | | |
|---|---|---|---|---|---|---|---|---|---|---|
| Gene | NCBI Gene ID / GenBank accession number (sequence source wild-type with nucleotide position ORF) | SEQ ID NO modified sequence | Ligand | wild-type | changed C-term. | Fold-improvement threshold | EC50 wild-type | EC50 changed C-term | Fold-improvement potency | Comments on efficacy |
| OR52A5 | 390054 / NM_001005160.3 (107-1057) | 178 | 4-ethyloctanoic acid | >1000 | 101 | >10 | n.a. | 300 | n.a. | 2.5-fold lower maximal induction for wild-type |
| OR5A2 | 219981 / NM_001001954.2 (335-1309) | 179 | Galaxolide | n.i. (>> 31) | 0.0016 | >20000 | n.i. (>> 31) | 0.1 | >300 | No induction wild-type |
| OR5A1 | 219982 / NM 001004728.2 (401-1348) | 199 | Beta-ionone | 19.1 | 3.2 | 5.9 | 5.5 | 3.1 | 1.8 | 4-fold lower maximal induction for wild-type |
| OR1N2 | 138882 / KP290194.1 (43-993) | 180 | Ambrettolide | 1.33 | 0.26 | 5.2 | 2.1 | 1.5 | 1.5 | 1.5 |
| OR52E8 | 390079 / NM_001005168.3 (1-942) | 181 | 3-Methyl-3-Hydroxy-Hexanoic acid | n.i. (>> 316) | 59 | >>5 | n.i. (>> 316) | 69 | >>5 | No induction wild-type |
| OR56A4 | 120793 / NM_001005179.4 (221-1162) | 182 | Undecanoic acid | 43 | 3.2 | 13.5 | 114 | 22 | 5.1 | |
| OR2C1 | 4993 / NM_012368.3 (53-991) | 183 | Ethyl 3-mercaptopropionate | 15.4 | 0.19 | 82.7 | 27.9 | 4.4 | 6.4 | 5-fold lower maximal in-duction for wild-type |

(continued)

| Receptor | | | | Detection threshold; concentration for 2-fold induction (μM) | | | Potency (μM) | | | |
|----------|--|--|--|--|--|--|--|--|--|--|
| Gene | NCBI Gene ID / GenBank accession number (sequence source wild-type with nucleotide position ORF) | SEQ ID NO modified sequence | Ligand | wild-type | changed C-term. | Fold-improvement threshold | EC50 wild-type | EC50 changed C-term | Fold-improvement potency | Comments on efficacy |
| OR2T11 | 127077 / KP290143.1 (1-951) | 184 | Diallyl disulfide | >200 | 2.03 | **>100** | n.i. (>>200) | 6.51 | >>40 | No induction wild-type |
| OR4S2 | 219431 / KP290251.1 (1-936) | 185 | Benzothiazole | 29.4 | 12.5 | **2.4** | 136.8 | 104 | 1.3 | 2-fold lower maximal induction for wild-type |

n.i. no luciferase induction above background at maximal test concentration
n.a. not applicable, not sufficient induction to calculate EC50
* Higher EC50 due to much higher efficacy of optimized C-terminus

Example 3: improved functional expression of modified OR using the optimized C-terminal sequence RNKEVKDALKRLLKRKCC (SEQ ID NO: 86) as compared to OR genes optimized by changing C-terminus towards consensus

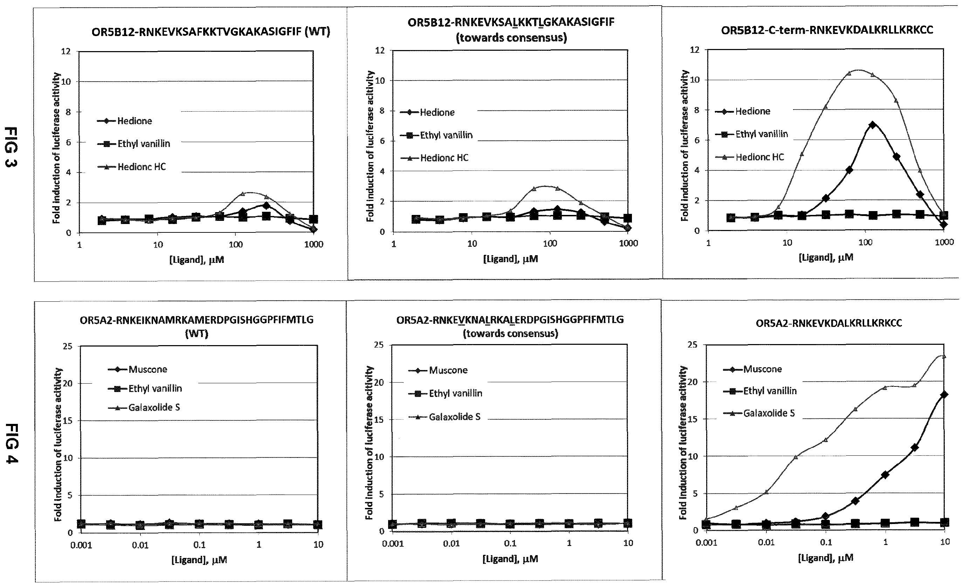

One option investigated before (Kotthoff et al. 2021, see supra) to optimize expression was based on the parent C-terminal sequence of a receptor which was changed towards the consensus sequence by restoring the consensus at single, non-conserved aminoacids, but leaving all the residues in the C-terminal sequence unchanged, for which there is no clear consensus residue from multiple sequence alignment. Thereby, for each receptor a specific C-terminal sequence was derived from the particular parent sequence of that receptor. This approach was compared to the approach with the same optimized, modified C-terminus added as described in Example 1 and 2 added to each receptor. Thus, the C-terminal sequence of OR5B12 was changed by introducing two leucine residues at positions where this amino acid is most common in the consensus sequence. At all other positions, OR5B12 already contains the typical residues of the consensus sequence (Kotthoff et al. 2021, see supra). As shown in Figure 3, changing the sequence of OR5B12 towards consensus had no significant effect whereas the optimized sequence described above led to strong functional expression as compared to the wild-type. Thus, restoring the consensus at conserved amino acids in the parent sequence as done by (Kotthoff et al. 2021, see supra) is not a generally applicable method for improved functional expression, whereas the generation of a modified receptor with the optimized C-terminal sequence as described herein is.

Similarly, for OR5A2 changing the native C-terminus towards consensus by exchanging three amino acids had no effect and, similar to the wild-type, no functional expression was achieved by this approach, whereas adding the optimized C-terminus led to functional expression (Figure 4).

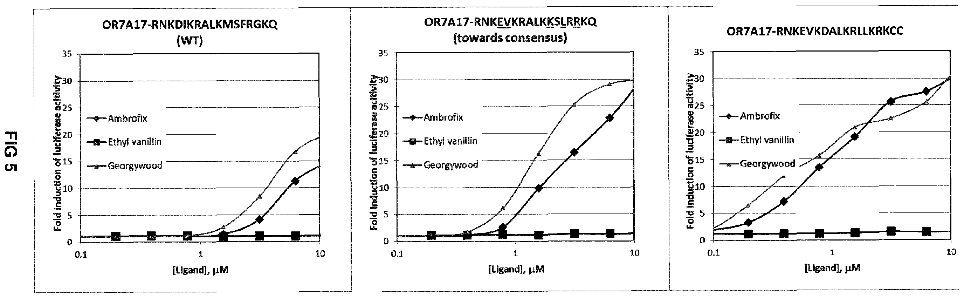

in the case of OR7A17, which already as wild-type has a relatively low detection threshold for two-fold induction of 1.9 $\mu$M for Ambrofix, altering the sequence towards consensus by exchanging 5 amino acids indeed had a positive effect, lowering the detection threshold 3-fold to 0.6 $\mu$M, however a much better effect was achieved using the optimized C-terminal sequence, as the detection threshold could be lowered 18-fold (Table 1 and Figure 5). Thus, optimal improvement of activity is not achieved by the approach of just using the parent C-terminus and mutating it towards consensus, but rather by exchanging the C-terminal sequence with the optimized sequence described herein.

Example 4: More comprehensive ligand spectrum and the discovery of novel ligand-OR pairs by using optimized C-terminus RNKEVKDALKRLLKRKCC (SEQ ID NO: 86)

The ligand spectrum of the modified OR with the optimized C-terminus sequence RNKEVKDALKRLLKRKCC (SEQ ID NO: 86) were further tested with multiple ligands and compared to the OR with the wild-type sequence.

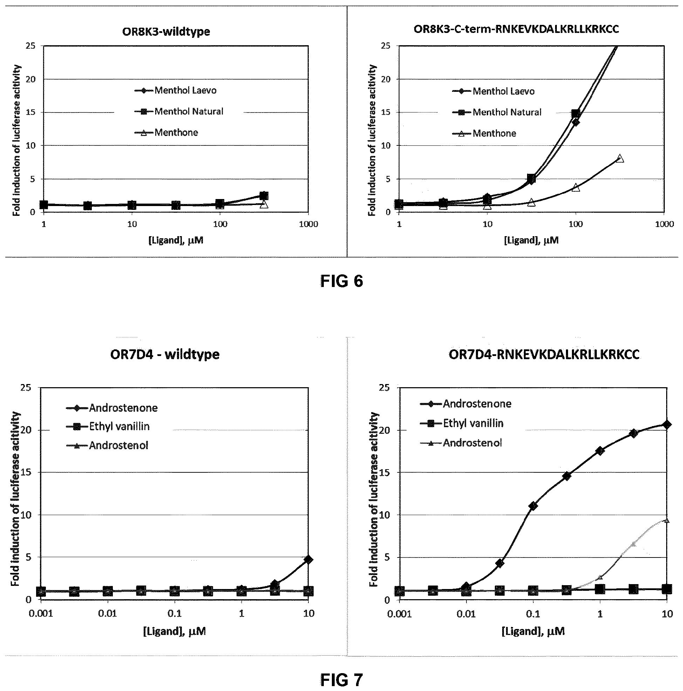

As shown in Figure 6, the wild-type receptor OR8K3 showed only a weak response to menthol (both natural menthol and (S)-menthol ("Menthol Laevo")), while the related molecule menthone is inactive up to 316 $\mu$M. Using the modified receptor variant, a stronger activation by menthol is observed, while in parallel menthone is also activating the receptor, although with a lower potency. Thus, using the improved sensitivity of the assay provided by the modified variant, activation of OR8K3 by a broader set of mint-smelling molecules can be detected.

Similarly, in Figure 7 the dose-response of OR7D4 is shown when tested both with androstenone and the closely related molecule androstenol. A significant activation by androstenone is shown for both variants, while androstenol can only activate the modified version of the receptor: Androstenol is a significantly weaker agonist, but it would appear as lacking activation of OR7D4 if only tested on the poorly expressed wild-type variant. Thus, a better understanding of the receptive space of OR7D4 is possible based on the improved sensitivity of the assay provided by the modified variant.

Example 5: identifying OR-ligand pairs using the optimized C-terminus sequence RNKEVKDALKRLLKRKCC (SEQ ID NO: 86)

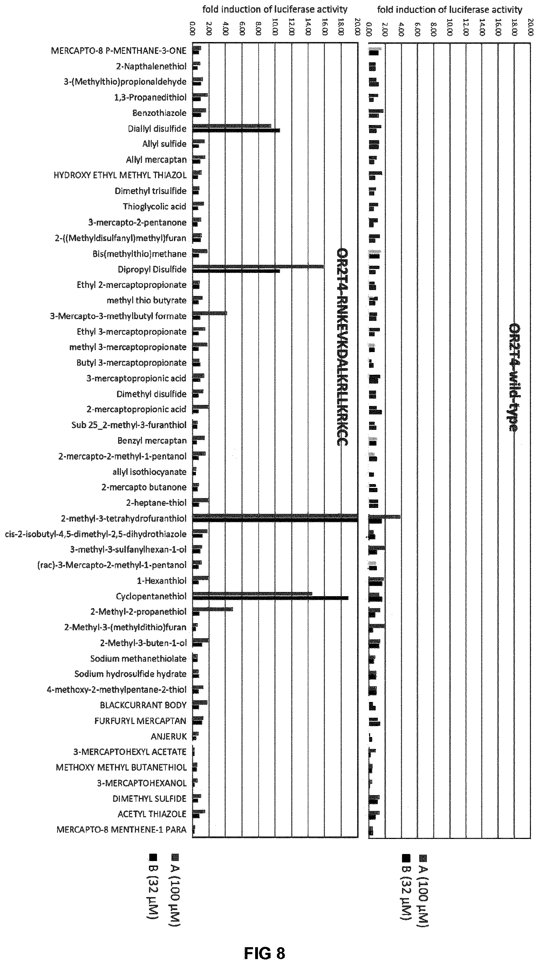

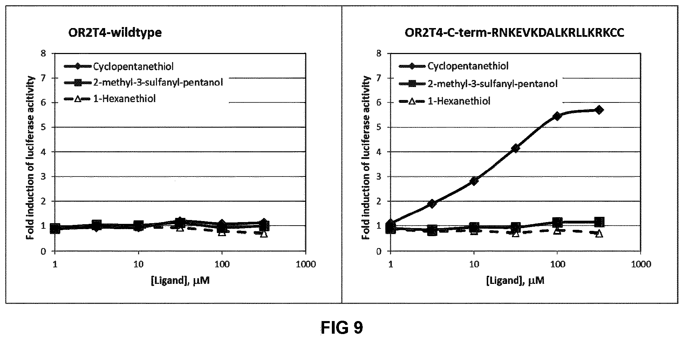

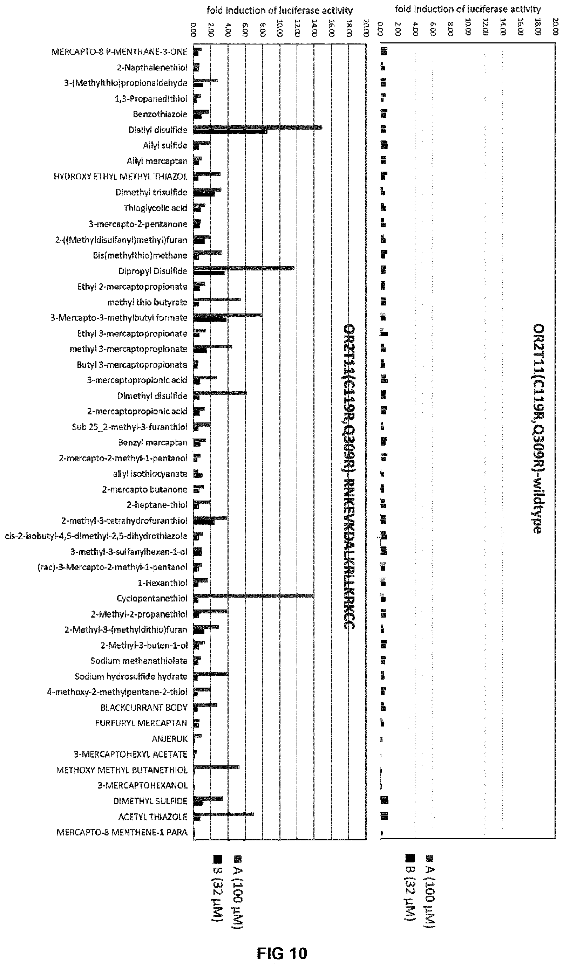

Little is known about the binding of key sulfur odorants to human ORs. Thus, a library of sulfur odorants was screened vs. a selection of potential sulfur compound receptors. As shown in Figure 8, no clear OR-ligand pairs were identified for OR2T4 with the wild-type sequence, whereas with the more sensitive modified variant with the optimized C-terminus diallyl disulfide, dipropyl disulfide, 2-methyl-3-tetrahydrofuranthiol and cyclopentanethiol were identified as good ligands for this receptor. This difference in screening efficacy was also verified by a dose-response analysis using the wild-type and the receptor variant (Figure 9). Similarly, for OR2T11, none of the 52 sulfur compounds was active when tested on the wild-type, whereas the more sensitive modified variant with the optimized C-terminus identified 8 ligands with > 4-fold induction for this receptor (Figure 10). Thus, receptor deorphanisation is facilitated with the optimized C-terminus sequence.

Example 6: Flexibility of the optimized C-terminal sequence: Base substitutions in the C-terminal sequence motif

As shown in Examples 1-5, the optimized C-terminal sequence RNKEVKDALKRLLKRKCC (SEQ ID NO: 86) allows for improved heterologous OR-activation experiments for a large variety of ORs, allowing to de-orphanize ORs, finding novel ligands for de-orphanized ORs, testing at lower ligand concentrations with fewer solubility issues and less cytotoxicity and obtaining higher efficacy for a better signal-to-noise ratio.

To test for possible variants of the sequence RNKEVKDALKRLLKRKCC (SEQ ID NO: 86) leading to similar improved functional expression, different point mutations were introduced into the first 16 amino acids of this sequence (corresponding to RNKEVKDALKRLLKRK, SEQ ID NO: 10) and the variants were fused after the TM7 with OR7C1. As shown in Figure 2, OR7C1 with the C-terminal sequence RNKEVKDALKRLLKRKCC (SEQ ID NO: 86) gives around 10-fold luciferase induction at 1 μM of Ambermax and 20-fold induction at 3.1 μM of Ambermax. Activation of the different modified variants were then compared to the variant with the optimized sequence described above at these two concentrations. Thus, all variants were transfected in HEK293T cells which had been stably transfected with a DNA sequence coding for functional variants of the human RTP1S (V227I, SEQ ID NO: 84) and RTP2 (L220R, SEQ ID NO: 85). Cells were then stimulated with 1 μM and 3.1 μM of Ambermax and the fold-induction of luciferase was compared to the fold-induction in the equal experiment conducted on the standard sequence RNKEVKDALKRLLKRKCC (SEQ ID NO: 86), which was set to 100%, and with the wild-type. The test concentrations were selected to fall into the range of partial activity of the wild-type sequence.

Sequence modifications that gave > 66% of the activation at 1 μM of Ambermax of the sequence RNKEVKDALKRLLKRKCC (SEQ ID NO: 86) were considered active variants, which can be preferentially employed for optimized expression. Sequence modifications that gave 33 - 66% of the activation at 1 μM of Ambermax of the reference sequence were considered partly active variants, which can optionally be employed for optimized expression. As shown in Table 2, while the optimized sequence employed in examples 1-5 is one possible option, other variants with single amino acid substitutions are similarly active and in some cases even provide improved activity.

Table 2. Activation by Ambermax of OR7C1 variants with the optimized C-terminus containing single base substitutions in the first 16 amino acids of the C-terminal motif RNKEVKDALKRLLKRKCC (SEQ ID NO: 86).

| C-terminal Sequence | % of the activation by the optimized sequence RNKEVKDALKRLLKRKCC | |
|---|---|---|
| | **1 μM Ambermax** | **3.1 μM Ambermax** |
| Wild Type | 0.5 | 4.4 |
| RNKEVKDALKRLLKRKCC (SEQ ID NO: 86) | 100 | 100 |
| **Active variants** | | |
| RN**R**EVKDALKRLLKRKCC (K295R) (SEQ ID NO: 87) | 119.1 | 128.7 |
| RNKE**I**KDALKRLLKRKCC (V297I) (SEQ ID NO: 88) | 66.5 | 98.9 |
| RNKE**M**KDALKRLLKRKCC (V297M) (SEQ ID NO: 89) | 76.7 | 67.0 |
| RNKEV**R**DALKRLLKRKCC (K298R) (SEQ ID NO: 90) | 117.4 | 153.9 |
| RNKEVK**K**ALKRLLKRKCC (D299K) (SEQ ID NO: 91) | 140.8 | 98.2 |
| RNKEVK**E**ALKRLLKRKCC (D299E) (SEQ ID NO: 92) | 81.3 | 113.6 |
| RNKEVK**N**ALKRLLKRKCC (D299N) (SEQ ID NO: 93) | 95.3 | 113.5 |
| RNKEVK**R**ALKRLLKRKCC (D299R) (SEQ ID NO: 94) | 176.6 | 176.9 |
| RNKEVK**V**ALKRLLKRKCC (D299V) (SEQ ID NO: 95) | 122.1 | 108.8 |
| RNKEVK**A**ALKRLLKRKCC (D299A) (SEQ ID NO: 96) | 115.3 | 111.0 |
| RNKEVK**Q**ALKRLLKRKCC (D299Q) (SEQ ID NO: 97) | 128.4 | 116.7 |
| RNKEVKDA**V**KRLLKRKCC (L301V) (SEQ ID NO: 98) | 120.1 | 104.6 |
| RNKEVKDA**I**KRLLKRKCC (L301I) (SEQ ID NO: 99) | 174.6 | 161.1 |
| RNKEVKDAL**R**RLLKRKCC (K302R) (SEQ ID NO: 100) | 86.0 | 88.1 |
| RNKEVKDALK**K**LLKRKCC (R303K) (SEQ ID NO: 101) | 86.8 | 67.6 |
| RNKEVKDALKRL**I**KRKCC (L305I) (SEQ ID NO: 102) | 88.6 | 80.8 |
| RNKEVKDALKRL**F**KRKCC (L305F) (SEQ ID NO: 103) | 207.1 | 127.1 |
| RNKEVKDALKRLL**R**RKCC (K306R) (SEQ ID NO: 104) | 96.3 | 92.8 |

(continued)

| Active variants | | |
|---|---|---|
| RNKEVKDALKRLL**K**KKCC (R307K) (SEQ ID NO: 105) | 106.7 | 88.0 |
| RNKEVKDALKRLLKR**R**CC (K308R) (SEQ ID NO: 106) | 104.1 | 93.1 |
| **Partly active variants** | | |
| RNK**D**VKDALKRLLKRKCC (E296D) (SEQ ID NO: 107) | 53.2 | *47.4* |
| RNK**Q**VKDALKRLLKRKCC (E296Q) (SEQ ID NO: 108) | 42.0 | 56.4 |
| RNKE**L**KDALKRLLKRKCC (V297L) (SEQ ID NO: 109) | 43.8 | 81.8 |
| RNKEVK**G**ALKRLLKRKCC (D299G) (SEQ ID NO: 110) | 41.6 | 59.8 |
| RNKEVKDAL**H**RLLKRKCC (K302H) (SEQ ID NO: 111) | 44.4 | 58.8 |
| RNKEVKDALKR**I**LKRKCC (L304I) (SEQ ID NO: 112) | 53.2 | 61.5 |
| RNKEVKDALKRLL**G**RKCC (K306G) (SEQ ID NO: 113) | 63.2 | 96.4 |
| **Examples of inactive variants** | | |
| **K**NKEVKDALKRLLKRKCC (R293K) (SEQ ID NO: 186) | 26.0 | 64.9 |
| R**T**KEVKDALKRLLKRKCC (N294T) (SEQ ID NO: 187) | 3.3 | 6.6 |
| R**Q**KEVKDALKRLLKRKCC (N294Q) (SEQ ID NO: 188) | 28.5 | 59.1 |
| RN**H**EVKDALKRLLKRKCC (K295Q) (SEQ ID NO: 189) | 16.2 | 33.3 |
| RNKEVKD**G**LKRLLKRKCC (A300G) (SEQ ID NO: 190) | 4.6 | 11.2 |
| RNKEVKD**V**LKRLLKRKCC (A300V) (SEQ ID NO: 191) | 3.9 | 3.9 |
| RNKEVKDA**M**KRLLKRKCC (L301M) (SEQ ID NO: 192) | 27.0 | 50.2 |
| RNKEVKDALK**H**LLKRKCC (R303H) (SEQ ID NO: 193) | 12.3 | 24.1 |
| RNKEVKDALKR**VV**KRKCC (L304V; L305V) (SEQ ID NO: 194) | 30.4 | 18.7 |
| RNKEVKDALKRL**R**KRKCC (L305R) (SEQ ID NO: 195) | 14.1 | 26.3 |
| RNKEVKDALKRL**K**KRKCC (L305K) (SEQ ID NO: 196) | 5.7 | 12.4 |
| RNKEVKDALKRLL**E**RKCC (K306E) (SEQ ID NO: 197) | 5.6 | 18.8 |

Example 7: Flexibility of the C-terminal sequence - Base substitutions in the amino acids at position 17 and 18 of the C-terminal sequence motif

To further test for possible variants of the sequence RNKEVKDALKRLLKRKCC (SEQ ID NO: 86) leading to similar benefits, 120 different point mutations were introduced into the optional last 2 amino acids of this sequence (positions 17 and 18) and all variants were fused after the TM7 of OR7C1 . Activation of the different modified variants was then compared to the variant with the optimized sequence described above in Examples 1-5.

Thus, all variants were transfected into HEK293T cells, which had been stably transfected with a DNA sequence coding for functional variants of the human RTP1S (V227I, SEQ ID NO: 84) and RTP2 (L220R, SEQ ID NO: 85). Cells were then stimulated with 1 $\mu$M and 3.1 $\mu$M of Ambermax and the fold-induction of luciferase was compared to fold-induction in the equal experiment conducted on the standard sequence RNKEVKDALKRLLKRKCC (SEQ ID NO: 86), which was set to 100%, and with the wild-type.

As shown in Table 3, there is flexibility in the last two amino acids, with a number of variants being active, but there can be also complete inactivation with the inappropriate amino acids in these positions.

Table 3. Activation by Ambermax of variants of the optimized C-terminus fused to OR7C1: Effect of base substitutions at amino acid positions 17 and 18 of the C-terminal motif RNKEVKDALKRLLKRKCC (SEQ ID NO: 86)

| C-terminal 2 amino acids (3 letter and single letter code) | | % of the activation by the optimized sequence RNKEVKDALKRLLKRKCC | |
|---|---|---|---|
| | | 1 μM Ambermax | 3.1 μM Ambermax |
| Wild Type | | 0.0 | 3.7 |
| Cys Cys | CC | 100 | 100 |
| **Examples of active variants** | | | |
| Ser Ile | SI | 225.3 | 110.4 |
| Tyr Pro | YP | 221.7 | 148.6 |
| Pro Gln | PQ | 211.8 | 130.8 |
| Phe Arg | FR | 181.7 | 128.1 |
| Cys Arg | CR | 173.2 | 179.6 |
| Arg Arg | RR | 167.7 | 77.0 |
| Glu Lys | EK | 153.0 | 81.0 |
| Pro Arg | PR | 142.3 | 97.0 |
| Cys Gly | CG | 142.3 | 126.5 |
| Phe Lys | FK | 141.9 | 141.2 |
| Arg Gly | RG | 139.9 | 128.8 |
| Arg Cys | RC | 134.0 | 184.1 |
| Arg Thr | RT | 133.7 | 79.8 |
| Arg Phe | RF | 130.8 | 146.6 |
| Gly Gly | GG | 128.9 | 103.4 |
| Tyr Arg | YR | 127.8 | 110.4 |
| Arg Stop | R | 127.2 | 116.1 |
| Gly Cys | GC | 126.8 | 112.1 |
| Thr Gly | TG | 122.0 | 59.2 |
| Pro Cys | PC | 120.8 | 156.6 |
| His Pro | HP | 114.6 | 105.9 |
| Pro Stop | P | 113.7 | 83.3 |
| Leu Stop | L | 113.6 | 121.4 |
| Pro Gly | PG | 111.9 | 103.5 |
| Lys Tyr | KY | 110.2 | 63.2 |
| Cys Pro | CP | 109.6 | 130.8 |
| Tyr Tyr | YY | 108.3 | 97.3 |
| Phe Phe | FF | 103.8 | 85.3 |
| Lys Stop | K | 103.4 | 105.7 |
| Gly Stop | G | 101.5 | 109.5 |
| Cys Phe | CF | 100.5 | 165.2 |
| Asn Pro | NP | 100.0 | 91.8 |
| Tyr Stop | Y | 95.8 | 92.5 |
| Tyr Leu | YL | 95.4 | 81.4 |

(continued)

| Examples of active variants | | | |
|---|---|---|---|
| Phe stop | F | 93.7 | 86.1 |
| Ile Cys | IC | 92.6 | 94.4 |
| His Cys | HC | 91.9 | 90.9 |
| Gly Leu | CL | 91.4 | 118.1 |
| Tyr Cys | YC | 91.3 | 136.8 |
| Glu Arg | ER | 85.0 | 101.5 |
| Met Stop | M | 84.7 | 99.7 |
| Trp stop | W | 84.6 | 68.0 |
| Arg Pro | RP | 83.9 | 77.6 |
| Pro Ala | PA | 75.6 | 95.3 |
| Phe Cys | FC | 72.9 | 110.5 |
| Arg Tyr | RY | 72.0 | 77.1 |
| Examples of inactive variants | | | |
| Pro Pro | PP | 36.2 | 49.5 |
| Val Ser | VS | 4.3 | 3.6 |
| Lys Ala | KA | 3.5 | 0.7 |
| Ala Ala | AA | 2.1 | 0.5 |
| Ala Glu | AE | 2.1 | 1.1 |
| Arg Lys | RK | 2.0 | 0.7 |
| His Asp | HN | 1.6 | 1.6 |
| Thr Asp | TN | 1.5 | 0.2 |
| Val Met | VM | 1.3 | 1.3 |
| Ser Gin | SQ | 1.0 | 0.6 |
| Ser Ser | SS | 0.9 | 4.3 |
| Gln Glu | QE | 0.0 | 0.0 |
| Ile Ala | IA | 0.0 | 0.0 |
| Ile Ser | IS | 0.0 | 0.0 |
| Leu Gln | LQ | 0.0 | 0.0 |
| Ser Ala | SA | 0.0 | 0.1 |
| Thr Ala | TA | 0.0 | 0.0 |

Example 8: Flexibility of the C-terminal sequence - Truncations of amino acids at position 17 and 18 of the C-terminal sequence motif

To further test for possible variants of the sequence RNKEVKDALKRLLKRKCC (SEQ ID NO: 86) leading to similar benefits in functional expression, further truncations by one or two amino acids were introduced and the sequence was fused after the TM7 with either OR7C1, OR2T4 or OR5B12. Activation of the different modified variants were then compared to the variant with the optimized sequence described above. For optimal activity, 30 µM of copper was added to the assay with OR2T4 (added as CuCl$_2$).

As shown in Table 4, the sequence motif with the first 16 amino acids of RNKEVKDALKRLLKRKCC (RNKEVKDALKRLLKRK, SEQ ID NO: 10) is sufficient for high functional expression of OR7C1, indicating that the additional amino acids in positions 17 and 18 are optional and do not need to be introduced for functional expression of all ORs. However, omitting these two additional amino acids leads to reduced activity in the case of OR2T4 and complete loss of activity in OR5B12, indicating that it is beneficial to add additional amino acids at position 17 and 18 in case these ORs are employed or if a library of many or all OR is generated as the optional amino acids allow for a more broadly improved expression of different ORs.

Table 4. Activation of variants of the modified OR7C1, OR2T4 and OR5B12 with optimized C-terminus: Effect of truncation of the optional amino acids at positions 17 and 18 of the C-terminal motif RNKEVKDALKRLLKRKCC (SEQ ID NO: 86)

| OR / C-terminal Sequence | % of the activation by the optimized sequence RNKEVKDALKRLLKRKCC | |
|---|---|---|
| | **1 $\mu$M Ambermax** | **3.1 $\mu$M Ambermax** |
| OR7C1 Wild Type | 0 | 3.7 |
| OR7C1 RNKEVKDALKRLLKRKCC (SEQ ID NO: 86) | 100 | 100 |
| OR7C1 RNKEVKDALKRLLKRKC (SEQ ID NO: 198) | 137.7 | 124.2 |
| OR7C1 RNKEVKDALKRLLKRK (SEQ ID NO: 10) | 89.5 | 89.2 |
| | **10 $\mu$M Cyclopentanethiol** | **31 $\mu$M Cyclopentanethiol** |
| OR2T4 Wild Type | 0 | 0 |
| OR2T4 RNKEVKDALKRLLKRKCC (SEQ ID NO: 86) | 100 | 100 |
| OR2T4 RNKEVKDALKRLLKRKC (SEQ ID NO: 198) | 37.7 | 63.5 |
| OR2T4 RNKEVKDALKRLLKRK (SEQ ID NO: 10) | 25.3 | 25.2 |
| | **31 $\mu$M Hedione HC** | **62.5 $\mu$M Hedione HC** |
| OR5B12 Wild Type | 18.3 | 8 |
| OR5B12 RNKEVKDALKRLLKRKCC (SEQ ID NO: 86) | 100 | 100 |
| OR5B12 RNKEVKDALKRLLKRKC (SEQ ID NO: 198) | 0 | 0 |
| OR5B12 RNKEVKDALKRLLKRK (SEQ ID NO: 10) | 0 | 0 |

Example 9: Flexibility of the C-terminal sequence - Addition of additional amino acids at positions 19 - 22

As shown in Example 1 - 5 and in Example 6, two cysteine residues in position 17 and 18 of the optimized C-terminal sequence RNKEVKDALKRLLKRKCC (SEQ ID NO: 86) provide improved functional expression of a wide variety of receptors. As shown in Example 8, these amino acids are optional and not needed for improved expression of all ORs. As shown in Example 7, there is flexibility in these two last amino acids, and particularly replacing one of the Cys-residues with a basic amino acid keeps or even improves activity, whereby particularly adding an arginine as the second residue improved activity for OR7C1. This effect is further exploited with variants elongated with further basic amino acids. For ease of reference, positions corresponding to elongated variants are denoted using the positioning of SEQ ID NO: 86 (which has 18 amino acids) as reference, thus positions 19-22 of SEQ ID NO: 86 as used herein correspond to positions present in the elongated variants. In other words, reference to e.g., position 19 of SEQ ID NO: 86 in a variant means that this variant has been elongated by 1 amino acid etc.

Thus having 1-6 basic amino acids in amino acid positions 17 - 22 improved activity for O7C1, as shown in Table 5. Similarly, having 2-3 basic amino acid residues at positions 18 - 20 improved activity of OR5B12 compared to only two C in position 17 and 18 (Table 6). For OR2T4 having 2-3 basic amino acid residues in positions 18 - 20 did not further increase activity, but it was not detrimental for the activity either (Table 7), indicating that in a library with the same C-terminus added to every OR, these additions are preferred, as they only improve or maintain the activity, but do not compromise the activity for the receptors tested. Furthermore, different amino acids added at position 19 improved activity of OR5B12 (Table 6), which is particularly dependent on the additional amino acids beyond position 16 (See Example 8). The method used and the way of calculating the results is the same as in Examples 6-8.

Table 5. Activation by Ambermax of OR7C1 variants with the optimized C-terminus containing additional basic residues at amino acid positions 18 - 22.

|  | % of the activation by the optimized sequence RNKEVKDALKRLLKRKCC | |
| --- | --- | --- |
| C-terminal Sequence | 1 µM Ambermax | 3.1 µM Ambermax |
| Wild Type | 0.5 | 4.4 |
| RNKEVKDALKRLLKRKCC (SEQ ID NO: 86) | 100 | 100 |
| RNKEVKDALKRLLKRKC**RR** (SEQ ID NO: 133) | 188.9 | 161.9 |
| RNKEVKDALKRLLKRKC**RRR** (SEQ ID NO: 149) | 265.3 | 192.2 |
| RNKEVKDALKRLLKRKC**RKK** (SEQ ID NO: 150) | 196.0 | 159.5 |
| RNKEVKDALKRLLKRKC**RRRR** (SEQ ID NO: 154) | 263.5 | 183.6 |
| RNKEVKDALKRLLKRKC**RRRRR** (SEQ ID NO: 157) | 204.3 | 160.7 |
| RNKEVKDALKRLLKRKC**RRRKK** (SEQ ID NO: 158) | 168.8 | 122.5 |

Table 6. Activation by Hedione HC of OR5B12 variants with the optimized C-terminus containing additional residues at amino acid positions 17-20

|  | % of the activation by the optimized sequence RNKEVKDALKRLLKRKCC | |
| --- | --- | --- |
| C-terminal Sequence | 31 µM Hedione HC | 62.5 µM Hedione HC |
| Wild Type | 18.3 | 8.0 |
| RNKEVKDALKRLLKRKCC (SEQ ID NO: 86) | 100 | 100 |
| RNKEVKDALKRLLKRKCC**C** (SEQ ID NO: 134) | 168.91 | 121.37 |
| RNKEVKDALKRLLKRKCC**F** (SEQ ID NO: 135) | 207.32 | 127.22 |
| RNKEVKDALKRLLKRKCC**L** (SEQ ID NO: 136) | 533.84 | 189.64 |
| RNKEVKDALKRLLKRKCC**M** (SEQ ID NO: 137) | 177.47 | 182.76 |
| RNKEVKDALKRLLKRKCC**S** (SEQ ID NO: 138) | 176.99 | 75.63 |
| RNKEVKDALKRLLKRKCC**P** (SEQ ID NO: 139) | 98.68 | 98.17 |
| RNKEVKDALKRLLKRKCC**A** (SEQ ID NO: 140) | 230.04 | 143.71 |
| RNKEVKDALKRLLKRKCC**Y** (SEQ ID NO: 141) | 298.48 | 274.09 |
| RNKEVKDALKRLLKRKCC**H** (SEQ ID NO: 142) | 142.62 | 129.19 |
| RNKEVKDALKRLLKRKCC**N** (SEQ ID NO: 143) | 137.13 | 91.94 |
| RNKEVKDALKRLLKRKCC**D** (SEQ ID NO: 144) | 86.54 | 56.54 |
| RNKEVKDALKRLLKRKCC**K** (SEQ ID NO: 145) | 280.71 | 237.30 |
| RNKEVKDALKRLLKRKCC**R** (SEQ ID NO: 146) | 619.88 | 248.52 |
| RNKEVKDALKRLLKRKCC**G** (SEQ ID NO: 147) | 394.57 | 300.08 |
| RNKEVKDALKRLLKRKC**RR** (SEQ ID NO: 148) | 181.2 | 189.0 |

(continued)

| C-terminal Sequence | % of the activation by the optimized sequence RNKEVKDALKRLLKRKCC | |
|---|---|---|
| | 31 μM Hedione HC | 62.5 μM Hedione HC |
| RNKEVKDALKRLLKRKC**RRR** (SEQ ID NO: 149) | 254.8 | 231.7 |
| RNKEVKDALKRLLKRKC**RKK** (SEQ ID NO: 150) | 182.3 | 145.7 |
| RNKEVKDALKRLLKRKCC**RR** (SEQ ID NO: 151) | 240.6 | 229.5 |
| RNKEVKDALKRLLKRKCC**RRR** (SEQ ID NO: 156) | 250.7 | 233.4 |
| RNKEVKDALKRLLKRKCC**RRRR** (SEQ ID NO: 219) | 244.3 | 239.6 |

Table 7. Activation by cyclopentanethiol of OR2T4 variants with the optimized C-terminus containing additional basic residues at amino acid positions 17 - 20.

| C-terminal Sequence | % of the activation by the optimized sequence RNKEVKDALKRLLKRKCC | |
|---|---|---|
| | 4 μM 2-methyl-3-tetrah yd rofu ran eth iol | 8 μM 2-methyl-3-tetrahyd rofu raneth iol |
| Wild Type | 18.3 | 8.0 |
| RNKEVKDALKRLLKRKCC (SEQ ID NO: 86) | 100 | 100 |
| RNKEVKDALKRLLKRKC**RR** (SEQ ID NO: 133) | 101.4 | 110.0 |
| RNKEVKDALKRLLKRKC**RRR** (SEQ ID NO: 149) | 108.0 | 135.7 |
| RNKEVKDALKRLLKRKC**RKK** (SEQ ID NO: 150) | 94.8 | 96.5 |
| * For optimal activity, 30 μM of copper was added to the assay with OR2T4 (added as CuCl$_2$). | | |

Example 10: Flexibility of the C-terminal sequence: Combinations of functional base substitutions

As shown in Examples 6-9, next to the optimized sequence of Examples 1-5, functional variants were identified at single amino acid positions within the first 16-amino acids of RNKEVKDALKRLLKRKCC (SEQ ID NO: 86), corresponding to RNKEVKDALKRLLKRK (SEQ ID NO: 10) and within the optional additional C-terminal cysteine residues at positions 17-22. it is here further demonstrated that these functional variants can be combined with each other, providing many different functional variants of the C-terminus and in some combinations even variants with further improved activity as compared to SEQ ID NO: 86.

Table 8 lists variants of the C-terminal sequence described in Examples 6, 7 and 9 combined into the same C-terminal sequence and fused to OR7C1. The method used and the way of calculating the results is the same as in Examples 6 -8.

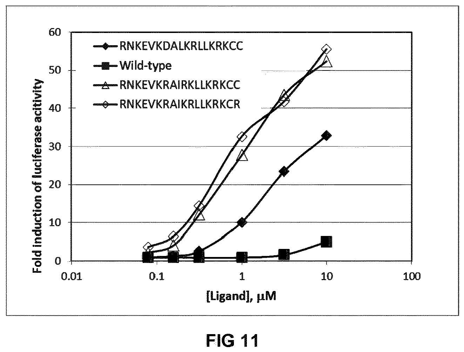

As is evident from the results, the functional variants combined in the same C-terminal sequence gave all functional combinations, and by combining variants with enhanced functionality, even further enhanced variants of the C-terminal sequence were obtained. Next to the improved response at two specific screening concentrations as shown in Table 8, this improvement is also obvious from a dose response analysis as shown in a representative example in Figure 11.

Table 8. Activation by Ambermax of OR7C1 variants with the optimized C-terminus containing multiple base substitutions selected from the active variants identified in examples 6, 7 and 9. Variant residues as compared to SEQ ID NO: 86 are in bold and underlined.

| C-terminal Sequence | % of the activation by the optimized sequence RNKEVKDALKRLLKRKCC | |
|---|---|---|
| | 1 μM Ambermax | 3.1 μM Ambermax |
| Wild Type | 0.5 | 4.4 |
| RNKEVKDALKRLLKRKCC (SEQ ID NO: 86) | 100 | 100 |

(continued)

| Active variants | | |
|---|---|---|
| RNKEVK**RAI**KRLLKRKCC (SEQ ID NO: 114) | 262.4 | 164.0 |
| RNKEVK**RAI**KRL**F**KRKCC (SEQ ID NO: 116)<br>*Complete OR7C1 modified sequence included as SEQ ID NO: 247* | 393.9 | 195.4 |
| RNKEVK**KAI**KRL**F**KRKCC (SEQ ID NO: 117) | 310.7 | 180.2 |
| RNKEVK**RAI RK**LLKRKCC (SEQ ID NO: 118) | 288.9 | 186.1 |
| RNKEVK**RAI**KRLLKRKC**R** (SEQ ID NO: 121) | 346.4 | 189.0 |
| RNKEVK**KAI**KRLLKRKC**R** (SEQ ID NO: 122) | 258.8 | 155.8 |
| RNKEVKDAL**RK**LLKRKCC (SEQ ID NO: 119) | 157.1 | 101.4 |
| RNKEVKDALKRLL**RRR**CC (SEQ ID NO: 120) | 90.4 | 101.5 |
| RNKEVK**R**ALKRLLKRK**RR** (SEQ ID NO: 123) | 312.9 | 252.4 |
| RNKEVK**R**ALKRLLKRK**YP** (SEQ ID NO: 124) | 239.7 | 140.9 |
| RNKEVK**R**ALKRLLKRK**RF** (SEQ ID NO: 125) | 254.2 | 181.8 |
| RNKEVK**R**ALKRLLKRK**FK** (SEQ ID NO: 126) | 280.9 | 174.8 |
| RNKEVK**K**ALKRLLKRK**RR** (SEQ ID NO: 127) | 308.5 | 189.5 |
| RNKEVK**K**ALKRLLKRK**YP** (SEQ ID NO: 128) | 206.9 | 144.4 |
| RNKEVK**K**ALKRLLKRK**RF** (SEQ ID NO: 129) | 294.8 | 187.8 |
| RNKEVK**K**ALKRLLKRK**FK** (SEQ ID NO: 130) | 286.4 | 185.9 |
| RNKEVK**KAI**KRL**F**KRKCC**RRR** (SEQ ID NO: 221)<br>*Complete OR7C1 modified sequence included as SEQ ID NO: 248* | 498.6 | 298.3 |

Example 11: Flexibility of the C-terminal sequence: Testing functional variants with different parent receptors

As shown in Example 2, the sequence RNKEVKDALKRLLKRKCC (SEQ ID NO: 86) is functionally improving the response of a multitude of receptors. As further shown in Examples 6-10, functional variants of the optimized C-terminal sequence of Examples 1-5 could be identified, which are still active or even have improved activity when tested with OR7C1 . It was further tested for functional variants and especially combinations of variants from Examples 6-10 whether they are also broadly applicable to different ORs.

Variants of the sequence RNKEVKDALKRLLKRKCC (SEQ ID NO: 86) were fused to OR5B12 and transfected in HEK293T cells which had been stably transfected with a DNA sequence coding for functional variants of the human RTP1S (V227I, SEQ ID NO: 84) and RTP2 (L220R, SEQ ID NO: 85). Cells were then stimulated with 31 µM and 62.5 µM of Hedione HC and the fold-induction of luciferase was compared to the fold-induction in the equal experiment conducted on the receptor with the standard sequence RNKEVKDALKRLLKRKCC (SEQ ID NO: 86), which was set to 100%, and with the wild-type.

As shown in Table 9, the combinations of variants identified to maintain or improve expression of OR7C1 (Example 10) also significantly further improved the expression of OR5B12 proving that the identified variants can be generalized and applied to other OR.

Table 9. Activation by Hedione HC of OR5B12 variants with the optimized C-terminus containing multiple base substitutions selected from the variants identified in Examples 6-9.

| | % of the activation by the optimized sequence RNKEVKDALKRLLKRKCC | |
|---|---|---|
| **C-terminal Sequence** | **31 µM Hedione HC** | **62.5 µM Hedione HC** |
| Wild Type | 18.3 | 8.0 |
| RNKEVKDALKRLLKRKCC (SEQ ID NO: 86) | 100 | 100 |
| RNKEVK**RAI**KRLLKRKC**R** (SEQ ID NO: 121) | 342.8 | 242.1 |

(continued)

| C-terminal Sequence | % of the activation by the optimized sequence RNKEVKDALKRLLKRKCC | |
|---|---|---|
| | 31 μM Hedione HC | 62.5 μM Hedione HC |
| RNKEVK**KAI**KRLLKRKC**R** (SEQ ID NO: 122) | 331.3 | 222.3 |
| RNKEVK**RAIRK**LLKRKCC (SEQ ID NO: 118) | 126.2 | 136.1 |
| RNKEVK**RAI**KRLLKRKCC (SEQ ID NO: 114) | 317.8 | 195.1 |
| RNKEVK**KAI**KRLLKRKCC (SEQ ID NO: 115) | 349.2 | 242.5 |
| RNKEVK**RAI**KRL**F**KRKCC (SEQ ID NO: 116) *Complete OR5B12 modified sequence included as SEQ ID NO: 251* | 433.2 | 303.9 |
| RNKEVK**KAI**KRL**F**KRKCC (SEQ ID NO: 117) | 435.4 | 282.5 |
| RNKEVK**KAI**KRLLKRK**CCRRR** (SEQ ID NO: 220) | 1092.9 | 922.2 |
| RNKEVK**KAI**KRL**F**KRK**CCRRR** (SEQ ID NO: 221) *Complete OR5B12 modified sequence included as SEQ ID NO: 252* | 1032.2 | 676.0 |

Similarly, variants of the sequence RNKEVKDALKRLLKRKCC (SEQ ID NO: 86) were fused to OR2T4 and transfected in HEK293T cells which had been stably transfected with a DNA sequence coding for functional variants of the human RTP1S (V227I, SEQ ID NO: 84) and RTP2 (L220R, SEQ ID NO: 85). Cells were then stimulated with 4 μM and 8 μM of 2-methyl-3-tetrahydrofuranethiol and the fold-induction of luciferase was compared to the fold-induction in the equal experiment conducted on the standard sequence RNKEVKDALKRLLKRKCC (SEQ ID NO: 86), which was set to 100%, and with the wild-type.

As shown in Table 10 the combinations of variants identified to maintain or improve expression of OR7C1 (Example 9) were all active when tested with OR2T4: For this receptor they did not further improve the functional activity; however, they were not detrimental for the activity either, indicating that in a library with the same C-terminus added to every OR, these variants may be still be preferred, as they only improve or maintain the activity, but do not compromise the activity of the receptors.

Table 10. Activation by 2-methyl-3-tetrahydrofuranethiol of OR2T4 variants with the optimized C-terminus containing multiple base substitutions selected from the variants identified in Examples 6-9.

| | % of the activation by the optimized sequence RNKEVKDALKRLLKRKCC | |
|---|---|---|
| | 4 μM 2-methyl-3-tetrahydrofuranethiol | 8 μM 2-methyl-3-tetrahydrofuranethiol |
| Wild Type | 18.3 | 8.0 |
| RNKEVKDALKRLLKRKCC (SEQ ID NO: 86) | 100 | 100 |
| RNKEVK**RAI**KRLLKRKCC (SEQ ID NO: 114) | 108.8 | 116.6 |
| RNKEVK**KAI**KRLLKRKCC (SEQ ID NO: 115) | 103.1 | 115.2 |
| RNKEVK**RAIRK**LLKRKCC (SEQ ID NO: 118) | 80.6 | 98.4 |
| RNKEVK**RAI**KRLLKRKC**R** (SEQ ID NO: 121) | 139.9 | 150.6 |
| RNKEVK**KAI**KRLLKRKC**R** (SEQ ID NO: 122) | 97.3 | 102.0 |
| RNKEVK**RAI**KRL**F**KRKCC (SEQ ID NO: 116) *Complete OR2T4 modified sequence included as SEQ ID NO: 249* | 63.3 | 77.0 |
| RNKEVK**KAI**KRL**F**KRKCC (SEQ ID NO: 117) | 96.2 | 88.2 |
| RNKEVK**KAI**KRLLKRKCC**RRR** (SEQ ID NO: 220) | 76.6 | 61.9 |

(continued)

| | % of the activation by the optimized sequence RNKEVKDALKRLLKRKCC | |
| --- | --- | --- |
| | 4 μM 2-methyl-3-tetrahydrofuranethiol | 8 μM 2-methyl-3-tetrahydrofuranethiol |
| RNKEVK**KA**IKRL**F**KRKCC**RRR** (SEQ ID NO: 221) *Complete OR2T4 modified sequence included as SEQ ID NO: 250* | 103.1 | 130.4 |

A variant of the sequence motif RNKEVKDALKRLLKRKCC was further also tested with OR5A2 as shown in Table 11. Similar to OR2T4 the identified variant was also fully functional when applied to OR5A2, although it did not further enhance activity (which is already very high with induction by Muscone starting at 0.03 μM).

Table 11. Dose-dependent induction OR5A2 with two different variants of the C-terminal sequence motif (Shown is fold-induction of luciferase)

| | Concentration of muscone (μM) | | | | | | |
| --- | --- | --- | --- | --- | --- | --- | --- |
| OR5A2 | 0.01 | 0.03 | 0.10 | 0.32 | 1.00 | 3.16 | 10.00 |
| RNKEVKDALKRLLKRKCC SEQ ID NO: 86) | 1.07 | 1.48 | 3.57 | 10.02 | 21.98 | 31.84 | 33.98 |
| RNKEVK**KA**IKRL**F**KRKCC (SEQ ID NO: 117) | 0.96 | 1.39 | 3.49 | 9.33 | 19.44 | 27.31 | 30.34 |

Example 12: Replacing the C-terminal sequence in Class I olfactory receptors with the optimal C-terminal sequence originally derived from Class II OR

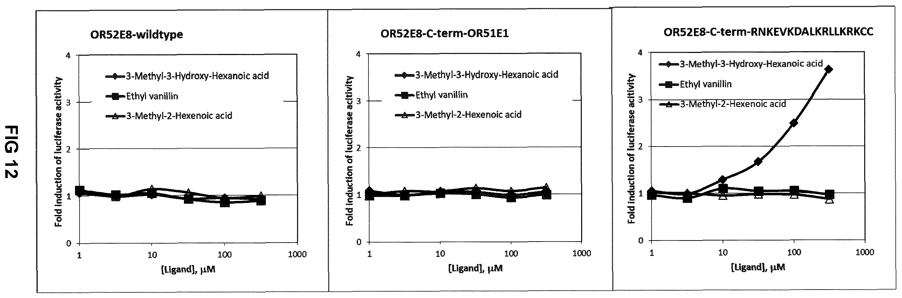

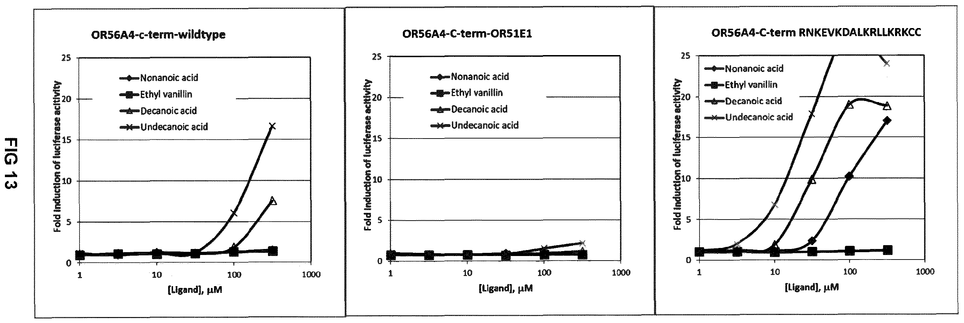

The optimized sequence in Examples 1-5 was originally derived by mutating the C-terminal sequence of the Class II OR5AN1. The C-terminal sequences of class I OR are widely diverging from the class II sequences, also leading to a different C-terminus consensus sequence for class I receptors (Kotthoff et al. 2021, see supra). It was thus further tested, whether the optimized C-terminal sequence developed starting from a class II receptor can also be applied to a class I receptor. Surprisingly, indeed this sequence also led to clearly enhanced expression of several class I receptors as already summarized in example 2 for OR52A5, OR52E8 and OR56A4. A more detailed comparison is given in Figure 12 and Figure 13.

Activation of OR52E8 by odorant acids present in human sweat (Natsch et al. A specific bacterial aminoacylase cleaves odorant precursors secreted in the human axilla. The Journal of biological chemistry 2003; 278(8):5718-5727) was investigated with different variants of the parent receptor sequence. The wild-type variant was not activated by the acids. Replacing the wild-type C-terminus with the the C-terminal sequence of the functional OR51E1 did not provide a functional OR52E8. However, using the optimized sequence from Examples 1-5 led to a strong signal upon addition of 3-methyl-3-hydroxy hexanoic acid.

Activation of OR56A4 by acids of different chain length was further tested. The wild-type was activated at high concentration by decanoic acid and undecanoic acid, but not by nonanoic acid. Replacing the wild-type with the C-terminal sequence of the functional OR51 E1 did reduce activity. However, using the optimized sequence form Examples 1-5 led to a strong signal and much lower detection threshold upon addition of all three acids.

Example 13: Replacing the C-terminal sequence of a poorly functional receptor with the C-terminal sequence of a functional receptor as compared to adding the optimal C-terminal sequence of this disclosure

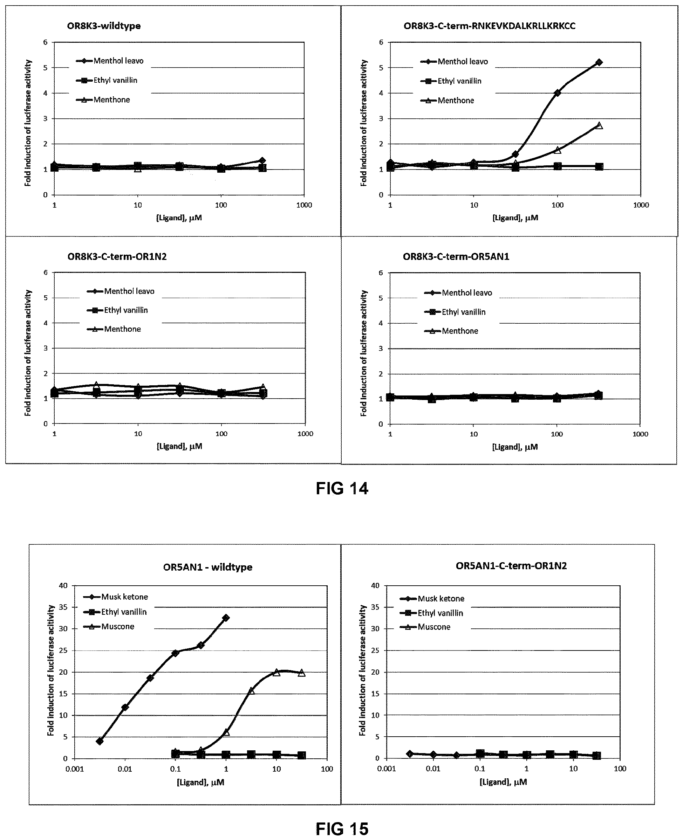

Knowing the importance of the C-terminal sequence for proper expression as observed in the previous examples, an obvious way to improve expression might be just to provide a hybrid receptor whereby the C-terminal domain in a non-functional or poorly functional receptor is replaced by the C-terminal domain of a functional receptor. Although this approach was tested in the past (Ikegami et al. 2020, see supra) whereby the C-terminus of the poorly expressed mouse Olf541 was replaced with the C-terminus of the well expressed Olfr539 and did not lead to enhanced expression of Olfr541, this approach was further tested in a comparative example with different human ORs. Thus the C-terminal sequence of OR8K3 was replaced with the C-terminal sequence of the ambrettolide-receptor OR1N2 or the musk receptor OR5AN1. For both of these receptors the wild-type is functional, indicating that they can properly function with their native C-terminal sequence and hence this C-terminal sequence in principle can provide for functional expression. However, modified OR8K3 variants with either of these C-terminal sequences did not show any functional response to the cognate ligand menthol (Figure 14), while the modified receptor with the C-terminal sequence according to examples 1-5 led to strong functional expression. Thus the improvement achieved with the modified C-terminal sequences of this disclosure cannot be achieved by simply generating chimeric receptors combining a functional C-terminal domain of a functional receptor with a poorly expressed receptor.

Similarly, the C-terminal sequence of OR5AN1 was replaced with the C-terminal sequence of the ambrettolide receptor OR1 N2. For both of these receptors the wild-type is functional, indicating that they can properly function with their native C-terminal sequence. However, the chimeric OR5AN1 variant with the C-terminal sequence of OR1N2 is not showing any functional response to the cognate ligands Musk ketone or Muscone (Figure 15).

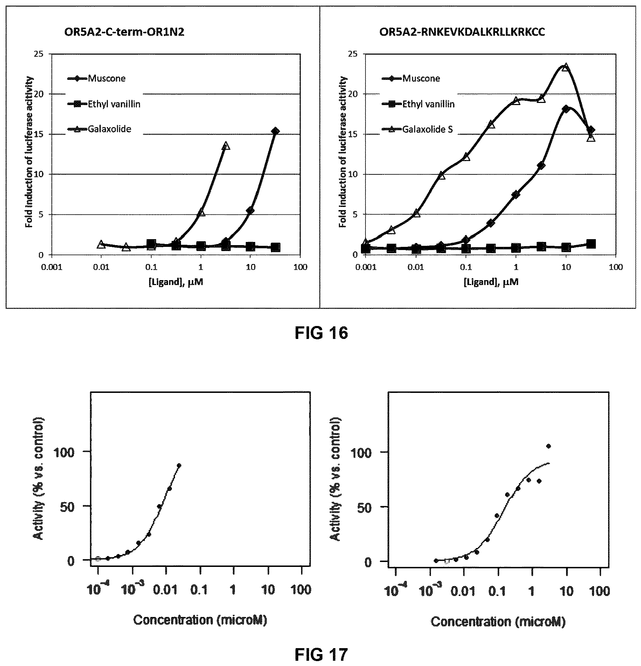

Similarly, musk receptor OR5A2 with the C-terminal sequence of OR1N2 has a poor sensitivity as compared to the variant receptor of this disclosure with the C-terminal sequence RNKEVKDALKRLLKRKCC (Figure 16). Thus, the improvement achieved with the modified C-terminal sequences of this disclosure cannot be achieved by simply generating chimeric receptors combining a functional C-terminal domain of a functional receptor with a poorly expressed receptor.

Example 14: Screening of odorants with the desired odor quality of Arborone using the optimized C-terminus sequence RNKEVKDALKRLLKRKCC (SEQ ID NO: 86)

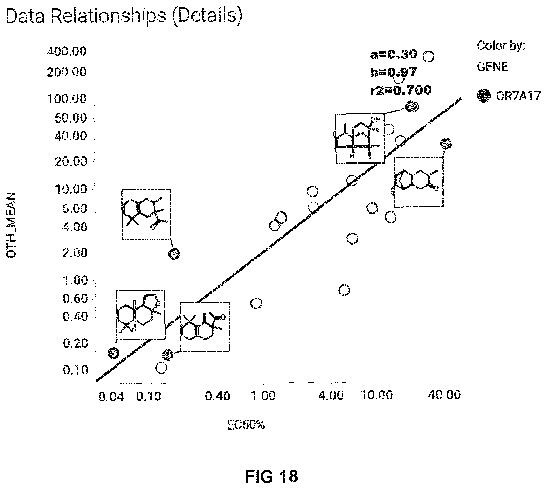

The modified variant of OR7A17 with the optimized C-terminus sequence RNKEVKDALKRLLKRKCC (SEQ ID NO: 86) as described in Example 2 was tested on Arborone, the key characteristic odorous component of the complex substance Iso E Super (Hong & Corey. Enantioselective syntheses of georgyone, arborone, and structural relatives. Relevance to the molecular-level understanding of olfaction. Journal of the American Chemical Society 2006;128(4):1346-1352). As shown in Figure 17, Arborone activates OR7A17 with the optimized C-terminus already down to a concentration of 1 nanomolar, i.e. at ca. 50 times lower concentration than Iso E super indicating that the hybrid OR7A17 with the optimized C-terminus is a powerful tool to screen for the desired woody character of Arborone. Thus, a number (n=22) of woody fragrance molecules were tested for both the odor detection threshold (OTH) in humans in vivo and the in vitro EC50 on the OR7A17 with the optimized C-terminus sequence RNKEVKDALKRLLKRKCC (SEQ ID NO: 86). As shown in Figure 18, the in vitro data predict well the low odour detection threshold of Ambrofix, Georgywood and Iso E super (Highlighted and with chemicals structure at the bottom left), discriminating them from odorants of intermediate potency and the much less active (both in vitro and in vivo) Cedrol (Highlighted top right).

Example 15. Screening for receptor activation by key human sweat odorant

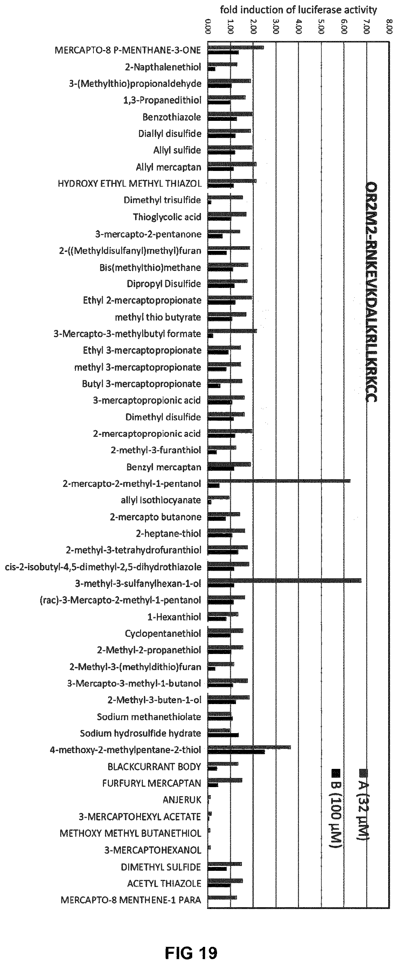

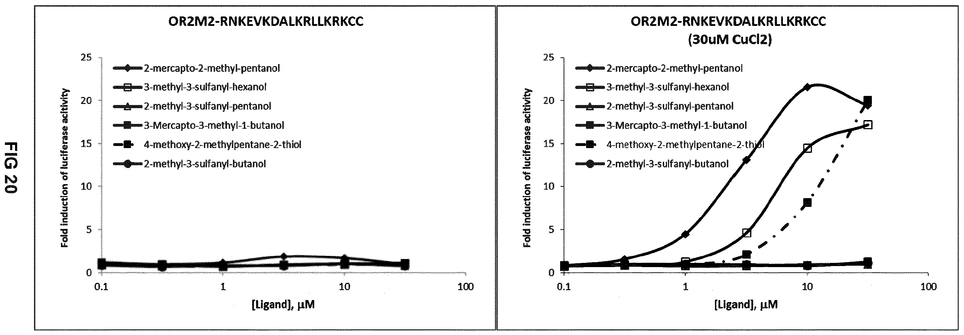

The most pungent odorant in human axillary sweat with an ODT of 1 picogram / L air is 3-methyl-3-sulfanyl-hexanol (also known as 3-methyl-3-mercapto-hexanol) (Natsch et al. identification of Odoriferous Sulfanylalkanols in Human Axilla Secretions and Their Formation through Cleavage of Cysteine Precursors by a C-S Lyase Isolated from Axilla bacteria. Chemistry and Biochemistry 2004; 1:1058-1072). So far, no OR has been described which can selectively detect this key odorant leading to undesired axilla odor in human subjects. By screening a library of receptor variants with the C-terminal sequence SEQ ID NO: 86 in presence of copper against a library of 52 sulfur compounds (see example 5), a hitherto orphan receptor, OR2M2, was identified, which is activated by 3-methyl-3-mercapto-hexanol and closely related chemicals, but not by other sulfur odorants (Figure 19). Dose-response analysis (Figure 20) indicated that these identified ligands can activate OR2M2 down to a concentration of 1 $\mu$M when tested in presence of copper, only, an effect which has been observed for other receptors responding to sulfur compounds. Thus, using the improved sequences of this disclosure it was for the first time possible to deorphanize OR2M2 and the modified OR2M2 with an optimized C-terminus can thus, in combination with one of its cognate ligands 3-methyl-3-sulfanyl-hexanol, 2-mercapto-2-methyl-pentanol or 4-methoxy-2-methylpentane-2-thiol in the presence of copper be used as a screening target for the most potent human axillary malodorant.

The full amino acid and DNA sequences of the OR2M2 modified sequence having the modified C-terminal domain of SEQ ID NO: 86 are provided as SEQ ID NO: 245 and 246.

Example 16: Random permutations of the variable positions within the modified C-terminus of the disclosure (SEQ ID NO: 1)

Random permutations of the general C-terminal sequence RN[KR][EDQ][VMIL][KR]**K**A[LIV][KRH][KR][LI][LIF][KRG][KR][KR]CC (SEQ ID NO: 822) linked to OR7D4 were generated based on degenerate oligonucleotides for the C-terminal domain. For this experiment, the Mfel / Xhol fragment from pcDNA3.1(+)-mmLucy-FLAG-rho-OR7D4 containing the CMV promoter and the OR coding sequence was used to replace the EcoRI / Sall fragment of pRDVCCB-CMV-dCas9-VPH-2A-Blast (Cellecta, Inc., Mountain View, USA). Complementary degenerate oligonucleotides were then used to replace the coding region of the C-terminal domain of OR7D4 (between Bsu36I and NotI). Random clones were selected, sequenced and tested for a correct open reading frame. A total of 55 clones were obtained with the correct open reading frame of OR7D4 and a permutation of the general sequence RN[KR][EDQ][VMIL][KR]**K**A[LIV][KRH][KR][LI][LIF][KRG][KR][KR]CC (SEQ ID NO: 822) as the C-terminal domain. All the different clones were compared to the wild-type sequence by the functional assay in HEK cells with the ligand Androstenone tested in a dose-response test. While the wild-type sequence is not significantly induced by 1 μM of the ligand, the induction by androstenone of the different variants is between 10.1-fold and 73.6-fold at this concentration. The EC2 for a two-fold induction of luciferase for the wild-type is at 1.71 μM, while it is reduced to 0.003 - 0.13 μM for the different variants, which corresponds to an improvement in the sensitivity of the assay by the modified C-terminal sequence of 13 - 545 fold, with a median of 125-fold improved sensitivity. These results indicate that all of the tested random permutations of the general sequence yield a clearly improved (> 10 fold) sensitivity of the functional assay.

Table 12. Functional assay with Androstenone activating OR7D4-variants with different random permutations of the general sequence RN[KR][EDQ][VMIL][KR]**K**A[LIV][KRH][KR][LI][LIF][KRG][KR][KR]**CC** as C-terminal domain (SEQ ID NO: 822).

|  | EC2 (μM; concentration for 2-fold luciferase induction) | | Fold induction luciferase | |
|---|---|---|---|---|
| **C-terminal sequence** | **EC2** | **Fold-improvement vs. wild-type** | **0.1 μM** | **1 μM** |
| **Wild-type**<br>**RNKDVKGALERLLSRADSCP** (SEQ ID NO: 253) | **1.724** | **1.00** | **0.98** | **1.20** |
| **RNKEVKDALKRLLKRKCC** (SEQ ID NO: 86) | **0.007** | **249.44** | **22.17** | **39.16** |
| RNREMRKALHRLLGKKCC (SEQ ID NO: 254) | 0.011 | 156.27 | 16.54 | 60.68 |
| RNREVKKAIHKLIGRKCC (SEQ ID NO: 255) | 0.010 | 164.23 | 26.43 | 51.99 |
| RNREVRKAVHRLFKRKCC (SEQ ID NO: 256) | 0.004 | 385.42 | 40.68 | 69.14 |
| RNKEMKKAIHKLFGKKCC (SEQ ID NO: 257) | 0.010 | 172.96 | 10.77 | 62.06 |
| RNRDVKKAVHKLFRRKCC (SEQ ID NO: 258) | 0.011 | 152.59 | 12.21 | 34.24 |
| RNRDMKKAVHKLFGKRCC (SEQ ID NO: 259) | 0.020 | 84.30 | 9.69 | 30.79 |
| RNKELRKALHKLLGRKCC (SEQ ID NO: 260) | 0.043 | 39.74 | 4.33 | 24.97 |
| RNRDVRKALRRILRRRCC (SEQ ID NO: 261) | 0.004 | 427.69 | 26.04 | 37.97 |
| RNKDVRKAVRKLIRRRCC (SEQ ID NO: 262) | 0.010 | 165.32 | 18.24 | 39.01 |
| RNRDVRKAVRRLFRKRCC (SEQ ID NO: 263) | 0.006 | 304.87 | 26.03 | 44.54 |
| RNKDIKKAVKKLIKKKCC (SEQ ID NO: 264) | 0.048 | 36.01 | 3.79 | 23.26 |
| RNRELRKAVRRLFKRRCC (SEQ ID NO: 265) | 0.022 | 78.45 | 9.89 | 34.10 |
| RNKELRKAVRKIIKKKCC (SEQ ID NO: 266) | 0.041 | 41.85 | 4.56 | 24.36 |
| RNRDVKKAVRRLFRRKCC (SEQ ID NO: 267) | 0.006 | 266.97 | 26.35 | 43.28 |
| RNREVRKALRRIIRKRCC (SEQ ID NO: 268) | 0.003 | 545.04 | 26.72 | 40.53 |
| RNKDIRKAVKKIFRRKCC (SEQ ID NO: 269) | 0.026 | 65.26 | 9.98 | 24.73 |
| RNKDVRKAVRRLIKRKCC (SEQ ID NO: 270) | 0.012 | 145.78 | 16.11 | 34.08 |
| RNRDLRKAVRKLFKKKCC (SEQ ID NO: 271) | 0.030 | 56.86 | 8.45 | 24.67 |
| RNRDLRKALRRIFKRRCC (SEQ ID NO: 272) | 0.013 | 136.52 | 16.54 | 35.68 |
| RNRDVRKAIKKLIRKRCC (SEQ ID NO: 273) | 0.005 | 373.12 | 19.39 | 29.11 |
| RNKELKKAIKRILKKKCC (SEQ ID NO: 274) | 0.095 | 18.20 | 2.07 | 17.21 |
| RNRDVRKAIRKLLKRKCC (SEQ ID NO: 275) | 0.005 | 323.20 | 20.67 | 28.85 |
| RNRDLRKAVRRIFKKRCC (SEQ ID NO: 276) | 0.034 | 50.55 | 7.49 | 21.84 |
| RNRDVRKAVRKLFKRRCC (SEQ ID NO: 277) | 0.005 | 325.72 | 18.86 | 33.06 |
| RNRDVRKALRRLFKKRCC (SEQ ID NO: 278) | 0.003 | 522.64 | 23.07 | 31.19 |

| | | | | |
|---|---|---|---|---|
| RNKELKKALRKLIGKKCC (SEQ ID NO: 279) | 0.098 | 17.53 | 2.05 | 15.68 |
| RNREMRKAIKKIIKKKCC (SEQ ID NO: 280) | 0.009 | 187.06 | 15.65 | 28.32 |
| RNKEIKKAIKKIIKKRCC (SEQ ID NO: 281) | 0.029 | 60.06 | 6.72 | 19.26 |
| RNRDVKKAIRRLFRRRCC (SEQ ID NO: 282) | 0.007 | 250.61 | 16.43 | 28.10 |
| RNREVKKAVKKLIGRCC (SEQ ID NO: 283) | 0.013 | 128.83 | 17.55 | 40.31 |
| RNREMRKALRRLFRKRCC (SEQ ID NO: 284) | 0.003 | 545.04 | 52.31 | 73.63 |
| RNKELKKALRRLIGRRCC (SEQ ID NO: 285) | 0.042 | 41.00 | 3.18 | 22.10 |
| RNRDVKKALRKLIGKRCC (SEQ ID NO: 286) | 0.003 | 545.04 | 38.03 | 58.63 |
| RNREVKKAVKKLIRRKCC (SEQ ID NO: 287) | 0.012 | 144.06 | 15.57 | 34.00 |
| RNKEVRKALKKLFGKKCC (SEQ ID NO: 288) | 0.006 | 294.52 | 17.84 | 28.81 |
| RNKEIRKALRRLFGKKCC (SEQ ID NO: 289) | 0.014 | 119.65 | 15.79 | 34.07 |
| RNKDVKKALRRLFGKKCC (SEQ ID NO: 290) | 0.008 | 225.85 | 17.08 | 27.45 |
| RNKELKKAIKRLIRRKCC (SEQ ID NO: 291) | 0.113 | 15.28 | 1.75 | 22.34 |
| RNKDVRKAVKRLLKKRCC (SEQ ID NO: 292) | 0.018 | 94.88 | 8.46 | 25.66 |
| RNKELRKAIRRLLRRRCC (SEQ ID NO: 293) | 0.068 | 25.22 | 2.76 | 19.39 |
| RNRDIRKALRKLFKKKCC (SEQ ID NO: 294) | 0.008 | 224.95 | 17.51 | 34.30 |
| RNRELKKALRRLLRRRCC (SEQ ID NO: 295) | 0.033 | 51.89 | 6.72 | 23.71 |
| RNREVKKALRRLFGKKCC (SEQ ID NO: 296) | 0.003 | 525.05 | 21.37 | 35.71 |
| RNRDVRKALRKLLKRKCC (SEQ ID NO: 297) | 0.005 | 337.39 | 14.08 | 29.20 |
| RNRDMRKAIRKLFGRKCC (SEQ ID NO: 298) | 0.011 | 162.22 | 15.47 | 28.10 |
| RNRELKKAIRKLLKRKCC (SEQ ID NO: 299) | 0.093 | 18.62 | 2.09 | 17.32 |
| RNRDIRKAVKKLFGKKCC (SEQ ID NO: 300) | 0.020 | 87.76 | 9.15 | 24.73 |
| RNKEVKKAIRKLFGRRCC (SEQ ID NO: 301) | 0.013 | 128.57 | 13.30 | 29.02 |
| RNREVRKAVRKLFRRKCC (SEQ ID NO: 302) | 0.014 | 126.99 | 12.90 | 24.63 |
| RNRDMKKALKKLFRRRCC (SEQ ID NO: 303) | 0.011 | 150.37 | 13.65 | 18.28 |
| RNRDVRKALKRLLGRRCC (SEQ ID NO: 304) | 0.006 | 292.59 | 14.06 | 24.75 |
| RNKDLKKAVKKLFGRKCC (SEQ ID NO: 305) | 0.131 | 13.15 | 1.63 | 10.13 |
| RNKDVRKAVRRLFGRRCC (SEQ ID NO: 306) | 0.017 | 103.17 | 9.29 | 22.60 |

The results of Table 12 were evaluated to derive, at each variable position in the general sequence, the best amino acid residue (i.e. the residue which gives the lowest median EC2 for sequences containing it at the given position). Based on this analysis, the statistically optimal sequence within the general sequence is **RNRDVRKALRRLFRKK** (SEQ ID NO: 307) and - because R at position 7 is at least as active as K (See Table 2 in example 6) - an optimal sequence is also **RNRDVRRALRRLFRKK** (SEQ ID NO: 308). The data from Table 12 were then further analysed for each sequence permutation as to how many residues differ from the statistically optimal sequence. As shown in Table 13, those sequences closest to SEQ ID NO: 307 have an overall better activity. Thus, while all tested random permutations within the general sequence are active, the most active sequences cluster among those which are closest to the optimal sequence. Thus, to name examples, the sequences with 2-4 residues difference from SEQ ID NO: 307 are particularly active and - with only one exception - give > 100 fold improvement in sensitivity over the wild-type.

Table 13. Relationship of the distance between the random sequence permutations in Table A to the optimal SEQ ID NO: 307 and activity.

| Residues differing from statistically best sequence | EC2 (μM; concentration for 2-fold luciferase induction) | | Median Fold induction luciferase | |
| --- | --- | --- | --- | --- |
| | **Median EC2** | **Median Fold-improvement vs. wild-type** | **0.1 μM** | **1 μM** |
| **Wild-type RNKDVKGALERLLS-RADSCP** (SEQ ID NO: 253) | **1.724** | **1.00** | **0.98** | **1.20** |
| 2 | 0.004 | 413.8 | 24.6 | 37.9 |
| 3 | 0.006 | 267.0 | 21.4 | 35.7 |
| 4 | 0.007 | 250.6 | 15.8 | 29.2 |
| 5 | 0.011 | 162.2 | 16.5 | 29.1 |
| 6 | 0.018 | 94.9 | 9.9 | 25.7 |
| 7 | 0.026 | 65.3 | 9.7 | 24.7 |
| 8 | 0.042 | 41.4 | 4.4 | 24.7 |
| 9 | 0.095 | 18.2 | 2.1 | 17.2 |
| 11 | 0.029 | 60.1 | 6.7 | 19.3 |

Example 17: Improved functional expression by different amino acids at position 7 of the general sequence

To test for possible variants of the sequence RN[KR][EDQ][VMIL][KR]**x**A[LIV][KRH][KR][LI][LIF][KRG][KR][KR] (SEQ ID NO: 1) leading to similar improved functional expression, different amino acids were introduced at the position 7 (denoted with an x) in the general sequence RNKEVK**x**ALKRLLKRK (SEQ ID NO: 319) and the variants were fused after the TM7 with OR7C1 . As shown in Figure 2, OR7C1 with the C-terminal sequence RNKEVKDALKRLLKRKCC (SEQ ID NO: 86) gives around 10-fold luciferase induction at 1 μM of Ambermax and 20-fold induction at 3.1 μM of Ambermax, while the wild-type is inactive. Activation of the different modified variants were then compared as described in Example 6. Results show that position 7 of SEQ ID NO: 86 has a high flexibility, with all of the 20 amino acids except Proline showing superior activity compared to the wild type.

Table 14. Activation by Ambermax of OR7C1 variants with the optimized C-terminus containing single base substitutions at position 7 of the C-terminal motif RNKEVKDALKRLLKRKCC (SEQ ID NO: 86).

| C-terminal Sequence | % of the activation by the optimized sequence RNKEVKDALKRLLKRKCC | |
| --- | --- | --- |
| | **1 μM Ambermax** | **3.1 μM Ambermax** |
| Wild Type | 0.5 | 4.4 |
| RNKEVKDALKRLLKRKCC (SEQ ID NO: 86) | 100 | 100 |
| RNKEVK**K**ALKRLLKRKCC (D299K) (SEQ ID NO: 91) | 140.8 | 98.2 |
| RNKEVK**E**ALKRLLKRKCC (D299E) (SEQ ID NO: 92) | 81.3 | 113.6 |
| RNKEVK**N**ALKRLLKRKCC (D299N) (SEQ ID NO: 93) | 95.3 | 113.5 |
| RNKEVK**R**ALKRLLKRKCC (D299R) (SEQ ID NO: 94) | 176.6 | 176.9 |
| RNKEVK**V**ALKRLLKRKCC (D299V) (SEQ ID NO: 95) | 122.1 | 108.8 |
| RNKEVK**A**ALKRLLKRKCC (D299A) (SEQ ID NO: 96) | 115.3 | 111.0 |
| RNKEVK**Q**ALKRLLKRKCC (D299Q) (SEQ ID NO: 97) | 128.4 | 116.7 |
| RNKEVK**G**ALKRLLKRKCC (D299G) (SEQ ID NO: 110) | 41.6 | 59.8 |
| RNKEVK**C**ALKRLLKRKCC (D299C) (SEQ ID NO: 321) | 46.0 | 97.2 |
| RNKEVK**F**ALKRLLKRKCC (D299F) (SEQ ID NO: 322) | 41.1 | 68.6 |

(continued)

| C-terminal Sequence | % of the activation by the optimized sequence RNKEVKDALKRLLKRKCC | |
|---|---|---|
| | 1 μM Ambermax | 3.1 μM Ambermax |
| RNKEVK**H**ALKRLLKRKCC (D299H) (SEQ ID NO: 323) | 65.6 | 78.6 |
| RNKEVK**I**ALKRLLKRKCC (D299I) (SEQ ID NO: 324) | 81.0 | 104.0 |
| RNKEVKDA**L**KRLLKRKCC (D299L) (SEQ ID NO: 325) | 108.0 | 100.5 |
| RNKEVK**M**ALKRLLKRKCC (D299M) (SEQ ID NO: 326) | 107.6 | 113.1 |
| RNKEVK**P**ALKRLLKRKCC (D299P) (SEQ ID NO: 327) | -3.7 | -1.8 |
| RNKEVK**S**ALKRLLKRKCC (D299S) (SEQ ID NO: 328) | 99.1 | 109.7 |
| RNKEVK**T**ALKRLLKRKCC (D299T) (SEQ ID NO: 329) | 129.9 | 85.8 |
| RNKEVK**W**ALKRLLKRKCC (D299W) (SEQ ID NO: 330) | 17.4 | 26.2 |
| RNKEVK**Y**ALKRLLKRKCC (D299Y) (SEQ ID NO: 331) | 59.4 | 69.9 |

Example 18: Screening of a library of all class II OR with an identical improved C-terminal domain with single odorants

All human class II OR and all key genetic variants of these OR were synthesized with a sequence coding for an identical modified C-terminal domain RNKEVK**K**AIKRL**F**KRKCC**RRR** (SEQ ID NO: 221) in the vector pcDNA3.1(+) to generate a library of all class II OR with an improved C-terminal domain. The complete list of class II OR included in this library is given in Table 15:

Table 15. Class II OR library members with modified C-terminal domains

| # | Receptor | SEQ ID NO DNA encoding the modified receptor including an N-terminal mmLucy-FLAG-rho tag (SEQ ID NO: 81) and a modified C-terminus (SEQ ID NO: 221) | # | Receptor | SEQ ID NO DNA encoding the modified receptor including an N-terminal mmLucy-FLAG-rho tag (SEQ ID NO: 81) and a modified C-terminus (SEQ ID NO: 221) |
|---|---|---|---|---|---|
| 1 | OR10A3 | 660 | 205 | OR4A5 | 456 |
| 2 | OR10A4 | 661 | 206 | OR4B1 | 457 |
| 3 | OR10A4(R262Q) | 662 | 207 | OR4C11 | 458 |
| 4 | OR10A5 | 663 | 208 | OR4C12(V283L) | 459 |
| 5 | OR10A6(A117V,V14 0G,L287P) | 664 | 209 | OR4C13(V133I) | 460 |
| 6 | OR10A6(L287P) | 665 | 210 | OR4C15 | 461 |
| 7 | OR10A7 | 666 | 211 | OR4C16(T76A) | 462 |
| 8 | OR10AD1 | 667 | 212 | OR4C3 | 463 |
| 9 | OR10AG1 | 668 | 213 | OR4C3(V257M,H 258L,P264S) | 464 |
| 10 | OR10C1 | 669 | 214 | OR4C45 | 465 |

(continued)

| # | Receptor | SEQ ID NO *DNA encoding the modified receptor including an N-terminal mmLucy-FLAG-rho tag (SEQ ID NO: 81) and a modified C-terminus (SEQ ID NO: 221)* | # | Receptor | SEQ ID NO *DNA encoding the modified receptor including an N-terminal mmLucy-FLAG-rho tag (SEQ ID NO: 81) and a modified C-terminus (SEQ ID NO: 221)* |
|---|---|---|---|---|---|
| 11 | OR10C1(M246V) | 670 | 215 | OR4C46 | 467 |
| 12 | OR10D3 | 671 | 216 | OR4C46(S240F) | 466 |
| 13 | OR10G2 | 672 | 217 | OR4C5 | 468 |
| 14 | OR10G3(S73G) | 673 | 218 | OR4C5(D77H) | 469 |
| 15 | OR10G4 | 674 | 219 | OR4C6 | 470 |
| 16 | OR10G6 | 675 | 220 | OR4D1 | 471 |
| 17 | OR10G7(T5S) | 676 | 221 | OR4D10 | 472 |
| 18 | OR10G8 | 677 | 222 | OR4D11 | 473 |
| 19 | OR10G9 | 678 | 223 | OR4D11(F197L) | 474 |
| 20 | OR10H1 | 679 | 224 | OR4D2 | 475 |
| 21 | OR10H2 | 680 | 225 | OR4D5 | 476 |
| 22 | OR10H3(R7S,R54H ) | 681 | 226 | OR4D6 | 477 |
| 23 | OR10H3(R7S,V224 M) | 682 | 227 | OR4D9 | 478 |
| 24 | OR10H4 | 683 | 228 | OR4E2 | 479 |
| 25 | OR10H5 | 684 | 229 | OR4E2(V118M,Q 234R) | 480 |
| 26 | OR10J1(M51I,I92M) | 685 | 230 | OR4F15 | 481 |
| 27 | OR10J5 | 686 | 231 | OR4F16 | 482 |
| 28 | OR10K1 | 687 | 232 | OR4F17 | 483 |
| 29 | OR10K2 | 688 | 233 | OR4F21 | 484 |
| 30 | OR10P1 | 689 | 234 | OR4F29 | 485 |
| 31 | OR10P1(P88L,V200 M) | 690 | 235 | OR4F3 | 486 |
| 32 | OR10Q1 | 691 | 236 | OR4F4 | 487 |
| 33 | OR10R2 | 692 | 237 | OR4F5 | 488 |
| 34 | OR10R2(E205G,L22 8F) | 693 | 238 | OR4F6 | 489 |
| 35 | OR10S1 | 694 | 239 | OR4K1 | 490 |
| 36 | OR10T2 | 695 | 240 | OR4K13 | 491 |
| 37 | OR10T2(I137M,V23 9A) | 696 | 241 | OR4K14 | 492 |
| 38 | OR10V1 | 697 | 242 | OR4K15 | 493 |
| 39 | OR10W1 | 698 | 243 | OR4K17 | 494 |

(continued)

| # | Receptor | SEQ ID NO DNA encoding the modified receptor including an N-terminal mmLucy-FLAG-rho tag (SEQ ID NO: 81) and a modified C-terminus (SEQ ID NO: 221) | # | Receptor | SEQ ID NO DNA encoding the modified receptor including an N-terminal mmLucy-FLAG-rho tag (SEQ ID NO: 81) and a modified C-terminus (SEQ ID NO: 221) |
|---|---|---|---|---|---|
| 40 | OR10W1(R263Q) | 699 | 244 | OR4K17(K128N) | 495 |
| 41 | OR10X1 | 700 | 245 | OR4K2 | 496 |
| 42 | OR10Z1 | 701 | 246 | OR4K5 | 497 |
| 43 | OR11A1 | 702 | 247 | OR4L1(D2N,M40 V,R52S,M101K, G109S) | 499 |
| 44 | OR11G2 | 703 | 248 | OR4L1(R52S) | 498 |
| 45 | OR11G2(I65N,V82I) | 704 | 249 | OR4M1 | 500 |
| 46 | OR11H1 | 705 | 250 | OR4M2 | 501 |
| 47 | OR11H12 | 706 | 251 | OR4M2M239V,R 284H) | 502 |
| 48 | OR11H2 | 707 | 252 | OR4N2 | 503 |
| 49 | OR11H4 | 708 | 253 | OR4N2(I76T) | 504 |
| 50 | OR11H6 | 709 | 254 | OR4N4 | 505 |
| 51 | OR11H6(I84T,L172 F,Y213H,C236R) | 710 | 255 | OR4N5 | 506 |
| 52 | OR11L1(G108S,F11 7L,A142T,R171P) | 711 | 256 | OR4P4 | 507 |
| 53 | OR12D2(S104F) | 712 | 257 | OR4Q3 | 508 |
| 54 | OR12D2(V47F,L56P ,S104F,F113L,L120 R,S121C,V159I) | 713 | 258 | OR4Q3(F238L) | 509 |
| 55 | OR12D3 | 714 | 259 | OR4S1 | 510 |
| 56 | OR13A1 | 715 | 260 | OR4S2 | 511 |
| 57 | OR13C2 | 716 | 261 | OR4X1 | 512 |
| 58 | OR13C3 | 717 | 262 | OR4X2 | 513 |
| 59 | OR13C4 | 718 | 263 | OR5A1 | 514 |
| 60 | OR13C5 | 719 | 264 | OR5A2 | 515 |
| 61 | OR13C5(V117M,C1 89Y,M258T,I282V,M 290T) | 720 | 265 | OR5AC2 | 516 |
| 62 | OR13C8 | 721 | 266 | OR5AC2(M200I) | 517 |
| 63 | OR13C8(A19D) | 722 | 267 | OR5AK2 | 518 |
| 64 | OR13C9 | 723 | 268 | OR5AK2(M92I) | 519 |
| 65 | OR13C9(E24D,T91 S) | 724 | 269 | OR5AN1 | 520 |

(continued)

| # | Receptor | SEQ ID NO *DNA encoding the modified receptor including an N-terminal mmLucy-FLAG-rho tag (SEQ ID NO: 81) and a modified C-terminus (SEQ ID NO: 221)* | # | Receptor | SEQ ID NO *DNA encoding the modified receptor including an N-terminal mmLucy-FLAG-rho tag (SEQ ID NO: 81) and a modified C-terminus (SEQ ID NO: 221)* |
|---|---|---|---|---|---|
| 66 | OR13D1 | 725 | 270 | OR5AP2 | 521 |
| 67 | OR13D1(Q159H,S2 45L) | 726 | 271 | OR5AR1 | 522 |
| 68 | OR13F1(F18S,M101 V,V134I,T254M) | 727 | 272 | OR5AS1 | 523 |
| 69 | OR13G1(I132V) | 728 | 273 | OR5AU1 | 524 |
| 70 | OR13G1(K46I,M146 L,R224C) | 729 | 274 | OR5B12 (C141R) | 525 |
| 71 | OR13H1 | 730 | 275 | OR5B17 | 526 |
| 72 | OR13J1 | 731 | 276 | OR5B17(L80I) | 527 |
| 73 | OR13J1(H133R) | 732 | 277 | OR5B2 | 528 |
| 74 | OR14A16(I238T) | 733 | 278 | OR5B2(M200T,V 208A) | 529 |
| 75 | OR14A2 | 734 | 279 | OR5B21 | 530 |
| 76 | OR14C36 | 735 | 280 | OR5B3 | 531 |
| 77 | OR14C36(G225R,D 231Y) | 736 | 281 | OR5B3(W49R,N 170S,A181T,I198 V,G247A) | 532 |
| 78 | OR14I1(V36A,D50N ,S170N) | 737 | 282 | OR5C1 | 533 |
| 79 | OR14J1 | 738 | 283 | OR5D13(C62Y) | 534 |
| 80 | OR14K1 | 739 | 284 | OR5D14(S249A) | 535 |
| 81 | OR1A1 | 332 | 285 | OR5D16 | 536 |
| 82 | OR1A2 | 333 | 286 | OR5D18(N136D) | 537 |
| 83 | OR1B1(L149S,C263 W) | 334 | 287 | OR5F1 | 538 |
| 84 | OR1C1 | 335 | 288 | OR5H1 | 539 |
| 85 | OR1D2 | 336 | 289 | OR5H14 | 540 |
| 86 | OR1D4(*172R) | 337 | 290 | OR5H14(G64R,Y 189C) | 541 |
| 87 | OR1D5 | 338 | 291 | OR5H15 | 542 |
| 88 | OR1E1 | 339 | 292 | OR5H15(V108I,S 148T,T167S,P16 5L) | 543 |
| 89 | OR1E2 | 340 | 293 | OR5H2 | 544 |
| 90 | OR1F1 | 341 | 294 | OR5H6(A129P,C 179R,T256A,D26 9N) | 545 |

(continued)

| # | Receptor | SEQ ID NO *DNA encoding the modified receptor including an N-terminal mmLucy-FLAG-rho tag (SEQ ID NO: 81) and a modified C-terminus (SEQ ID NO: 221)* | # | Receptor | SEQ ID NO *DNA encoding the modified receptor including an N-terminal mmLucy-FLAG-rho tag (SEQ ID NO: 81) and a modified C-terminus (SEQ ID NO: 221)* |
|---|---|---|---|---|---|
| 91 | OR1F1(F75S) | 342 | 295 | OR5I1 | 546 |
| 92 | OR1F12 | 343 | 296 | OR5J2 | 547 |
| 93 | OR1G1 | 344 | 297 | OR5K1 | 548 |
| 94 | OR1I1 | 345 | 298 | OR5K2 | 549 |
| 95 | OR1I1(P139R,F211 L,Y252S) | 346 | 299 | OR5K3 | 550 |
| 96 | OR1J1 | 347 | 300 | OR5K4 | 551 |
| 97 | OR1J2 | 348 | 301 | OR5L1 | 552 |
| 98 | OR1J4 | 349 | 302 | OR5L2 | 553 |
| 99 | OR1K1 | 350 | 303 | OR5M1 | 554 |
| 100 | OR1L1 | 351 | 304 | OR5M1(S282T) | 555 |
| 101 | OR1L3 | 352 | 305 | OR5M10 | 556 |
| 102 | OR1L4 | 353 | 306 | OR5M11 | 557 |
| 103 | OR1L6 | 354 | 307 | OR5M11(S171N) | 558 |
| 104 | OR1L8 | 355 | 308 | OR5M3 | 559 |
| 105 | OR1L8(T27P,R211P ) | 356 | 309 | OR5M8 | 560 |
| 106 | OR1M1 | 357 | 310 | OR5M9 | 561 |
| 107 | OR1N1 | 358 | 311 | OR5P2 | 562 |
| 108 | OR1N2(W23R,V230 G,T287M) | 359 | 312 | OR5P2(Y145C,V 212I,C213S) | 563 |
| 109 | OR1Q1(Q24R) | 360 | 313 | OR5P3 | 564 |
| 110 | OR1S1 | 361 | 314 | OR5R1(C122R,A 274V) | 565 |
| 111 | OR1S1(I110T,H122 R,N170D,S214I) | 362 | 315 | OR5R1(I7T,C122 R,S128G) | 566 |
| 112 | OR1S2 | 363 | 316 | OR5T1 | 567 |
| 113 | OR2A1 | 364 | 317 | OR5T1(S157G) | 568 |
| 114 | OR2A12 | 365 | 318 | OR5T2(V46L) | 569 |
| 115 | OR2A14 | 366 | 319 | OR5T3 | 570 |
| 116 | OR2A14(S133I, S164R) | 367 | 320 | OR5V1 | 571 |
| 117 | OR2A2 | 368 | 321 | OR5W2 | 572 |

(continued)

| # | Receptor | SEQ ID NO DNA encoding the modified receptor including an N-terminal mmLucy-FLAG-rho tag (SEQ ID NO: 81) and a modified C-terminus (SEQ ID NO: 221) | # | Receptor | SEQ ID NO DNA encoding the modified receptor including an N-terminal mmLucy-FLAG-rho tag (SEQ ID NO: 81) and a modified C-terminus (SEQ ID NO: 221) |
|---|---|---|---|---|---|
| 118 | OR2A2(F280L) | 369 | 322 | OR6A2 | 573 |
| 119 | OR2A25 | 370 | 323 | OR6B1 | 574 |
| 120 | OR2A25(S75N,A209 P) | 371 | 324 | OR6B2 | 575 |
| 121 | OR2A4 | 372 | 325 | OR6B3 | 576 |
| 122 | OR2A42 | 373 | 326 | OR6B3(C234Y) | 577 |
| 123 | OR2A5 | 374 | 327 | OR6C1(C130Y,H 165D,V246I) | 578 |
| 124 | OR2A7 | 375 | 328 | OR6C2 | 579 |
| 125 | OR2AE1 | 376 | 329 | OR6C3 | 580 |
| 126 | OR2AE1(I77T) | 377 | 330 | OR6C4 | 581 |
| 127 | OR2AG1 | 378 | 331 | OR6C6 | 582 |
| 128 | OR2AG2(Y28C) | 379 | 332 | OR6C65 | 583 |
| 129 | OR2AJ1 | 380 | 333 | OR6C65(T222A) | 584 |
| 130 | OR2AK2(S84N) | 382 | 334 | OR6C68(A45T) | 585 |
| 131 | OR2AK2(V188M) | 381 | 335 | OR6C70(L181P) | 586 |
| 132 | OR2AP1 | 383 | 336 | OR6C74 | 587 |
| 133 | OR2AT4 | 834 | 337 | OR6C75 | 588 |
| 134 | OR2B11(I13OS,V19 8M) | 386 | 338 | OR6C76 | 589 |
| 135 | OR2B11(V198M,G2 23D) | 385 | 339 | OR6F1 | 590 |
| 136 | OR2B2 | 387 | 340 | OR6J1 | 591 |
| 137 | OR2B3 | 388 | 341 | OR6J1(P250S) | 592 |
| 138 | OR2B6 | 389 | 342 | OR6K2 | 593 |
| 139 | OR2C1 | 390 | 343 | OR6K3 | 594 |
| 140 | OR2C3(T20A,P68S) | 391 | 344 | OR6K3(P228S,P 248L) | 595 |
| 141 | OR2D2 | 392 | 345 | OR6K6 | 596 |
| 142 | OR2D2(M202T) | 393 | 346 | OR6M1 | 597 |
| 143 | OR2D3 | 394 | 347 | OR6N1 | 598 |
| 144 | OR2D3(L66I,W149S ) | 395 | 348 | OR6N1(I194T,F2 45L,Q261R) | 599 |

EP 4 664 463 A1

(continued)

| # | Receptor | SEQ ID NO DNA encoding the modified receptor including an N-terminal mmLucy-FLAG-rho tag (SEQ ID NO: 81) and a modified C-terminus (SEQ ID NO: 221) | # | Receptor | SEQ ID NO DNA encoding the modified receptor including an N-terminal mmLucy-FLAG-rho tag (SEQ ID NO: 81) and a modified C-terminus (SEQ ID NO: 221) |
|---|---|---|---|---|---|
| 145 | OR2F1 | 396 | 349 | OR6N2 | 600 |
| 146 | OR2F2 | 397 | 350 | OR6P1 | 601 |
| 147 | OR2G2 | 398 | 351 | OR6Q1 | 602 |
| 148 | OR2G2(V120L,L167 P,R236G) | 399 | 352 | OR6Q1((D100G, Y173C) | 603 |
| 149 | OR2G3 | 400 | 353 | OR6S1(R237H) | 605 |
| 150 | OR2G6 | 401 | 354 | OR6S1(T42I,V15 6I) | 604 |
| 151 | OR2H1 | 402 | 355 | OR6T1 | 606 |
| 152 | OR2H2(L30F) | 403 | 356 | OR6V1 | 607 |
| 153 | OR2H2(L30F,A48V) | 404 | 357 | OR6X1 | 608 |
| 154 | OR2J2 | 405 | 358 | OR6Y1 | 609 |
| 155 | OR2J2(Y74H,V146A ,T218A) | 406 | 359 | OR7A10 | 610 |
| 156 | OR2J3(M261I) | 407 | 360 | OR7A17 | 611 |
| 157 | OR2K2 | 408 | 361 | OR7A5 | 612 |
| 158 | OR2L13 | 409 | 362 | OR7C1(V126I) | 613 |
| 159 | OR2L2 | 410 | 363 | OR7C2 | 614 |
| 160 | OR2L2(V259L) | 411 | 364 | OR7D2 | 615 |
| 161 | OR2L3 | 412 | 365 | OR7D4 | 616 |
| 162 | OR2L5 | 413 | 366 | OR7E24 | 617 |
| 163 | OR2L8(G196C,A202 T,Y217C,H226R) | 414 | 367 | OR7E24(P242S) | 618 |
| 164 | OR2M2 | 415 | 368 | OR7G1 | 619 |
| 165 | OR2M3 | 416 | 369 | OR7G1(V83A,W 141C,Y252C) | 620 |
| 166 | OR2M4 | 417 | 370 | OR7G2 | 621 |
| 167 | OR2M5 | 418 | 371 | OR7G3 | 622 |
| 168 | OR2M7 | 419 | 372 | OR7G3(M29V) | 623 |
| 169 | OR2M7(F35L,V78A, D191N) | 420 | 373 | OR8A1 | 624 |
| 170 | OR2S2 | 421 | 374 | OR8B12 | 625 |
| 171 | OR2S2(R17G,M143 V) | 422 | 375 | OR8B2 | 626 |
| 172 | OR2T1 | 423 | 376 | OR8B3 | 627 |

(continued)

| # | Receptor | SEQ ID NO *DNA encoding the modified receptor including an N-terminal mmLucy-FLAG-rho tag (SEQ ID NO: 81) and a modified C-terminus (SEQ ID NO: 221)* | # | Receptor | SEQ ID NO *DNA encoding the modified receptor including an N-terminal mmLucy-FLAG-rho tag (SEQ ID NO: 81) and a modified C-terminus (SEQ ID NO: 221)* |
|---|---|---|---|---|---|
| 173 | OR2T10 | 424 | 377 | OR8B4(E22G) | 628 |
| 174 | OR2T11 | 425 | 378 | OR8B4(Y131H,C 140F,C178R) | 629 |
| 175 | OR2T12 | 426 | 379 | OR8B8 | 630 |
| 176 | OR2T12(R55T) | 427 | 380 | OR8D1 | 631 |
| 177 | OR2T2 | 428 | 381 | OR8D2 | 632 |
| 178 | OR2T27(L36V) | 429 | 382 | OR8D4(R133K,L 283P) | 633 |
| 179 | OR2T29 | 430 | 383 | OR8G1(stop259 Y) | 634 |
| 180 | OR2T3 | 431 | 384 | OR8G5 | 635 |
| 181 | OR2T33(A169V) | 432 | 385 | OR8G5(G204E) | 636 |
| 182 | OR2T34 | 433 | 386 | OR8H1 | 637 |
| 183 | OR2T35 | 434 | 387 | OR8H2 | 638 |
| 184 | OR2T4 | 435 | 388 | OR8H3(P137S) | 639 |
| 185 | OR2T5 | 436 | 389 | OR8I2 | 640 |
| 186 | OR2T6 | 437 | 390 | OR8J1 | 641 |
| 187 | OR2T6(S243A) | 438 | 391 | OR8J3 | 642 |
| 188 | OR2T7 | 439 | 392 | OR8K1 | 643 |
| 189 | OR2T8 | 440 | 393 | OR8K3(L122R) | 644 |
| 190 | OR2V1 | 441 | 394 | OR8K5 | 645 |
| 191 | OR2V2 | 443 | 395 | OR8S1(M48V,L8 2P,A198T) | 646 |
| 192 | OR2V2(H221R) | 442 | 396 | OR8U1 | 647 |
| 193 | OR2W1 | 444 | 397 | OR8U8 | 648 |
| 194 | OR2W3 | 446 | 398 | OR8U9 | 649 |
| 195 | OR2W3(R179C,V19 0I,E196D,M272K,M2 75T) | 445 | 399 | OR9A2 | 650 |
| 196 | OR2W5 | 447 | 400 | OR9A4 | 651 |
| 197 | OR2Y1 | 448 | 401 | OR9G1(T62I) | 652 |
| 198 | OR2Z1 | 449 | 402 | OR9G4(N191D) | 653 |

(continued)

| # | Receptor | SEQ ID NO *DNA encoding the modified receptor including an N-terminal mmLucy-FLAG-rho tag (SEQ ID NO: 81) and a modified C-terminus (SEQ ID NO: 221)* | # | Receptor | SEQ ID NO *DNA encoding the modified receptor including an N-terminal mmLucy-FLAG-rho tag (SEQ ID NO: 81) and a modified C-terminus (SEQ ID NO: 221)* |
|---|---|---|---|---|---|
| 199 | OR3A1 | 451 | 403 | OR9G9 | 654 |
| 200 | OR3A1(R125Q) | 450 | 404 | OR9I1 | 655 |
| 201 | OR3A2 | 452 | 405 | OR9K2 | 656 |
| 202 | OR3A3 | 453 | 406 | OR9K2(R23C,E81A,R185H) | 657 |
| 203 | OR4A47 | 455 | 407 | OR9Q1 | 658 |
| 204 | OR4A47(I104L,V145 M) | 454 | 408 | OR9Q2 | 659 |

SEQ ID NOs 331-739 also include a 5' BamHI restriction site (GGATCC) and Kozak sequence (GCCACC), and a 3' NotI restriction site (GCGGCCGC) for cloning and expression purposes.

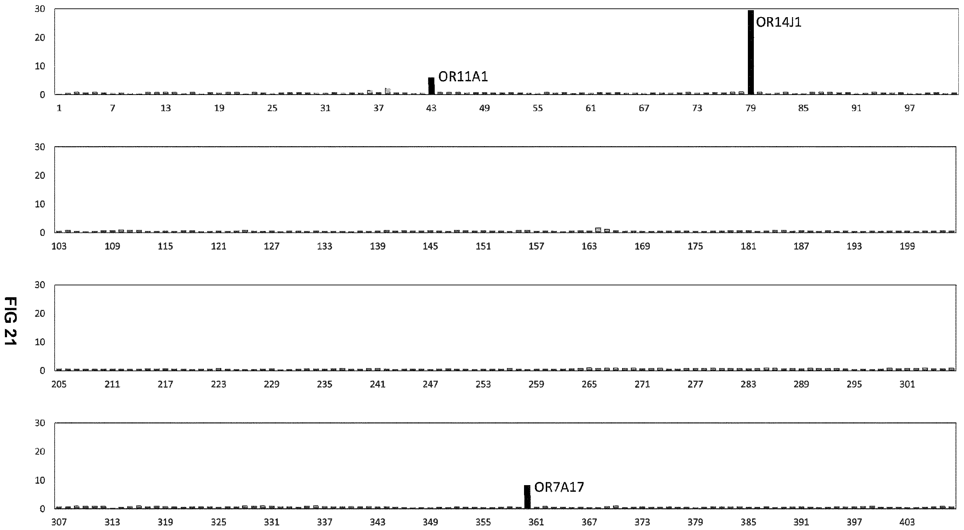

Parallel transfection experiments were conducted with all these plasmids (n = 408) and cells expressing the different OR were stimulated with one given ligand at a time, tested at the maximal, clearly non-cytotoxic concentration. In total 26 different ligands were tested on the full library of class II receptors. For 25 of these ligands, at least one receptor was identified with a > 4-fold luciferase induction over the background, indicating that a cognate receptor can be identified with these improved OR library for a majority of odorants. In general, the screening with this library yields a very good signal-to-noise ratio, clearly separating positive screening hits from inactive ligand - OR associations. As an example, the screening of the full library with the ligand Patchoulol is shown in Figure 21. Patchoulol gave a strong induction of OR14J1 and a weak induction of OR11A1 and OR7A17. The full set of identified OR form this deorphanisation campaign is shown in Table 16.

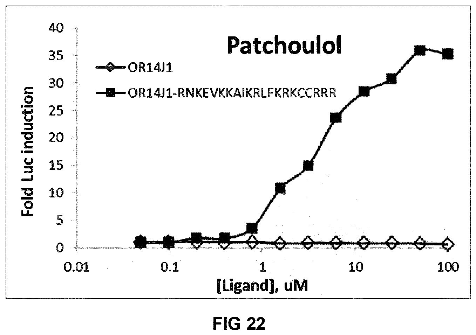

Selected ligand - OR pairs were then tested in a confirmatory test with a dose response analysis. For all tested ligand - OR pairs from newly de-orphanized receptors(n = 31), a clear dose-response was obtained when these screening hits were verified in the confirmatory tests (for examples see ORs listed in example 19 and the dose-response of Patchoulol on OR14J1 in Figure 22), showing that with this improved OR library with a high signal-to-noise ratio, this screening leads to very reproducible ligand-OR associations. This can be compared with the state of the art technology described in Mainland et al. (2015) Sci Data 2:150002 applying OR expression with RTP1S and a N-terminal rho-tag, but employing wild-type OR sequences. In that screening a comprehensive OR library (Class I and class II; 511 clones including key variants) was tested on 73 odorants. The initial screen gave 1572 odor/receptor pair hits (covering 394 OR), yet only for 63 clones representing only 27 OR, the associations could be verified in a dose-response analysis indicating a significant noise in the primary screen due to poor or absent expression of the wild-type sequences.

Table 16. Ligands tested on the full library of OR and cognate receptors identified for these ligands

| Ligand | Activated OR and OR variants |
|---|---|
| (E,S)-3,7-dimethylnon-6-en-1-ol (rosabloom) | OR1D2, OR2A25, OR2A25(S75N,A209P), OR2A5 |
| 3-Mercapto-3-methylhexan-1 -ol | OR2M2, OR2V1 |
| Ambrettolide | OR1N2(W23R,V230G,T287M); OR5A2 |
| Ambrofix | OR7A17, OR7E24, OR7E24(P242S) |

(continued)

| Ligand | Activated OR and OR variants |
|---|---|
| 7-(3-Methylbutyl)-benzo[b][1,4]dioxepin-3-one (Azurone) | OR10H1, OR10K1 |
| Benzaldehyde | OR5P3, OR6P1 |
| Benzyl salicylate | OR2AG2(Y28C) |
| Calone | OR10H2, OR10H5 |
| delta-Damascone | OR8K3(L122R) |
| Ethyl cyclohexanecarboxylate (Esterly) | OR2L2, OR2L2(V259L), OR2L3, OR2L5, OR11G2, OR11G2(I65N, V82I) |
| Geosmin | OR11A1 |
| Mahonial | OR10J5 |
| 3-(4-isobutyl-2-methylphenyl)propanal (Nympheal) | OR10J5 |
| Isoeugenol | OR10D3, OR10G7(T5S) |
| Muscone | OR5AN1, OR5A2 |
| Patchoulol | OR14J1, OR7A17, OR11A1 |
| Peonile | OR2A25, OR2A25(S75N,A209P), OR8H1 |
| Rotundone | OR7A10, OR7A5, OR7A17 |
| Indole | OR5P3 |
| Heliotropine | OR5P3 |
| Methyl salicylate | OR5P3 |
| Galbanone | OR11G2, OR11G2(I65N,V82I) |
| Javanol | OR7A17 |
| Timberol | OR7C1 |
| trans 2, cis 6-Nonadienal | OR10J5 |
| 2,4,6-trichloroanisol | OR5V1 |

Example 19: Activation of OR identified in a screening with a library of all human OR with an improved C-terminal domain - comparison of wild-type OR with the improved OR

The improved functional expression of receptors of the OR-library described in example 18 with the C-terminal domain RNKEVK**KAI**KRL**F**KRKCC**RRR** (SEQ ID NO: 221) was further analyzed. For selected receptors, the wild-type sequence was synthesized and cloned into pcDNA3.1(+). Dose-response analysis with the cognate ligand was then performed for both the wild-type and the modified sequence. Data were analyzed for induction threshold (concentration for 2-fold luciferase induction) and for the EC50 (potency) and for maximal induction (efficacy), as done also in Table 1. As shown in Table 17, for 13 OR identified in the screening in Example C, the wild-type was completely inactive, while the OR with the optimized C-terminal domain was activated by the cognate ligands in the low micromolar range (all-or-nothing effect). This proves, that these OR could only be de-orphanized by this library of OR with improved expression by the C-terminal modification. For 9 more tested OR, the improved sequence leads to a clearly reduced detection threshold (5.2 - 163-fold) and increased efficacy (1.9 - 16.8 fold).

Table 17. improved functional expression of modified OR-genes containing the optimized C-terminus RNKEVK**KA**IKRL**F**KRKCC**RRR** (SEQ ID NO: 221) as compared to the wild-type sequence

| Receptor | | | | Detection threshold; concentration for 2-fold induction (μM) | | | Potency (μM) | | | Fold improvement efficacy |
|---|---|---|---|---|---|---|---|---|---|---|
| OR (variant) | NCBI Gene ID (seque nce source wild-type) | SEQ ID NO modifie d sequen ce | Ligand | wild-type | chang ed C-term. | Fold-improvem ent | EC 50 wild-type | EC50 chang ed C-term | Fold-impro vement | |
| a) OR which are inactive as wild-type and only active with modified C-terminal domain, only de-orphanized by the screening of the library as described in Example B | | | | | | | | | | |
| OR2L3 | 391192 | SEQ ID NO: 412 | Esterly | no inducti on | 11.2 | n.a. | n.a. | 86.2 | n.a. | 17.1 |
| OR2AG2(Y 28C) | 338755 | SEQ ID NO: 379 | Benzyl salicylat e | no inducti on | 1.3 | n.a. | n.a. | 4.5 | n.a. | 10.2 |
| OR7A5 | 26659 | SEQ ID NO: 612 | Rotund one | no inducti on | 0.67 | n.a. | n.a. | 5.6 | n.a. | 24.1 |
| OR7E24 | 26648 | SEQ ID NO: 617 | Ambrofi x | no inducti on | 0.47 | n.a. | n.a. | 7.7 | n.a. | 20.7 |
| OR7A10 | 390892 | SEQ ID NO: 610 | Rotund one | no inducti on | 0.005 8 | n.a. | n.a. | 0.16 | n.a. | 5.9 |
| OR10H2 | 26538 | SEQ ID NO: 680 | Cascalo ne | no inducti on | 108.6 | n.a. | n.a. | 226 | n.a. | 7.0 |
| OR10H1 | 26539 | SEQ ID NO: 679 | Azurone | no inducti on | 3.13 | n.a. | n.a. | 7.0 | n.a. | 10.0 |
| OR10D3 | 26497 | SEQ ID NO: 671 | Isoeuge nol | no inducti on | 1.4 | n.a. | n.a. | 8.4 | n.a. | 6.1 |
| OR1D2 | 4991 | SEQ ID NO: 336 | rosablo om | no inducti on | 3.0 | n.a. | n.a. | 34.6 | n.a. | 14.9 |
| OR2A5 | 393046 | SEQ ID NO: 374 | Rosyfoli a | no inducti on | 17.6 | n.a. | n.a. | 18 | n.a. | 3.0 |
| OR2A25 | 392138 | SEQ ID NO: 370 | Rosyfoli a | no inducti on | 1.1 | n.a. | n.a. | 11.0 | n.a. | 7.6 |
| OR11G2 | 390439 | SEQ ID NO: 703 | Galban one | no inducti on | 8.8 | n.a. | n.a. | 2.2 | n.a. | 3.1 |
| OR14J1 | 442191 | SEQ ID NO: 738 | Patchou lol | no inducti on | 0.45 | n.a. | n.a. | 2.2 | n.a. | 33.4 |
| b) OR which are active with the wild-type sequence, but for which the assay sensitivity is significantly improved by the modified C-terminal domain | | | | | | | | | | |
| OR5A1 | 219982 | SEQ ID NO: 514 | β-Ionone | 20.36 | 2. 1 | 9.9 | 48. 3 | 15.1 | 3.2 | 2.4 |
| OR5M3 | 219482 | SEQ ID NO: 559 | Furaneo l | 30.5 | 0.3 | 100.7 | 65. 3 | 8.7 | 7.5 | 16.8 |
| OR8D1 | 283159 | SEQ ID NO: 631 | Sotolon e | 2.5 | 0.48 | 5.2 | 19. 0 | 7.5 | 2.5 | 2.1 |
| OR10G3(S7 3G) | 26533 | SEQ ID NO: 673 | Vanillin | 49.0 | 0.3 | 163.4 | 261 | 15.3 | 17.0 | 2.9 |
| OR10G9 | 219870 | SEQ ID NO: 678 | Ultravan il | 14.6 | 1.7 | 8.62 | 91. 4 | 19.0 | 4.8 | 3.4 |
| OR2L5 | 81466 | SEQ ID NO: 413 | Esterly | 40.5 | 0.26 | 158.8 | 114 .6 | 4.76 | 24.1 | 2.6 |
| OR8H1 | 21946 9 | SEQ ID NO: 637 | Peonile | 6.9 | 0.4 | 15.6 | 17. 3 | 5.8 | 2.95 | 4.2 |

(continued)

| Receptor | | | | Detection threshold; concentration for 2-fold induction (μM) | | | Potency (μM) | | | Fold improvement efficacy |
|---|---|---|---|---|---|---|---|---|---|---|
| OR (variant) | NCBI Gene ID (seque nce source wild-type) | SEQ ID NO modifie d sequen ce | Ligand | wild-type | chang ed C-term. | Fold-improvem ent | EC 50 wild -type | EC50 chang ed C-term | Fold-impro vement | |
| OR10K1 | 391109 | SEQ ID NO: 687 | Azurone | 9. 4 | 0.15 | 63.4 | 6.8 | 2.2 | 3.1 | 12.4 |
| OR11A1 | 26531 | SEQ ID NO: 702 | Geosmi n | 1.23 | 0.04 | 30.7 | 6.8 | 2.2 | 3.1 | 1.9 |
| n.a. not applicable, cannot be calculated as wild-type is inactive | | | | | | | | | | |

Example 20: Optimized expression with SEQ ID NO: 221 as compared to SEQ ID NO: 86

All tested receptors of Table 1 gave improved functional expression when the wild-type C-terminal domain was replaced with SEQ ID NO: 86. This functional expression was further improved when SEQ ID NO:86 was replaced with SEQ ID NO: 221 as shown for OR7C1 in Table 8 and for OR5B12 in Table 9. This additive improvement was further tested with OR10H5 (Table 18) and OR7A17 (Table 19). In both cases, SEQ ID NO: 221 further improved functional expression as compared to the already strongly improved functional expression vs. wild-type when using SEQ ID NO: 86. The full DNA sequences encoding the modified receptors are SEQ ID NO: 684 for OR10H5 and SEQ ID NO: 611 for OR7A17.

Table 18. improved functional expression of OR10H5

| OR10H5 | fold induction of luciferase | | |
|---|---|---|---|
| C-terminal Sequence | | | |

| | 10 $\mu$M Calone | 31 $\mu$M Calone | 100 $\mu$M Calone |
|---|---|---|---|
| wild-type | 1.00 | 1.21 | 2.71 |
| RNKEVKDALKRLLKRKCC (SEQ ID NO: 86) | 1.09 | 1.92 | 5.52 |
| RNKEVKKAIKRLFKRKCCRRR (SEQ ID NO: 221) | 2.09 | 8.00 | 10.74 |

Table 19. Improved functional expression of OR7A17

| OR7A17 | fold induction of luciferase | | | |
|---|---|---|---|---|
| C-terminal Sequence | 0.01 $\mu$M Ambrofix | 0.03 $\mu$M Ambrofix | 0.1 $\mu$M Ambrofix | 0.3 $\mu$M Ambrofix |
| wild-type | 1.05 | 1.10 | 1.10 | 1.17 |
| RNKEVKDALKRLLKRKCC (SEQ ID NO: 86) | 1.55 | 2.72 | 5.88 | 9.60 |
| RNKEVKKAIKRLFKRKCCRRR (SEQ ID NO: 221) | 2.63 | 5.24 | 7.75 | 11.85 |

Example 21: improved C-terminal domains for class I OR

Further variants of the improved C-terminal domain sequence RNKEVKDALKRLLKRKCC (SEQ ID NO: 86) and falling within the general sequence RN[KR][EDQ][VMIL][KR]xA[LIV][KRH][KR][LI][LIF][KRG][KR][KR] (SEQ ID NO: 1) with further amino acids at the C-terminal end (CC) were tested on the class I receptor OR52A5 activated by 4-ethyloctanoic acid. As shown in Table 20, SEQ ID NO: 86 gives a clearly improved expression as compared to the wild-type. This activation is further improved by either replacing amino acid position 4 - 6 with the sequence QIR or by adding the terminal sequence RRR.

These two improvements were further combined and the combination was tested on OR52A5 and two further class I receptors. As indicated in Table 21, all three class I receptors are highly active with the modified C-terminal sequence RNK**QIR**DALKRLLKRKCC**RRR** (SEQ ID NO: 741), with no or weak activity with the wild-type. In the case of OR52A4, 4-ethyloctanoic acid, which is detected at low concentrations by the human nose, is detected with a high luciferase response already at 0.19 $\mu$M by the receptor with the C-terminal modification showing very sensitive detection of this carboxylic acid by the modified class I receptor.

Table 20. Optimized C-terminal domains for Class I OR52A5

| C-terminal Sequence | fold induction of luciferase by 4-ethyloctanoic acid | | | |
|---|---|---|---|---|
| | 1 $\mu$M | 3.1 $\mu$M | 10 $\mu$M | 31 $\mu$M |
| Wild-type | 0.98 | 1.13 | 1.67 | 2.13 |
| RNKEVKDALKRLLKRKCC (SEQ ID NO: 86) | 2.21 | 2.81 | 3.53 | 7.64 |
| RNK**QIR**DALKRLLKRKCC (SEQ ID NO: 740) | 10.09 | 12.84 | 12.30 | 19.31 |
| RNKEVKDALKRLLKRKCC**RRR** (SEQ ID NO: 156) | 9.39 | 13.73 | 14.90 | 22.00 |

Table 21. Optimized C-terminal domains for Class I OR52E8, OR56A4 and OR52A5

| | fold induction of luciferase | | | |
|---|---|---|---|---|
| | 1 μM 3-methyl-3-hydroxyhexanoi c acid | 3.1 μM 3-methyl-3-hydroxyhexanoi c acid | 10 μM 3-methyl-3-hydroxyhexanoi c acid | 31 μM 3-methyl-3-hydroxyhexanoi c acid |
| OR52E8-wild-type | 1.07 | 0.98 | 1.03 | 0.93 |
| OR52E8-RNK**QIR**DALKRLLKRKCC (SEQ ID NO: 740) | 4.79 | 8.52 | 13.13 | 14.20 |
| OR52E8-RNK**QIR**DALKRLLKRKCC**RR** (SEQ ID NO: 741) | 9.76 | 19.80 | 24.48 | 34.54 |
| | 1 μM 2-methylundecano ic acid | 3.1 μM 2-methylundecano ic acid | 10 μM 2-methylundecano ic acid | 31 μM 2-methylundecano ic acid |
| OR56A4-Wild Type | 0.82 | 0.85 | 0.91 | 1.82 |
| OR56A4-RNK**QIR**DALKRLLKRKCC (SEQ ID NO: 740) | 4.05 | 6.94 | 9.99 | 12.60 |
| OR56A4-RNK**QIR**DALKRLLKRKCC**RR** (SEQ ID NO: 741) | 9.19 | 14.53 | 16.91 | 22.23 |
| | 0.19 μM 4-ethyloctanoic acid | 0.78 μM 4-ethyloctanoic acid | 3.15 μM 4-ethyloctanoic acid | 12.5 μM 4-ethyloctanoic acid |
| OR52A5-RNKEVKDALKRLLKRKCC (SEQ ID NO: 86) | 1.03 | 1.28 | 1.35 | 1.43 |
| OR52A5-RNKEVKDALKRLLKRKCC**RR** (SEQ ID NO: 156) | 1.80 | 3.59 | 4.49 | 8.99 |
| OR52A5-RNK**QIR**DALKRLLKRKCC (SEQ ID NO: 740) | 2.48 | 3.88 | 5.00 | 7.28 |
| OR52A5-RNK**QIR**DALKRLLKRKCC**RR** (SEQ ID NO: 741) | 6.04 | 7.86 | 13.96 | 12.83 |

Thus, additional improved C-terminal domain general sequences are

RN[KR]QIRxA[LIV][KRH][KR][LI][LIF][KRG][KR][KR] (SEQ ID NO: 822),
RN[KR][EDQ][VMIL][KR]xA[LIV][KRH][KR][LI][LIF][KRG][KR][KR]RRR (SEQ ID NO: 823), and
RN[KR]QIRxA[LIV][KRH][KR][LI][LIF][KRG][KR][KR]RRR (SEQ ID NO: 824).

Example 22: Screening with a library of all class I OR with an identical improved C-terminal domain

All human class I OR and all key genetic variants of these OR were synthesized with a sequence coding for the identical C-terminal domain RNK**QIR**DALKRLLKRKCC**RRR** (SEQ ID NO: 741) in the vector pcDNA3.1(+) to generate a library of all class I OR with an improved C-terminal domain. The complete list of class II ORs included in this library is given in Table 22:

Table 22. Class I OR library members

| # | Receptor | SEQ ID NO *DNA encoding the modified receptor including an N-terminal mmLucy-FLAG-rho tag (SEQ ID NO: 81) and a modified C-terminus (SEQ ID NO: 741)* |
|---|---|---|
| 1 | OR51A2 | 742 |
| 2 | OR51A2(S218F,K289N) | 743 |
| 3 | OR51A4(T288M) | 744 |
| 4 | OR51A7 | 745 |
| 5 | OR51B2 | 746 |
| 6 | OR51B2(C120R,C209S,P283S) | 747 |
| 7 | OR51B2(C120R,L134F,C209S) | 748 |
| 8 | OR51B4 | 749 |
| 9 | OR51B4(V36I) | 750 |
| 10 | OR51B4(V36I,M147T) | 751 |
| 11 | OR51B5 | 752 |
| 12 | OR51B5(G5S) | 753 |
| 13 | OR51B5(I102T,P160L) | 754 |
| 14 | OR51B6 | 755 |
| 15 | OR51B6(var 3) | 756 |
| 16 | OR51D1 | 757 |
| 17 | OR51E1 | 758 |
| 18 | OR51E2 | 759 |
| 19 | OR51F1 | 760 |
| 20 | OR51F2 | 761 |
| 21 | OR51G1 | 762 |
| 22 | OR51G2 | 763 |
| 23 | OR51I1 | 764 |
| 24 | OR51I1(A252S) | 765 |
| 25 | OR51I2 | 766 |
| 26 | OR51J1 | 767 |
| 27 | OR51L1 | 768 |
| 28 | OR51M1 | 769 |
| 29 | OR51Q1 | 770 |

(continued)

| # | Receptor | SEQ ID NO *DNA encoding the modified receptor including an N-terminal mmLucy-FLAG-rho tag (SEQ ID NO: 81) and a modified C-terminus (SEQ ID NO: 741)* |
|---|---|---|
| 30 | OR51S1 | 771 |
| 31 | OR51S1(Q45E,L163R,L249F) | 772 |
| 32 | OR51T1 | 773 |
| 33 | OR51V1 | 774 |
| 34 | OR51V1(L30F) | 775 |
| 35 | OR52A1 | 776 |
| 36 | OR52A5 | 777 |
| 37 | OR52B2 | 778 |
| 38 | OR52B4 | 779 |
| 39 | OR52B6(V267I) | 780 |
| 40 | OR52B6(T36A,V267I) | 781 |
| 41 | OR52D1 | 782 |
| 42 | OR52E2(N5S) | 783 |
| 43 | OR52E2(H174R,R264C) | 784 |
| 44 | OR52E4 | 785 |
| 45 | OR52E4(V176I,R184M) | 786 |
| 46 | OR52E5(P234L) | 787 |
| 47 | OR52E6 | 788 |
| 48 | OR52E6 (var2) | 789 |
| 49 | OR52E8 | 790 |
| 50 | OR52H1(H124R) | 791 |
| 51 | OR52H1(A83T,H124R,G229C) | 792 |
| 52 | OR52I1(T411) | 793 |
| 53 | OR52I2 | 794 |
| 54 | OR52I2(T141M) | 795 |
| 55 | OR52J3 | 796 |
| 56 | OR52J3(T77A,V128I,Q141L) | 797 |
| 57 | OR52K1 | 798 |
| 58 | OR52K1(Q52R) | 799 |
| 59 | OR52K2 | 800 |
| 60 | OR52L1(C125R,C146R,K168T,W282R) | 801 |
| 61 | OR52M1 | 802 |
| 62 | OR52N1(R167C,F247I) | 803 |
| 63 | OR52N1(A101T,C125Y,F247I) | 804 |
| 64 | OR52N2 | 805 |
| 65 | OR52N2(S249A,H264R) | 806 |
| 66 | OR52N4 (L167R,N218I) | 807 |
| 67 | OR52N5 | 808 |

(continued)

| # | Receptor | SEQ ID NO *DNA encoding the modified receptor including an N-terminal mmLucy-FLAG-rho tag (SEQ ID NO: 81) and a modified C-terminus (SEQ ID NO: 741)* |
|---|---|---|
| 68 | OR52R1 | 809 |
| 69 | OR52R1(I129T,N201Y,S245A) | 810 |
| 70 | OR52W1(H239R,L254Q) | 811 |
| 71 | OR56A1 | 812 |
| 72 | OR56A3 | 813 |
| 73 | OR56A3(M51T) | 814 |
| 74 | OR56A4 | 815 |
| 75 | OR56A5 | 816 |
| 76 | OR56B1(C103R) | 817 |
| 77 | OR56B4 | 818 |
| 78 | OR56B4(P277S) | 819 |

SEQ ID NOs 742-819 also include a 5' BamHI restriction site (GGATCC) and Kozak sequence (GCCACC), and a 3' NotI restriction site (GCGGCCGC) for cloning and expression purposes.

Parallel transfection experiments were conducted with all these plasmids (n = 77, SEQ ID NO: 760, OR51F1 was excluded due to poor plasmid yield) and cells expressing the different OR were stimulated with one given ligand at a time. In total 10 different carboxylic acids ligands were tested on the full library of class I receptors. For 8 of these ligands, at least one receptor was identified with a > 3-fold luciferase induction over the background (Table 23), indicating that a cognate receptor can be identified with these improved OR library especially for carboxylic acids.

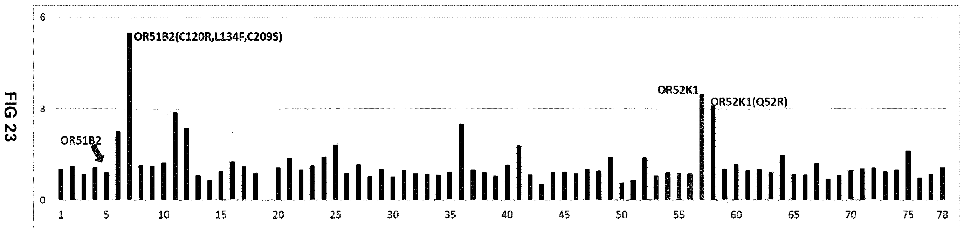

As an example, the screening of the full library with 3-methyl-2-hexenoic acid, which is an important constituent of human sweat (Natsch et al. A specific bacterial aminoacylase cleaves odorant precursors secreted in the human axilla. The Journal of biological chemistry 2003; 278(8):5718-5727), is shown in Figure 23. 3-methyl-2-hexenoic acid gave a strong induction of the OR51B2(C120R, L134F, C209S) variant but not of the wild-type variant of OR51B2. This is in contradiction to Li et al. (PLoS Genet. 2022 Feb 3;18(2):e1009564) who reported that the wild-type but not the (C120R, L134F, C209S)-variant of OR51B2 is activated by 3-methyl-2-hexenoic acid. Thus, contrary to the expression data in that publication, the (C120R, L134F, C209S)-variant of OR51B2 is a preferred target to screen antagonists for human sweat odor. In addition, 3-methyl-2-hexenoic acid activates also both tested variants of OR52K1, which are thus further new targets to screen for malodor antagonists. Next to 3-methyl-2-hexenoic acid, 4-methyl-3-hexenoic acid is also activating the (C120R, L134F, C209S)-variant of OR51B2. This acid also activates both tested variants of OR51B5. 4-methyl-3-hexenoic acid is an important contributor to the typical malodor in laundry (Kubota et al., Appl Environ Microbiol. 2012;78(9):3317-24). Thus, screening additionally antagonists for OR51 B5 can be used to target this specific malodor. For the most dominant carboxylic acid in human sweat odor, namely 3-methyl-3-hydroxyhexanoic acid, screening the complete library of class I OR found only one hit, OR52E8 confirming above results that for screening antagonists to this acid, OR52E8 variants with an improved expression by an optimized C-terminal domain are a key target. Next to OR for these short chain odorant acids, OR52K1, OR52K1(Q52R), OR56A1, OR56A3, OR56A3(M51T) and OR56A4 are activated by C8 - C11 chain acids tested in this screening.

Table 23. Ligands tested on the full library of class I OR and cognate receptors identified for these ligands

| Ligand | Activated OR and OR variants |
|---|---|
| 3-Methyl-3-hydroxyhexanoic acid | OR52E8 |
| 3-Methyl-2-hexenoic acid | OR51B2(C120R,L134F,C209S); OR52K1; OR52K1(Q52R) |
| Octanoic acid | OR51B2(C120R,L134F,C209S); OR52A5; OR52K1; OR52K1(Q52R); OR56A1; OR56A4 |
| 4-Isopropylcyclohex-3-ene carboxylic acid | OR52E8 |
| Phenyl acetic acid | OR51B2(C120R,L134F,C209S); OR51B5; OR51B5(G5S); OR51L1 |

(continued)

| Ligand | Activated OR and OR variants |
|---|---|
| 2-Methyl-undecanoic acid | OR52K1(Q52R); OR56A1; OR56A3; OR56A3(M51T); OR56A4 |
| 4-Methyl-3-hexenoic acid | OR51B2(C120R,L134F,C209S); OR51B5; OR51B5(G5S) |
| Citronellic acid | OR52K1; OR52K1(Q52R); OR56A1; OR56A4 |
| 3-Methylbutanoic acid | No activation observed |
| 2-Methyl-2-pentenoic acid | No activation observed |

Example 23: Screening and matching of complex perfumes (i.e. a complex odorant mixture) with a library of all class II OR with an identical improved C-terminal domain

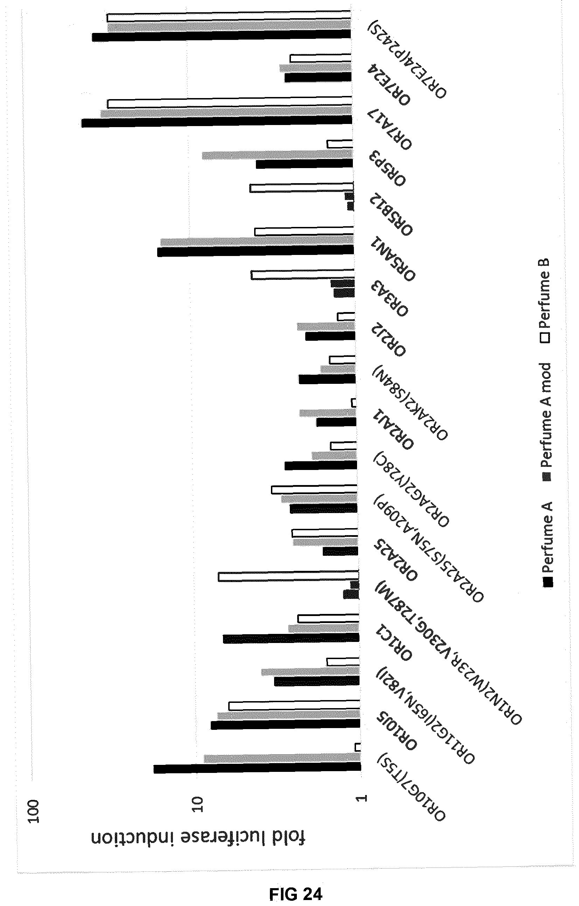

The library of human class II OR of example 18 was further tested with three perfume oils, i.e. complex mixtures of ≥ 24 single perfume ingredients, which are mixed in a specific ratio to give the desired overall odor. "Perfume A" and "Perfume B" are independent perfume formulations with a different overall odor impression, while "Perfume A mod" shares 66% of its ingredient with "Perfume A" and has been modified to keep the overall odor of Perfume A, but by replacing 33% of the single ingredients. In total, 18 OR and OR variants were activated by at least one of these oils with an at least 2-fold luciferase induction. The induction of these activated subset of OR by these three oils is shown in Figure 24. As is obvious from the black and grey bars, "perfume A" and "perfume A mod" elicit a similar pattern of OR activation, while "Perfume B" is clearly different, especially in regards to the activation of OR10G7, OR11G2, OR1N2, OR2AJ1, OR2J2, OR3A3, OR5AN1, OR5B12 and OR5P3. Thus, Perfume B has a different 'fingerprint' of OR activation reflecting its different olfactive character. The difference / similarity of the different oils can also be quantified using a distance measure. For example, the distance between two compositions i and j can e.g. be calculated according to the following formula:

$$Distance = \sum_{OR_1}^{OR_x} |Log(fold\ induction\ perfume\ i) - Log(fold\ induction\ perfume\ j)|$$

For the 18 OR of Figure 24, the distance between "perfume A" and "perfume A mod" is 2.2, while the distance between "Perfume A" and "Perfume B" is 6.4, and the distance between "Perfume A mod" and "Perfume B" is 5.5, showing the similarity between "perfume A" and "perfume A mod" and the difference of both from "perfume B".

These results indicate that the approach of screening the sensitive library of OR with a modified C-terminal domain with complex mixtures can be used to measure similarity of complex odorant mixtures such as perfumes or flavours and give an objective representation of the olfactive profile of such mixtures.

Due to regulatory pressure, enigmatic perfumery ingredient(s) become banned in some countries. It is then desirable, to design a perfume which smells very similar to a consumer but in which the banned ingredient(s) are replaced. Classically, a single ingredient was used to try to replace the olfactory missing part after the removal of a banned ingredient. However, since single ingredients may activate multiple OR and oftentimes multiple ingredients have to be removed, it is important to replace the OR activation pattern of the removed ingredient(s) and reconstruct the overall OR activation pattern of the original perfume, which can be done by one or multiple ingredients.

This can be achieved using the method of screening single ingredients for OR activation as shown in example 18 (class II) and 22 (class I) on the full OR library with a modified C-terminal domain and recording their OR activation pattern to generate a database of activation patterns of single ingredients. Then the original perfume oil and the original perfume oil with the scrutinized ingredients removed are both screened on the full OR library as shown above. After adding selected replacing ingredients, the resulting perfume can then be validated by testing it again on the full library of OR as shown above and the similarity to the original perfume can be calculated in an objective manner.

Example 24: Detection of a cognate odorant in a complex mixture or reaction mixture of low purity

Classically, perfumery ingredients and experimental perfumery ingredients (research samples) need to be highly pure to be evaluated by a perfumer - because the human nose has all ca. 400 OR being functional at the same time, and any odorant impurity thus will affect the overall olfactory impression of a sample, and humans thus have difficulties in judging samples of limited purity. An assay with a single or few expressed receptor(s) on the other hand is focused on one particular odor description, and can thus in principle detect an odorant with that particular odor description against a complex background. However, with a poorly expressed receptor, the matrix will interfere with the assay as the matrix ingredients will quickly reach cytotoxic concentrations in case the active ingredient for the target odor direction to be searched is present at low concentration.

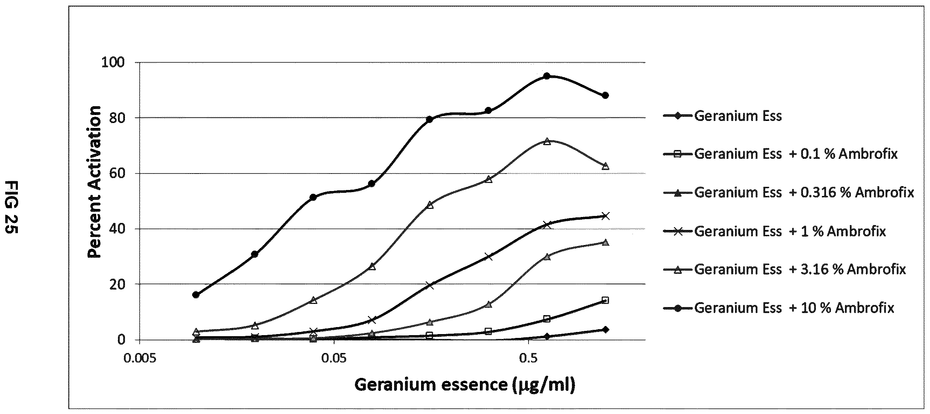

To investigate sensitivity of the modified olfactory receptors of this disclosure to detect an odorant in a complex odorant matrix, the ligand Ambrofix was spiked into a complex essential oil, namely Geranium oil sourced from Egypt, containing 13 different ingredients above 1% serving as an example of a complex background matrix of strongly odorant materials. The spiking levels of Ambrofix were 0%, 0.1%, 0.316%, 1%, 3.16% and 10%. These mixtures were tested with OR7A17 with a C-terminal domain of SEQ ID 221 (a DNA sequence encoding the modified receptor is included as SEQ ID NO: 611) as described in example 20. As shown in Figure 25, the spiked oils significantly induced luciferase expression over the background. Thus, for a potent ligand like Ambrofix, the sensitive assay can detect concentrations down to at least 0.1% of target ingredient against a complex matrix.

Example 25: Screening a library of OR with a mixture of odorants with a particular odor description

Single odorants may trigger activation of multiple OR as shown by the case of Ambrofix in example 18, which activates the specific receptors OR7E24 and OR7A17. On the other hand, multiple odorants may be perceived as and described by certain common odor descriptors due to their common activation of a given set of OR. To trigger a specific odor sensation, thus a specific set of OR may need to be activated. This specific OR set can be identified by mixing several odorants with a given odor description. The mixture is then screened on the full library of OR. The identified set of OR can then be further used to screen for that particular odor description. For example, to identify a typical set of OR for the odor description of "fruity esters", a mixture of the following compounds was made: Pentanoic acid, 2-methyl ethyl ester; Butanoic acid, 3-methyl-, ethyl ester; Acetic acid, phenoxy-, 2-propenyl ester; Hexanoic acid, ethyl ester; Acetic acid, (3-methylbutoxy)-, 2-propenyl ester; Acetic acid, (cyclohexyloxy)-, 2-propenyl ester; Butanoic acid, 2-methyl-, (3Z/E)-3-hexenyl ester; Cyclohexanecarboxylic acid, ethyl ester and Oxiranecarboxylic acid, 3-phenyl, ethyl ester. This mixture was screened against the full library of OR as described in example 18. With this approach a specific set of OR activated by ligands for this specific odor description were identified: Thus OR11G2, OR11G2(I65N,V82I), OR1D2, OR2AK2(S84N) and OR2L5 are identified as the set of OR most strongly activated by the mixture of fruity esters.

Table 24. OR identified by screening class II OR with a mixture of fruity esters.

| OR | Fold luciferase induction (20 ppm fruity ester mix) |
|---|---|
| OR11G2 | 7.2 |
| OR11G2(I65N,V82I) | 22.6 |
| OR1D2 | 12.3 |
| OR2AK2(S84N) | 6.0 |
| OR2L5 | 8.9 |

Similarly, a mixture of odorants with fruity-lactonic descriptors was made containing equal amounts of dodecalactone delta, heptalactone gamma, octalactone gamma, nonalactone gamma, undecalactone gamma, decalactone gamma, dodecalactone gamma, decalactone delta, methyl tuberate and nectaryl. Screening the full library of class II OR with this mixture, a defined set of OR, namely OR10A3, OR10A6(A117V, V140G, L287P), OR10J1(M51I, I92M), OR1D2 and OR2J2 was found to be activated.

Table 25. OR identified by screening class II OR with a mixture of fruity lactones

| OR | Fold luciferase induction (20 ppm lactone mix) |
|---|---|
| OR10A3 | 14.3 |
| OR10A6(A117V,V140G,L287P) | 29.1 |
| OR10J1(M51I,I92M) | 23.7 |
| OR1D2 | 12.6 |
| OR2J2 | 13.8 |

In a subsequent de-convolution experiment, the single ingredients were tested in a dose-response test on all the identified OR. Table 26 lists the concentration for 2-fold luciferase induction (10-fold in the case of OR10A3). These data indicate that all the OR identified are activated by the single ingredients of the mix, with some difference in specificity. Thus, OR2AP1 and OR2J2 are particularly strongly activated by the long-chain dodecalactone gamma, while OR10A3 is very sensitive to several longer chain lactones. Overall, undecalactone gamma is the most potent ligand for four of the five identified OR which is in line with the fact that among the lactones, undecalactone gamma has the lowest olfactory detection threshold *in vivo.* Among the five OR, OR10A3 is the most sensitive. Thus for the most potent ligand undecalactone gamma, already at 0.31µM, 10.2-fold activation is observed.

Table 26. OR identified by screening class II OR with a mixture of fruity lactones tested with individual lactones in a dose-response analysis

| | OR10A3 | OR10A6 (A117V, V140G, L287P) | OR10J1(M51I, I92M) | OR2AP1 | OR2J2 |
|---|---|---|---|---|---|
| | (EC10; µM) | (EC2; µM) | (EC2; µM) | (EC2; µM) | (EC2; µM) |
| Lactone mix [1] | 1.59 | 1.17 | 8.48 | 10.67 | 2.39 |
| Heptalactone gamma | 298.04 | inactive | inactive | inactive | inactive |
| Octalactone gamma | 26.93 | inactive | 101.73 | inactive | 65.20 |
| Nonalactone gamma | 2.83 | 11.34 | 11.14 | inactive | 12.85 |
| Decalactone gamma | 0.84 | 1.62 | 3.63 | 33.66 | 3.27 |
| Decalactone delta | 2.10 | 5.31 | 31.73 | inactive | 98.89 |
| Dodecalactone gamma | 0.85 | 0.48 | 5.31 | **1.31** | 1.41 |
| Dodecalactone delta | 2.59 | 1.11 | 31.38 | 15.76 | 35.12 |
| Methyl Tuberate Pure | 1.44 | 10.35 | 10.89 | 189.82 | 120.58 |
| Nectaryl | 2.32 | 1.12 | inactive | 19.72 | inactive |
| Undecalactone gamma | **0.28** | **0.34** | **2.11** | 6.53 | **0.71** |

[1] An arbitrary molecular weight of 200 was assumed for the lactone mix
OR10A3 is a very sensitive receptor with a high efficacy, therefore here the EC10, concentration for 10-fold OR activation is indicated.

Example 26: Screening odorants with cells expressing multiple receptors

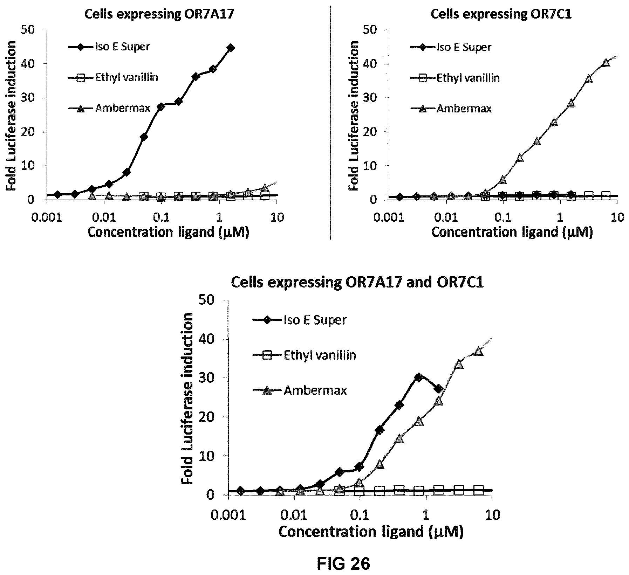

in order to screen for a specific odor description, some or all members of a given set of OR which was identified to be specific for a given odor description as shown in example 25 can be combined in a screening for new odorants or new odorant mixtures. The screening on the different OR can be performed sequentially or in parallel assays with the individual OR of the OR set. Additionally, some or all of the OR of the OR set specific for the odor-description can also be co-expressed in a single cell line. Thus, to screen for woody-ambery molecules, cells were either separately or simultaneously transfected with two plasmids coding for the optimized OR7A17 and OR7C1 both with the C-terminal domain of SEQ ID NO: 221. Cells were then stimulated with the ligand Iso E super, which is a specific ligand for OR7A17 and with Ambermax, which is a specific ligand for OR7C1. As shown in Figure 26, cells solely expressing OR7A17 respond only to Iso E super, while cells expressing OR7C1 only respond to Ambermax. Cells expressing both receptors become functional sensors for the Ambery-woody odor description and detect both ligands down to low concentrations.

Example 27 Determination of more potent ligand for OR10J5 among (S,E)-10-hydroxy-4,8-dimethyldec-4-enal or (R,E)-10-hydroxy-4,8-dimethyldec-4-enal

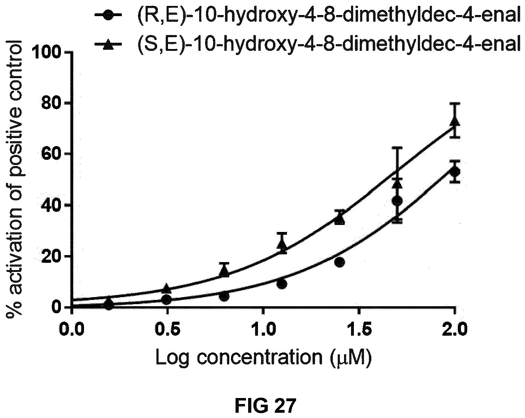

Activation of OR10J5 with the C-terminal domain of SEQ ID NO: 86 by (S,E)-10-hydroxy-4,8-dimethyldec-4-enal or (R,E)-10-hydroxy-4,8-dimethyldec-4-enal was measured in a dose-response analysis.
Potency of the two tested OR10J5 ligands is expressed as the EC20% value, which is the concentration that leads to a 20 % increase of the luciferase activity relative to the positive control (100µM, Mahonial; (4E)-9-hydroxy-5,9-dimethyl-4-Decenal). (S,E)-10-hydroxy-4,8-dimethyldec-4-enal has an EC20% value of 10.1 µM while (R,E)-10-hydroxy-4,8-dimethyldec-4-enal has an EC20% value of 24.4 µM. Because of the difference in EC20%, a much lower concentration of the (S,E)-isomer is needed to activate the receptor compared to the concentration needed of the (R,E)-isomer. Figure 27 shows the response of both test compounds graphically. This example further illustrates the usefulness of the improved assay to determine the most odor-active isomers.

Example 28: Characterization of OR activation vectors

Equipped with OR activation vectors $V_{act,i}$ for a number (n) of compounds or compositions, computational techniques may be used to gain insight into the activation vectors and underlying trends or patterns thereof. Each OR activation vector contains a measured level of activation for a large library of ORs (a 408-dimension vector for the Class II OR library; a 78-dimension vector for the class I OR library; etc.).

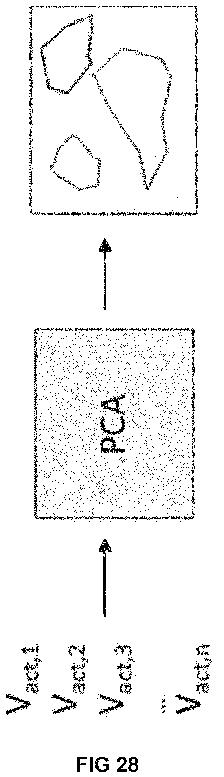

Considering the Class II OR library, principal component analysis (PCA) techniques successfully map the 408-dimension vectors to a lower (2- or 3-) dimension space and show clusters of compounds or compositions that are related to each other. The PCA process on activation vectors is shown schematically in Figure 28, where the resultant plot reduces the data to 2-dimensions and includes indicators of clusters.

Of course, alternative dimensionality reduction techniques may be utilised for a similar purpose, including t-Distributed Stochastic Neighbor Embedding (t-SNE) and Uniform Manifold Approximation and Projection (UMAP).

Using such dimensionality reduction techniques for OR activation vectors, one is able to search, compare, and cluster compounds and compositions (including fragrances and flavours). For example, determination of cosine similarity (or other distance metric, such as that discussed in example 23) on the data may be used to provide a measure of similarity between compounds.

Example 29: Predicting olfactory properties of a compound or a composition based on OR activation data

OR activation data, such as that in the form of Table 28, is labelled data. Labels can include typical olfactory properties including odor descriptors, such as "sweet", "citrus", "baked", "vanilla", "odorless", etc. Each of the (n) tabulated compounds or compositions with activation vector $V_{act,i}$ includes labelled olfactory properties, including odor descriptor(s) and/or physiochemical parameter(s), including vapor pressure, partition coefficient, distribution coefficient, etc.

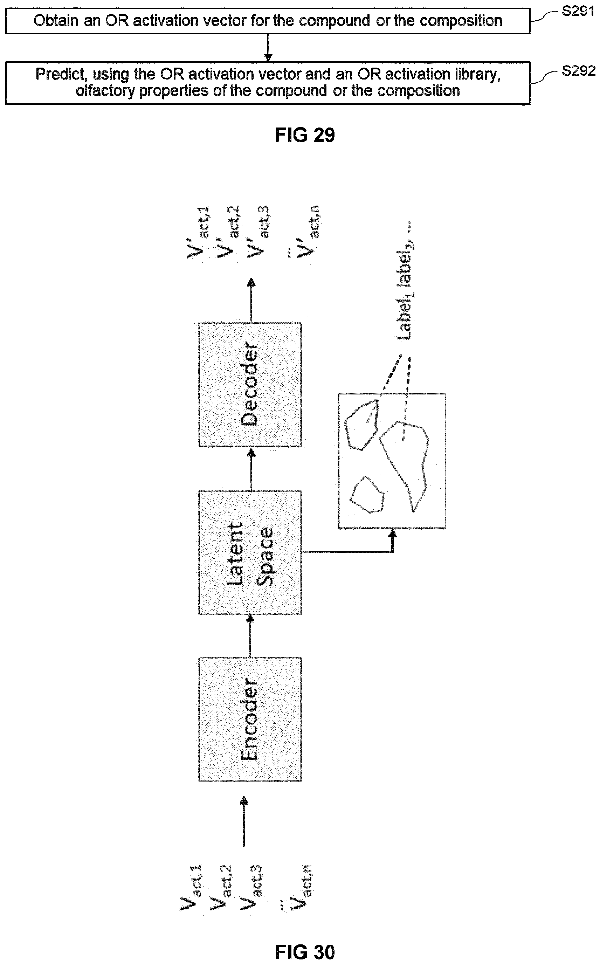

Figure 29 provides an example general method of predicting olfactory properties of a compound or a composition based on OR activation data. At S291, the computer implementing the method obtains an OR activation vector for the compound or the composition. For example, the computer may be configured to obtain the OR activation vector (or plurality thereof) from a table stored in memory, such as that depicted in Table 28. At S292, the computer predicts, using the obtained OR activation vector, olfactory properties of the compound or composition under consideration.

In some embodiments, the method for predicting olfactory properties of a sample compound or a sample composition based on OR activation data may use a lookup table, so as to identify known and measured compounds or compositions for which the known and measured OR activation vector most closely matches the sample compound or sample composition OR activation vector.

In some embodiments, the method for predicting olfactory properties of a sample compound or a sample composition based on OR activation data may use proximity searches such as nearest neighbour searches, including k-nearest neighbour (kNN) search. The machine learning Python library scikit-learn is particularly suitable for implementing nearest neighbour searches.

Multi-label classification techniques may be used to predict labels (olfactory properties) for previously unseen OR activation data for a compound or composition of interest. Formally, a binary output is assigned to each class (i.e., each distinct odor descriptor), for every sample. Positive classes are indicated with 1 and negative classes with 0 or -1. For example, with reference to Table 28, with the set of odor descriptors {Floral, Sweet, Honey, Woody, Earth, Ambery, Musky}, compound 1 (with digital identifier CAS 1) may give rise to a binary output in the form of {1, 1, 1, 0, 0, 0}.

In some embodiments, the scikit-learn Python package may be used to implement such muti-label classification, in which various modules may be implemented as meta-estimators that require a base estimator to be provided in their constructor. Meta-estimators extend the functionality of the base estimator to support multi-learning problems, which is accomplished by transforming the multi-learning problem into a set of simpler problems, then fitting one estimator per problem. An example suitable multi-label classification estimator is the OneVsRestClassifier. Similarly, multiclass-multioutput classification or multitask classification may be used to predict multiple labels for previously activation data for a compound or composition of interest.

The scikit-multilearn Python package is particularly well suited for prediction of olfactory properties, in that it provides multi-label implementation of several well-known techniques including SVM and kNN.

in some embodiments, machine learning techniques may be used to predict the olfactory properties. For example, a machine learning model may be trained using acquired OR activation data in the form of Table 28 such that the trained machine learning model may be configured to output olfactory properties. An artificial neural network is a particularly suitable form of machine learning model, where a hidden layer (or hidden layers) may be configured to accept features from an input layer (features corresponding to aspects of OR activation data) and - after application of weighting and biasing for multiple neurons - pass data onwards towards an output layer, which contains the prediction. Where input data further includes, for example, two-dimensional graphical representations of compound chemical structure, a graph neural network (GNN) is a particularly suitable machine learning model, which may be used in combination with other forms of neural networks (e.g., in an ensemble neural network, comprising multiple underlying neural networks). For example, a GNN may process compound chemical structure data to provide molecule embeddings, which may be processed in combination with OR activation data using another neural network to ultimately generate olfactory properties.

In some embodiments, an autoencoder is found to be well suited for the task of predicting olfactory properties of a compound or a composition based on OR activation data. The autoencoder is a model architecture that, through training, learns to extract the most important information from the input (i.e., the input OR activation vector). Autoencoders are a combination of an encoder and decoder. Autoencoders rely on the following two-step process:

- The encoder maps the input to a (typically) lossy lower-dimensional (intermediate) format.
- The decoder builds a lossy version of the original input by mapping the lower-dimensional format to the original higher-dimensional input format.

Autoencoders may be trained end-to-end by having the decoder attempt to reconstruct the original input from the encoder's intermediate format as closely as possible. As the intermediate format is smaller (lower-dimensional) than the original format, the autoencoder is forced to learn what information in the input is particularly important. Use of a trained autoencoder is depicted schematically in Figure 30.

The encoder stage maps each OR activation vector $V_{act,I}$ to a lower dimensional latent space. The decoder stage is trained to reconstruct a close approximation of the original activation vector, output as $V'_{act,i}$.

When trained, the trained encoder component(s) of the autoencoder may be isolated and used without use of the decoder component(s). The representation in the latent space may be visualised (the dimensions may need to be brought down with PCA, t-SNE or UMAP for visualization in 2D or 3D) and any labels may be plotted to identify regions of interest, such as in Example 28 above. In this manner, the encoder component(s) acts as a "virtual nose". Provided with an OR activation vector, the encoder maps the OR activation vector to a point in the latent space. By detecting which labelled points are nearby (using any suitable vector distance measure, such as an L1 or L2 norm), the overall model may synthesise or predict a label for the associated compound or composition. For example, if the top 3 nearby points are labelled (in order of increasing distance): "citrus", "fresh" and "lemon", this labelling will give a fairly good indication of the predicted flagrance.

In some embodiments, a variational autoencoder (VAE) is a particularly suitable form of autoencoder. A VAE leverages the discrepancy between inputs and outputs to generate modified versions of the inputs. Variational autoencoders are particularly useful for generative AI applications.

Example 30: Generation of supplementary OR activation vectors

The above-described generative model (e.g., an autoencoder or a VAE), when trained, allows one to generate new, previously unseen (supplementary) OR activation vectors. When the trained decoder is used with input of a point in the latent space that is nearby to points for the training set (i.e., the library of known OR activation vector for known compounds and compositions) with a desired label, newly generated supplementary OR activation vectors are likely to share the same label.

This process allows one to generate OR activation vectors for promising new fragrances, which are expected to share olfactory properties with known and measured compounds or compositions.

Example 31: Predicting OR activation data for a compound or a composition

Libraries of OR activation data (OR activation libraries) and properties of corresponding known and measured compounds and compositions may be used to predict OR activation data for new sample compounds and new sample compositions. In this way, models used to predict OR activation data may be used without the need to perform the arduous tasks of fabricating the compound or composition and measuring OR activations in a laboratory setting. In turn, the user may, for instance, prepare a shortlist of compounds or compositions, which are expected to demonstrate desired OR activations, thereby significantly shortening fabrication timespans.

Various aspects of the sample compound or sample composition - when coupled with OR activation data - are well-suited for use in such methods, including olfactory properties of the compound or the composition (e.g., odor descriptors); compound concentration within the composition (quantified in terms of mass concentration, molar concentration, number concertation, and/or volume concentration); unique identification details for the compound or the composition (e.g., CAS number; SMILES string; International Chemical Identifier); and emotion or mood portraits (e.g., image collections that are considered to be representative of the compound or composition, such as pictures of lemons for compositions that exhibit citrus-like odors or are labeled with such odor descriptors as "citrus" or "lemon" or the like).

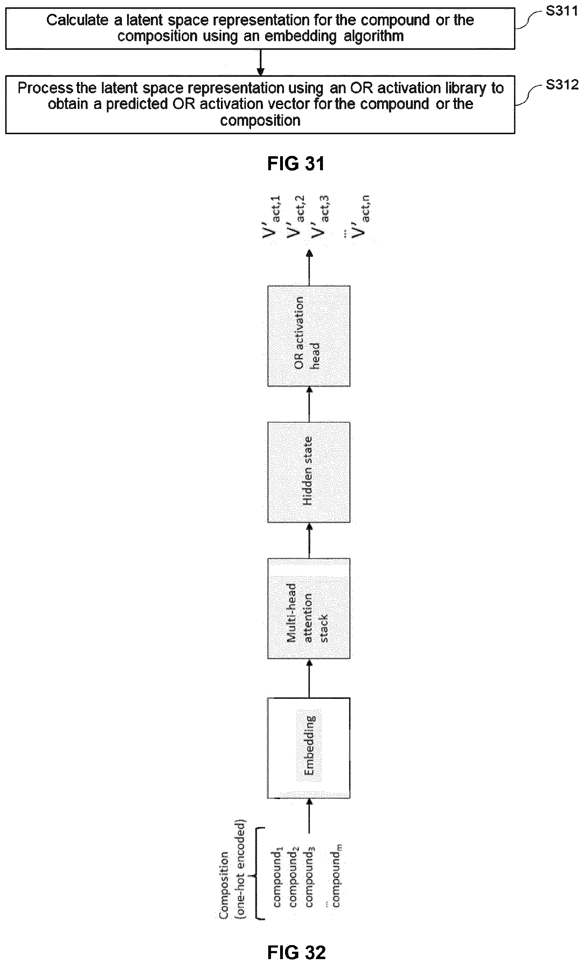

Figure 31 provides an example general method of predicting OR activation data for a compound or a composition. At S311, the computer implementing the method calculates a latent space representation (i.e., an embedding) for the compound or the composition using an embedding algorithm. At S312, the computer processes the latent space representation to obtain a predicted OR activation vector for the compound or the composition under consideration.

In effect, complementary techniques to those described in Example 29 may be applied in order to predict OR activation data for a compound or a composition. That is, an autoencoder, for example, may be used, which - through training - learns to extract the most important information from the input (i.e., the input compound or composition properties in this instance). The encoder stage maps each set of compound or compound properties (e.g., concatenated strings including olfactory property and physiochemical parameter) to a lower dimensional latent space. The decoder stage is trained to reconstruct a close approximation of the original feature vector. An isolated, trained encoder component(s) of the autoencoder may then be used to provide representations of the input feature vector in the latent space. Proximity measurement techniques (such as those described above) may be used within the latent space to predict expected OR activation vector for the sample compound or composition; e.g., the predicted OR activation vector may be determined as the same as (or similar to) OR activation data for known, nearby compounds within the latent space.

Of course, the techniques described in Example 31 may be combined with those described in Example 29 to predict olfactory properties of a sample compound or sample composition. For instance, the above techniques may be used to predict an OR activation vector for a sample composition; the predicted OR activation vector may be used within the techniques described in Example 29 to predict olfactory properties, such as expected odor descriptor(s), of the sample composition. As an example, a machine-learned model (or combination thereof) may provide output comprising a description of predicted olfactory properties of a compound, such as, for example, a list of odor descriptors, descriptive of what the compound may smell like to a human. For instance, a SMILES string may be provided, such as the SMILES string "O=C(OCCC(C)C)C" for the chemical structure of isoamyl acetate, and a first machine-learned model may provide as output a predicted OR activation vector. When used as input to a second machine learning model, trained in accordance with Example 29, the predicted OR activation vector may give rise to an output of what that compound may smell like to a human, which may for example include a group of odor descriptors, such as "fruit, banana, apple."

in some embodiments, an encoder stage of a Transformer-based large language model (LLM) may be used as suitable processing layer of a machine learning model for predicting OR activation data. In some embodiments, the encoder stage (comprising one encoder components or multiple encoder components) may use non-causal attention, meaning that the attention mechanism is able to look at all compounds of the composition at once. Transformer models are deep learning models that use self-attention to find differential weightings for a sequence of input data.

The originally introduced Transformer models comprise at least one encoder and at least one decoder. Each encoder contains a self-attention mechanism and a feed-forward neural network and generally attends over all the tokens in the sequence that the encoder is processing (that is, the encoder focuses on all tokens in the sequence when processing, allowing the encoder to associate each specific token with other tokens in the sequence). In this context, a "token" is the smallest unit on which the model is training and making predictions and may correspond, for example, to an EC50 value representative of the activation threshold for an OR. The first encoder takes an embedding (a vector representation, often reduced in terms of dimensionality) of an input sequence of defined tokens. Later encoders take the output of a preceding encoder as input. Each encoder outputs a vector representative of a self-attention score (how much focus to place on other parts of the input sequence as the model encodes a token at a certain position). Each decoder contains a self-attention mechanism, an attention mechanism over the encodings (over the output of the final encoder), and a feed-forward neural network and generally attends in a "causal" manner. That is, the decoder attends only to tokens in the past of an attended token (masked self-attention). The first decoder takes an embedding of the output element following each step of the decoder stack. Each decoder takes the output of the final encoder and generates an output vector representative of a sequence of tokens (usually sequentially generated). Each step in the decoding phase outputs an element from the output sequence (i.e., OR activation vector).

The OR activation data, such as that depicted in Table 28 may be seen to correspond to a "compound vocabulary" similar to the token vocabulary of a LLM (such as BERT or GPT). Compounds in a composition may be one-hot encoded using this compound vocabulary, in the same way as tokens are one-hot encoded in a LLM.

An example Transformer-based model, suitable for OR activation vector prediction is provided in Figure 32. The first stage of the model is a learned/trained embedding module, which transforms the one-hot encoded composition data into a set of embedding vectors (one value for each compound in the composition). The positional embedding used in conventional LLMs to encode the order of the words in a sentence, may be omitted as a compound in a composition have no order that needs to be fed into the model. However, while the techniques are not limited to a specific type or architecture of machine learning model, a preferable model should be able to predict data from potentially sequential input data (e.g., the sequential proportional presence of compounds within a composition, which may be seen as a relative "importance" of compounds within a composition).

The set of embeddings may then be processed by a stack of multi-head attention layers, with each layer resulting in a hidden state that serves as an input for the next layer. The hidden state delivered by the last layer is processed by an OR activation head layer, which predicts an OR activation vector $V'_{act,l}$ for the composition.

In some embodiments, a recurrent network may be used as suitable processing layer of a machine learning model for predicting OR activation data. A recurrent neural network is intentionally run multiple times, where parts of each run feed into the next run. Specifically, hidden layers from the previous run provide part of the input to the same hidden layer in the next run. Recurrent neural networks are particularly useful for evaluating sequences, so that the hidden layers can learn from previous runs of the neural network on earlier parts of the sequence. A recurrent neural network is then well suited for instance where feature vectors (input properties of the compound or composition) include sequential data, such as ordered compounds in a composition based on concentration or cost, for instance. A long short-term memory, LSTM, recurrent neural network is particularly well suited. Any or all of the above described processing layer models may be readily implemented using such software libraries as PyTorch and Tensorflow.

The available datasets of OR activation data and corresponding compound and composition properties are well-suited for training machine learning models for the present task. The available datasets, as seen above in many of the Examples, include OR activation data for many compounds in the vocabulary, and also for many combinations of compounds in various compositions (with varying concentrations). As the interaction of OR activations of compounds in a composition is generally a non-linear interaction, training models using such abundant data is particularly successful in learning how to predict OR activation vectors for new compositions.

Example 32: Replacement of a compound in a composition based on OR activation data

Techniques herein are suited for suggestion of replacement compounds in an existing composition. Given a desired OR activation vector (such as that of the existing composition), and given one or more "forbidden" compounds, some embodiments may generate a composition of compounds that omits any forbidden compound and that presents a same or a similar OR activation vector. Forbidden compounds in this context may be determined based on toxicity, price, availability, etc. Example methods may then, for example, replace expensive compounds in compositions with cheaper alternatives that nonetheless provide the same or similar OR activations. Similarly, methods may consider toxicity data so as to calculate an OR activation data for a composition minimizing toxicological properties.

In some embodiments, the computer-implemented method for suggesting replacement of a compound in a given composition includes a step of predicting a first OR, activation vector for the given composition. The method includes a step of predicting a second OR activation vector for the given composition in the absence of the compound (i.e., the same given composition only lacking the compound). Techniques such as those described in Example 29 above may be employed here for prediction of OR activation vectors. The method includes a step of selecting one or more candidate replacement compounds (i.e., potential replacement compounds) based on a difference between the first OR activation vector and the second OR activation vector.

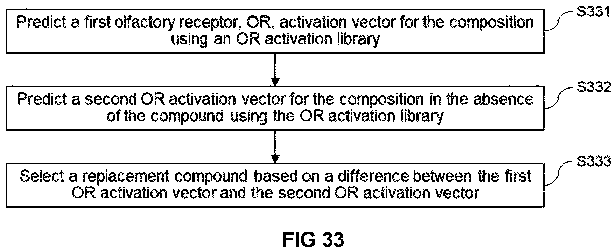

Figure 33 provides an example general method of replacing a compound in a composition based on OR activation data. At S331, the computer implementing the method predicts a first OR activation vector for the composition including the compound for which a replacement is sought (if already known, the computer may of course simply obtain this vector from memory or from the appropriate resource as appropriate). At S332, the computer predicts a second OR activation vector for the composition now excluding the compound for which a replacement is sought. At S333, the computer selects a replacement compound based on the difference between the first and second OR activation vectors.

The step of selecting candidate replacement compounds may be achieved by looking for the candidate replacement compound that provides the OR activation vector that is expected to most likely supplement the second OR activation vector so as to obtain at the first OR activation vector. That is, the technique looks to identify the candidate compound for which the composition OR activation vector (in the presence of the candidate compound) most closely matches the target OR activation vector (i.e., the OR activation vector of the original composition). For instance, if one expects a linear relationship, one may select a candidate compound expected to "fill the gap". In an example with multiple candidate compounds, this may be achieved by - for each candidate compound - predicting a third OR activation vector for the composition in the absence of the compound and in the presence of the candidate compound. The replacement compound may then be selected as the candidate compound for which the difference between the first OR activation vector and the second OR activation vector most closely matches the difference between the second OR activation vector and the third OR activation vector. Of course, multiple replacement compounds may be used to replace a sole given compound; multiple replacement compounds may be used to replace multiple given compounds; and a sole replacement compound may be used to replace multiple given compounds.

An example iterative process for replacing compounds in a composition is set out below:

1) The OR response is measured or predicted for the composition including the compound to be replaced.
2) The OR response is measured or predicted for the composition without the compound to be replaced.
3) The difference in OR responses is calculated, a set of candidate replacement compounds is determined based on an overlap between the difference and the N-dimensional OR response of the candidate replacement.
4) For each of the candidate replacements:

a) The OR response is calculated for the composition without the compound to be replaced, and with the candidate replacement.
b) The difference in OR response of step 1) and 4)a) is calculated.
c) If the difference is below a predetermined threshold, the candidate replacement is selected as the replacement compound.

5) If the difference for all candidate replacements exceeds a predetermined threshold, a set of additional replacement compounds is determined.
6) For each of the additional candidate replacements:

a) The OR response is calculated for the composition without the compound to be replaced, and with the additional candidate replacement.
b) The difference in OR response of step 1) and 6)a) is calculated. [etc.]

This nested search may be performed for a set of up to D candidate replacement compounds, where D is the nesting level. If each time C candidates are taken, the total number of compositions to evaluate is $C^D$, e.g. with C=10 and D=3, a maximum of 1000 compositions are considered. The composition most closely approximately the given composition may then be selected.

Example 33: Hardware for use and exploitation of acquired data

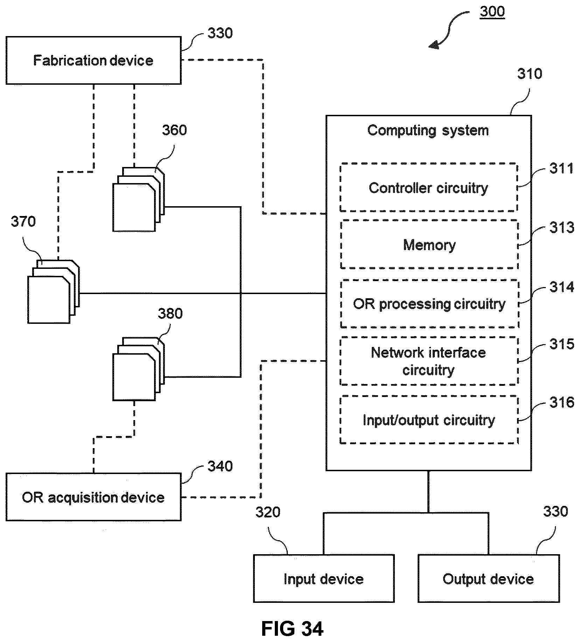

Figure 34 is a block diagram of an implementation of a OR prediction system 300, suitable for executing methods for use of acquired OR activation data and for prediction of OR activation data. The example OR prediction system 300 comprises a computing system 310 within which a set of instructions, for causing the computing system 310 to perform any one or more of the methods (or steps thereof) discussed herein, may be executed. The computing system 310 may also be referred to as a computer. In particular, the methods described herein may be implemented by a processor or controller circuitry 311 of the computing system 310.

The computing system 310 shall be taken to include any number or collection of machines, e.g., computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 310 includes controller circuitry 311 and a memory 313 (e.g., read-only memory (ROM), flash memory, dynamic random access memory (DRAM) such as synchronous DRAM (SDRAM) or Rambus DRAM (RDRAM), etc.). The memory 313 may comprise a static memory (e.g., flash memory, static random access memory (SRAM), etc.), and/or a secondary memory (e.g., a data storage device), which communicate with each other via a bus (not shown).

Controller circuitry 311 represents one or more general-purpose processors such as a microprocessor, central processing unit, accelerated processing units, or the like. More particularly, the controller circuitry 311 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 311 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 311 is configured to execute the processing logic for performing the operations and steps discussed herein.

The computing system 310 may further include a network interface circuitry 315. The computing system 310 may be communicatively coupled to an input device 320 and/or an output device 330, via input/output circuitry 316. in some implementations, the input device 320 and/or the output device 330 may be elements of the computing system 310. The input device 320 may include an alphanumeric input device (e.g., a keyboard or touchscreen), a cursor control device (e.g., a mouse or touchscreen), an audio device such as a microphone, and/or a haptic input device. The output device 330 may include an audio device such as a speaker, a video display unit (e.g., a liquid crystal display (LCD) or a cathode ray tube (CRT)), and/or a haptic output device. In some implementations, the input device 320 and the output device 330 may be provided as a single device, or as separate devices.

In some implementations, the computing system 310 may comprise OR processing circuitry 314. OR processing circuitry 314 may be configured to process OR activation data 380 (e.g., individual compound OR activation data, or broader libraries), such as OR activation data obtained from one or more data sources, including an OR acquisition device 340. OR processing circuitry 314 may be configured to process, or pre-process, OR activation data 380. For example, OR processing circuitry 314 may convert received OR activation data into a particular format, size, resolution or the like. In some implementations, OR processing circuitry 314 may be combined with controller circuitry 311.

in some implementations, the OR prediction system 300 may further comprise an OR acquisition device 340 and/or a fabrication device 330. The OR acquisition device 340 and the fabrication device 330 may be provided as a single device. In some implementations, fabrication device 330 is configured to perform composition fabrication.

OR acquisition device 340 may be configured to automate the above-described methods for acquiring OR activation data, including automated C-terminus modification and insertion, and automated contacting ORs with test compounds or compositions, and automated detection of activation of the OR.

OR acquisition device 340 may be configured to output OR activation data 380, which may be accessed by computing system 310. Fabrication device 330 may be configured to output fabrication data 360, which may be accessed by computing system 310.

Computing system 310 may be configured to access or obtain fabrication data 360, fabrication instruction data 370 and/or OR activation data 380. Fabrication data 360 may be obtained from an internal data source (e.g., from memory 313) or from an external data source, such as OR fabrication device 330 or an external database. Fabrication instruction data 370 may be obtained from memory 313 and/or from an external source, such as a fabrication planning database. Fabrication instruction data 370 may comprise information obtained or derived from one or more of the OR acquisition device 340 and the fabrication device 330, and which may be adapted to control a fabrication device 330 to fabricate a compound or a composition using a digital representation of the compound or the composition.

In cases where a liquid perfume formulation is to be fabricated, fabrication instruction data 370 may include weight data per composition compound, which is to be dispensed into the liquid perfume formula. If fabrication device 330 is to fabricate gas phase (which can be the case when one is fabricating demonstration samples rather than commercial formulae in bulk for sale), then fabrication data 360 may include physicochemical properties, such as volatility to adjust for a final level in the gas phase.

The various methods described above may be implemented by a computer program. The computer program may include computer code (e.g., instructions) arranged to instruct a computer to perform the functions of one or more of the various methods described above. For example, the steps of the methods described in relation to any of Figures 30, 32, and 34 may be performed by the computer code. The steps of the methods described above may be performed in any suitable order. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-RIW or DVD. The instructions may also reside, completely or at least partially, within the memory 313 and/or within the controller circuitry 311 during execution thereof by the computing system 310, the memory 313 and the controller circuitry 311 also constituting computer-readable storage media.

In an implementation, the modules, components and other features described herein may be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components may be implemented as firmware or functional circuitry within hardware devices. Further, the modules and components may be implemented in any combination of hardware devices and software components, or only in software (e.g., code stored or otherwise embodied in a machine-readable medium or in a transmission medium).

**Claims**

1. A computer-implemented method for predicting olfactory properties of a compound or a composition based on olfactory receptor, OR, activation data, the method comprising:

   obtaining an OR activation vector for the compound or the composition; and
   predicting, using the OR activation vector and an OR activation library, olfactory properties of the compound or the composition.

2. The computer-implemented method of claim 1, wherein predicting olfactory properties of the compound or the composition comprises:
   passing the OR activation vector through a trained machine learning model, the trained machine learning model configured to output olfactory properties.

3. The computer-implemented method of claim 2, wherein predicting olfactory properties of the compound or the composition comprises:

   calculating, from the OR activation vector, a latent space representation using a hidden layer of the trained machine learning model;
   predicting, from the latent space representation, olfactory properties of the compound or the composition using an output layer of the trained machine learning model, optionally wherein calculating the latent space representation uses an encoder of an auto-encoder model, optionally a variational auto-encoder model.

4. The computer implemented method of claim 2 or claim 3, further comprising generating a supplementary OR activation vector using a decoder of an auto-encoder model, optionally a variational auto-encoder model.

5. The computer-implemented method of any of claims 2 to 4, wherein the machine learning model is trained using the OR activation library including labels of olfactory properties for one or more OR activation vectors.

6. The computer-implemented method of any preceding claim, wherein obtaining the OR activation vector comprises measuring activations of a plurality of ORs when contacted with one or more test compounds or test compositions, wherein each OR of the plurality of Ors comprises OR proteins having a modified C-terminal domain comprising an amino acid sequence motif RN[KR][EDQ][VMIL][KR]xA[LIV][KRH][KR][LI][LIF][KRG][KR][KR] (SEQ ID NO: 1), and optionally wherein the modified C-terminal domain is fused to a seventh transmembrane helix of the OR protein.

7. The computer-implemented method of claim 6, wherein the OR protein is a class I or class II OR with a modified C-terminal domain, preferably a human, dog, or cat class I or class II OR with a modified C-terminal domain, more preferably a human class I or class II OR with a modified C-terminal domain.

8. A computer-implemented method for predicting olfactory receptor, OR, activation data for a compound or a composition, the method comprising:

   calculating a latent space representation for the compound or the composition using an embedding algorithm; and
   processing the latent space representation using an OR activation library to obtain a predicted OR activation vector for the compound or the composition.

9. The computer-implemented method of claim 8, wherein calculating the latent space representation for the compound or the composition uses any or all of the following: olfactory properties of the compound or the composition; unique identification details for the compound or the composition; emotion or mood portraits; toxicological data; and compound or composition concentration.

10. The computer-implemented method of claim 8 or claim 9, wherein processing the latent space representation comprises use of an ensemble learning method, such as random forests or gradient-boosting.

11. The computer-implemented method of any of claims 8 to 10, wherein processing the latent space representation comprises:

processing the latest space representation using a processing layer to obtain a hidden state representation; and processing the hidden state representation using an OR activation layer to obtain the predicted OR activation vector for the compound or the composition,
optionally wherein the processing layer is a multi-headed attention layer of a transformer encoder or a recurrent network, such as a long short-term memory, LSTM, network.

12. The computer-implemented method of claim 11, wherein the processing layer and the OR activation layer are trained using the OR activation library.

13. The computer-implemented method of claim 12, wherein the OR activation vector library comprises one or more measured activations of one or more ORs when contacted with one or more test compounds or test compositions, optionally wherein each OR of the one or more ORs comprises OR proteins having a modified C-terminal domain comprising an amino acid sequence motif RN[KR][EDQ][VMIL][KR]xA[LIV][KRH][KR][LI][LIF][KRG][KR][KR] (SEQ ID NO: 1), and optionally wherein the modified C-terminal domain is fused to a seventh transmembrane helix of the OR protein.

14. The computer-implemented method of claim 13, wherein the OR protein is a class I or class II OR with a modified C-terminal domain, preferably a human, dog, or cat class I or class II OR with a modified C-terminal domain, more preferably a human class I or class II OR with a modified C-terminal domain.

15. The computer-implemented method of any of claims 8 to 14, further comprising:

predicting olfactory properties of the predicted OR activation vector using the method of any of claims 1 to 10; and optionally predicting a compound or a composition based on the olfactory properties for the predicted OR activation vector to design a desired fragrance or flavour.

16. A computer-implemented method for the creation, modification, or improvement of a fragrance or flavour composition, for example by replacement of one or more compounds in a composition, the method comprising:

obtaining a first olfactory receptor, OR, activation vector for the composition using an OR activation library; obtaining a second OR activation vector for the composition in the absence of the compound using the OR activation library;
selecting a replacement compound based on a difference between the first OR activation vector and the second OR activation vector.

17. The computer-implemented method of claim 16, wherein selecting the replacement compound comprises, foreach candidate compound from a plurality of candidate compounds:

obtaining a third OR activation vector for the composition in the absence of the compound and in the presence of the candidate compound;
selecting the replacement compound as the candidate compound for which the difference between the first OR activation vector and the second OR activation vector most closely matches the difference between the second OR activation vector and the third OR activation vector.

18. The computer-implemented method of claim 16 or claim 17, comprising selecting one or more replacement compounds for replacing the compound, or selecting one or more replacement compounds for replacing one or more compounds.

19. The computer-implemented method of any preceding claim, further comprising outputting operating instructions adapted to control a fabrication device to fabricate the composition.

20. The computer-implemented method of any preceding claim, wherein the compound is an OR agonist or an OR antagonist, or wherein the composition includes an OR agonist or an OR antagonist.

21. A computer-readable medium storing data that defines a digital representation of the compound or the composition of any of claims 1 to 20, including a representation of the OR activation vector for the compound or the composition,

22. The computer-readable medium of claim 21, wherein the data includes operating instructions adapted to control a fabrication device to fabricate the composition using the digital representation of the compound or the composition when the data is relayed to the fabrication device, or wherein the digital representation is formatted for control of a fabrication device to fabricate the composition.

23. A data processing apparatus comprising a memory storing computer-executable instructions and a processor configured to execute the instructions to perform the method of any of claims 1 to 20.

24. A computer program comprising instructions which, when the program is executed by a computer, cause the computer to perform the method of any of claims 1 to 20.

25. A computer-readable storage medium comprising instructions which, when executed by a computer, cause the computer to perform the method of any of claims 1 to 20.

EP 4 664 463 A1

FIG 1

FIG 2

99

FIG 3

FIG 4

OR5B12-RNKEVKSAFKKTVGKAKASIGFIF (WT)

OR5B12-RNKEVKSALKKTLGKAKASIGFIF
(towards consensus)

OR5B12-C-term-RNKEVKDALKRLLKRKCC

OR5A2-RNKEIKNAMRKAMERDPGISHGGPFIFMTLG
(WT)

OR5A2-RNKEVKNALRKALERDPGISHGGPFIFMTLG
(towards consensus)

OR5A2-RNKEVKDALKRLLKRKCC

**FIG 5**

FIG 6

FIG 7

FIG 8

**FIG 9**

FIG 10

**FIG 11**

**FIG 12**

FIG 13

FIG 14

FIG 15

FIG 16

FIG 17

**FIG 18**

FIG 19

FIG 20

FIG 21

FIG 22

FIG 23

FIG 24

FIG 25

FIG 26

FIG 27

FIG 28

Obtain an OR activation vector for the compound or the composition — S291

Predict, using the OR activation vector and an OR activation library, olfactory properties of the compound or the composition — S292

**FIG 29**

**FIG 30**

Calculate a latent space representation for the compound or the composition using an embedding algorithm — S311

Process the latent space representation using an OR activation library to obtain a predicted OR activation vector for the compound or the composition — S312

**FIG 31**

Composition (one-hot encoded) { $compound_1$, $compound_2$, $compound_3$ ⋮ $compound_m$ } → Embedding → Multi-head attention stack → Hidden state → OR activation head → $V'_{act,1}$, $V'_{act,2}$, $V'_{act,3}$ ⋮ $V'_{act,n}$

**FIG 32**

| Predict a first olfactory receptor, OR, activation vector for the composition using an OR activation library | S331 |

| Predict a second OR activation vector for the composition in the absence of the compound using the OR activation library | S332 |

| Select a replacement compound based on a difference between the first OR activation vector and the second OR activation vector | S333 |

**FIG 33**

FIG 34

Europäisches
Patentamt

European
Patent Office

Office européen
des brevets

## EUROPEAN SEARCH REPORT

**Application Number**

EP 24 18 2426

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 130 736 A1 (AJINOMOTO KK [JP]) 8 February 2023 (2023-02-08) | 1,2,5, 21-25 | INV. G16B35/20 |
| Y | * abstract * * paragraphs [0001], [0012], [0091] - [0102], [0104], [0124] - [0142], [0147] - [0160], [0155], [0156], [0210] - [0212] * | 3,4,6,7 | G16B40/20 G16C20/70 |
| | ----- | | |
| Y | US 2020/176087 A1 (COLBY SEAN M [US] ET AL) 4 June 2020 (2020-06-04) * abstract * * paragraphs [0005] - [0010], [0048] - [0050] * | 3,4 | |
| | ----- | | |
| Y | JONES ERIC M. ET AL: "A Scalable, Multiplexed Assay for Decoding GPCR-Ligand Interactions with RNA Sequencing", CELL SYSTEMS, vol. 8, no. 3, 1 March 2019 (2019-03-01), pages 254-260.e6, XP055943033, US ISSN: 2405-4712, DOI: 10.1016/j.cels.2019.02.009 * abstract * * paragraphs [0010] - [0015], [0025]; sequence 56 * | 6,7 | |
| | | | **TECHNICAL FIELDS SEARCHED (IPC)** |
| | | | G16B G16C |
| | ----- | | |
| A | US 2021/223232 A1 (MATSUNAMI HIROAKI [US] ET AL) 22 July 2021 (2021-07-22) * the whole document * | 1-7, 21-25 | |
| | ----- | | |
| | -/-- | | |

The present search report has been drawn up for all claims

| Place of search | Date of completion of the search | Examiner |
|---|---|---|
| Munich | 26 November 2024 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Kowalewski Joel ET AL: "Predicting Human Olfactory Perception from Activities of Odorant Receptors odorant activates multiple ORs odor perception AI chemical structure predicts OR activity", , 21 August 2020 (2020-08-21), XP093227593, DOI: 10.1016/j.isci Retrieved from the Internet: URL:https://doi.org/10.1016/ j.isci.2020.101361 * abstract * * page 2, paragraph 6 - page 4, paragraph 3 * | 1-7, 21-25 | |

TECHNICAL FIELDS
SEARCHED (IPC)

The present search report has been drawn up for all claims

| Place of search | Date of completion of the search | Examiner |
|---|---|---|
| Munich | 26 November 2024 | Thumb, Werner |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

................................................................

& : member of the same patent family, corresponding
   document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-7(completely); 21-25(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

Application Number

**EP 24 18 2426**

The Search Division considers that the present European patent application does not comply with the requirements of unity of invention and relates to several inventions or groups of inventions, namely:

      1. claims: 1-7(completely); 21-25(partially)

          Predicting olfactory properties of a compound or composition using olfactory receptor activation data
                  ---

      2. claims: 8-15(completely); 21-25(partially)

          Predicting OR activation data for a compound or composition
                  ---

      3. claims: 16-20(completely); 21-25(partially)

          Method for improving or changing the quality of a fragrance or flavour composition by replacing a compound based on OR activation data
                  ---

## ANNEX TO THE EUROPEAN SEARCH REPORT
## ON EUROPEAN PATENT APPLICATION NO.

EP 24 18 2426

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2024

| Patent document cited in search report | | Publication date | Patent family member(s) | | Publication date |
|---|---|---|---|---|---|
| EP 4130736 | A1 | 08-02-2023 | EP | 4130736 A1 | 08-02-2023 |
| | | | JP WO2021200780 A1 | | 07-10-2021 |
| | | | US | 2023085282 A1 | 16-03-2023 |
| | | | WO | 2021200780 A1 | 07-10-2021 |
| US 2020176087 | A1 | 04-06-2020 | NONE | | |
| US 2021223232 | A1 | 22-07-2021 | EP | 3814371 A2 | 05-05-2021 |
| | | | US | 2021223232 A1 | 22-07-2021 |
| | | | WO | 2020006108 A2 | 02-01-2020 |

EPO FORM P0459

For more details about this annex : see Official Journal of the European Patent Office, No. 12/82

## REFERENCES CITED IN THE DESCRIPTION

**Patent documents cited in the description**

- WO 2019110630 A1
- WO 2014037800 A
- WO 2006002161 A
- WO 2019110630 A
- WO 201911630 A

**Non-patent literature cited in the description**

- **FELDMESSER et al.** Widespread ectopic expression of olfactory receptor genes. *BMC Genomics*, 2006, vol. 7, 121
- **MASSBERG, D.** ; **H. HATT**. Human Olfactory Receptors: Novel Cellular Functions Outside of the Nose. *Physiol Rev*, 2018, vol. 98 (3), 1739-1763
- **LEE et al.** Therapeutic potential of ectopic olfactory and taste receptors. *Nature Reviews Drug Discovery*, 2019, vol. 18 (2), 116-138
- **YU et al.** Receptor-transporting protein (RTP) family members play divergent roles in the functional expression of odorant receptors. *PLoS One*, 2017, vol. 12 (6), e0179067
- **KRAUTWURST et al.** identification of ligands for olfactory receptors by functional expression of a receptor library. *Cell*, 1998, vol. 95 (7), 917-26
- **SAITO et al.** RTP family members induce functional expression of mammalian odorant receptors. *Cell*, 2004, vol. 119 (5), 679-691
- **IKEGAMI et al.** Structural instability and divergence from conserved residues underlie intracellular retention of mammalian odorant receptors. *PNAS*, 2020, vol. 117 (6), 2957-2967
- **KOTTHOFF et al.** *The FASEB Journal*, 2021, vol. 35, e21274
- **KADATA et al.** Structural determinants for membrane trafficking and G protein selectivity of a mouse olfactory receptor. *Journal of Neurochemistry*, 2004, vol. 90 (6), 1453-1463
- **KATO et al.** Amino acids involved in conformational dynamics and G protein coupling of an odorant receptor: targeting gain-of-function mutation. *Journal of Neurochemistry*, 2008, vol. 107 (5), 1261-1270
- **SATO et al.** Functional Role of the C-Terminal Amphipathic Helix 8 of Olfactory Receptors and Other G Protein-Coupled Receptors. *Int. J. Mol. Sci.*, 2016, vol. 17 (11), 1930
- **ZHANG** ; **FIRESTEIN**. *Nature Neurosci*, 2002, vol. 5 (2), 124-33
- **MALNIC et al.** *PNAS*, 2004, vol. 101 (8), 2584-9
- **OLENDER et al.** *Methods Mol Biol*, 2013, vol. 1003, 23-38
- UniProt: the universal protein knowledgebase in 2021. *Nucleic Acids Research*, 08 January 2021, vol. 49 (D1), D480-D489, www.uniprot.org
- **SAITO et al.** *Cell*, 2004, vol. 119 (5), 679-691
- The Measuring of Odors. **NEUNER-JEHLE, N** ; **ETZWEILER, F.** Perfumes. Springer, 1994
- **MAINLAND et al.** *Sci Data*, 2015, vol. 2, 150002
- **HAN et al.** *Science China. Life sciences*
- **SHEPARD et al.** *PLoS One*, 2013, vol. 8 (7), e68758
- **ZHUANG** ; **MATSUNAMI**. *J Biol Chem*, 2007, vol. 282 (20), 15284-15293
- **NOE et al.** A bi-functional IL-6-HaloTag® as a tool to measure the cell-surface expression of recombinant odorant receptors and to facilitate their activity quantification. *J Biol Methods*, 2017, vol. 4 (4), e82
- **TAN et al.** *Scientific reports*, 2022, vol. 12, 17658
- **TAN et al.** *Scientific Reports*, 2022, vol. 12, 17658
- **KAUSHAL et al.** *Proc Natl Acad Sci U S A*, 1998, vol. 91 (9), 4024-4028
- **AUSUBEL et al.** Current Protocols in Molecular Biology. John Wiley & Sons Inc, 2003
- **SAMBROOK** ; **GREEN**. Molecular Cloning: A Laboratory Manual. Cold Spring Harbor Laboratory Press, 2012
- **KUNKEL**. *Proc. Natl. Acad. Sci.*, 1985, vol. 82, 488
- **ROBERTS et al.** *Nature*, 1987, vol. 328, 731-734
- **WELLS, J.A. et al.** *Gene*, 1985, vol. 34, 315
- *CHEMICAL ABSTRACTS*, 83108-07-0
- *CHEMICAL ABSTRACTS*, 4674-50-4
- *CHEMICAL ABSTRACTS*, 406488-30-0

- **SUNDERKOTTER, A. et al.** *Chemistry*, 2010, vol. 16 (25), 7404-21
- **YOSHIKAWA, K. et al.** An odorant receptor that senses four classes of musk compounds. *Curr Biol*, 2022, vol. 32 (23), 5172-5179, e5
- **IKEGAMI, K. et al.** Structural instability and divergence from conserved residues underlie intracellular retention of mammalian odorant receptors. *Proc Natl Acad Sci USA*, 2020, vol. 117 (6), 2957-2967
- Principles and Strategies Related to the Testing of Degradation of Organic Chemicals;. *OECD 25 Guidelines for the Testing of Chemicals*, July 2003
- **VEITHEN et al.** Springer Handbook of Odor. Springer International publishing, 2017
- **BUETTNER**. Springer Handbook of Odor. Springer International publishing, 2017
- **SHARMA et al.** OlfactionBase: a repository to explore odors, odorants, olfactory receptors and odorant-receptor interactions. *Nucleic Acids Res.*, 07 January 2022, vol. 50 (D1), D678-D686
- **XIA X.** Bioinformatics and the Cell: Modern Computational Approaches in Genomics, Proteomics and transcriptomics. Springer International Publishing, 2018
- **MOUNT D.** Bioinformatics: Sequence and Genome Analysis. Cold Spring Harbor Laboratory Press, 2004
- **HENIKOFF** ; **HENIKOFF**. *PNAS*, 1992, vol. 89, 915-919
- **ALTSCHUL et al.** *J. Mol. Biol.*, 1990, vol. 215, 403-10
- **ALTSCHUL et al.** *Nucleic Acids Res.*, 1997, vol. 25 (17), 3389-3402
- **NATSCH et al.** A specific bacterial aminoacylase cleaves odorant precursors secreted in the human axilla. *The Journal of biological chemistry*, 2003, vol. 278 (8), 5718-5727
- **HONG** ; **COREY**. Enantioselective syntheses of georgyone, arborone, and structural relatives. Relevance to the molecular-level understanding of olfaction. *Journal of the American Chemical Society*, 2006, vol. 128 (4), 1346-1352
- **NATSCH et al.** identification of Odoriferous Sulfanylalkanols in Human Axilla Secretions and Their Formation through Cleavage of Cysteine Precursors by a C-S Lyase Isolated from Axilla bacteria. *Chemistry and Biochemistry*, 2004, vol. 1, 1058-1072
- **LI et al.** *PLoS Genet.*, 03 February 2022, vol. 18 (2), e1009564
- **KUBOTA et al.** *Appl Environ Microbiol.*, 2012, vol. 78 (9), 3317-24